# EUROPEAN PATENT APPLICATION

(11) **EP 3 763 726 A1**
(43) Date of publication of application: **13.01.2021**
(21) Application number: 19000325.1
(22) Date of filing: 08.07.2019
(51) Int. Cl.: C07K 7/08, C07K 14/81, A61K 38/08, G01N 33/543

(54) **COMPOUNDS COMPRISING A FIBROBLAST ACTIVATION PROTEIN LIGAND AND USE THEREOF**

(71) Applicant: 3B Pharmaceuticals GmbH, 12489 Berlin (DE)
(72) Inventor: OSTERKAMP, Frank, 12489 Berlin (DE); ZBORALSKI, Dirk, 10827 Berlin (DE); SCHNEIDER, Eberhard, 14552 Michendorf OT Wildenbruch (DE); HAASE, Christian, 10409 Berlin (DE); PASCHKE, Matthias, 10163 Berlin (DE); HÖHNE, Aileen, 12524 Berlin (DE); UNGEWISS, Jan, 12355 Berlin (DE); SMERLING, Christiane, 12205 Berlin (DE); REINEKE, Ulrich, 14193 Berlin (DE); BREDENBECK, Anne, 13187 Berlin (DE)
(74) Representative: Bohmann, Armin K.

(57) **Abstract**

The present invention is related to a compound comprising a cyclic peptide of formula (I) 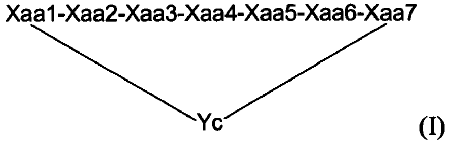 and an N-terminal modification group A attached to Xaa1,
wherein
each and any one of Xaa1, Xaa2, Xaa3, Xaa4, Xaa5, Xaa6 and Xaa7 is a residue of an amino acid, and
Yc is a structure of formula (X)

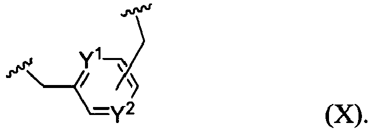

SEQUENCE LISTING

<110>  3B Pharmaceuticals GmbH

<120>  Compounds comprising a fibroblast activation protein ligand and
       use thereof

<130>  D 10023 EP

<160>  3

<170>  PatentIn version 3.5

<210>  1
<211>  760
<212>  PRT
<213>  Homo sapiens

<220>
<221>  MISC_FEATURE
<223>  Fibroblast activation protein (FAP)

<400>  1

Met Lys Thr Trp Val Lys Ile Val Phe Gly Val Ala Thr Ser Ala Val
1               5                   10                  15

Leu Ala Leu Leu Val Met Cys Ile Val Leu Arg Pro Ser Arg Val His
            20                  25                  30

Asn Ser Glu Glu Asn Thr Met Arg Ala Leu Thr Leu Lys Asp Ile Leu
        35                  40                  45

Asn Gly Thr Phe Ser Tyr Lys Thr Phe Phe Pro Asn Trp Ile Ser Gly
    50                  55                  60

Gln Glu Tyr Leu His Gln Ser Ala Asp Asn Asn Ile Val Leu Tyr Asn
65                  70                  75                  80

Ile Glu Thr Gly Gln Ser Tyr Thr Ile Leu Ser Asn Arg Thr Met Lys
                85                  90                  95

Ser Val Asn Ala Ser Asn Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Ser Asn Gly Glu Phe Val Arg Gly Asn
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Phe Asn Gly Arg Glu Asn Lys Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asn Gly Lys Phe Leu Ala Tyr Ala
    210                 215                 220

Glu Phe Asn Asp Thr Asp Ile Pro Val Ile Ala Tyr Ser Tyr Tyr Gly
225                 230                 235                 240

Asp Glu Gln Tyr Pro Arg Thr Ile Asn Ile Pro Tyr Pro Lys Ala Gly
                245                 250                 255

Ala Lys Asn Pro Val Val Arg Ile Phe Ile Ile Asp Thr Thr Tyr Pro
            260                 265                 270

Ala Tyr Val Gly Pro Gln Glu Val Pro Val Pro Ala Met Ile Ala Ser
        275                 280                 285

Ser Asp Tyr Tyr Phe Ser Trp Leu Thr Trp Val Thr Asp Glu Arg Val
    290                 295                 300

Cys Leu Gln Trp Leu Lys Arg Val Gln Asn Val Ser Val Leu Ser Ile
305                 310                 315                 320

Cys Asp Phe Arg Glu Asp Trp Gln Thr Trp Asp Cys Pro Lys Thr Gln
                325                 330                 335

Glu His Ile Glu Glu Ser Arg Thr Gly Trp Ala Gly Gly Phe Phe Val
            340                 345                 350

Ser Thr Pro Val Phe Ser Tyr Asp Ala Ile Ser Tyr Tyr Lys Ile Phe
        355                 360                 365

Ser Asp Lys Asp Gly Tyr Lys His Ile His Tyr Ile Lys Asp Thr Val
    370                 375                 380

Glu Asn Ala Ile Gln Ile Thr Ser Gly Lys Trp Glu Ala Ile Asn Ile
385                 390                 395                 400

Phe Arg Val Thr Gln Asp Ser Leu Phe Tyr Ser Ser Asn Glu Phe Glu

                405                 410                 415

Glu Tyr Pro Gly Arg Arg Asn Ile Tyr Arg Ile Ser Ile Gly Ser Tyr
            420                 425                 430

Pro Pro Ser Lys Lys Cys Val Thr Cys His Leu Arg Lys Glu Arg Cys
        435                 440                 445

Gln Tyr Tyr Thr Ala Ser Phe Ser Asp Tyr Ala Lys Tyr Tyr Ala Leu
    450                 455                 460

Val Cys Tyr Gly Pro Gly Ile Pro Ile Ser Thr Leu His Asp Gly Arg
465                 470                 475                 480

Thr Asp Gln Glu Ile Lys Ile Leu Glu Glu Asn Lys Glu Leu Glu Asn
                485                 490                 495

Ala Leu Lys Asn Ile Gln Leu Pro Lys Glu Glu Ile Lys Lys Leu Glu
            500                 505                 510

Val Asp Glu Ile Thr Leu Trp Tyr Lys Met Ile Leu Pro Pro Gln Phe
        515                 520                 525

Asp Arg Ser Lys Lys Tyr Pro Leu Leu Ile Gln Val Tyr Gly Gly Pro
    530                 535                 540

Cys Ser Gln Ser Val Arg Ser Val Phe Ala Val Asn Trp Ile Ser Tyr
545                 550                 555                 560

Leu Ala Ser Lys Glu Gly Met Val Ile Ala Leu Val Asp Gly Arg Gly
                565                 570                 575

Thr Ala Phe Gln Gly Asp Lys Leu Leu Tyr Ala Val Tyr Arg Lys Leu
            580                 585                 590

Gly Val Tyr Glu Val Glu Asp Gln Ile Thr Ala Val Arg Lys Phe Ile
        595                 600                 605

Glu Met Gly Phe Ile Asp Glu Lys Arg Ile Ala Ile Trp Gly Trp Ser
    610                 615                 620

Tyr Gly Gly Tyr Val Ser Ser Leu Ala Leu Ala Ser Gly Thr Gly Leu
625                 630                 635                 640

Phe Lys Cys Gly Ile Ala Val Ala Pro Val Ser Ser Trp Glu Tyr Tyr
                645                 650                 655

Ala Ser Val Tyr Thr Glu Arg Phe Met Gly Leu Pro Thr Lys Asp Asp
            660                 665                 670

Asn Leu Glu His Tyr Lys Asn Ser Thr Val Met Ala Arg Ala Glu Tyr
        675                 680                 685

Phe Arg Asn Val Asp Tyr Leu Leu Ile His Gly Thr Ala Asp Asp Asn
    690                 695                 700

Val His Phe Gln Asn Ser Ala Gln Ile Ala Lys Ala Leu Val Asn Ala
705                 710                 715                 720

Gln Val Asp Phe Gln Ala Met Trp Tyr Ser Asp Gln Asn His Gly Leu
                725                 730                 735

Ser Gly Leu Ser Thr Asn His Leu Tyr Thr His Met Thr His Phe Leu
            740                 745                 750

Lys Gln Cys Phe Ser Leu Ser Asp
        755                 760

<210> 2
<211> 766
<212> PRT
<213> Homo sapiens

<220>
<221> MISC_FEATURE
<223> Dipeptidyl peptidase 4 (DPP4)

<400> 2

Met Lys Thr Pro Trp Lys Val Leu Leu Gly Leu Leu Gly Ala Ala Ala
1               5                   10                  15

Leu Val Thr Ile Ile Thr Val Pro Val Val Leu Leu Asn Lys Gly Thr
            20                  25                  30

Asp Asp Ala Thr Ala Asp Ser Arg Lys Thr Tyr Thr Leu Thr Asp Tyr
        35                  40                  45

Leu Lys Asn Thr Tyr Arg Leu Lys Leu Tyr Ser Leu Arg Trp Ile Ser
    50                  55                  60

Asp His Glu Tyr Leu Tyr Lys Gln Glu Asn Asn Ile Leu Val Phe Asn
65                  70                  75                  80

Ala Glu Tyr Gly Asn Ser Ser Val Phe Leu Glu Asn Ser Thr Phe Asp
                85                  90                  95

Glu Phe Gly His Ser Ile Asn Asp Tyr Ser Ile Ser Pro Asp Gly Gln
            100                 105                 110

Phe Ile Leu Leu Glu Tyr Asn Tyr Val Lys Gln Trp Arg His Ser Tyr
        115                 120                 125

Thr Ala Ser Tyr Asp Ile Tyr Asp Leu Asn Lys Arg Gln Leu Ile Thr
    130                 135                 140

Glu Glu Arg Ile Pro Asn Asn Thr Gln Trp Val Thr Trp Ser Pro Val
145                 150                 155                 160

Gly His Lys Leu Ala Tyr Val Trp Asn Asn Asp Ile Tyr Val Lys Ile
                165                 170                 175

Glu Pro Asn Leu Pro Ser Tyr Arg Ile Thr Trp Thr Gly Lys Glu Asp
            180                 185                 190

Ile Ile Tyr Asn Gly Ile Thr Asp Trp Val Tyr Glu Glu Glu Val Phe
        195                 200                 205

Ser Ala Tyr Ser Ala Leu Trp Trp Ser Pro Asn Gly Thr Phe Leu Ala
    210                 215                 220

Tyr Ala Gln Phe Asn Asp Thr Glu Val Pro Leu Ile Glu Tyr Ser Phe
225                 230                 235                 240

Tyr Ser Asp Glu Ser Leu Gln Tyr Pro Lys Thr Val Arg Val Pro Tyr
                245                 250                 255

Pro Lys Ala Gly Ala Val Asn Pro Thr Val Lys Phe Phe Val Val Asn
            260                 265                 270

Thr Asp Ser Leu Ser Ser Val Thr Asn Ala Thr Ser Ile Gln Ile Thr
        275                 280                 285

Ala Pro Ala Ser Met Leu Ile Gly Asp His Tyr Leu Cys Asp Val Thr
    290                 295                 300

Trp Ala Thr Gln Glu Arg Ile Ser Leu Gln Trp Leu Arg Arg Ile Gln
305                 310                 315                 320

Asn Tyr Ser Val Met Asp Ile Cys Asp Tyr Asp Glu Ser Ser Gly Arg
                325                 330                 335

Trp Asn Cys Leu Val Ala Arg Gln His Ile Glu Met Ser Thr Thr Gly
            340                 345                 350

Trp Val Gly Arg Phe Arg Pro Ser Glu Pro His Phe Thr Leu Asp Gly
        355                 360                 365

Asn Ser Phe Tyr Lys Ile Ile Ser Asn Glu Glu Gly Tyr Arg His Ile
    370                 375                 380

Cys Tyr Phe Gln Ile Asp Lys Lys Asp Cys Thr Phe Ile Thr Lys Gly
385                 390                 395                 400

Thr Trp Glu Val Ile Gly Ile Glu Ala Leu Thr Ser Asp Tyr Leu Tyr
                405                 410                 415

Tyr Ile Ser Asn Glu Tyr Lys Gly Met Pro Gly Gly Arg Asn Leu Tyr
            420                 425                 430

Lys Ile Gln Leu Ser Asp Tyr Thr Lys Val Thr Cys Leu Ser Cys Glu
        435                 440                 445

Leu Asn Pro Glu Arg Cys Gln Tyr Tyr Ser Val Ser Phe Ser Lys Glu
    450                 455                 460

Ala Lys Tyr Tyr Gln Leu Arg Cys Ser Gly Pro Gly Leu Pro Leu Tyr
465                 470                 475                 480

Thr Leu His Ser Ser Val Asn Asp Lys Gly Leu Arg Val Leu Glu Asp
                485                 490                 495

Asn Ser Ala Leu Asp Lys Met Leu Gln Asn Val Gln Met Pro Ser Lys
            500                 505                 510

Lys Leu Asp Phe Ile Ile Leu Asn Glu Thr Lys Phe Trp Tyr Gln Met
        515                 520                 525

Ile Leu Pro Pro His Phe Asp Lys Ser Lys Lys Tyr Pro Leu Leu Leu
    530                 535                 540

Asp Val Tyr Ala Gly Pro Cys Ser Gln Lys Ala Asp Thr Val Phe Arg
545                 550                 555                 560

Leu Asn Trp Ala Thr Tyr Leu Ala Ser Thr Glu Asn Ile Ile Val Ala
                565                 570                 575

Ser Phe Asp Gly Arg Gly Ser Gly Tyr Gln Gly Asp Lys Ile Met His
            580                 585                 590

Ala Ile Asn Arg Arg Leu Gly Thr Phe Glu Val Glu Asp Gln Ile Glu

595                 600                 605

Ala Ala Arg Gln Phe Ser Lys Met Gly Phe Val Asp Asn Lys Arg Ile
    610                 615                 620

Ala Ile Trp Gly Trp Ser Tyr Gly Gly Tyr Val Thr Ser Met Val Leu
625                 630                 635                 640

Gly Ser Gly Ser Gly Val Phe Lys Cys Gly Ile Ala Val Ala Pro Val
                645                 650                 655

Ser Arg Trp Glu Tyr Tyr Asp Ser Val Tyr Thr Glu Arg Tyr Met Gly
            660                 665                 670

Leu Pro Thr Pro Glu Asp Asn Leu Asp His Tyr Arg Asn Ser Thr Val
        675                 680                 685

Met Ser Arg Ala Glu Asn Phe Lys Gln Val Glu Tyr Leu Leu Ile His
    690                 695                 700

Gly Thr Ala Asp Asp Asn Val His Phe Gln Gln Ser Ala Gln Ile Ser
705                 710                 715                 720

Lys Ala Leu Val Asp Val Gly Val Asp Phe Gln Ala Met Trp Tyr Thr
                725                 730                 735

Asp Glu Asp His Gly Ile Ala Ser Ser Thr Ala His Gln His Ile Tyr
            740                 745                 750

Thr His Met Ser His Phe Ile Lys Gln Cys Phe Ser Leu Pro
        755                 760                 765

<210>  3
<211>  710
<212>  PRT
<213>  Homo sapiens

<220>
<221>  MISC_FEATURE
<223>  Prolyl endopeptidase (PREP)

<400>  3

Met Leu Ser Leu Gln Tyr Pro Asp Val Tyr Arg Asp Glu Thr Ala Val
1               5                   10                  15

Gln Asp Tyr His Gly His Lys Ile Cys Asp Pro Tyr Ala Trp Leu Glu
            20                  25                  30

Asp Pro Asp Ser Glu Gln Thr Lys Ala Phe Val Glu Ala Gln Asn Lys
        35                  40                  45

Ile Thr Val Pro Phe Leu Glu Gln Cys Pro Ile Arg Gly Leu Tyr Lys
    50                  55                  60

Glu Arg Met Thr Glu Leu Tyr Asp Tyr Pro Lys Tyr Ser Cys His Phe
65                  70                  75                  80

Lys Lys Gly Lys Arg Tyr Phe Tyr Phe Tyr Asn Thr Gly Leu Gln Asn
                85                  90                  95

Gln Arg Val Leu Tyr Val Gln Asp Ser Leu Glu Gly Glu Ala Arg Val
            100                 105                 110

Phe Leu Asp Pro Asn Ile Leu Ser Asp Asp Gly Thr Val Ala Leu Arg
        115                 120                 125

Gly Tyr Ala Phe Ser Glu Asp Gly Glu Tyr Phe Ala Tyr Gly Leu Ser
    130                 135                 140

Ala Ser Gly Ser Asp Trp Val Thr Ile Lys Phe Met Lys Val Asp Gly
145                 150                 155                 160

Ala Lys Glu Leu Pro Asp Val Leu Glu Arg Val Lys Phe Ser Cys Met
                165                 170                 175

Ala Trp Thr His Asp Gly Lys Gly Met Phe Tyr Asn Ser Tyr Pro Gln
            180                 185                 190

Gln Asp Gly Lys Ser Asp Gly Thr Glu Thr Ser Thr Asn Leu His Gln
        195                 200                 205

Lys Leu Tyr Tyr His Val Leu Gly Thr Asp Gln Ser Glu Asp Ile Leu
    210                 215                 220

Cys Ala Glu Phe Pro Asp Glu Pro Lys Trp Met Gly Gly Ala Glu Leu
225                 230                 235                 240

Ser Asp Asp Gly Arg Tyr Val Leu Leu Ser Ile Arg Glu Gly Cys Asp
                245                 250                 255

Pro Val Asn Arg Leu Trp Tyr Cys Asp Leu Gln Gln Glu Ser Ser Gly
            260                 265                 270

Ile Ala Gly Ile Leu Lys Trp Val Lys Leu Ile Asp Asn Phe Glu Gly
        275                 280                 285

Glu Tyr Asp Tyr Val Thr Asn Glu Gly Thr Val Phe Thr Phe Lys Thr
    290                 295                 300

Asn Arg Gln Ser Pro Asn Tyr Arg Val Ile Asn Ile Asp Phe Arg Asp
305                 310                 315                 320

Pro Glu Glu Ser Lys Trp Lys Val Leu Val Pro Glu His Glu Lys Asp
                325                 330                 335

Val Leu Glu Trp Ile Ala Cys Val Arg Ser Asn Phe Leu Val Leu Cys
            340                 345                 350

Tyr Leu His Asp Val Lys Asn Ile Leu Gln Leu His Asp Leu Thr Thr
        355                 360                 365

Gly Ala Leu Leu Lys Thr Phe Pro Leu Asp Val Gly Ser Ile Val Gly
    370                 375                 380

Tyr Ser Gly Gln Lys Lys Asp Thr Glu Ile Phe Tyr Gln Phe Thr Ser
385                 390                 395                 400

Phe Leu Ser Pro Gly Ile Ile Tyr His Cys Asp Leu Thr Lys Glu Glu
                405                 410                 415

Leu Glu Pro Arg Val Phe Arg Glu Val Thr Val Lys Gly Ile Asp Ala
            420                 425                 430

Ser Asp Tyr Gln Thr Val Gln Ile Phe Tyr Pro Ser Lys Asp Gly Thr
        435                 440                 445

Lys Ile Pro Met Phe Ile Val His Lys Lys Gly Ile Lys Leu Asp Gly
    450                 455                 460

Ser His Pro Ala Phe Leu Tyr Gly Tyr Gly Gly Phe Asn Ile Ser Ile
465                 470                 475                 480

Thr Pro Asn Tyr Ser Val Ser Arg Leu Ile Phe Val Arg His Met Gly
                485                 490                 495

Gly Ile Leu Ala Val Ala Asn Ile Arg Gly Gly Gly Glu Tyr Gly Glu
            500                 505                 510

Thr Trp His Lys Gly Gly Ile Leu Ala Asn Lys Gln Asn Cys Phe Asp
        515                 520                 525

Asp Phe Gln Cys Ala Ala Glu Tyr Leu Ile Lys Glu Gly Tyr Thr Ser
    530                 535                 540

Pro Lys Arg Leu Thr Ile Asn Gly Gly Ser Asn Gly Gly Leu Leu Val
545                 550                 555                 560

Ala Ala Cys Ala Asn Gln Arg Pro Asp Leu Phe Gly Cys Val Ile Ala
                565                 570                 575

Gln Val Gly Val Met Asp Met Leu Lys Phe His Lys Tyr Thr Ile Gly
            580                 585                 590

His Ala Trp Thr Thr Asp Tyr Gly Cys Ser Asp Ser Lys Gln His Phe
        595                 600                 605

Glu Trp Leu Val Lys Tyr Ser Pro Leu His Asn Val Lys Leu Pro Glu
    610                 615                 620

Ala Asp Asp Ile Gln Tyr Pro Ser Met Leu Leu Leu Thr Ala Asp His
625                 630                 635                 640

Asp Asp Arg Val Val Pro Leu His Ser Leu Lys Phe Ile Ala Thr Leu
                645                 650                 655

Gln Tyr Ile Val Gly Arg Ser Arg Lys Gln Ser Asn Pro Leu Leu Ile
            660                 665                 670

His Val Asp Thr Lys Ala Gly His Gly Ala Gly Lys Pro Thr Ala Lys
        675                 680                 685

Val Ile Glu Glu Val Ser Asp Met Phe Ala Phe Ile Ala Arg Cys Leu
    690                 695                 700

Asn Val Asp Trp Ile Pro
705                 710

## Description

### FIELD OF INVENTION

The present invention is related to a chemical compound; an inhibitor of fibroblast activation protein (FAP); a composition comprising the compound and inhibitor, respectively; the compound, the inhibitor and the composition, respectively, for use in a method for the diagnosis of a disease; the compound, the inhibitor and the composition, respectively, for use in a method for the treatment of a disease; the compound, the inhibitor and the composition, respectively, for use in a method of diagnosis and treatment of a disease which is also referred to as "thera(g)nosis" or "thera(g)nostics"; the compound, the inhibitor and the composition, respectively, for use in a method for delivering an effector to a FAP-expressing tissue; a method for the diagnosis of a disease using the compound, the inhibitor and the composition, respectively; a method for the treatment of a disease using the compound, the inhibitor and the composition, respectively; a method for the diagnosis and treatment of a disease which is also referred to as "thera(g)nosis" or "thera(g)nostics, using the compound, the inhibitor and the composition, respectively; a method for the delivery of an effector to a FAP-expressing tissue using the compound, the inhibitor and the composition, respectively.

### BACKGROUND

Despite the increasing availability of therapeutic options, cancer is still the second leading cause of death globally. Therapeutic strategies mainly focus on targeting malignant cancer cells itself, ignoring the ever-present surrounding tumor microenvironment (TME) that limit the access of therapeutic cancer cell agents (Valkenburg, et al., Nat Rev Clin Oncol, 2018, 15: 366). The TME is part of the tumor mass and consists not only of the heterogeneous population of cancer cells but also of a variety of resident and infiltrating host cells, secreted factors, and extracellular matrix proteins (Quail, et al., Nat Med, 2013, 19: 1423). A dominant cell type found in the TME is the cancer associated fibroblast (CAF) (Kalluri, Nat Rev Cancer, 2016, 16: 582). Many different cell types have been described as the source and origin for CAFs, such as e.g. fibroblasts, mesenchymal stem cells, smooth muscle cells, cells of epithelial origin, or endothelial cells (Madar, et al., Trends Mol Med, 2013, 19: 447). CAFs exhibit mesenchymal-like features and often are the dominant cell type within a solid tumor mass. CAFs have attracted increasing attention as a player in tumor progression and homeostasis (Gascard, et al., Genes Dev, 2016, 30: 1002; LeBleu, et al., Dis Model Mech, 2018, 11).

During recent years, fibroblast activation protein (FAP) has gained notoriety as a marker of CAFs (Shiga, et al., Cancers (Basel), 2015, 7: 2443; Pure, et al., Oncogene, 2018, 37: 4343; Jacob, et al., Curr Mol Med, 2012, 12: 1220). Due to the omnipresence of CAFs and stroma within tumors, FAP was discovered as a suitable marker for radiopharmaceutical diagnostics and as a suitable target for radiopharmaceutical therapy (Siveke, J Nucl Med, 2018, 59: 1412).

Fibroblast activation protein α (FAP) is a type II transmembrane serine protease and a member of the S9 prolyl oligopeptidase family (Park, et al., J Biol Chem, 1999, 274: 36505). The closest family member DPP4 shares 53% homology with FAP. Like other DPP enzymes (DPP4, DPP7, DPP8, DPP9), FAP has post-proline exopeptidase activity. In addition, FAP possesses endopeptidase activity, similar to prolyl oligopeptidase/endopeptidase (POP/PREP). The FAP gene is highly conserved across various species. The extracellular domain of human FAP shares 90% amino acid sequence identity with mouse and rat FAP. Mouse FAP has 97% sequence identity with rat FAP.

Structurally, FAP is a 760 amino acid transmembrane protein composed of a short N-terminal cytoplasmic tail (6 amino acids), a single transmembrane domain (20 amino acids), and a 734 amino acid extracellular domain (Aertgeerts, et al., J Biol Chem, 2005, 280: 19441). This extracellular domain consists of an eight-bladed β-propeller and an α/β hydrolase domain. The catalytic triad is composed of Ser624, Asp702, and His734 and is located at the interface of the β-propeller and the hydrolase domain. The active site is accessible through a central hole of the β-propeller domain or through a narrow cavity between the β-propeller and the hydrolase domain. FAP monomers are not active, but form active homodimers as well as heterodimers with DPP4 (Ghersi, et al., Cancer Res, 2006, 66: 4652). Soluble homodimeric FAP has also been described (Keane, et al., FEBS Open Bio, 2013, 4: 43; Lee, et al., Blood, 2006, 107: 1397).

FAP possesses dual enzyme activity (Hamson, et al., Proteomics Clin Appl, 2014, 8: 454). Its dipeptidyl peptidase activity allows cleaving two amino acids of the N-terminus after a proline residue. FAP substrates that are cleaved rapidly via its dipeptidyl peptidase activity are neuropeptide Y, Peptide YY, Substance P, and B-type natriuretic peptide. Collagen I and III, FGF21 and α₂-antiplasmin have been shown to be cleaved by the endopeptidase activity of FAP. While FAP is unable to cleave native collagens, pre-digestion by other proteases, such as matrix metalloproteinases, facilitates further collagen cleavage by FAP. Processing of collagen may influence migratory capacities of cancer cells. Besides increasing invasiveness of cancer cells through remodeling of the extracellular matrix, several other FAP-mediated tumor promoting roles have been proposed, including proliferation and increasing angiogenesis. Furthermore, stromal expression of FAP is linked to escape from immunosurveillance in various cancers, suggesting a role in anti-tumor immunity (Pure, et al., Oncogene, 2018, 37: 4343).

FAP is transiently expressed during normal development, but only rarely in healthy adult tissues. In transgenic mice, it was demonstrated that FAP is expressed by adipose tissue, skeletal muscle, skin, bone and pancreas (Pure, et al., Oncogene, 2018, 37: 4343; Roberts, et al., J Exp Med, 2013, 210: 1137). However, a FAP knockout mouse has a healthy phenotype, suggesting a redundant role under normal conditions (Niedermeyer, et al., Mol Cell Biol, 2000, 20: 1089). At sites of active tissue remodeling, including wound healing, fibrosis, arthritis, atherosclerosis and cancer, FAP becomes highly upregulated in stromal cells (Pure, et al., Oncogene, 2018, 37: 4343).

FAP expression in the tumor stroma of 90% of epithelial carcinomas was first reported in 1990 under use of a monoclonal antibody, F19 (Garin-Chesa, et al., Proc Natl Acad Sci USA, 1990, 87: 7235; Rettig, et al., Cancer Res, 1993, 53: 3327). FAP-expressing stromal cells were further characterized as cancer-associated fibroblasts (CAF) and cancer-associated pericytes (Cremasco, et al., Cancer Immunol Res, 2018, 6: 1472). FAP expression on malignant epithelial cells has also been reported but its significance remains to be defined (Pure, et al., Oncogene, 2018, 37: 4343). The following Table 1, taken from Busek *et al.* (Busek, et al., Front Biosci (Landmark Ed), 2018, 23: 1933), summarizes the expression of FAP in various malignancies indicating the tumor type and the cellular expression.

**Table 1: FAP expression in human malignancies (from Busek et al.)**

| **Tumor Type** | **Expression of FAP In Malignant Cells** | **Expression of FAP In Stroma Cells** | **Notes** |
|---|---|---|---|
| Basal cell carcinoma, squamous cell carcinoma of the skin | - | + | Expression in fibroblasts strongest in close proximity to cancer cells. FAP expression is absent in benign epithelial tumors, its positivity in the stroma may be a useful criterion for differentiating between morpheaform/infiltrative basal cell carcinomas and FAP-negative desmoplastic trichoepithelioma. |
| Oral squamous cell carcinoma | + | + | FAP is a negative prognostic marker - elevated expression is associated with greater tumor size, lymph-node metastasis, advanced clinical stage, and worse overall survival. |
| Melanoma | - (*in situ*) | + | FAP expression present in a subset of melanocytes in 30% of benign melanocytic nevi, but not detectable in malignant melanoma cells in melanoma tissues. The quantity of FAP-positive stromal cells is positively associated with ECM content and inflammatory cell infiltration. Normal melanocytes express FAP *in vitro.* Conflicting data for FAP in melanoma cells: several human melanoma cell lines express FAP and FAP contributes to their invasiveness *in vitro,* but immunopositivity has not been detected in melanoma tissues. Mouse melanoma cell lines are FAP-negative and mouse FAP is a tumor suppressor independently of its enzymatic activity. |
| Esophageal cancer | + | + | FAP is expressed in cancer cells as well as in premalignant metaplastic cells of the esophagus in both adenocarcinoma and squamous cell carcinoma. |
| Gastric cancer | + | + (incl. low expression in endothelial cells) | A higher stromal FAP expression at the invasion front is associated with low tumor cell differentiation, more advanced TNM stage, serosal invasion, and poor survival. A higher stromal FAP is associated with worse survival. A higher FAP expression in intestinal-type gastric cancer (in stroma, moderately differentiated cancer cells, and endothelial cells) than in the diffuse type (mainly in cancer cells with poor cell-to-cell contacts, endothelial cells). A higher stromal FAP expression in the intestinal-type gastric cancer is associated with the presence of liver and lymph node metastases. |
| Colorectal cancer | + | + | A higher stromal FAP positivity found in earlier-stage disease, but in patients with stage IV tumors high FAP is associated with worse survival. A higher FAP expression is associated with advanced Duke stage. A high FAP expression in the tumor center is a negative prognostic factor. Stromal FAP expression in stage II/III rectal cancer after chemoradiotherapy is associated with a worse prognosis. A higher FAP mRNA expression is associated with worse disease-free survival and a trend for worse overall survival. |
| Pancreatic adenocarcinoma | + | + | FAP expression in carcinoma cells is associated with a larger tumor size, presence of a fibrotic focus, perineural invasion, and a worse prognosis. Stromal FAP expression correlates with lymph node metastasis and reduced survival. Nevertheless, a recent retrospective Korean study reports an association between a lower number of FAP+ fibroblasts and a decreased overall survival based on a univariate analysis. |
| Hepatocellular carcinoma | + | | FAP expression detected especially in tumors with abundant fibrous stroma. FAP mRNA expression increased in peritumoral tissue, positively correlating with the density of peritumoral activated HSCs. Higher levels are associated with more frequent early recurrence, larger tumor size, presence of vascular invasion, and an advanced TNM stage. |
| Non-small cell lung cancer | -/+ | + | Absence of stromal FAP expression (24% of cases) in NSCLC is associated with better survival. Reports regarding expression in cancer cells are inconsistent. |
| Mesothelioma | + | + | Expression, although to a variable extent, has been detected in all subtypes. |
| Breast tumors | + (ductal adenocarci noma) | + (incl. endothelial cells | FAP positivity detected mainly in the stroma; another study proposes a predominant localization in cancer cells in ductal adenocarcinoma. Jung *et al.* observed expression in cancer and stromal cells in 50% of cases where stroma is rich in adipose tissue (approximately 1/3 of all tumors); in these cases, FAP expression was associated with a higher tumor grade. In tumors with fibrous stroma, FAP expression was virtually absent (2/3 of all tumors) |
| | | | FAP expression is higher in cancer cells in lobular cancer than in ductal carcinoma. Stromal FAP and calponin positivity may be an ancillary marker for detecting microinvasion in ductal carcinoma. FAP expression increases with the malignant progression of phyllodes tumors, but a later study detected stromal FAP expression only in 12.5% of the malignant phyllodes tumors by IHC. Conflicting data regarding a possible association with breast cancer survival: smaller studies have reported that a higher total FAP mRNA expression is associated with worse survival, while a higher stromal FAP expression detected by IHC was associated with a longer overall survival and disease-free survival. A recent larger study involving 939 breast cancer patients did not prove any association between FAP expression in the cancer or stromal cells and survival. |
| Renal cancer | - | + | Stromal FAP expression (detected in 23% of cases) associated with markers of aggressiveness and worse survival in clear cell renal cell carcinoma. In metastatic clear cell renal carcinoma, stromal FAP expression was detected in 36% of primary and 44% of metastatic lesions, and was associated with several parameters of tumor aggressiveness and worse survival. |
| Prostate cancer | - | + | Only small patient cohorts reported in literature. Expression in stromal cells detected in 7/7 cases, most intense in stromal cells adjacent to cancer cells. |
| Cervical cancer | + | + | No FAP expression was detected in preinvasive cervical neoplasia (CIN1, 2), occasional positivity in stroma in CIN3 with moderate or severe inflammatory infiltrates. Enhanced expression of FAP was found in cancer cells and subepithelial stromal cells in some of the microinvasive and all of the invasive carcinomas. |
| Ovary | + | + | FAP positivity increases with tumor stage; negative FAP expression is associated with longer disease-free survival. FAP positivity detected in cancer cells in 21% of tumors, stromal positivity in 61%. Another study reported stromal positivity in 92% of cancer tissues with extremely rare FAP expression in malignant cells; it also reported an association with advanced tumor stage and presence of lymph node metastases. FAP-positive malignant cells are present in malignant pleural and peritoneal effusions: strong positivity is associated with worse survival. |
| Glioma | + | + | FAP expression increased in glioblastoma, highest expression found in the mesenchymal subtype and gliosarcoma. Low expression in glioma stem-like cells. In glioblastoma, overall FAP quantity is not associated with survival. |
| Thyroid cancer | - | + | FAP upregulated in aggressive papillary thyroid carcinomas. In medullary thyroid carcinoma, FAP expression in the peritumoral and intratumoral stromal compartment correlates with the degree of desmoplasia and presence of lymph node metastases. |
| Parathyroid tumors | n.d. | + | FAP mRNA expression was significantly higher in parathyroid carcinomas than in adenomas. |
| Sarcomas | + (see note) | + (reactive fibroblasts in Ewing's sarcomas) | FAP expression found in malignant cells in fibrosarcomas, leiomyosarcoma, malignant fibrous histiocytoma, low grade myofibroblastic sarcoma, fibroblastic areas in osteosarcomas, osteoid osteoma, and in osteosarcoma. FAP is negative in malignant cells with "small round cell" phenotype (embryonal rhabdomyosarcoma, Ewing sarcoma, or mesenchymal chondrosarcoma). A higher expression in osteosarcoma associated with more advanced clinical stage, presence of distant metastasis, high histological grade, and a worse progression-free and overall survival. FAP is expressed in both malignant and benign tumors and its positivity reflects their histogenetic origin rather than malignant potential. |
| Myeloma | - | + | FAP expression was detected in osteoclasts, endothelial cells, adipocytes, fibrotic stroma, but not in multiple myeloma cells. FAP is upregulated in osteoclasts co-cultured with myeloma cells. |

FAP expression in CAFs was shown for almost all carcinomas and sarcomas (Pure, et al., Oncogene, 2018, 37: 4343; Busek, et al., Front Biosci (Landmark Ed), 2018, 23: 1933). Furthermore, CAFs are present in hematological malignancies (Raffaghello, et al., Oncotarget, 2015, 6: 2589). Utilization of FAP as a therapeutic target is therefore not limited to certain tumor entities.

The abundance of FAP-expressing CAFs is described to correlate with poor prognosis. Across a wide range of human tumor indications, FAP expression is described to correlate with higher tumor grade and worse overall survival (Pure, et al., Oncogene, 2018, 37: 4343).

As described above, it is indicated that FAP as well as FAP-expressing cells present in the tumor microenvironment significantly influence tumor progression (Hanahan, et al., Cancer Cell, 2012, 21: 309). Additionally, due to its relatively selective expression in tumors, FAP is regarded as a suitable target for therapeutic and diagnostic agents as described below (Siveke, J Nucl Med, 2018, 59: 1412; Christiansen, et al., Neoplasia, 2013, 15: 348; Zi, et al., Mol Med Rep, 2015, 11: 3203).

Soon after its discovery, FAP was utilized as a therapeutic target in cancer. Until today, various strategies have been explored, including e.g. inhibition of FAP enzymatic activity, ablation of FAP-positive cells, or targeted delivery of cytotoxic compounds.

In 2007, an inhibitor of FAP and DPP4, Talabostat (Val-boro-Pro, PT-100), was developed by Point Therapeutics (for example as described in U.S. patent No. 6,890,904, WO9916864). Pennisi et al. (Pennisi, et al., Br J Haematol, 2009, 145: 775) observed a reduced tumor growth in a multiple myeloma animal model as well as in cancer syngeneic mouse models. Furthermore, several other prolyl boronic acid derivatives have been developed and reported as putative selective inhibitors for FAP. These derivatives show instability in aqueous environments at physiologic pH (Coutts, et al., J Med Chem, 1996, 39: 2087) and a nonspecific reactivity with other enzymes.

WO 2008/116054 disclosed hexapeptide derivatives wherein compounds comprise a C-terminal bis-amino or boronic acid functional group.

US 2017/0066800 disclosed pseudopeptide inhibitors, such as M83, effective against FAP. These inhibitors were assessed in lung and colon cancer xenografts in immunodeficient mice. A suppression of tumor growth was observed (Jackson, et al., Neoplasia, 2015, 17: 43). These pseudopeptides inhibit the activity of both prolyl oligopeptidase (POP/PREP) and FAP, thereby excluding their use as specific therapeutic FAP inhibitors.

US 2008/280856 disclosed a nanomolar boronic acid-based inhibitor. The inhibitor shows a bispecific inhibition of FAP and PREP, thereby excluding their use as specific therapeutic FAP inhibitors.

FAP inhibitors based on cyclic peptides were disclosed, e.g., in WO 2016/146174 and WO 2006/042282. WO 2016/146174 disclosed peptides for diagnosis and treatment of tumors expressing FAP showing specificity for FAP, whereby closely related homologue DPP4 was not recognized by said peptides. WO 2006/042282 disclosed polypeptides for treatment of melanoma. In nude mice, inhibition of melanoma growth and melanoma metastasis was shown.

WO 99/75151 and WO 01/68708 disclosed a humanized FAP monoclonal antibody, F19, (Sibrotuzumab). Furthermore, the anti-FAP antibody F19 and humanized versions thereof were disclosed in WO 99/57151 and WO 01/68708. Development approaches involved e.g. the generation of high affinity, species cross-reactive, FAP-specific scFvs converted into a bivalent derivative (Brocks, et al., Mol Med, 2001, 7: 461). In Phase I and II clinical trials, Sibrotuzumab showed specific tumor enrichment whilst failing to demonstrate measurable therapeutic activity in patients with metastatic colorectal cancer, with only 2 out of 17 patients having stable disease (Hofheinz, et al., Onkologie, 2003, 26: 44). This F19 antibody has not been shown to block any cellular or protease function of FAP, which might explain the lack of therapeutic effects (Hofheinz, et al., Onkologie, 2003, 26: 44; Scott, et al., Clin Cancer Res, 2003, 9: 1639).

US 2018/022822 disclosed novel molecules specifically binding to human FAP and epitopes thereof, as human-derived antibodies and chimeric antigen receptors (CARs) useful in the treatment of diseases and conditions induced by FAP. Treatment of mice bearing orthotopic syngeneic MC38 colorectal tumors with an anti-FAP antibody reduced the tumor diameter and number of metastasis. WO 2012/020006 disclosed glycoengineered antibodies that bear modified oligosaccharides in the Fc region. Subsequently, bispecific antibodies specific for FAP and DR5 were developed as subject to WO 2014/161845. These antibodies trigger tumor cell apoptosis in vitro and in in vivo preclinical tumor models with FAP-positive stroma (Brunker, et al., Mol Cancer Ther, 2016, 15: 946). Antibody drug conjugates and immunotoxins that target FAP are described in WO 2015/118030. In vitro toxicity as well as in vivo inhibition of tumor growth was shown following application of anti-hu/moFAP hu36:cytolysin ADC candidates. It is unclear whether these antibodies were capable of inhibiting FAP activity.

Small molecule FAP inhibitors based on (4-quinolinoyl)glycyl-2-cyanopyrrolidine displaying low nanomolar inhibitory potency and high selectivity against related DPPs and PREP were described by Jansen et al. (Jansen, et al., J Med Chem, 2014, 57: 3053; Jansen, et al., ACS Med Chem Lett, 2013, 4: 491) and disclosed in WO 2013/107820. However, the compounds are structurally unrelated to the compounds of the present invention and include a war-head leading to covalent binding to FAP.

In recent years, several FAP-targeted radiopharmaceutical approaches were developed which are exemplarily described herein.

WO 2010/036814 disclosed small molecule inhibitors of FAP for use as therapeutic agents through inhibition of FAPs enzyme activity or as radiopharmaceuticals through binding to FAP.

WO 2019/083990 disclosed imaging and radiotherapeutic agents based on small molecule FAP-inhibitors described by Jansen *et al.* (Jansen, et al., J Med Chem, 2014, 57: 3053; Jansen, et al., ACS Med Chem Lett, 2013, 4: 491). Furthermore, several authors described selective uptake in tumors of cancer patients of imaging and radiotherapeutic agents (Lindner, et al., J Nucl Med, 2018, 59: 1415; Loktev, et al., J Nucl Med, 2018, 59: 1423; Giesel, et al., J Nucl Med, 2019, 60: 386; Loktev, et al., J Nucl Med, 2019, Mar 8 *(epub ahead ofprint);* Giesel, et al., Eur J Nucl Med Mol Imaging, 2019, 46: 1754; Kratochwil, et al., J Nucl Med, 2019, 60: 801) based on FAP-inhibitors described by Jansen *et al.* (Jansen, et al., J Med Chem, 2014, 57: 3053; Jansen, et al., ACS Med Chem Lett, 2013, 4: 491).

Clinical assessments of a ¹³¹I-labeled, humanized form of the F19 antibody (sibrotuzumab) revealed a selective uptake by tumors but not by normal tissues in patients with colorectal carcinoma or non-small cell lung cancer (Scott, et al., Clin Cancer Res, 2003, 9: 1639). This may be due to the long circulation time of antibodies that makes them unsuitable for a diagnostic, therapeutic, or theragnostic approach involving radionuclides.

WO 2011/040972 disclosed high-affinity antibodies recognizing both human and murine FAP antigen as potent radioimmunoconjugates. ESC11 1gG1 induces down modulation and internalization of surface FAP (Fischer, et al., Clin Cancer Res, 2012, 18: 6208). WO 2017/211809 disclosed tissue targeting thorium-227 complexes wherein the targeting moiety has specificity for FAP. However, the long circulation time of antibodies makes them unsuitable for a diagnostic, therapeutic, or theragnostic approach involving radionuclides.

FAP has also been described as being involved in other diseases than oncology indications, examples of which are given below.

Fibroblast-like synoviocytes in rheumatoid arthritic joints of patients show a significantly increased expression of FAP (Bauer, et al., Arthritis Res Ther, 2006, 8: R171; Milner, et al., Arthritis Res Ther, 2006, 8: R23). In rheumatoid arthritis, stromal cells play an important role in organizing the structure of synovial tissue of joints by producing extracellular matrix components, recruiting infiltrating immune cells and secreting inflammatory mediators. Considerable evidence exists supporting a role for these cells in driving the persistence of inflammation and joint damage (Bartok, et al., Immunol Rev, 2010, 233: 233; Turner, et al., Curr Opin Rheumatol, 2015, 27: 175). In rheumatoid arthritis FAP has a pathological role in cartilage turnover at least by promotion of proteoglycan loss and subsequently cartilage degradation (Bauer, et al., Arthritis Res Ther, 2006, 8: R171; Waldele, et al., Arthritis Res Ther, 2015, 17: 12). Therefore, it might serve as a marker for patient stratification, for evaluation and follow-up of treatment success, or as a therapeutic target (Bauer, et al., Arthritis Res Ther, 2006, 8: R171). In mice, a treatment response was demonstrated using SPECT/CT imaging of a ^{99m}Tc-labeled anti-FAP antibody (van der Geest, et al., Rheumatology (Oxford), 2018, 57: 737; Laverman, et al., J Nucl Med, 2015, 56: 778; van der Geest, et al., J NuclMed, 2017, 58: 151).

Additionally, FAP was recognized not only as a marker of activated fibroblasts in the injury response (Tillmanns, et al., Int J Cardiol, 2013, 168: 3926) but also as an important player in the healing process of wounds (Ramirez-Montagut, et al., Oncogene, 2004, 23: 5435). Jing et al. demonstrated a time-dependent course of change in FAP expression following burn wounds in rats (Jing et al., Nan Fang Yi Ke Da Xue Xue Bao, 2013, 33: 615). Inhibiting of FAP activity in reactive wound fibroblasts in Keloid scars, common benign fibroproliferative reticular dermal lesions, might offer therapeutic option to prevent disease progression (Dienus, et al., Arch Dermatol Res, 2010, 302: 725).

In fibrotic diseases, upregulated expression of FAP was observed e.g. in idiopathic pulmonary fibrosis, Crohn's disease, and liver fibrosis. In an *ex vivo* model for Crohn's disease, a chronic bowel inflammatory disease characterized by an excessive, misbalanced extracellular matrix (ECM) deposition, upregulated FAP expression was observed. FAP inhibition reconstituted extracellular matrix homeostasis (Truffi, et al., Inflamm Bowel Dis, 2018, 24: 332). Similar observations were made by Egger et al. (Egger, et al., Eur J Pharmacol, 2017, 809: 64) under use of a murine model of pulmonary fibrosis. Inhibition of FAP leads to reduced fibrotic pathology. FAP is also expressed in the tissue remodelling region in chronically injured liver (Wang, et al., Front Biosci, 2008, 13: 3168), and FAP expression by hepatic stellate cells correlates with the histological severity of liver disease (Gorrell, et al., Adv Exp Med Biol, 2003, 524: 235). Therefore, FAP is also a promising target in the treatment of liver fibrosis (Lay, et al., Front Biosci (Landmark Ed), 2019, 24: 1).

FAP is expressed in arteriosclerotic lesions and upregulated in activated vascular smooth muscle cells (Monslow, et al., Circulation, 2013, 128: A17597). Monslow et al. showed that targeted inhibition of FAP in arteriosclerotic lesions may decrease overall lesion burden, inhibit inflammatory cell homing, and increase lesion stability through its ability to alter lesion architecture by favoring matrix-rich lesions over inflammation. More importantly, most of the arteriosclerotic pathologies share a common pathogenic feature: the rupture of an atherosclerotic plaque inducing arteriosclerotic lesions (Davies, et al., Br Heart J, 1985, 53: 363; Falk, Am J Cardiol, 1989, 63: 114e). Rupture of the fibrous cap in advanced atherosclerotic plaques is a critical trigger of acute coronary syndromes that may lead to myocardial infarction and sudden cardiac death. One of the key events in promoting plaque instability is the degradation of the fibrous cap, which exposes the underlying thrombogenic plaque core to the bloodstream, thereby causing thrombosis and subsequent vessel occlusion (Farb, et al., Circulation, 1996, 93: 1354; Virmani, et al., J Am Coll Cardiol, 2006, 47: C13). Brokopp et al. showed that FAP contributes to type I collagen breakdown in fibrous caps (Brokopp, et al., Eur Heart J, 2011, 32: 2713). A radiolabeled tracer was developed and its applicability for atherosclerosis imaging shown (Meletta, et al., Molecules, 2015, 20: 2081).

### DETAILED DESCRIPTION OF THE INVENTION

The problem underlying the present invention is the provision of a compound which is suitable as a diagnostic agent and/or a pharmaceutical agent, particularly if conjugated to a diagnostically and/or therapeutically active effector. A further problem underlying the present invention is the provision of a compound which is suitable as a diagnostic agent and/or a pharmaceutical agent, particularly if conjugated to a diagnostically and/or therapeutically active effector, whereby the compound is a potent inhibitor of FAP activity; preferably the pIC50 of the compound is equal to or greater than 6.0. A further problem underlying the present invention is the provision of a compound which is suitable as a diagnostic agent and/or a pharmaceutical agent, particularly if conjugated to a diagnostically and/or therapeutically active effector, in the diagnosis and/or therapy of a disease where the diseased cells and/or diseased tissues express FAP. A still further problem underlying the instant invention is the provision of a compound which is suitable for delivering a diagnostically and/or therapeutically effective agent to a diseased cell and/or diseased tissue, respectively, and more particularly a FAP-expressing diseased cell and/or diseased tissue, preferably the diseased tissue comprises or contains cancer associated fibroblasts. Also, a problem underlying the present invention is the provision of a method for the diagnosis of a disease, of a method for the treatment and/or prevention of a disease, and a method for the combined diagnosis and treatment of a disease; preferably such disease is a disease involving FAP-expressing cells and/or tissues, more particularly a FAP-expressing diseased cell and/or diseased tissue, preferably the diseased tissue comprises or contains cancer associated fibroblasts. A still further problem underlying the present invention is the provision of a method for the identification of a subject, wherein the subject is likely to respond or likely not to respond to a treatment of a disease, a method for the selection of a subject from a group of subjects, wherein the subject is likely to respond or likely not to respond to a treatment of a disease. Also, a problem underlying the present invention is the provision of a pharmaceutical composition containing a compound having the characteristics as outlined above. Furthermore, a problem underlying the present invention is the provision of a kit which is suitable for use in any of the above methods.

These and other problems are solved by the subject matter of the attached independent claims. Preferred embodiments may be taken from the attached dependent claims.

These and other problems underlying the present invention are also solved by the following embodiments.
Embodiment 1. A compound comprising a cyclic peptide
   of formula (I) 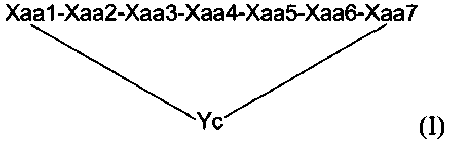 and an N-terminal modification group A attached to Xaa1,
   wherein
   the peptide sequence is drawn from left to right in N to C-terminal direction,
   Xaa1 is a residue of an amino acid of formula (II) 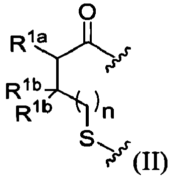 wherein
      R^{1a} is -NH-
      R^{1b} is H or CH₃,
      n = 0 or 1,
      the N-terminal modification group A is covalently attached to the nitrogen atom of Xaa1,
      the carbonyl group of Xaa1 is covalently attached to the nitrogen of Xaa2,
      and the sulfur atom of Xaa1 is covalently attached as thioether to Yc;
   Xaa2 is a residue of an amino acid of formula (III), (IV) or (XX) 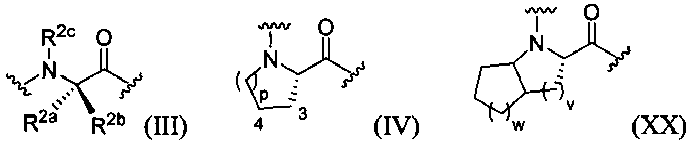 wherein
      R^{2a}, R^{2b}and R^{2c} are each and independently selected from the group consisting of (C₁-C₂)alkyl and H, wherein said (C₁-C₂)alkyl maybe substituted by a substituent selected from the group consisting of OH, NH₂, halogen, (C₅-C₇)cycloalkyl,
      p = 0, 1 or 2
      v = 1 or 2
      w = 1, 2 or 3 and
      the amino acid of formula (IV) maybe substituted by one or two substituents selected from the group consisting of methyl, OH, NH₂ and F, at indicated ring positions 3 and 4;
   Xaa3 is a residue of an amino acid of formula (V) or (XX) 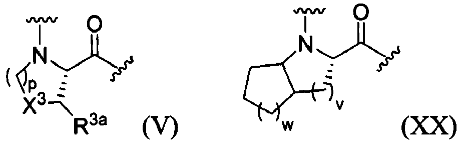 wherein
      X³ is selected from the group consisting of CH₂, CF₂, CH-R^{3b}, S, O and NH,
      p= 1 or2
      v= 1 or2
      w = 1,2 or 3,
      R^{3a} is H, methyl, OH, NH₂ or F,
      R^{3b} is methyl, OH, NH₂ or F;
   Xaa4 is a residue of an amino acid of formula (VI) 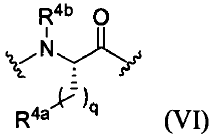 wherein
      R^{4a} is selected from the group consisting of H, OH, COOH, CONH₂, X⁴ and-NH-CO-X⁴, wherein X⁴ is selected from the group consisting of (C₁-C₆)alkyl, (C₅-C₆)aryl and (C₅-C₆)heteroaryl, and X⁴ may be substituted by one or two substituents selected from the group consisting of methyl, CONH₂, halogen, NH₂ and OH;
      q = 1, 2 or 3, wherein optionally, one or two hydrogens of said one, two, or three CH₂-groups are each and individually substituted by methyl, ethyl, (C₅-C₆)aryl or (C₅-C₆)heteroaryl,
      R^{4b} is methyl or H;
   Xaa5 is a residue of an amino acid of structure (VII) 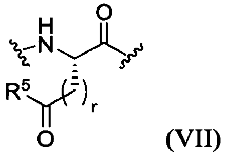 wherein
      R⁵ is selected from the group of OH and NH₂, and
      r = 1, 2 or 3;
   Xaa6 is an amino acid selected from the group consisting of an aromatic L-α-amino acid and a heteroaromatic L-α-amino acid;
   Xaa7 is a residue of an amino thiol or an amino acid of formula (IX), 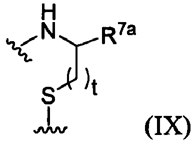 wherein
      R^{7a} is -CO-, -COOH,-CONH₂,-CH2-OH,-(CO)-NH-R^{7b}, -(CO)-(NR^{7c})-R^{7b} or H, wherein
      R^{7b} and R^{7c} are each and independently (C₁-C₄)alkyl and
      t is 1 or 2;
   Yc is a structure of formula (X) 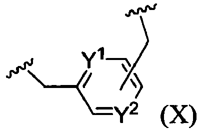 linking the S atom of Xaa1 and the S atom of Xaa7 under the formation of two thioether linkages thus forming a cyclic structure of formula (XXI) 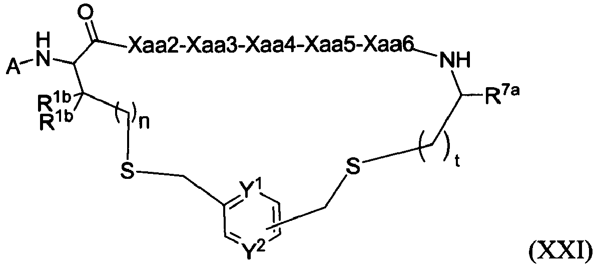 wherein
      the substitution pattern of the aromatic group in formula (X) is *ortho, meta* or *para,*
      n = 0 or 1,
      t = 1 or 2,
      Y¹ is C-H or N,
      Y² is N or C-R^{c1},
      R^{c1} is H or CH₂-R^{c2} and
      R^{c2} is a structure of formula (XI), (XII) or (XXII) 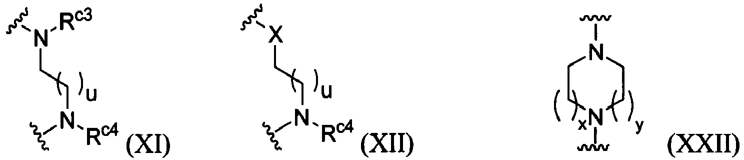 wherein
         R^{c3} and R^{c4} are each and independently selected from the group consisting of H and (C₁-C₄)alkyl and
            u = 1, 2, 3, 4, 5 or 6,
            x and y are each and independently 1, 2 or 3, and
            X = O or S
            wherein in formulae (XI) and (XXII) one of the nitrogen atoms is attached to -CH₂- of R^{c1} and in formula (XII) -X- is attached to -CH₂- of R^{cl}; and
         wherein the N-terminal modification group A is either a blocking group Abl or an amino acid Aaa.
Embodiment 2. The compound of Embodiment 1, wherein the blocking group Abl is selected from the group consisting of R^{a1}-C(O)-, R^{a1}-S(O₂)-, R^{a1}-NH-C(O)- and R^{a1}-O-C(O)-; wherein R^{a1} is (C₁-C₈)alkyl optionally substituted by up to two substituents each and independently selected from the group consisting of OH, F, COOH, (C₃-C₈)cycloalkyl, aryl, heteroaryl and (C₃-C₈)heterocycle, and wherein in (C₁-C₈)alkyl one of the -CH₂-groups is optionally replaced by -S- or -0- .
Embodiment 3. The compound of Embodiment 2, wherein the blocking group Abl is R^{a1}-C(O)- or R^{a1}-S(O₂)- and R^{a1} is (C₁-C₆)alkyl, wherein optionally one of the -CH₂- groups is replaced by -S- or -O-.
Embodiment 4. The compound of Embodiment 3, wherein the blocking group Abl is hexanoyl or pentyl sulfonyl, preferably blocking group Abl is hexanoyl.
Embodiment 5. The compound of Embodiment 1, wherein the amino acid Aaa is a D-amino acid residue or an L- amino acid residue, each of structure (XIV): 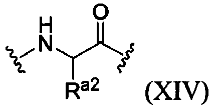 wherein
   R^{a2} is selected from the group consisting of (C₁-C₆)alkyl, modified (C₁-C₆)alkyl, (C₁-C₃)alkyl, modified (C₁-C₃), (C₃-C₈)carbocycle, aryl, heteroaryl and (C₃-C₈)heterocycle, wherein in modified (C₁-C₆)alkyl one -CH₂- group is replaced by -S- or -O-, and in modified (C₁-C₃)alkyl one of the H is substituted by OH, F or COOH, or two of the H are substituted by F.
Embodiment 6. The compound of Embodiment 5, wherein R³² is a C₁-₆ alkyl and one-CH₂- group of said C₁-C₆ is replaced by -S- .
Embodiment 7. The compound of any one of Embodiments 5 to 6, wherein Aaa is selected from the group consisting of the amino acid residues of Nle, nle, Met and met, and their derivatives.
Embodiment 8. The compound of any one of Embodiments 1 to 7, wherein Xaa1 is a D-amino acid residue selected from the group consisting of cys, hcy and pen, or Xaa1 is an L-amino acid residue selected from the group consisting of Cys, Hcy and Pen.
Embodiment 9. The compound of Embodiment 8, wherein Xaa1 is Cys.
Embodiment 10. The compound of any one of Embodiments 1 to 9, wherein Xaa2 is an amino acid residue selected from the group consisting of Pro, Gly, Nmg and their derivatives.
Embodiment 11. The compound of any one of Embodiments 1 to 10, wherein Xaa3 is an amino acid residue selected from the group consisting of Pro, Hyp, Tfp, Cfp, Dmp, Aze and Pip, and their derivatives.
Embodiment 12. The compound of any one of Embodiments 1 to 11, wherein Xaa4 is an amino acid residue selected from the group consisting of Thr, Hse, Asn, Gln and Ser, and their derivatives.
Embodiment 13. The compound of any one of Embodiments 1 to 12, wherein Xaa5 is an amino acid residue selected from the group consisting of Gln and Glu, and their derivatives.
Embodiment 14. The compound of any one of Embodiments 1 to 13, wherein Xaa6 is an amino acid residue of any one of formulae (VIIIa), (VIIIb), (VIIIc) and (VIIId): 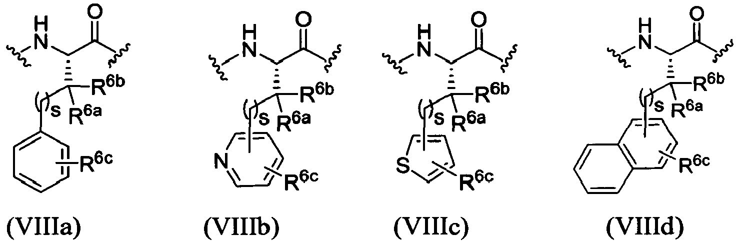 wherein
   R^{6a} and R^{6b} are each and independently selected from the group consisting of H, methyl, ethyl, propyl and isopropyl,
   R^{6c} represents from 0 to 3 substituents, each such substituent being each and independently selected from the group consisting of Cl, F, Br, NO₂, NH₂, CN, CF₃, OH, OR^{6d} and C₁-C₄ alkyl,
   R^{6d} is selected from the group consisting of methyl, ethyl, propyl, and isopropyl, and
   s is 0 or 1.
Embodiment 15. The compound of Embodiment14, wherein Xaa6 is an amino acid residue of any one of formulae (VIIIa), (VIIIb), (VIIIc) and (VIIId):
   wherein
   R^{6a} and R^{6b} are each H
   R^{6c} represents from 0 to 2 substituents, each such substituent being each and independently selected from the group consisting of Cl, F, Br, NO₂, NH₂, CN, CF₃, OH, OR^{6d} and methyl,
   R^{6d} is selected from the group consisting of methyl, ethyl, propyl, and isopropyl, and s is 0.
Embodiment 16. The compound of any one of Embodiments 14 to 15, wherein Xaa6 is an amino acid residue selected from the group consisting of Phe, Ocf, Ppa, Thi, 1Ni, Otf, and Mpa, and their derivatives.
Embodiment 17. The compound of any one of Embodiments 1 to 16, wherein Xaa7 is an amino thiol residue selected from the group consisting of Cys, Cysol, AET, Hcy, cys and hcy.
Embodiment 18. The compound of Embodiment 17, wherein Xaa7 is an amino thiol residue selected from the group consisting of Cys, Cysol and AET.
Embodiment 19. The compound of any one of Embodiments 1 to 18, wherein the compound is a compound of formula (LI), (LII), (LIII) or (LIV): 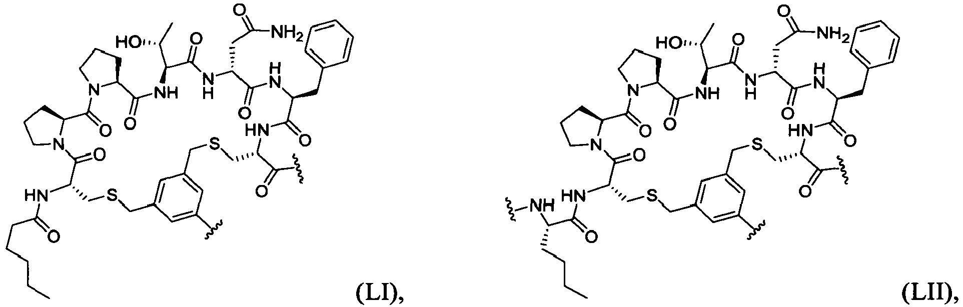 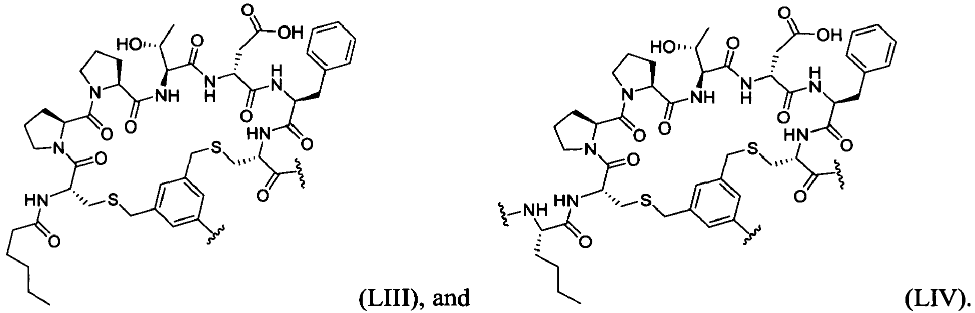
Embodiment 20. The compound of any one of Embodiments 1 to 19, wherein R^{c2} is a structure of any one of formulae (XXIIa), (XIb) and (XIIa): 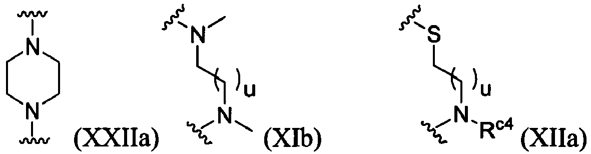
   wherein R^{c4} is H or methyl and
   u = 1, 2, 3, 4 or 5,
   in formulae (XIb) and (XXIIa) any one of the nitrogen atoms is attached to -CH₂- of R^{c1} and in formula (XIIa) -S- is attached to -CH₂- of R^{c1}.
Embodiment 21. The compound of any one of Embodiments 1 to 20, wherein Yc is a structure of formula (XIII): 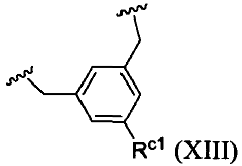
Embodiment 22. The compound of any one of Embodiments 1 to 21, wherein Yc comprises a NH group, preferably a reactive NH group, wherein the NH group allows conjugation of a moiety to Yc, preferably, the NH group is provided by the structure R^{c2}, wherein R^{c2} is selected from the group consisting of a structure of any one of formulae (XXIb), (XIc) and (XIIb): 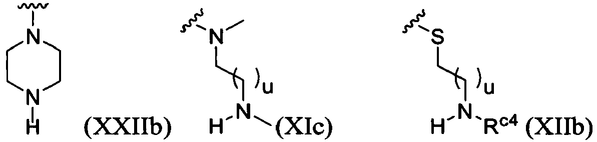
   wherein R^{c4} is H or methyl and
   u = 1, 2, 3,4 or 5.
Embodiment 23. The compound of Embodiment 22, wherein the structure R^{c2} is of formula (XXIIb) or (XIIc): 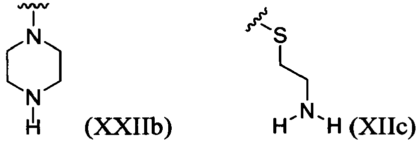
Embodiment 24. The compound of any one of Embodiments 22 to 23, wherein the compound comprises a Z group, wherein the Z group is covalently attached to Yc, preferably to the structure of formula (X), wherein the Z group comprises a chelator and optionally a linker.
Embodiment 25. The compound of Embodiment 24, wherein the Z group is covalently attached to R^{c2}, forming a structure of any one of formulae (XXIIc), (XId) and (XIId): 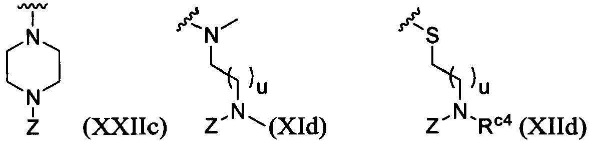
   wherein R^{c4} is H or methyl and
   u = 1, 2, 3,4 or 5.
Embodiment 26. The compound of any one of Embodiments 24 to 25, wherein the Z group comprises a linker, wherein the linker covalently links the chelator to Yc, preferably to R^{c2}.
Embodiment 27. The compound of Embodiment 26, wherein the covalent linkage between Yc and the linker, preferably between Rc2 and the linker, is an amide.
Embodiment 28. The compound of any one of Embodiments 26 to 27, wherein the chelator is covalently linked to the linker, wherein the covalent linkage is selected from the group comprising an amide linkage, a urea linkage, a carbamate linkage, an ester linkage, an ether linkage, a thioether linkage, a sulfonamide, a triazole and a disulfide linkage.
Embodiment 29. The compound of any one of Embodiments 24 to 28, preferably of any one of claims 26 to 28, wherein the linker is selected from the group comprising Ttds, 020c, Apac, Gly, Bal, Gab, Mamb, Pamb, Ppac, 4Amc, Inp, Sni, Rni, Nmg, Cmp, PEG6, PEG12 and other PEG-amino acids, and most preferably Ttds, 020c, Apac, 4Amc, PEG6 and PEG12.
Embodiment 30. The compound of any one of Embodiments 24 to 25, wherein the chelator is covalently linked to Yc, preferably covalently linked to R^{c2}.
Embodiment 31. The compound of Embodiment 30, wherein the chelator is directly linked to Yc.
Embodiment 32. The compound of any one of Embodiments 30 to 31, wherein the Z group is devoid of any linker.
Embodiment 33. The compound of any one of Embodiments 30 to 32, wherein the covalent linkage between Yc and the chelator, preferably between R^{c2} and the chelator, is an amide.
Embodiment 34. The compound of any one of Embodiments 24 to 33, wherein the chelator is selected from the group consisting of DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, NₓS₄₋ₓ (N4, N2S2, N3S), Hynic, ^{99m}Tc(CO)₃-Chelators, more preferably DOTA, DOTAGA, NOTA, NODAGA, NODA-MPAA, HBED, CB-TE2A, DFO, THP, N4 and most preferred DOTA, DOTAGA, NOTA and NODAGA.
Embodiment 35. The compound of any one of Embodiments 1 to 34, preferably any one of claims 1 and 5 to 34, wherein the N-terminal modification group A is the amino acid Aaa and wherein the compound comprises a Z group covalently attached to the amino acid Aaa, wherein the Z group comprises a chelator and optionally a linker.
Embodiment 36. The compound of Embodiment 35, wherein the Z group comprises a linker, wherein the linker covalently links the chelator to the amino acid Aaa, preferably to the α-nitrogen of the amino acid Aaa.
Embodiment 37. The compound of Embodiment 36, wherein the covalent linkage between the linker and the α-nitrogen of the amino acid Aaa is an amide.
Embodiment 38. The compound of any one of Embodiments 36 to 37, wherein the chelator is covalently linked to the linker, wherein the covalent linkage is selected from the group comprising an amide linkage, a urea linkage, a carbamate linkage, an ester linkage, an ether linkage, a thioether linkage, a sulfonamide, a triazole and a disulfide linkage.
Embodiment 39. The compound of any one of Embodiments 35 to 38, wherein the linker is selected from the group comprising Ttds, 020c, Apac, Gly, Bal, Gab, Mamb, Pamb, Ppac, 4Amc, Inp, Sni, Rni, Nmg, Cmp, PEG6, PEG12 and other PEG-amino acids, and most preferably Ttds, 020c, Apac, 4Amc, PEG6 and PEG12, preferably the linker amino acid is selected from the group consisting of Ttds, 020c and PEG6.
Embodiment 40. The compound of any one of Embodiments 35 to 39, wherein the chelator is covalently linked to the amino acid Aaa.
Embodiment 41. The compound of Embodiment 40, wherein the chelator is directly linked to the amino acid Aaa.
Embodiment 42. The compound of any one of Embodiments 40 to 41, wherein the Z group is devoid of any linker.
Embodiment 43. The compound of any one of Embodiments 40 to 42, wherein the covalent linkage between the amino acid Aaa and the chelator is an amide.
Embodiment 44. The compound of any one of Embodiments 35 to 43, wherein the chelator is selected from the group consisting of DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, NₓS₄₋ₓ (N4, N2S2, N3S), Hynic, ^{99m}Tc(CO)₃-Chelators, more preferably DOTA, DOTAGA, NOTA, NODAGA, NODA-MPAA, HBED, CB-TE2A, DFO, THP, N4 and most preferred DOTA, DOTAGA, NOTA and NODAGA.
Embodiment 45. The compound of any one of Embodiments 1 to 47, preferably any one of claims 1 to 34, wherein an amino acid or a peptide is attached to Xaa7, wherein a majority of the amino acids of this peptide are charged or polar and the net charge of the peptide is -2, - 1, 0, +1 or +2.
Embodiment 46. The compound of Embodiment 45, wherein the peptide is selected from the group consisting of peptides of formula (XXXa-f)

   Xaa10-Xaa11-Xaa12-Xaa13-Xaa14 -Xaa15-Xaa16 (XXXa)

   Xaa10-Xaa11-Xaa12-Xaa13-Xaa14-Xaa15 (XXXb)

   Xaa10-Xaa11-Xaa12-Xaa13-Xaa14 (XXXc)

   Xaa10-Xaa11-Xaa12-Xaa13 (XXXd)

   Xaa10-Xaa11-Xaa12 (XXXe)

   Xaa10-Xaa11 (XXXf)

   wherein
   Xaa10 is Asp, asp, Bal, Gly, Gab, Ser, Nmg, Bhf. Lys, Ttds or Bhk
   Xaa11 is His, his, Lys, Ttds, Arg, Ape or Ala,
   Xaa12 is Phe, Nmf, Tic, Aic, Ppa, Mpa, Amf, Nmf, phe, Lys, Ape, Ttds and Ppa
   Xaa13 is Arg, Lys, Ape, Ttds or arg,
   Xaa14 is Asp, Ala, asp, Lys, Ape or Ttds,
   Xaal5 is Ttds, Ape or Lys, and
   Xaa16 is Lys or Ape,
   wherein, optionally,
   Xaa11 and Xaa12 together form a single amino acid selected from the group consisting of Gab, Pamb, Cmp, Pamb, Mamb, and, optionally,
   XaalO, Xaa11 and Xaa12 form together a single amino acid selected from the group consisting of Gab, Pamb, Cmp, Pamb, and Mamb,
   under the proviso that in the peptides of formulae (XXXa-f) Ape, if present, is the C-terminal building block.
Embodiment 47. The compound of any one of Embodiments 45 to 46, wherein the amino acid attached to Xaa7 is Xaa10 of claim 46, preferably the amino acid attached to Xaa7 is Asp, asp, Bal, Gly, Gab, Ser, Nmg, Bhf, Lys, Ape, Ttds or Bhk.
Embodiment 48. The compound of any one of Embodiments 45 to 47, wherein a Z-group is covalently attached to the peptide, preferably to the C-terminal amino acid of the peptide, wherein the Z group comprises a chelator and optionally a linker.
Embodiment 49. The compound of Embodiment 48, wherein the Z-group is covalently attached to the C-terminal amino acid of the peptide, preferably to the C-terminal amino acid of any one of peptides of formulae (XXXa), (XXXb), (XXXc), (XXXd), (XXXe) and (XXXf).
Embodiment 50. The compound of any one of Embodiments 45 to 47, wherein a Z-group is covalently attached to the amino acid attached to Xaa7, wherein the Z group comprises a chelator and optionally a linker.
Embodiment 51. The compound of any one of Embodiments 45 to 50, wherein the Z-group comprises a linker, wherein the linker covalently links the chelator to the amino acid attached to Xaa7, preferably in case no peptide is attached to Xaa7, or the linker covalently links the chelator to the C-terminal amino acid of the peptide, preferably the C-terminal amino acid of any one of peptide of formulae (LI), (LII), (LIII) and (LIV).
Embodiment 52. The compound of Embodiment 51, wherein the covalent linkage is an amide bond.
Embodiment 53. The compound of any one of Embodiments 51 to 52, wherein the chelator is covalently linked to the linker, wherein the covalent linkage is selected from the group comprising an amide linkage, a urea linkage, a carbamate linkage, an ester linkage, an ether linkage, a thioether linkage, a sulfonamide, a triazole and a disulfide linkage.
Embodiment 54. The compound of any one of Embodiments 51 to 53, wherein the linker is selected from the group consisting of Ttds, 020c, Apac, Gly, Bal, Gab, Mamb, Pamb, Ppac, 4Amc, Inp, Sni, Rni, Nmg, Cmp, PEG6, PEG12 and other PEG-amino acids.
Embodiment 55. The compound of Embodiment 54, wherein the linker is selected from the group consisting of Ttds, 020c, Apac, 4Amc, PEG6 and PEG12.
Embodiment 56. The compound of any one of Embodiments 48 to 50, wherein the chelator is covalently linked to the amino acid attached to Xaa7 or the chelator is covalently linked to the C-terminal amino acid of the peptide, preferably the C-terminal amino acid of any one of peptide of formulae (LI), (LII), (LIII) and (LIV).
Embodiment 57. The compound of Embodiment 56, wherein the chelator is directly linked to the amino acid attached to Xaa7 or to the C-terminal amino acid of the peptide, preferably the C-terminal amino acid of any one of peptide of formulae (LI), (LII), (LIII) and (LIV).
Embodiment 58. The compound of any one of Embodiments 56 to 57, wherein the Z group is devoid of any linker.
Embodiment 59. The compound of any one of Embodiments 56 to 58, wherein the covalent linkage between the chelator and the amino acid attached to Xaa7 and the covalent linkage between the chelator and the C-terminal amino acid of the peptide, preferably the C-terminal amino acid of any one of peptide of formulae (LI), (LII), (LIII) and (LIV), is an amide bond.
Embodiment 60. The compound of any one of Embodiments 48 to 59, wherein the chelator is selected from the group consisting of DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, NₓS₄₋ₓ (N4, N2S2, N3S), Hynic, ^{99m}Tc(CO)₃-Chelators, more preferably DOTA, DOTAGA, NOTA, NODAGA, NODA-MPAA, HBED, CB-TE2A, DFO, THP, N4 and most preferred DOTA, DOTAGA, NOTA and NODAGA.
Embodiment 61. The compound of any one of Embodiments 1 to 60, wherein the compound is selected from the group consisting of compound
   H-Met-[Cys(3MeBn)-Pro-Pro-Thr-Glu-Phe-Cys]-Asp-His-Phe-Arg-Asp-Ttds-Lys(Bio)-NH2 (3BP-2881) of the following formula 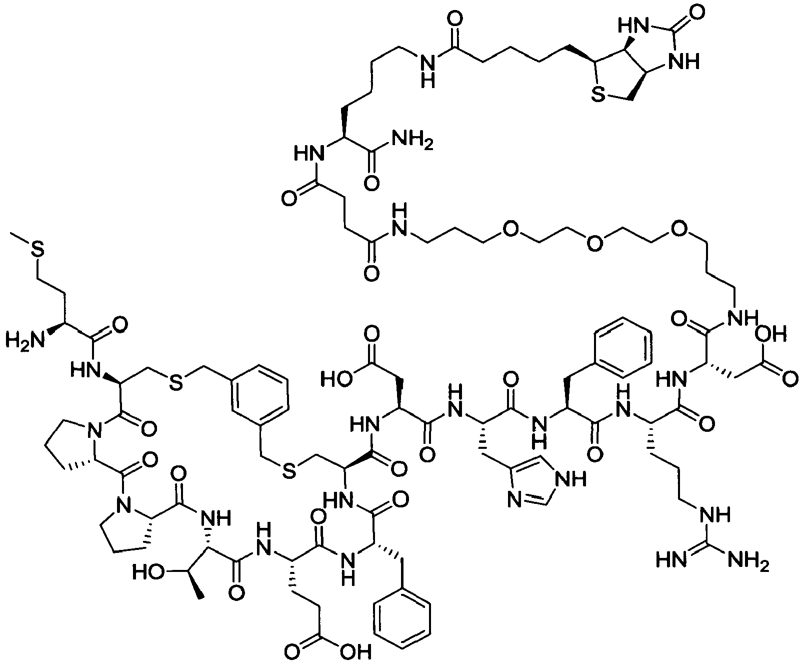
   compound Hex-[Cys(3MeBn)-Pro-Pro-Thr-Glu-Phe-Cys]-Asp-His-Phe-Arg-Asp-NH2 (3BP-2974) of the following formula 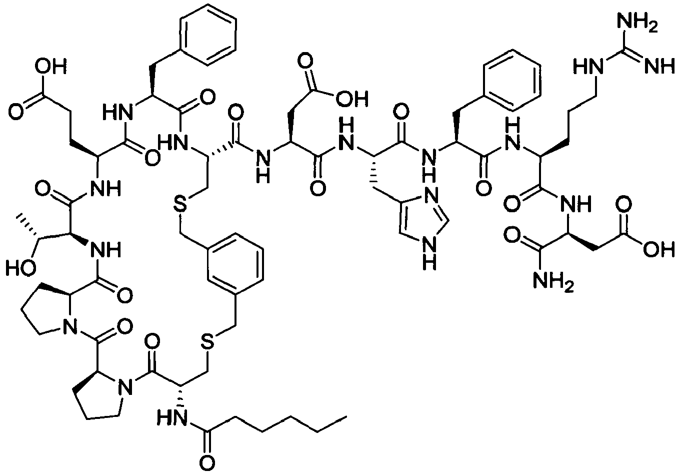
   compound Ac-Met-[Cys(3MeBn)-Pro-Pro-Thr-Glu-Phe-Cys]-Asp-His-Phe-Arg-Asp-NH2 (3BP-2975) of the following formula 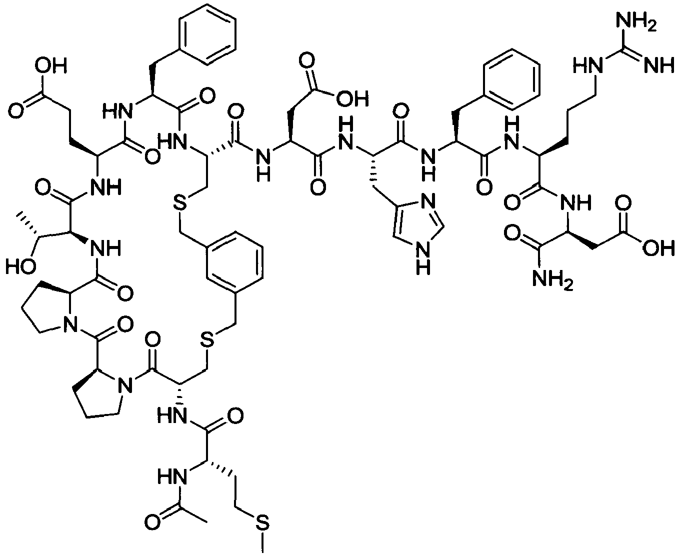
   compound H-met-[Cys(3MeBn)-Pro-Pro-Thr-Glu-Phe-Cys]-Asp-His-Phe-Arg-Asp-NH2 (3BP-2976) of the following formula 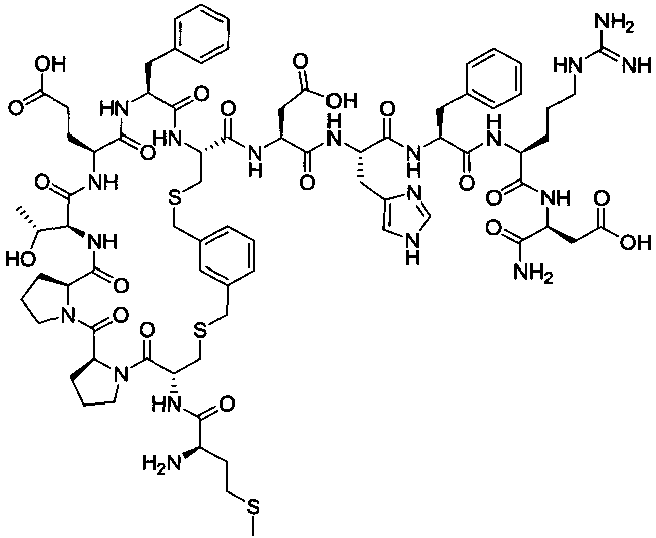
   compound Hex-[Cys(3MeBn)-Pro-Pro-Thr-Glu-Phe-Cys]-Asp-His-Phe-Arg-Asp-Ttds-Lys(DOTA)-NH2 (3BP-3105) of the following formula 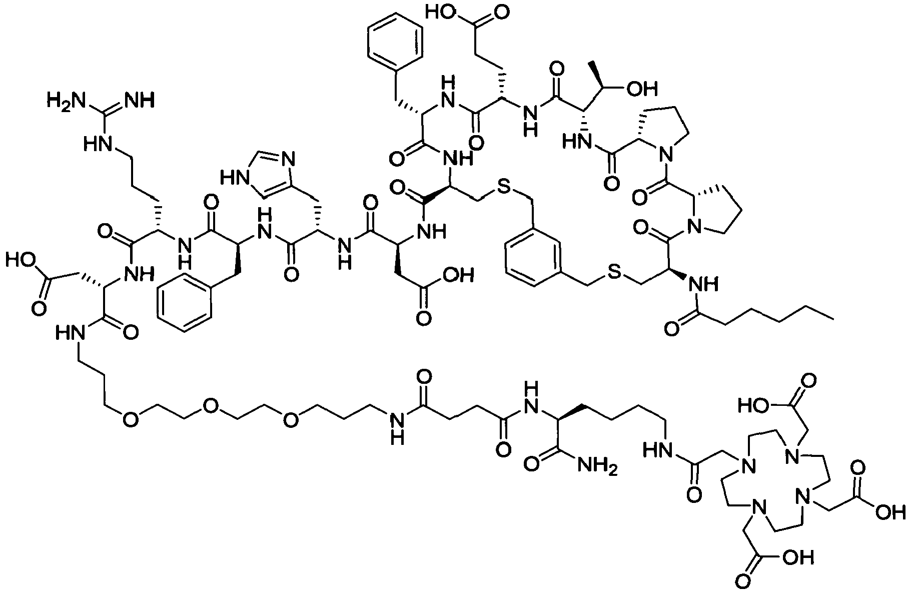
   compound DOTA-Ttds-Nle-[Cys(3MeBn)-Pro-Pro-Thr-Glu-Phe-Cys]-Asp-His-Phe-Arg-Asp-NH2 (3BP-3168) of the following formula 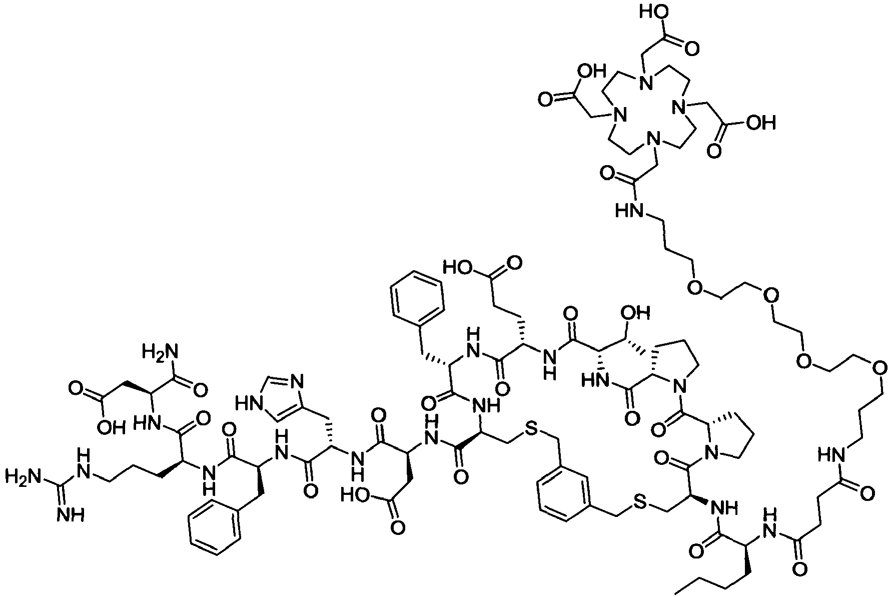
   compound DOTA-Ttds-Met-[Cys(3MeBn)-Pro-Pro-Thr-Glu-Phe-Cys]-Asp-His-Phe-Arg-Asp-NH2 (3BP-3169) of the following formula 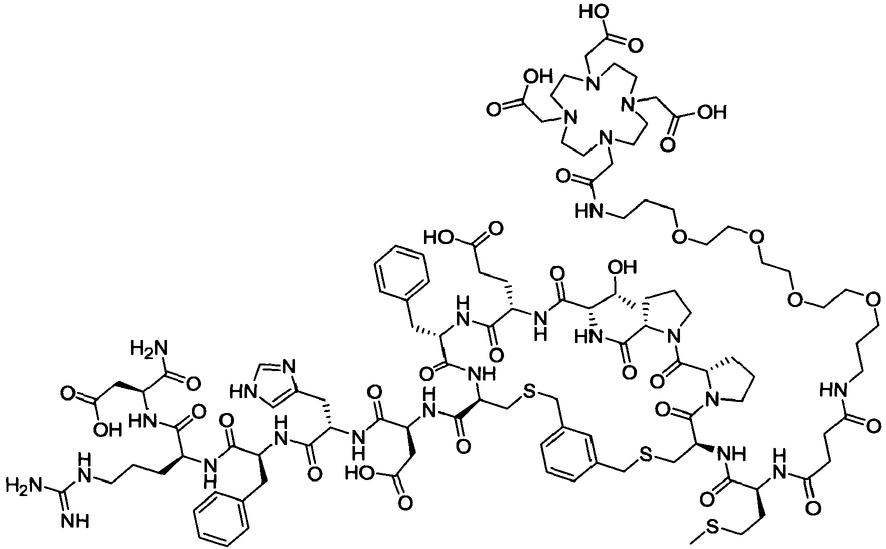
   compound DOTA-Ttds-Leu-[Cys(3MeBn)-Pro-Pro-Thr-Glu-Phe-Cys]-Asp-His-Phe-Arg-Asp-NH2 (3BP-3172) of the following formula 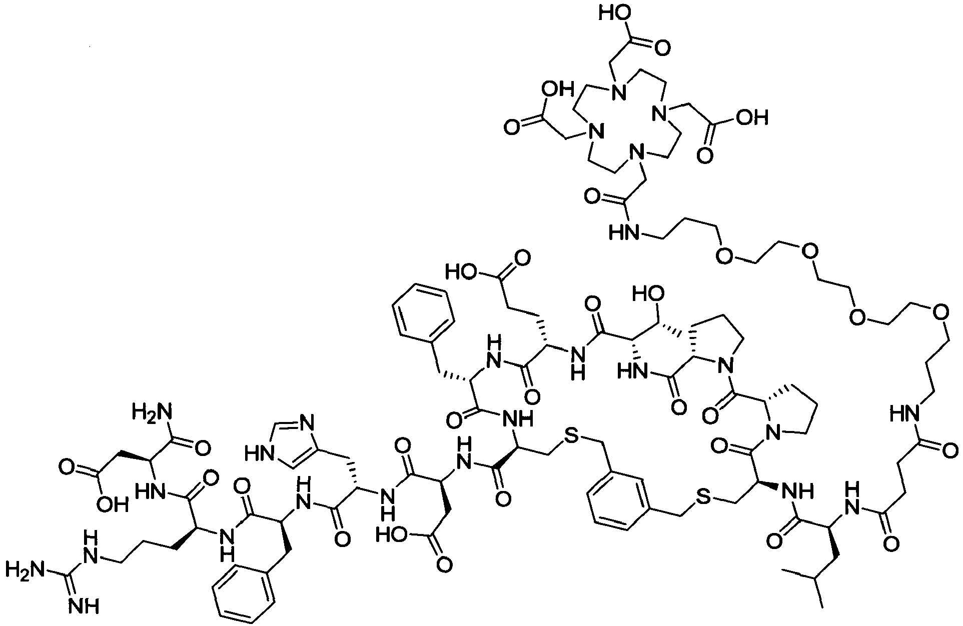
   compound Ac-Met-[cys(3MeBn)-Pro-Pro-Thr-Glu-Phe-Cys]-Asp-His-Phe-Arg-Asp-NH2 (3BP-3175) of the following formula 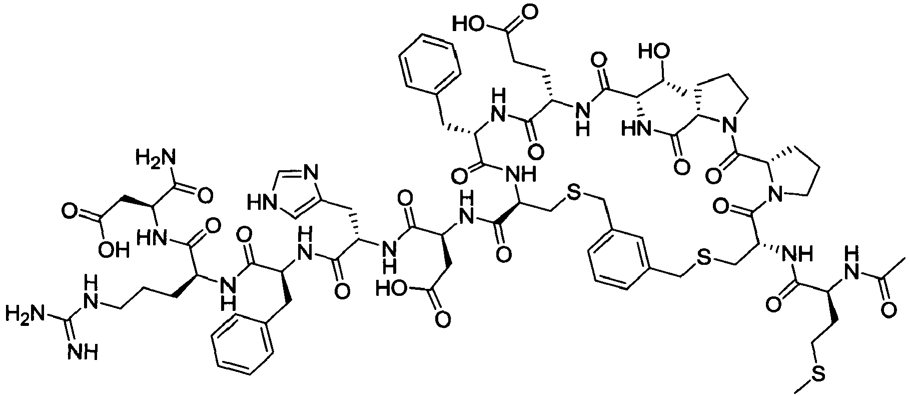
   compound Ac-met-[Cys(3MeBn)-Pro-Pro-Thr-Glu-Phe-Cys]-Asp-His-Phe-Arg-Asp-NH2 (3BP-3187) of the following formula 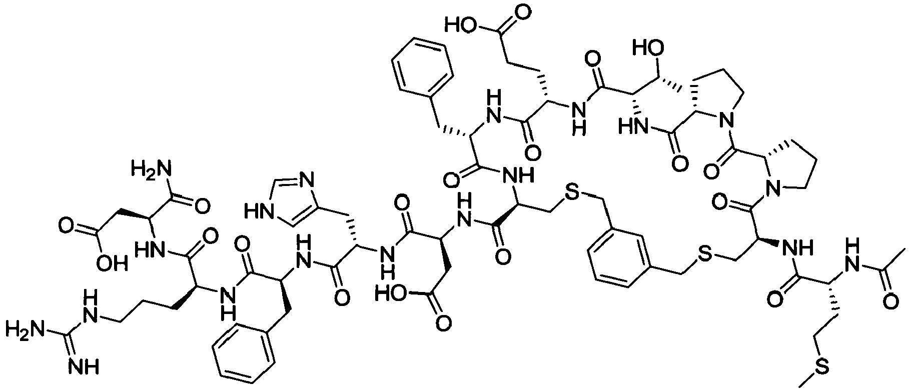
   compound Ac-Met-[Cys(3MeBn)-Pro-Pro-Thr-Glu-Phe-Cys]-Asp-His-Nmf-Arg-Asp-NH2 (3BP-3188) of the following formula 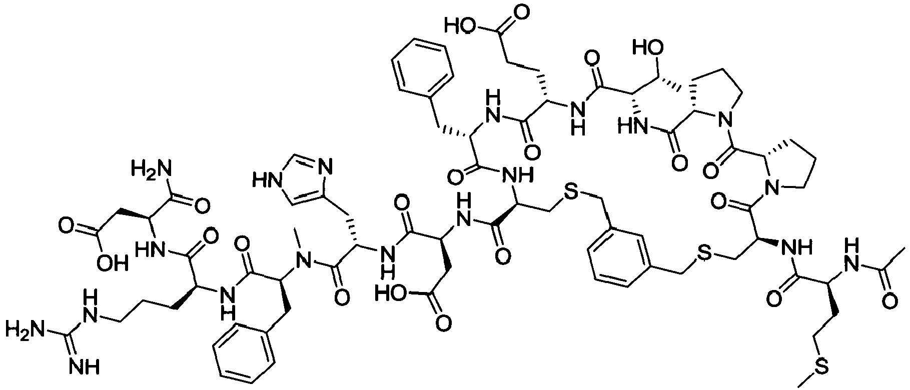
   compound Ac-Met-[Cys(3MeBn)-Pro-Pro-Thr-Glu-Phe-Cys]-Asp-His-Tic-Arg-Asp-NH2 (3BP-3189) of the following formula 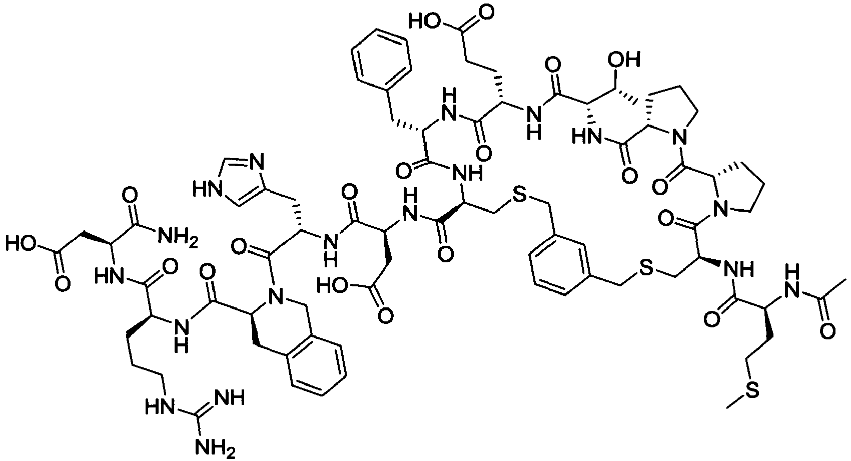
   compound Ac-Met-[Cys(3MeBn)-Pro-Pro-Thr-Glu-Phe-Cys]-Asp-His-Aic-Arg-Asp-NH2 (3BP-3190) of the following formula 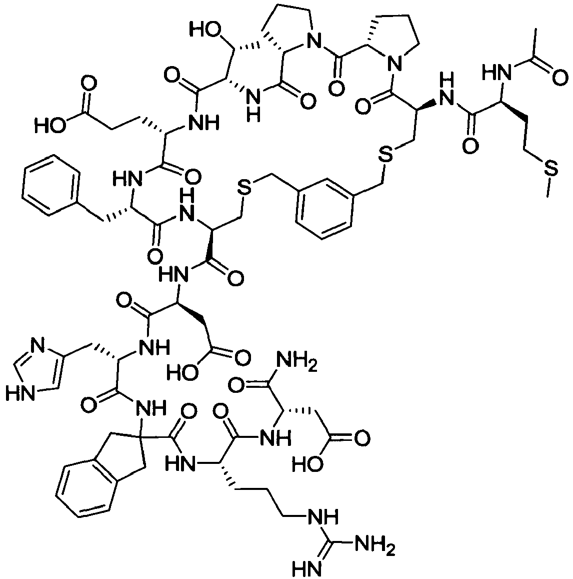
   compound Ac-Met-[Cys(3MeBn)-Pro-Pro-Thr-Glu-Phe-Cys]-Asp-His-Ppa-Arg-Asp-NH2 (3BP-3191) of the following formula 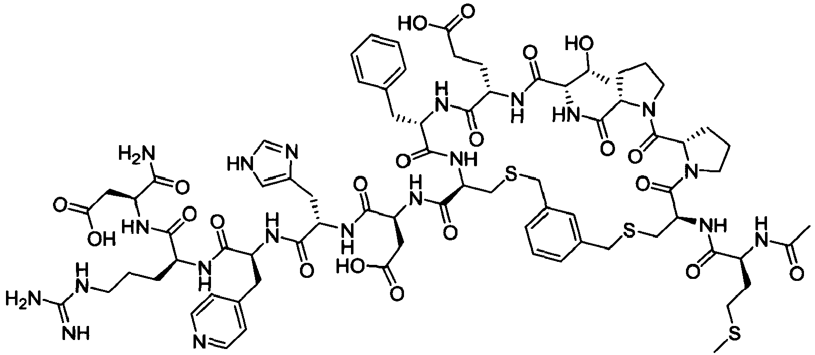
   compound Ac-Met-[Cys(3MeBn)-Pro-Pro-Thr-Glu-Phe-Cys]-Asp-His-Mpa-Arg-Asp-NH2 (3BP-3192) of the following formula 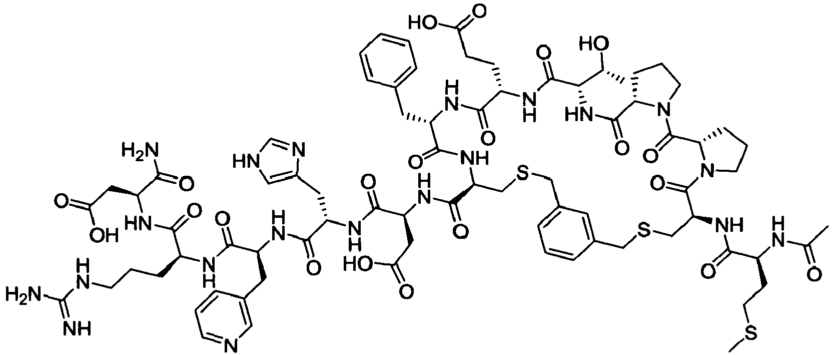
   compound Ac-Met-[Cys(3MeBn)-Pro-Pro-Thr-Glu-Thi-Cys]-Asp-His-Phe-Arg-Asp-NH2 (3BP-3193) of the following formula 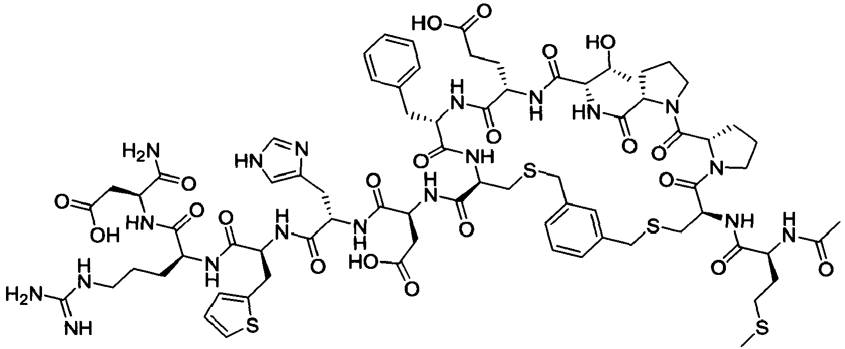
   compound Ac-Met-[Cys(3MeBn)-Pro-Pro-Thr-Glu-Phe-Cys]-Asp-Ala-Phe-Arg-Asp-NH2 (3BP-3195) of the following formula 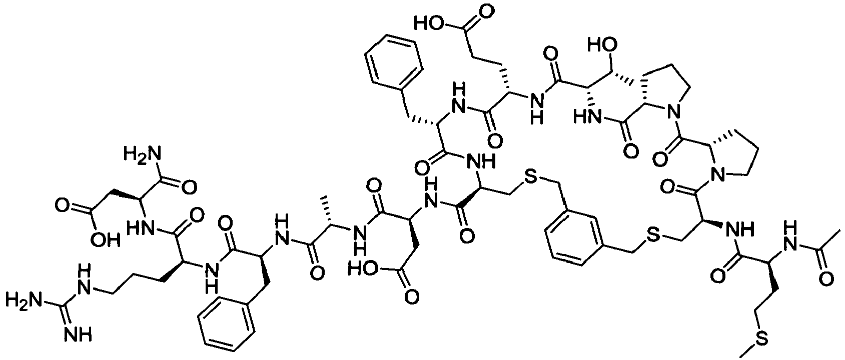
   compound Ac-Met-[Cys(3MeBn)-Pro-Pro-Thr-Glu-Phe-Cys]-Asp-His-Ala-Arg-Asp-NH2 (3BP-3196) of the following formula 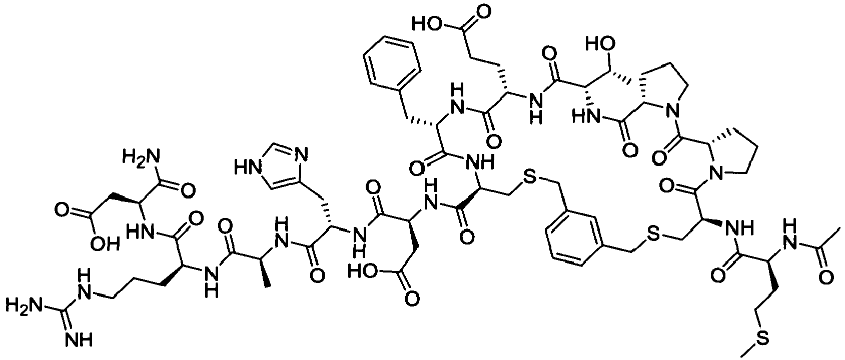
   compound Ac-Met-[Cys(3MeBn)-Pro-Pro-Thr-Glu-Phe-Cys]-Asp-His-Phe-Arg-Ala-NH2 (3BP-3198) of the following formula 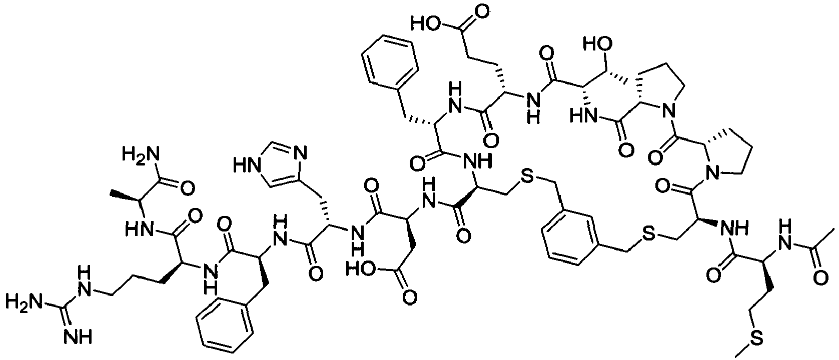
   compound Ac-Met-[Cys(3MeBn)-Pro-Pro-Thr-Glu-Phe-Cys]-Asp-His-Phe-Arg-NH2 (3BP-3200) of the following formula 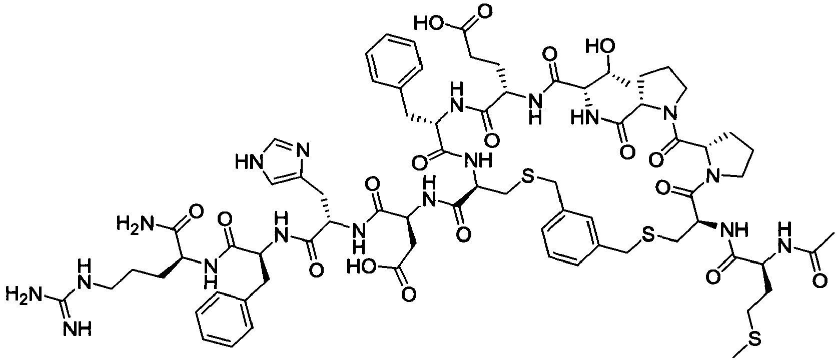
   compound Ac-[Cys(3MeBn)-Pro-Pro-Thr-Glu-Phe-Cys]-Asp-His-Phe-Arg-Asp-NH2 (3BP-3202) of the following formula 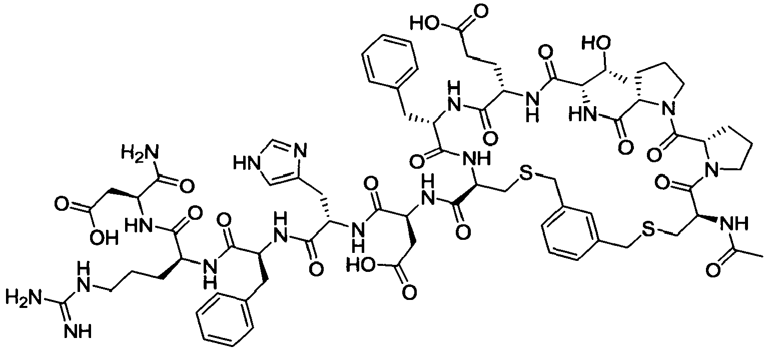
   compound Ac-Met-[Cys(3MeBn)-Pro-Pro-Thr-Glu-Phe-Cys]-Asp-His-Amf-Arg-Asp-NH2 (3BP-3203) of the following formula 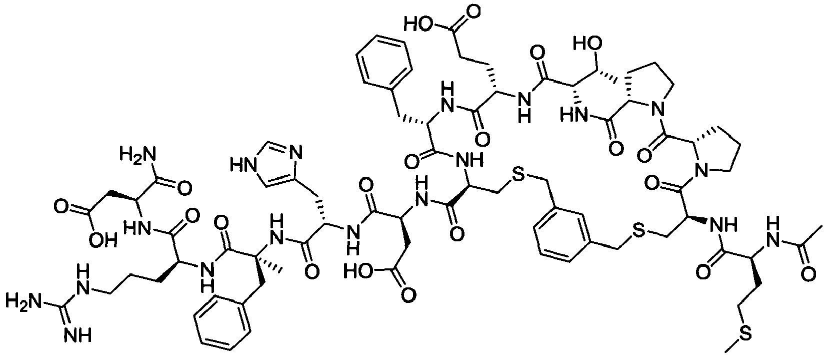
   compound Ac-Met-[Cys(3MeBn)-Pro-Pro-Thr-Glu-Phe-Cys]-Asp-his-Phe-Arg-Asp-NH2 (3BP-3210) of the following formula 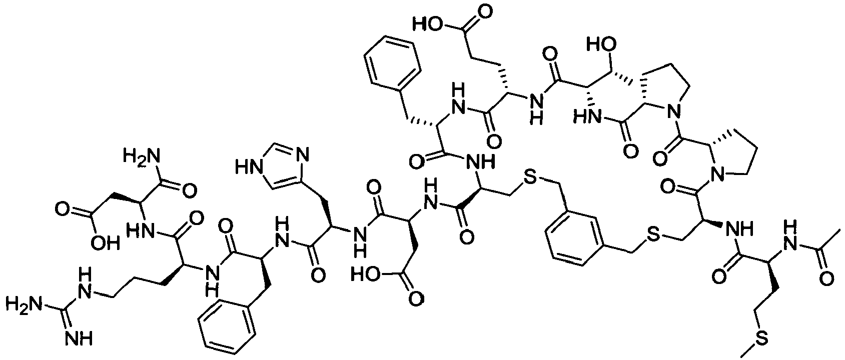
   compound Ac-Met-[Cys(3MeBn)-Pro-Pro-Thr-Glu-Phe-Cys]-Asp-His-phe-Arg-Asp-NH2 (3BP-3211) of the following formula 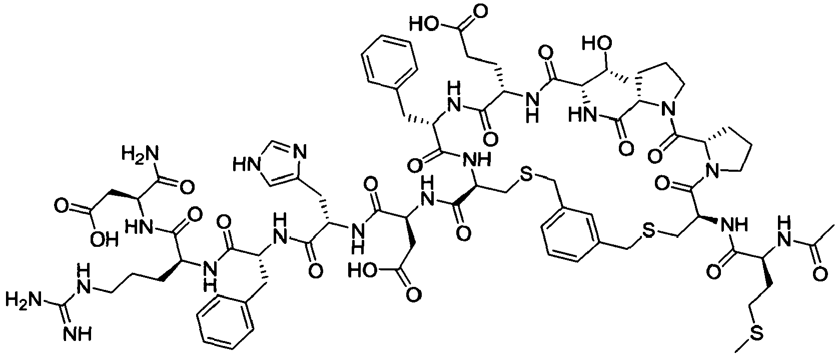
   compound Ac-Met-[Cys(3MeBn)-Pro-Pro-Thr-Glu-Phe-Cys]-Asp-His-Phe-arg-Asp-NH2 (3BP-3212) of the following formula 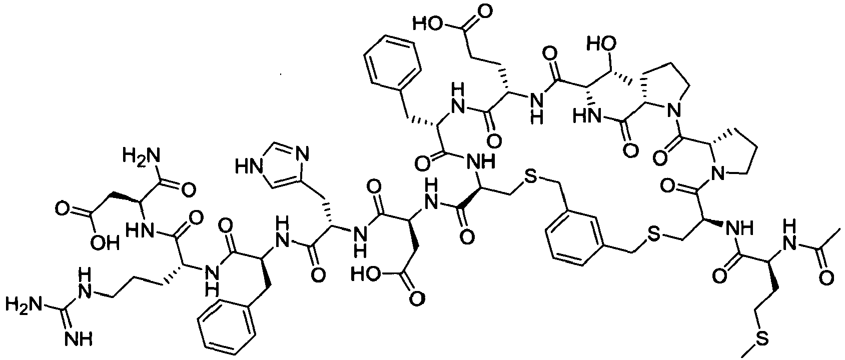
   compound Ac-Met-[Cys(3MeBn)-Pro-Pro-Thr-Glu-Phe-Cys]-Asp-His-Phe-Arg-asp-NH2 (3BP-3213) of the following formula 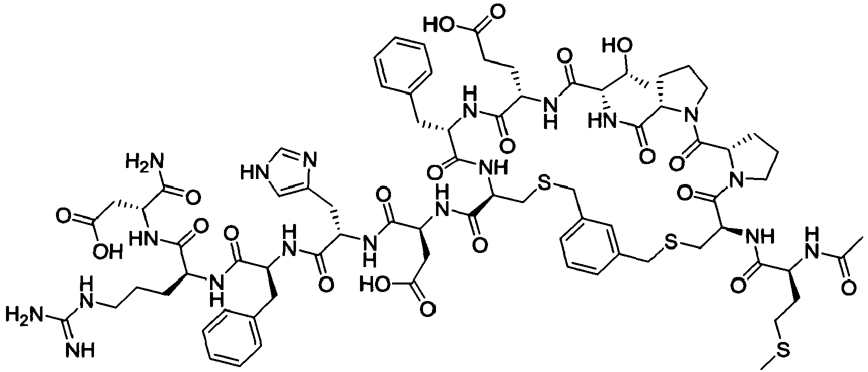
   compound Ac-Met-[Cys(3MeBn)-Gly-Pro-Thr-Glu-Phe-Cys]-Asp-His-Phe-Arg-Asp-NH2 (3BP-3214) of the following formula 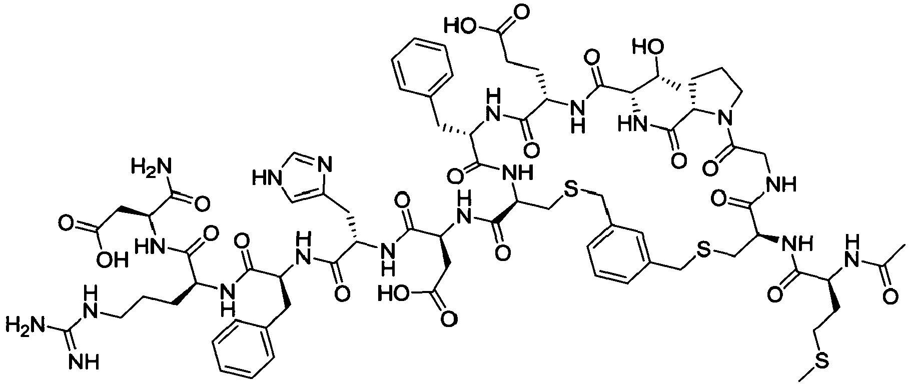
   compound Hex-[Cys(3MeBn)-Pro-Pro-Thr-Glu-Phe-Cys]-Asp-His-Nmf-Arg-Ttds-Lys(DOTA)-NH2 (3BP-3275) of the following formula 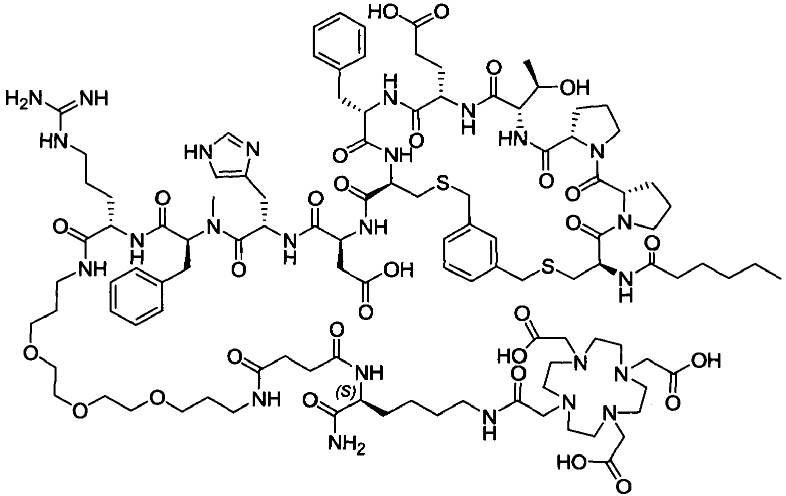
   compound Hex-[Cys(3MeBn)-Pro-Pro-Thr-Glu-Phe-Cys]-Asp-His-phe-Arg-Ttds-Lys(DOTA)-NH2 (3BP-3276) of the following formula 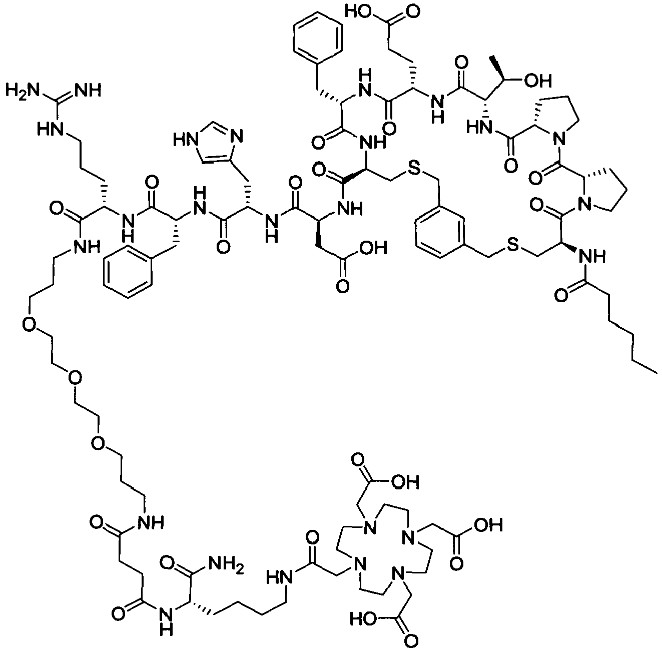
   compound Hex-[Cys(3MeBn)-Pro-Pro-Thr-Glu-Phe-Cys]-Asp-His-Ppa-arg-Ttds-Lys(DOTA)-NH2 (3BP-3277) of the following formula 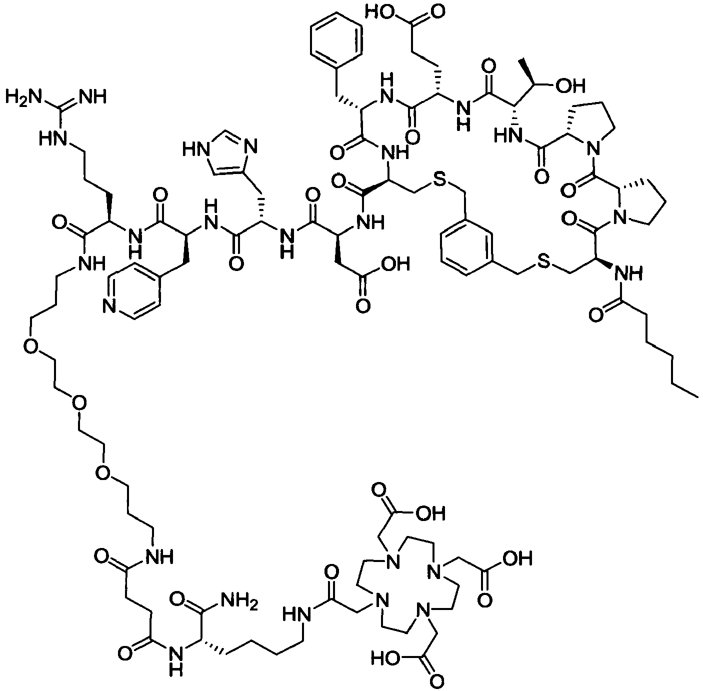
   compound Hex-[Cys(3MeBn)-Pro-Pro-Thr-Glu-Phe-Cys]-Asp-NH2 (3BP-3288) of the following formula 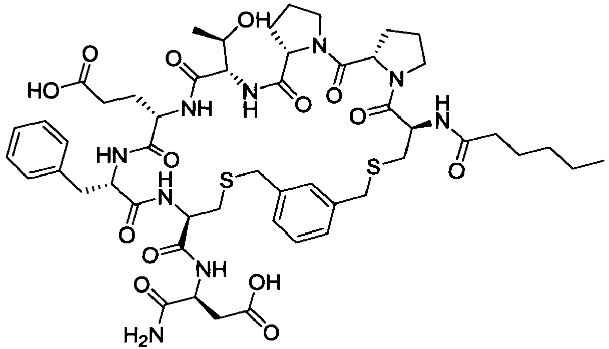
   compound Hex-[Cys(3MeBn)-Pro-Pro-Thr-Glu-Phe-Cys]-Asp-Arg-NH2 (3BP-3299) of the following formula 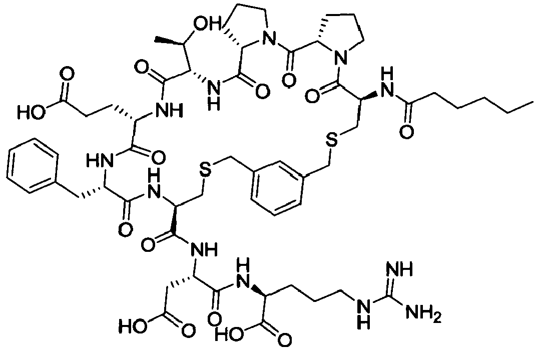
   compound Hex-[Cys(3MeBn)-Pro-Pro-Thr-Glu-Phe-Cys]-Asp-Gab-Arg-NH2 (3BP-3300) of the following formula 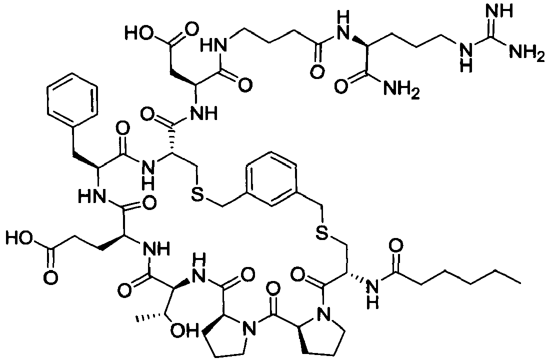
   compound Hex-[Cys(3MeBn)-Pro-Pro-Thr-Glu-Phe-Cys]-Asp-Pamb-Arg-NH2 (3BP-3301) of the following formula 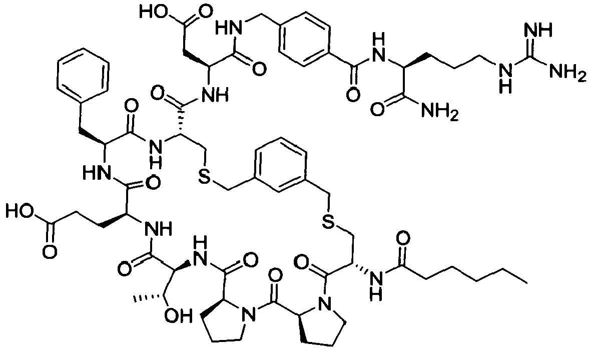
   ompound Hex-[Cys(3MeBn)-Pro-Pro-Thr-Glu-Phe-Cys]-Asp-Cmp-Arg-NH2 (3BP-3302) of the following formula 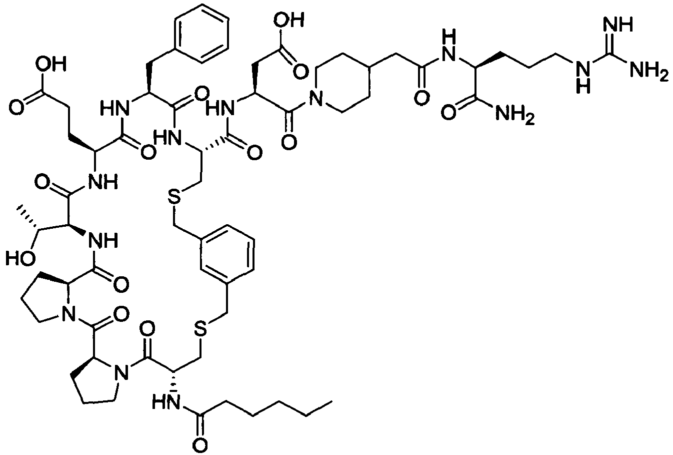
   compound Hex-[Cys(3MeBn)-Pro-Pro-Thr-Glu-Phe-Cys]-Pamb-Arg-NH2 (3BP-3303) of the following formula 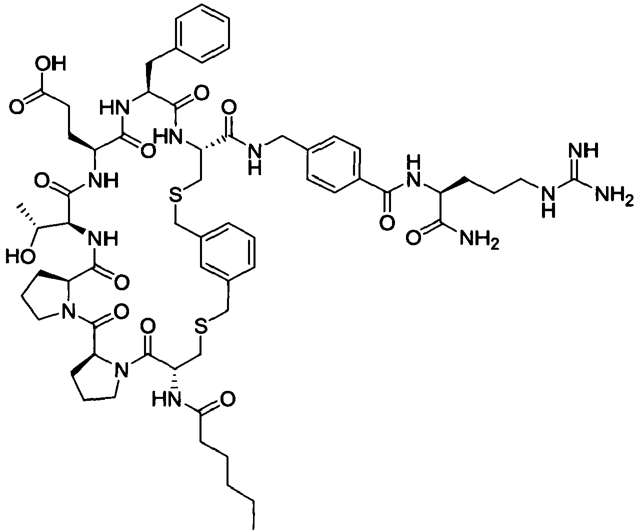
   compound DOTA-Ttds-Nle-[Cys(3MeBn)-Pro-Pro-Thr-Glu-Phe-Cys]-NH2 (3BP-3319) of the following formula 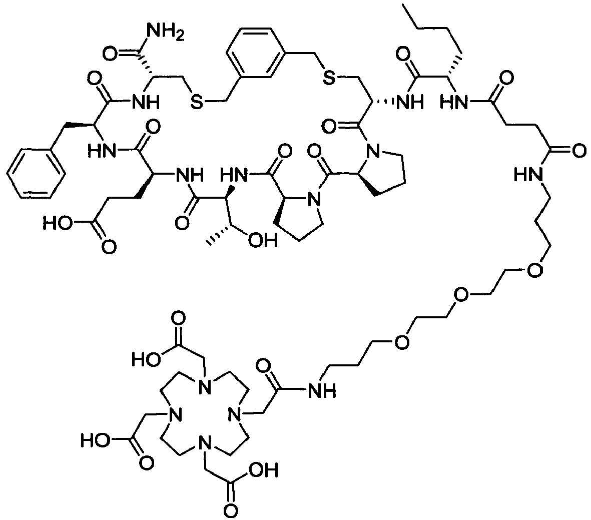
   compound DOTA-Ttds-Nle-[Cys(3MeBn)-Pro-Pro-Thr-Glu-Phe-Cys]-Asp-NH2 (3BP-3320) of the following formula 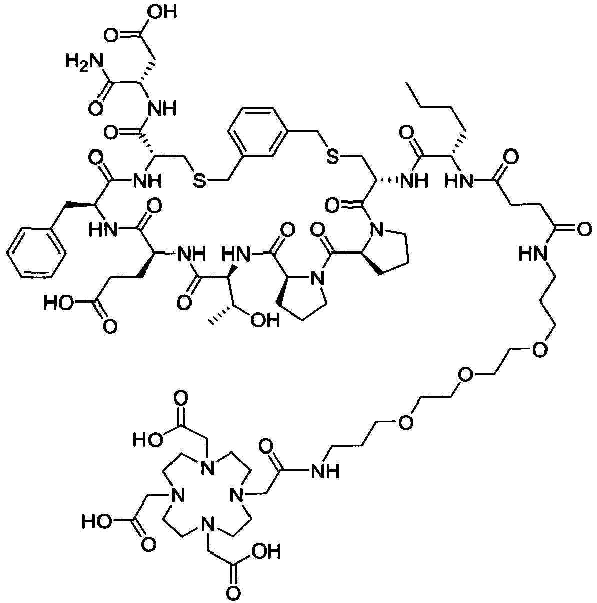
   compound DOTA-Ttds-Nle-[Cys(3MeBn)-Pro-Pro-Thr-Glu-Phe-Cys]-Asp-Pamb-Arg-NH2 (3BP-3321) of the following formula 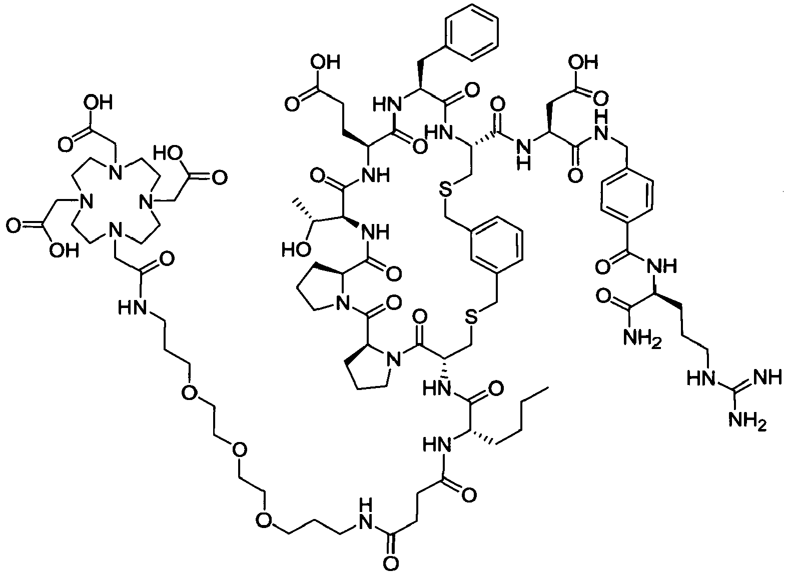
   compound Hex-[Cys(3MeBn)-Pro-Pro-Thr-Glu-Phe-Cys]-Asp-Mamb-Arg-NH2 (3BP-3324) of the following formula 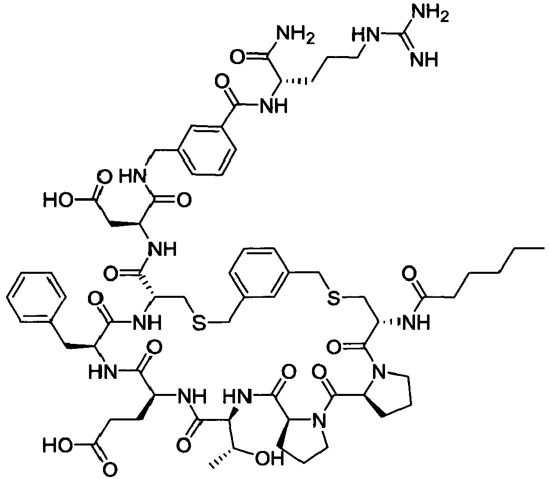
   compound Hex-[Cys(3MeBn)-Pro-Pro-Thr-Gln-Phe-Cys]-NH2 (3BP-3349) of the following formula 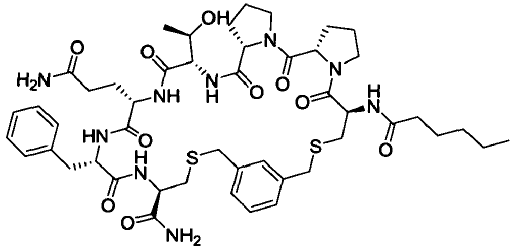
   compound Hex-[Cys(3MeBn)-Pro-Pro-Thr-Glu-Phe-Cys]-Bal-OH (3BP-3371) of the following formula 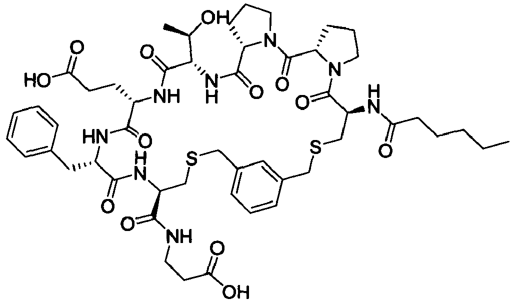
   compound Hex-[Cys(3MeBn)-Pro-Pro-Thr-Gln-Phe-Cys]-Asp-Ttds-Lys(DOTA)-NH2 (3BP-3395) of the following formula 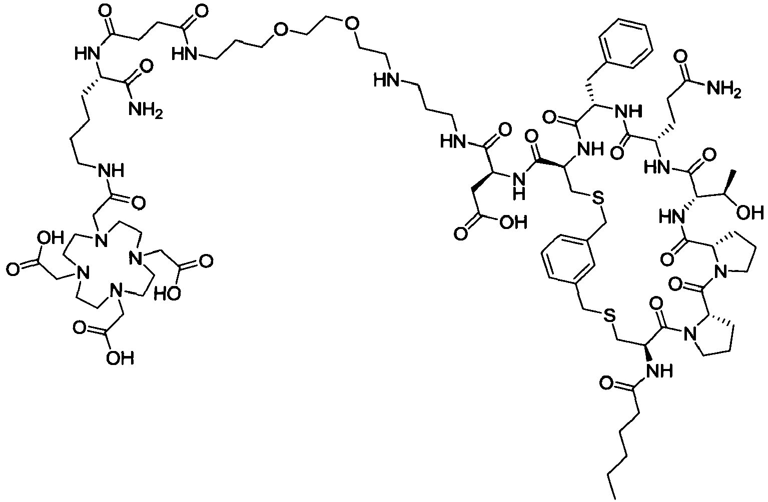
   compound Hex-[Cys(3MeBn)-Pro-Pro-Thr-Glu-Phe-Cys]-Asp-Ttds-Lys(DOTA)-NH2 (3BP-3396) of the following formula 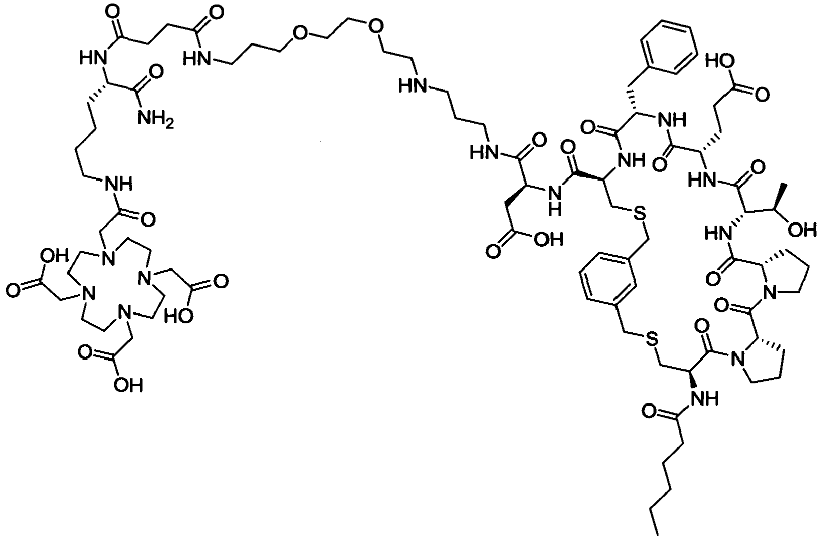
   compound Hex-[Cys(3MeBn)-Pro-Pro-Thr-Gln-Phe-Cys]-Bhk(DOTA)-OH (3BP-3397) of the following formula 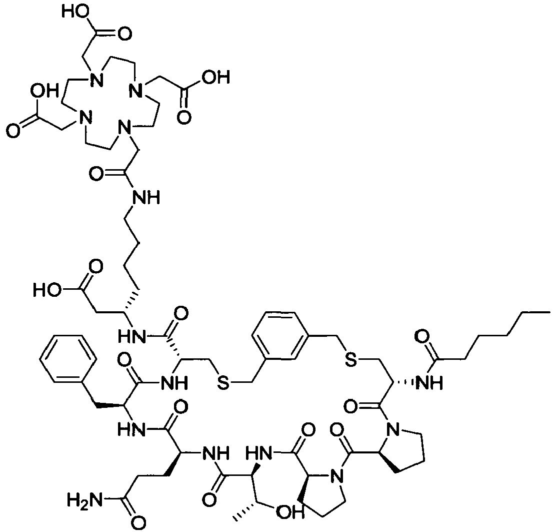
   compound DOTA-Ttds-Nle-[Cys(3MeBn)-Pro-Pro-Thr-Gln-Phe-Cys]-Bal-OH (3BP-3398) of the following formula 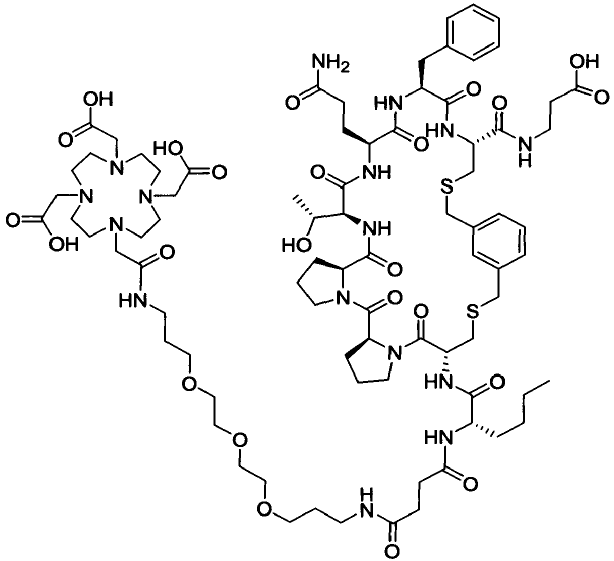
   compound DOTA-Ttds-Nle-[Cys(3MeBn)-Pro-Pro-Thr-Gln-Phe-Cys]-Asp-NH2 (3BP-3401) of the following formula 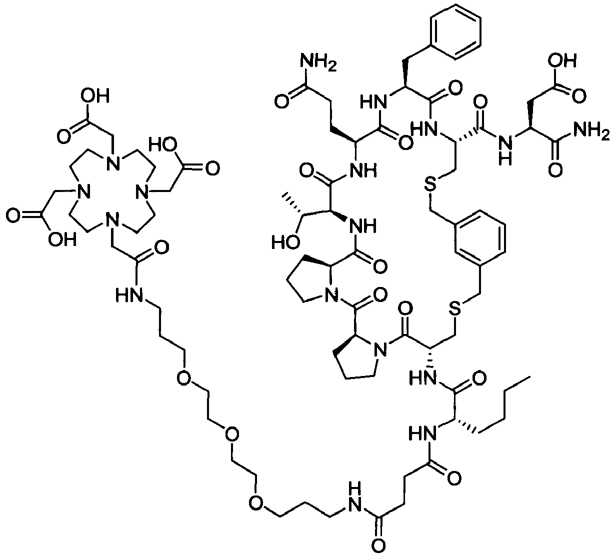
   compound Hex-[Cys(3MeBn)-Pro-Pro-Thr-Gln-Phe-Cys]-Asp-Ape(DOTA) (3BP-3403) of the following formula 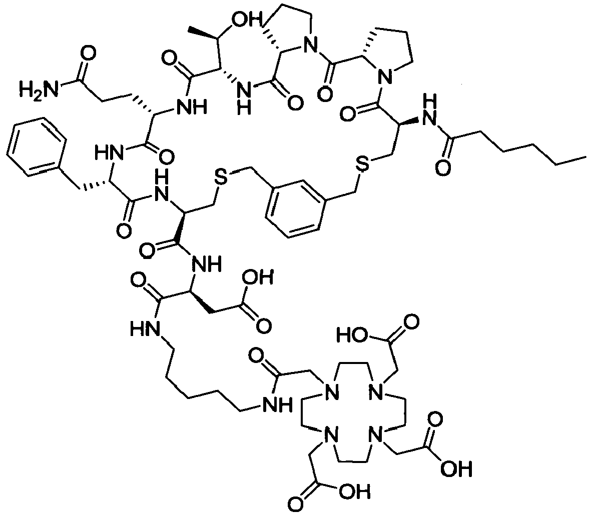
   compound Hex-[Cys(3MeBn)-Pro-Pro-Thr-Gln-Phe-Cys]-Asp-Ttds-Ape(DOTA) (3BP-3404) of the following formula 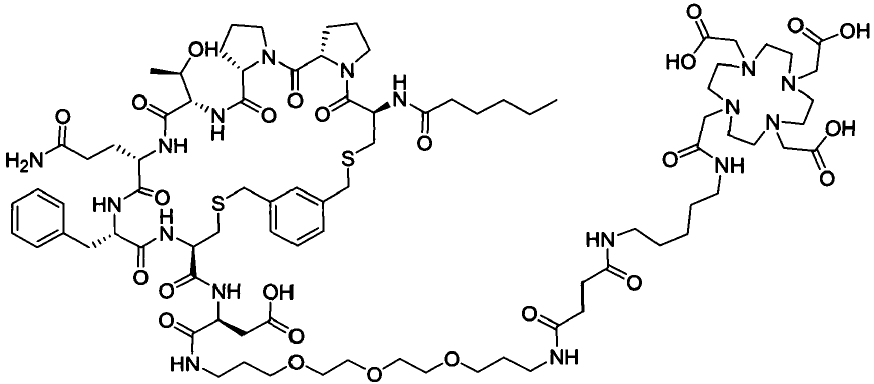
   compound Hex-[Cys(tMeBn(DOTA-PP))-Pro-Pro-Thr-Gln-Phe-Cys]-Asp-NH2 (3BP-3407) of the following formula 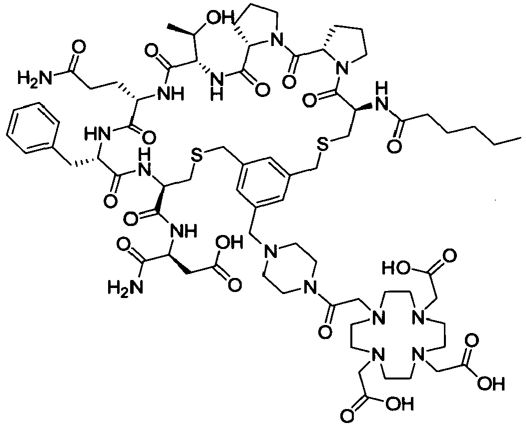
   compound Hex-[Cys(3MeBn)-Pro-Pro-Thr-Gln-Otf-Cys]-NH2 (3BP-3409) of the following formula 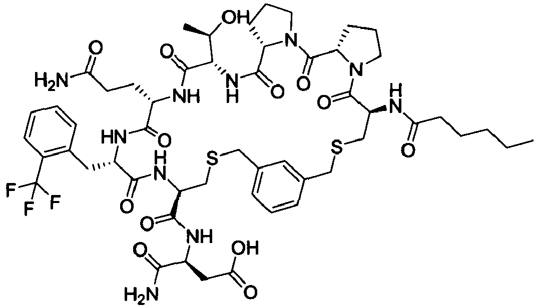
   compound PentylNH-urea-[Cys(3MeBn)-Pro-Pro-Thr-Gln-Phe-Cys]-Asp-NH2 (3BP-3425) of the following formula 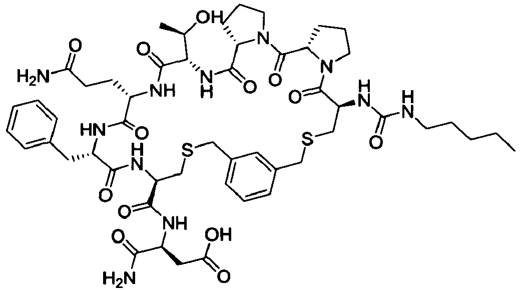
   compound Hex-[Cys(tMeBn(DOTA-AET))-Pro-Pro-Thr-Gln-Phe-Cys]-Asp-NH2 (3BP-3426) of the following formula 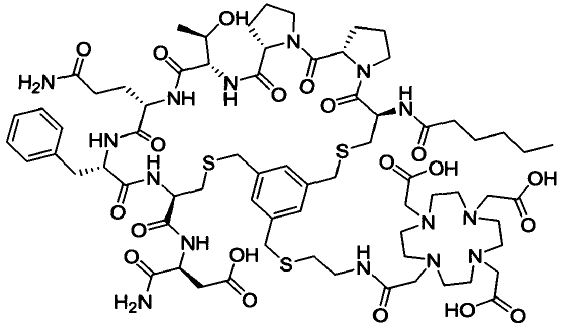
   compound Hex-[Cys(3MeBn)-Pro-Pro-Thr-Gln-Phe-Cys]-Asp-NH2 (3BP-3476) of the following formula 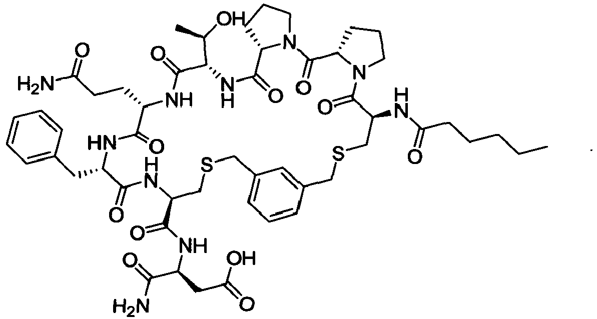
   compound Hex-[Cys(3MeBn)-Pro-Pro-Thr-Gln-Phe-Cys]-Bhk(DOTA-Ttds)-OH (3BP-3489) of the following formula 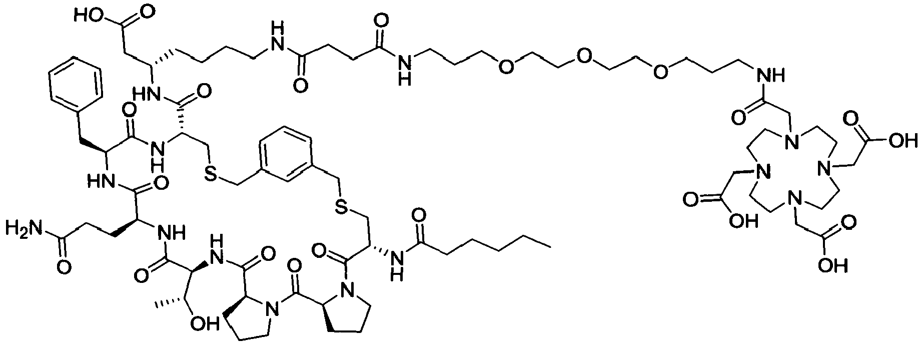
   compound Pentyl-SO2-[Cys(3MeBn)-Pro-Pro-Thr-Gln-Phe-Cys]-Asp-NH2 (3BP-3514) of the following formula 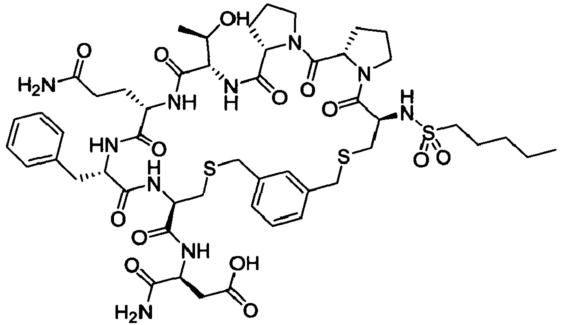
   compound Hex-[Cys(2Lut)-Pro-Pro-Thr-Gln-Phe-Cys]-Asp-NH2 (3BP-3518) of the following formula 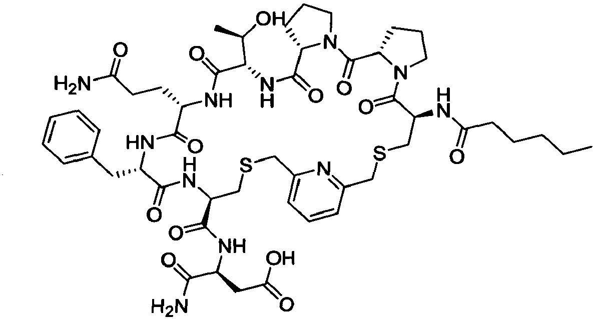
   compound Hex-[Cys(3Lut)-Pro-Pro-Thr-Gln-Phe-Cys]-Asp-NH2 (3BP-3519) of the following formula 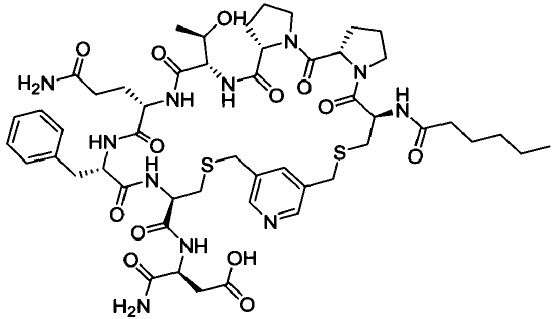
   compound Hex-[Cys(tMeBn(DOTA-AET))-Pro-Pro-Thr-Gln-Phe-Cys]-OH (3BP-3554) of the following formula 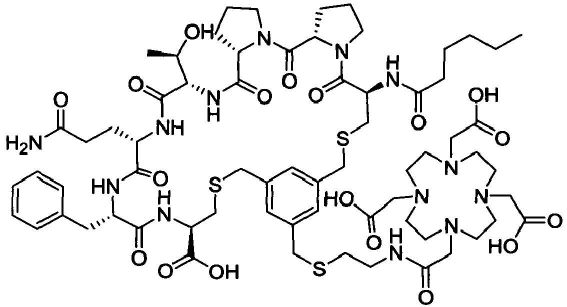
   compound Hex-[Cys(tMeBn(DOTA-PP))-Pro-Pro-Thr-Gln-Phe-Cys]-OH (3BP-3555) of the following formula 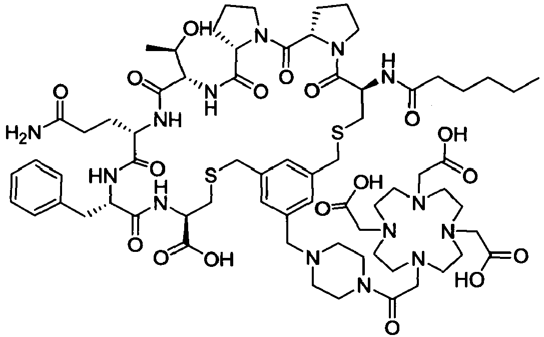
   compound Hex-[Cys(tMeBn(InDOTA-PP))-Pro-Pro-Thr-Gln-Phe-Cys]-Asp-NH2 (3BP-3590) of the following formula 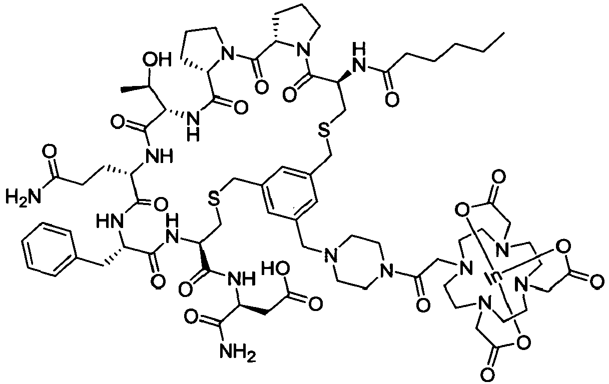
   compound Hex-[Cys(tMeBn(LuDOTA-PP))-Pro-Pro-Thr-Gln-Phe-Cys]-Asp-NH2 (3BP-3591) of the following formula 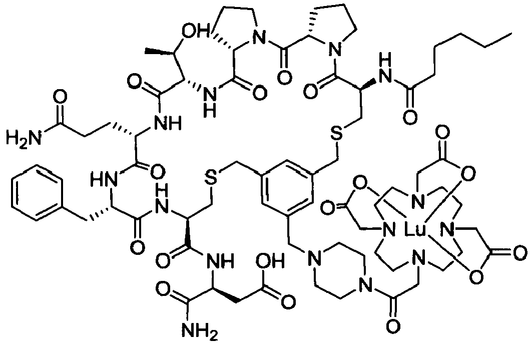
   compound Hex-[Cys(tMeBn(GaDOTA-PP))-Pro-Pro-Thr-Gln-Phe-Cys]-Asp-NH2 (3BP-3592) of the following formula 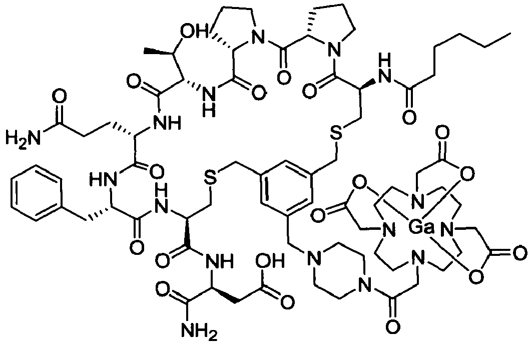
   compound Hex-[Cys(tMeBn(InDOTA-AET))-Pro-Pro-Thr-Gln-Phe-Cys]-OH (3BP-3623) of the following formula 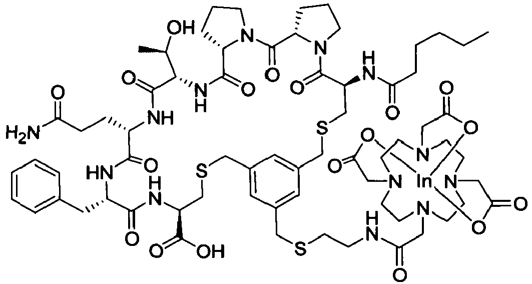
   compound Hex-[Cys(tMeBn(LuDOTA-AET))-Pro-Pro-Thr-Gln-Phe-Cys]-OH (3BP-3624) of the following formula 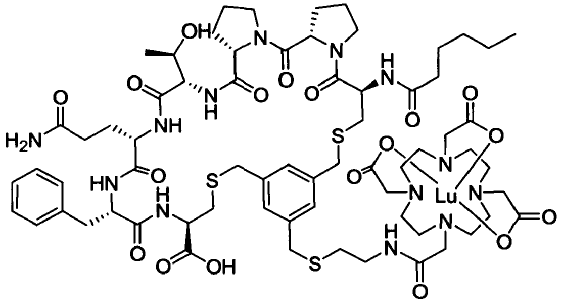
   compound Hex-[Cys(tMeBn(DOTA-AET))-Pro-Pro-Thr-Gln-1Ni-Cys]-OH (3BP-3650) of the following formula 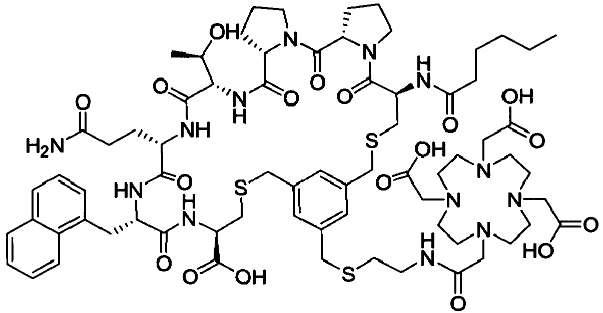
   compound Hex-[Cys(tMeBn(DOTA-AET))-Pro-Pro-Thr-Gln-Phe-Cys]-Bal-OH (3BP-3651) of the following formula 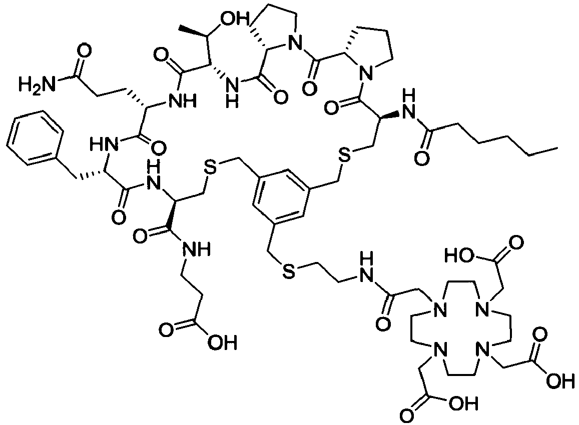
   compound Hex-[Cys(tMeBn(DOTA-AET))-Pro-Pro-Thr-Gln-Phe-Cys]-NH2 (3BP-3652) of the following formula 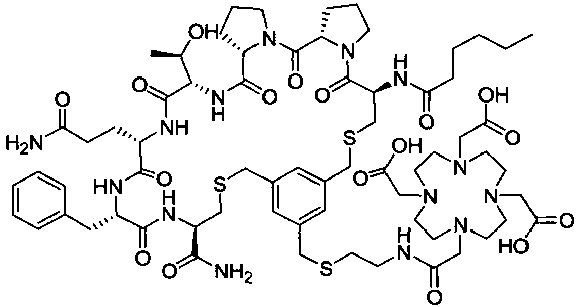
   compound Hex-[Cys(tMeBn(DOTA-AET))-Pro-Pro-Thr-Glu-Phe-Cys]-NH2 (3BP-3653) of the following formula 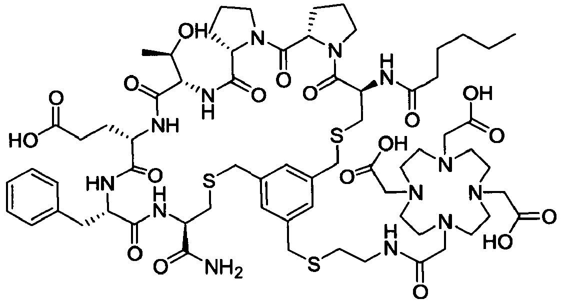
   compound Hex-[Cys(tMeBn(DOTA-AET))-Pro-Pro-Thr-Gln-Phe-AET] (3BP-3654) of the following formula 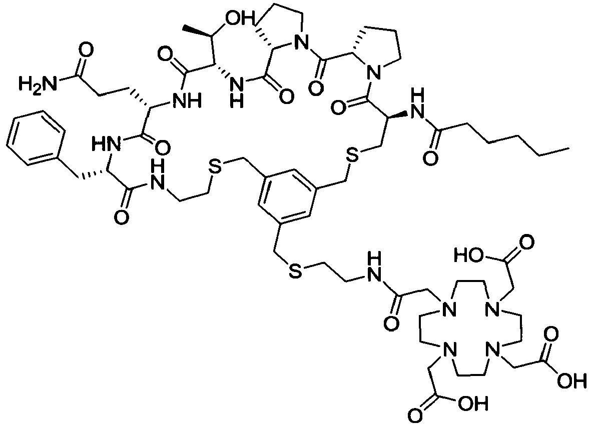
   compound Hex-[Cys(tMeBn(DOTA-AET))-Pro-Pro-Thr-Gln-Phe-Cys]-Gly-OH (3BP-3656) of the following formula 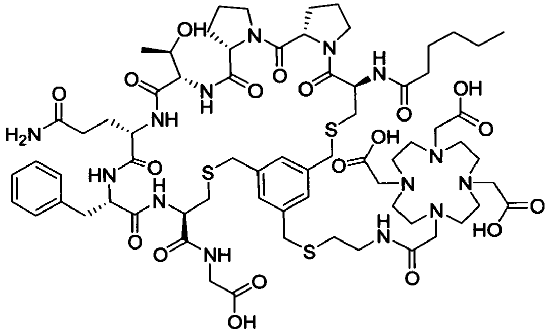
   compound Hex-[Cys(tMeBn(DOTA-AET))-Pro-Pro-Thr-Gln-Phe-Cys]-Gab-OH (3BP-3657) of the following formula 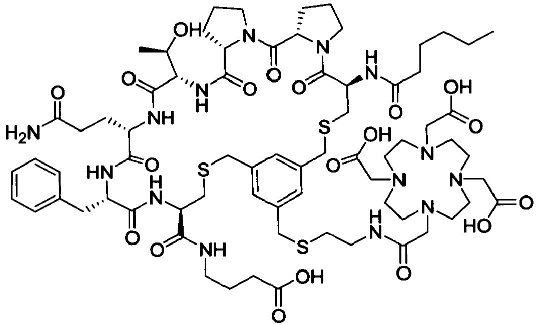
   compound Hex-[Cys(tMeBn(DOTA-AET))-Pro-Pro-Thr-Gln-Phe-Cys]-Ser-OH (3BP-3658) of the following formula 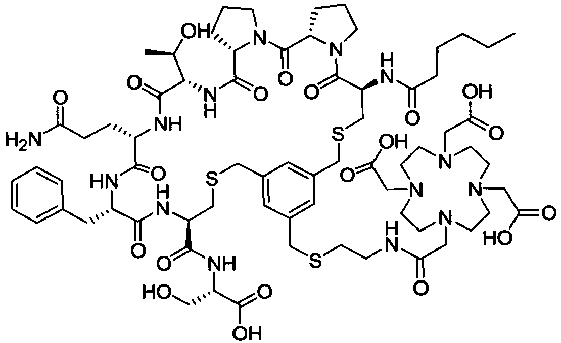
   compound Hex-[Cys(tMeBn(DOTA-AET))-Pro-Pro-Thr-Gln-Phe-Cys]-Nmg-OH (3BP-3659) of the following formula 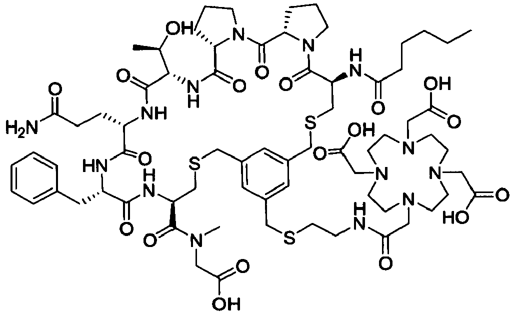
   compound Hex-[Cys(tMeBn(DOTA-AET))-Pro-Pro-Thr-Gln-Phe-Cys]-Bhf-OH (3BP-3660) of the following formula 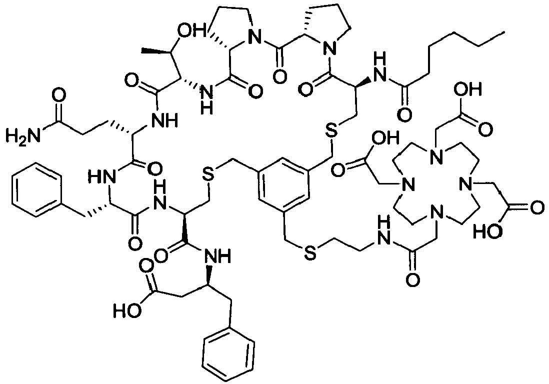
   compound Hex-[Cys(tMeBn(EuDOTA-PP))-Pro-Pro-Thr-Gln-Phe-Cys]-Asp-NH2 (3BP-3661) of the following formula 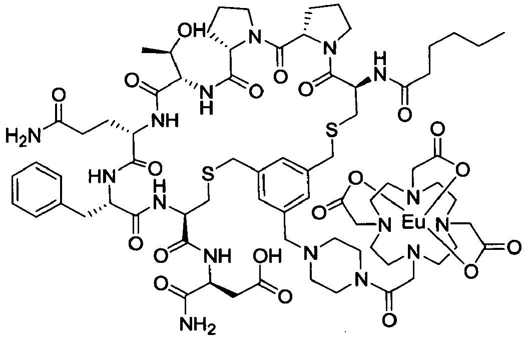
   compound Hex-[Cys(tMeBn(EuDOTA-AET))-Pro-Pro-Thr-Gln-Phe-Cys]-OH (3BP-3662) of the following formula 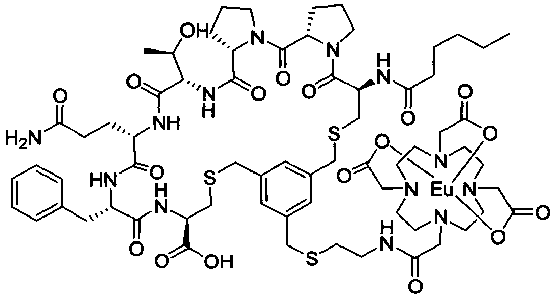
   compound Hex-[Cys(tMeBn(DOTA-AET))-Pro-Pro-Thr-Gln-Mpa-Cys]-OH (3BP-3664) of the following formula 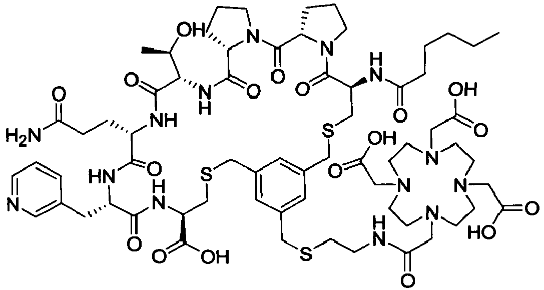
   compound Hex-[Cys(tMeBn(DOTA-AET))-Pro-Pro-Thr-Gln-Phe-Cys]-Asp-OH (3BP-3665) of the following formula 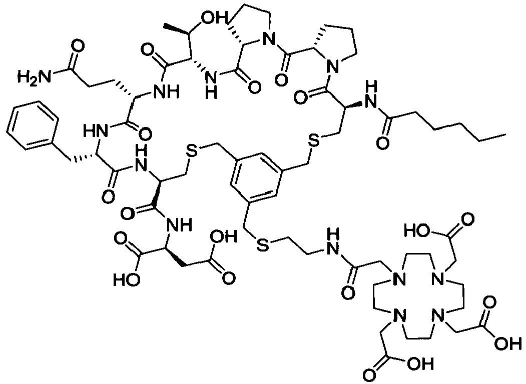
   compound Hex-[Cys(tMeBn(DOTA-AET))-Nmg-Pro-Thr-Gln-Phe-Cys]-OH (3BP-3678) of the following formula 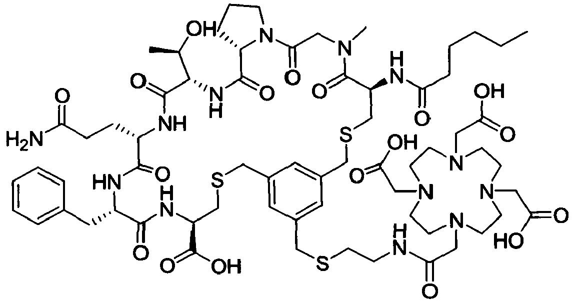
   compound Hex-[Cys(tMeBn(DOTA-AET))-Pro-Hyp-Thr-Gln-Phe-Cys]-OH (3BP-3679) of the following formula 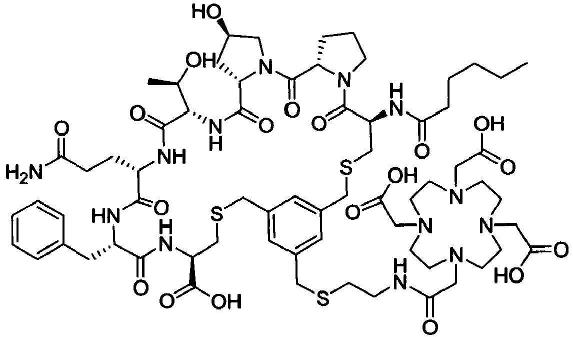
   compound Hex-[Cys(tMeBn(DOTA-AET))-Pro-Pro-Thr-Gln-Otf-Cys]-OH (3BP-3680) of the following formula 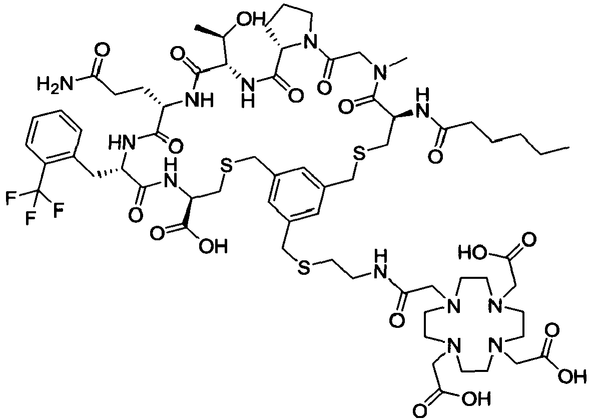
   compound Hex-[Cys(tMeBn(DOTA-AET))-Pro-Pro-Thr-Gln-Phe-Cys]-asp-NH2 (3BP-3681) of the following formula 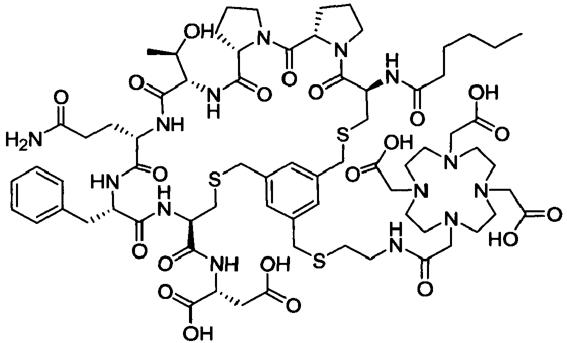
   compound Pentyl-SO2-[Cys(tMeBn(DOTA-AET))-Pro-Pro-Thr-Gln-Phe-Cys]-OH (3BP-3690) of the following formula 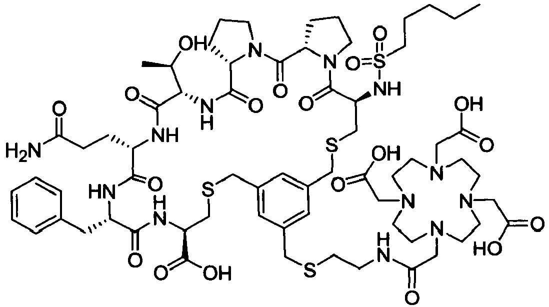
   compound Pentyl-SO2-[Cys(tMeBn(DOTA-AET))-Pro-Pro-Thr-Glu-Phe-Cys]-OH (3BP-3691) of the following formula 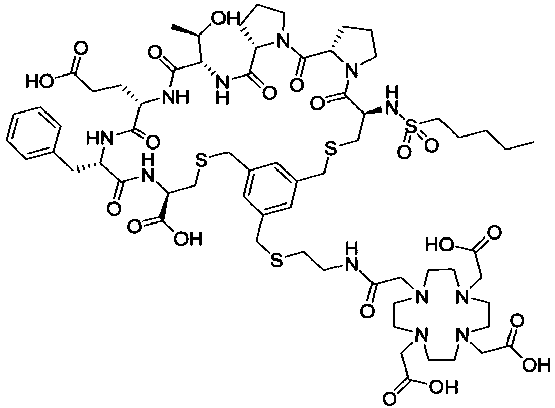
   compound Pentyl-SO2-[Cys(tMeBn(DOTA-PP))-Pro-Pro-Thr-Gln-Phe-Cys]-Asp-NH2 (3BP-3692) of the following formula 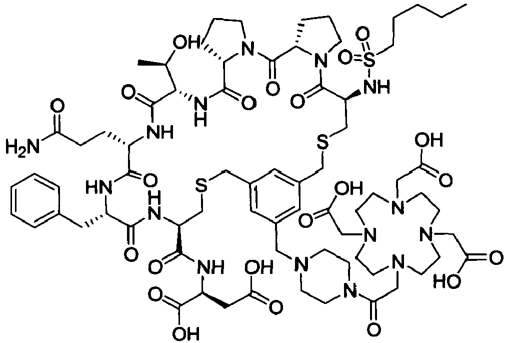
   compound Hex-[Cys(tMeBn(InDOTA-AET))-Pro-Pro-Thr-Gln-Phe-Cys]-NH2 (3BP-3712) of the following formula 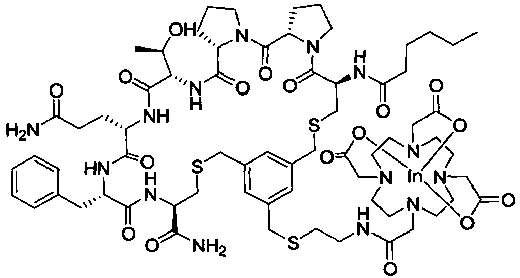
   compound Hex-[Cys(tMeBn(InDOTA-AET))-Pro-Pro-Thr-Gln-Phe-AET] (3BP-3713) of the following formula 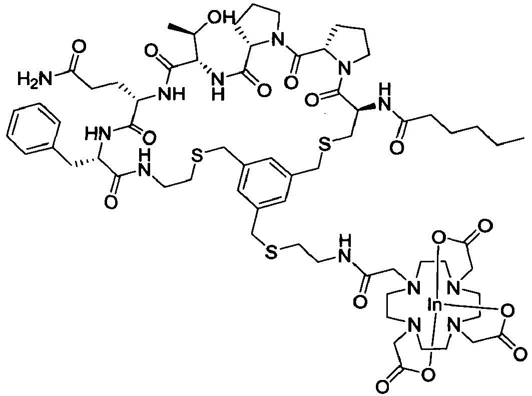
   compound Hex-[Cys(tMeBn(InDOTA-AET))-Pro-Pro-Thr-Gln-Phe-Cys]-Gly-OH (3BP-3714) of the following formula 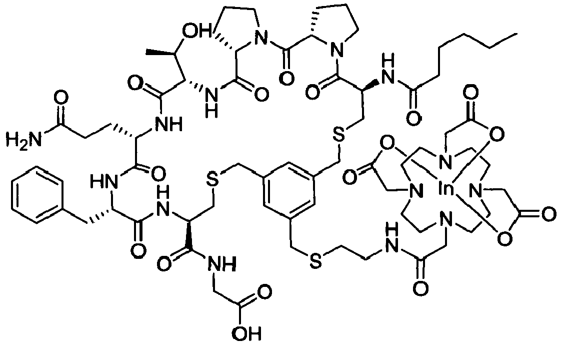
   compound Hex-[Cys(tMeBn(InDOTA-AET))-Pro-Pro-Thr-Gln-Phe-Cys]-Nmg-OH (3BP-3715) of the following formula 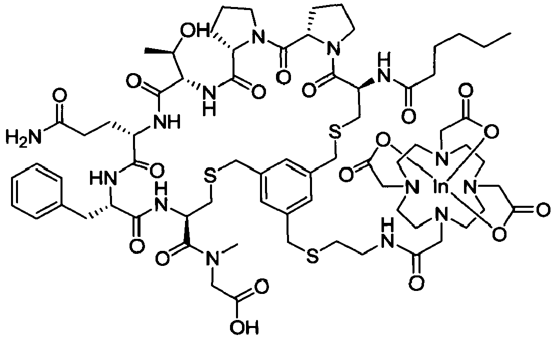
   compound Hex-[Cys(tMeBn(InDOTA-AET))-Nmg-Pro-Thr-Gln-Phe-Cys]-OH (3BP-3716) of the following formula 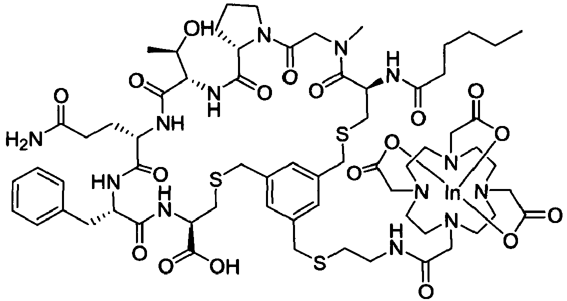
   compound Pentyl-SO2-[Cys(tMeBn(InDOTA-PP))-Pro-Pro-Thr-Gln-Phe-Cys]-Asp-NH2 (3BP-3717) of the following formula 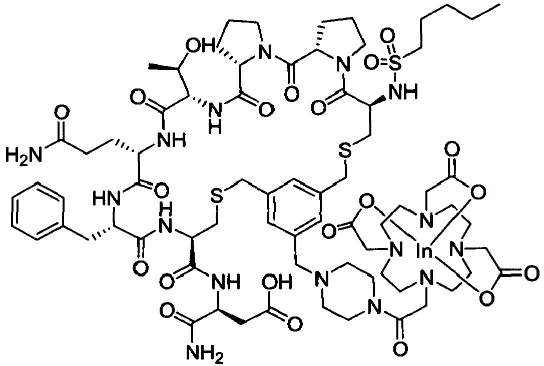
   compound Hex-[Cys(tMeBn(DOTA-AET))-Pro-Pro-Thr-Gln-Phe-Cys]-Bal-NH2 (3BP-3736) of the following formula 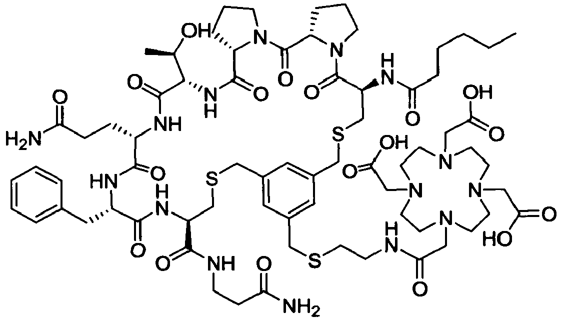
   compound Hex-[Cys(tMeBn(DOTA-AET))-Pro-Pro-Thr-Gln-Phe-Cys]-Nmg-NH2 (3BP-3737) of the following formula 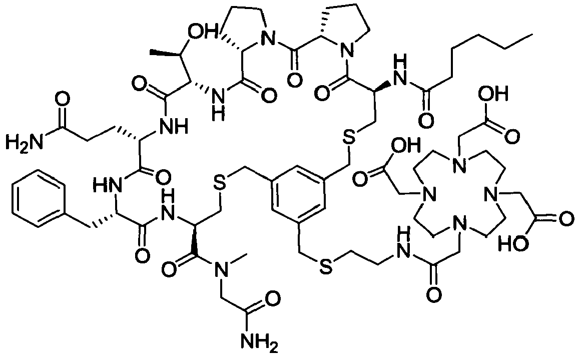
   compound Hex-[Cys(tMeBn(DOTA-AET))-Nmg-Pro-Thr-Gln-Phe-Cys]-NH2 (3BP-3744) of the following formula 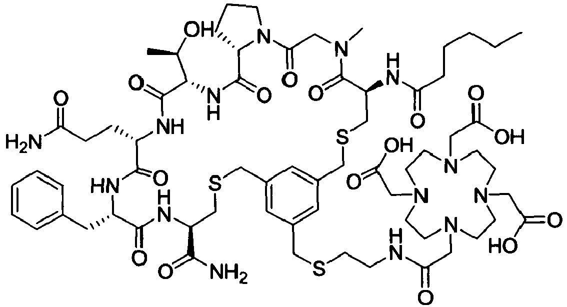
   compound Hex-[Cys(tMeBn(DOTA-AET))-Pro-Pro-Thr-Gln-Phe-Cysol] (3BP-3767) of the following formula 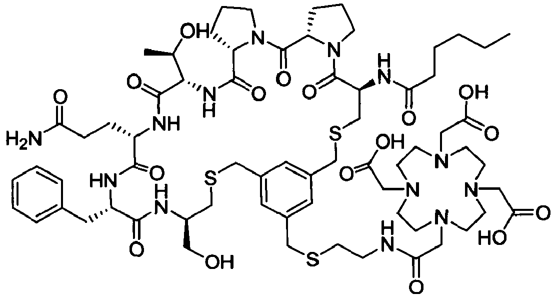
   compound Hex-[Cys(tMeBn(InDOTA-PP))-Pro-Pro-Thr-Gln-Phe-Cys]-OH (3BP-3770) of the following formula 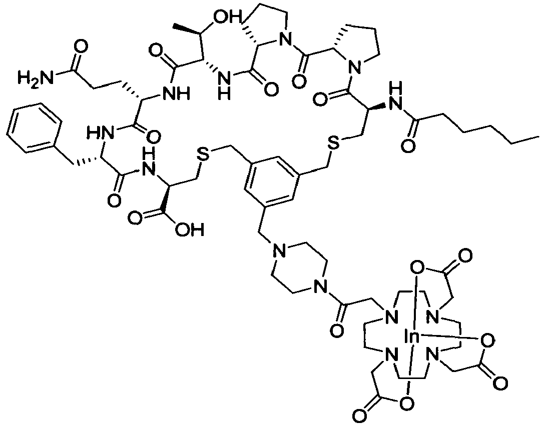
   and compound Hex-[Cys(tMeBn(DOTA-PP))-Nmg-Pro-Thr-Gln-Phe-Cys]-OH (3BP-3771) of the following formula 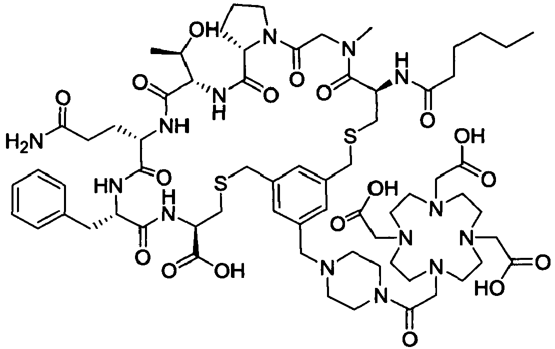
Embodiment 62. The compound of any one of Embodiments 1 to 61, wherein any S atom which can be oxidized, preferably S atoms of thioether groups, is present as -S-, -S(O)- or-S(O₂)- or a mixture thereof.
Embodiment 63. The compound of any one of Embodiments 1 to 62, wherein the compound is capable of binding to fibroblast activation protein (FAP).
Embodiment 64. The compound of any one of Embodiments 1 to 63, wherein the compound comprises a diagnostically active nuclide or a therapeutically active nuclide.
Embodiment 65. The compound of Embodiment 64, wherein the diagnostically active nuclide is a diagnostically active radionuclide.
Embodiment 66. The compound of Embodiment 65, wherein the diagnostically active radionuclide is selected from the group consisting of ⁴³Sc, ⁴⁴SC, ⁵¹Mn, ⁵²Mn, ⁶⁴Cu, ⁶⁷Ga, ⁶⁸Ga, ⁸⁶Y,⁸⁹Zr, ^{94m}TC, ^{99m}Tc, ¹¹¹In, ¹⁵²Tb, ¹⁵⁵Tb, ²⁰¹Tl, ²⁰³Pb, ¹⁸F, ⁷⁶Br , ⁷⁷Br, ¹²³I, ¹²⁴I, ¹²⁵I, preferably ⁴³Sc, ⁴⁴Sc, ⁶⁴Cu, ⁶⁷Ga, ⁶⁸Ga, ⁸⁶Y, ⁸⁹Zr, ^{99m}Tc, ¹¹¹In, ¹⁵²Tb, ¹⁵⁵Tb, ²⁰³Pb, ¹⁸F, ⁷⁶Br, ⁷⁷Br, ¹²³I, ¹²⁴I, ¹²⁵I and most preferably ⁶⁴Cu, ⁶⁸Ga, ⁸⁹Zr, ^{99m}Tc, ¹¹¹In, ¹⁸F, ¹²³I, and ¹²⁴I.
Embodiment 67. The compound of Embodiment 64, wherein the therapeutically active nuclide is a therapeutically active radionuclide.
Embodiment 68. The compound of Embodiment 67, wherein the therapeutically active radionuclide is selected from the group consisting of ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu , ¹⁸⁶Re, ¹⁸⁸Re, ²¹²Pb , ²¹³Bi, ²²³Ra, ²²⁵Ac, ²²⁶Th, ²²⁷Th, ¹³¹I, ²¹¹At, preferably ⁴⁷Sc, ⁶⁷Cu, ⁹⁰Y, ¹⁷⁷Lu, ¹⁸⁸Re, ²¹²Pb , ²¹³Bi, ²²⁵Ac, ²²⁷Th, ¹³¹I, ²¹¹At and most preferably ⁹⁰Y, ¹⁷⁷Lu, ²²⁵Ac, ²²⁷Th, ¹³¹I and ²¹¹At.
Embodiment 69. The compound of any one of Embodiments 1 to 68, wherein the compound interacts with a fibroblast activation protein (FAP), preferably with human FAP having an amino acid sequence of SEQ ID NO: 1 or a homolog thereof, wherein the amino acid sequence of the homolog has an identity of at least 85% to the amino acid sequence of SEQ ID NO: 1.
Embodiment 70. The compound of Embodiment 69, wherein the compound is an inhibitor of the fibroblast activation protein (FAP).
Embodiment 71. The compound of any one of Embodiments 1 to 70, wherein the compound has a pIC₅₀ value for human FAP of SEQ ID NO: 1 of ≥ 6.0, preferably of ≥ 7.0, and most preferably of ≥ 8.0.
Embodiment 72. The compound of any one of Embodiments 1 to 71, for use in a method for the diagnosis of a disease.
Embodiment 73. The compound for use of Embodiment 72, wherein the disease is a disease involving fibroblast activation protein (FAP), preferably upregulated expression of fibroblast activation protein (FAP).
Embodiment 74. The compound for use of any one of Embodiments 72 to 73, wherein the disease involves cells showing upregulated expression of fibroblast activation protein (FAP), preferably diseased tissue containing cells showing upregulated expression of fibroblast activation protein (FAP), more preferably disease involving tumor associated fibroblasts.
Embodiment 75. The compound for use of any one of Embodiments 72 to 74, wherein the disease is a neoplasm, preferably a cancer or tumor.
Embodiment 76. The compound for use of Embodiment 75, wherein the neoplasm, cancer, and tumor are each and individually selected from the group comprising a solid tumor, an epithelial tumor, bladder cancer, breast cancer, cervical cancer, colorectal cancer, cholangiocarcinoma, endometrial cancer, esophageal cancer, gastric cancer, gastrointestinal stromal tumors, head and neck cancer, liver cancer, lung cancer, melanoma, mesothelioma, neuroendocrine tumors and carcinomas, ovarian cancer, pancreatic cancer, prostate cancer, renal cell carcinoma, salivary carcinoma, sarcoma, squamous cell carcinoma, and thyroid cancer.
Embodiment 77. The compound for use of Embodiment 76, wherein the neoplasm, cancer, and tumor are each and individually selected from the group comprising breast cancer, colorectal cancer, cholangiocarcinoma, head and neck cancer, lung cancer, mesothelioma, neuroendocrine tumors and carcinomas, ovarian cancer, pancreatic cancer, prostate cancer, sarcoma, and squamous cell carcinoma.
Embodiment 78. The compound for use of any one of Embodiments 72 to 74, wherein the disease is selected from the groups comprising inflammatory disease, cardiovascular disease, autoimmune disease, and fibrotic disease.
Embodiment 79. The compound for use of Embodiment 78, wherein the disease is an inflammatory disease.
Embodiment 80. The compound for use of Embodiment 79, wherein the disease is atherosclerosis, arthritis, or rheumatoid arthritis.
Embodiment 81. The compound for use of Embodiment 78, wherein the disease is a cardiovascular disease.
Embodiment 82. The compound for use of Embodiment 81, wherein the disease is a cardiovascular disease involving atherosclerotic plaques.
Embodiment 83. The compound for use of Embodiment 82, wherein the disease is an atherosclerotic pathology caused by rupture of plaques, acute coronary syndrome, myocardial infarction, thrombosis, or vessel occlusion.
Embodiment 84. The compound for use of Embodiment 78, wherein the disease is a fibrotic disease.
Embodiment 85. The compound for use of Embodiment 84, wherein the disease is selected form the group comprising idiopathic pulmonary fibrosis, Crohn's disease, and liver fibrosis.
Embodiment 86. The compound for use of any one of Embodiments 72 to 85, wherein the compound comprises a diagnostically active nuclide, preferably a diagnostically active radionuclide.
Embodiment 87. The compound for use of Embodiment 86, wherein the diagnostically active nuclide is selected from the group comprising ⁴³Sc, ⁴⁴SC, ⁵¹Mn, ⁵²Mn, ⁶⁴Cu, ⁶⁷Ga, ⁶⁸Ga, ⁸⁶Y, ⁸⁹Zr, ^{94m}TC, ^{99m}Tc, ¹¹¹In , ¹⁵²Tb, ¹⁵⁵Tb, ²⁰¹Tl, ²⁰³Pb, ¹⁸F, ⁷⁶Br, ⁷⁷Br, ¹²³I, ¹²⁴I, ¹²⁵I, preferably ⁴³Sc, ⁴⁴Sc, ⁶⁴Cu, ⁶⁷Ga, ⁶⁸Ga, ⁸⁶Y, ⁸⁹Zr, ^{99m}Tc, ¹¹¹In, ¹⁵²Tb, ¹⁵⁵Tb, ²⁰³Pb, ¹⁸F, ⁷⁶Br, ⁷⁷Br, ¹²³I, ¹²⁴I, ¹²⁵I, and more preferably ⁶⁴Cu, ⁶⁸Ga, ⁸⁹Zr, ^{99m}Tc, ¹¹¹In, ¹⁸F, ¹²³I, and ¹²⁴I.
Embodiment 88. The compound for use of any one of Embodiments 72 to 87, wherein the method for the diagnosis is an imaging method.
Embodiment 89. The compound for use of Embodiment 88, wherein the imaging method is selected from the group consisting of scintigraphy, Single Photon Emission Computed Tomography (SPECT) and Positron Emission Tomography (PET).
Embodiment 90. The compound for use of any one of Embodiments 72 to 89, wherein the method comprises the administration of a diagnostically effective amount of the compound to a subject, preferably to a mammal, wherein the mammal is selected from the group comprising man, companion animals, pets, and livestock, more preferably the subject is selected from the group comprising man, dog, cat, horse, and cow, and most preferably the subject is a human being.
Embodiment 91. The compound of any one of Embodiments 1 to 71, for use in a method for the treatment of a disease.
Embodiment 92. The compound for use of Embodiment 91, wherein the disease is a disease involving fibroblast activation protein (FAP), preferably upregulated expression of fibroblast activation protein (FAP).
Embodiment 93. The compound for use of any one of Embodiments 91 to 92, wherein the disease involves cells showing upregulated expression of fibroblast activation protein (FAP), preferably diseased tissue containing cells showing upregulated expression of fibroblast activation protein (FAP), more preferably disease involving tumor associated fibroblasts.
Embodiment 94. The compound for use of any one of Embodiments 91 to 93, wherein the disease is a neoplasm, preferably a cancer or tumor.
Embodiment 95. The compound for use of Embodiment 94, wherein the neoplasm, cancer, and tumor are each and individually selected from the group comprising a solid tumor, an epithelial tumor, bladder cancer, breast cancer, cervical cancer, colorectal cancer, cholangiocarcinoma, endometrial cancer, esophageal cancer, gastric cancer, gastrointestinal stromal tumors, head and neck cancer, liver cancer, lung cancer, melanoma, mesothelioma, neuroendocrine tumors and carcinomas, ovarian cancer, pancreatic cancer, prostate cancer, renal cell carcinoma, salivary carcinoma, sarcoma, squamous cell carcinoma, and thyroid cancer.
Embodiment 96. The compound for use of Embodiment 95, wherein the neoplasm, cancer, and tumor are each and individually selected from the group comprising breast cancer, colorectal cancer, cholangiocarcinoma, head and neck cancer, lung cancer, mesothelioma, neuroendocrine tumors and carcinomas, ovarian cancer, pancreatic cancer, prostate cancer, sarcoma, and squamous cell carcinoma.
Embodiment 97. The compound for use of any one of Embodiments 91 to 93, wherein the disease is selected from the groups comprising inflammatory disease, cardiovascular disease, autoimmune disease, and fibrotic disease.
Embodiment 98. The compound for use of Embodiment 97, wherein the disease is an inflammatory disease.
Embodiment 99. The compound for use of Embodiment 98, wherein the disease is atherosclerosis, arthritis, or rheumatoid arthritis.
Embodiment 100. The compound for use of Embodiment 97, wherein the disease is a cardiovascular disease.
Embodiment 101. The compound for use of Embodiment 100, wherein the diseases is a cardiovascular disease involving atherosclerotic plaques.
Embodiment 102. The compound for use of Embodiment 101, wherein the diseases is an atherosclerotic pathology caused by rupture of plaques, acute coronary syndrome, myocardial infarction, thrombosis, or vessel occlusion.
Embodiment 103. The compound for use of Embodiment 97, wherein the disease is a fibrotic disease.
Embodiment 104. The compound for use of Embodiment 103, wherein the disease is selected form the group comprising idiopathic pulmonary fibrosis, Crohn's disease, and liver fibrosis.
Embodiment 105. The compound for use of any one of Embodiments 91 to 96, wherein the compound comprises a therapeutically active nuclide, preferably a therapeutically active radionuclide.
Embodiment 106. The compound for use of Embodiment 105, wherein the therapeutically active nuclide is selected from the group comprising ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I, ²¹¹At, preferably ⁴⁷Sc, ⁶⁷Cu, ⁹⁰Y, ¹⁷⁷Lu, ¹⁸⁸Re, ²¹²Pb, ²¹³Bi, ²²⁵Ac, ²²⁷Th, ¹³¹I, ²¹¹At and most preferably ⁹⁰Y, ¹⁷⁷Lu, ²²⁵Ac, ²²⁷Th, ¹³¹I and ²¹¹At.
Embodiment 107. The compound for use of any one of Embodiments 91 to 106, wherein the method comprises the administration of a therapeutically effective amount of the compound to a subject, preferably to a mammal, wherein the mammal is selected from the group comprising man, companion animals, pets, and livestock, more preferably the subject is selected from the group comprising man, dog, cat, horse, and cow, and most preferably the subject is a human being.
Embodiment 108. The compound of any one of Embodiments 1 to 71, for use in a method for the identification of a subject, wherein the subject is likely to respond or likely not to respond to a treatment of a disease, wherein the method for the identification of a subject comprises carrying out a method of diagnosis using the compound of any one of Embodiments 1 to 71, preferably a method for the diagnosis of a disease as described in any one of Embodiments 72 to 90.
Embodiment 109. The compound of any one of Embodiments 1 to 71, for use in a method for the selection of a subject from a group of subjects, wherein the subject is likely to respond or likely not to respond to a treatment of a disease, wherein the method for the selection of a subject from a group of subjects comprises carrying out a method of diagnosis using the compound of any one of Embodiments 1 to 71, preferably a method for the diagnosis of a disease as described in any one of Embodiments 72 to 90.
Embodiment 110. The compound of any one of Embodiments 1 to 71, for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosis using the compound of any one of Embodiments 1 to 71, preferably a method for the diagnosis of a disease as described in any one of Embodiments 72 to 90.
Embodiment 111. The compound for use of any one of Embodiments 108 to 110, wherein the disease is a disease involving fibroblast activation protein (FAP), preferably upregulated expression of fibroblast activation protein (FAP).
Embodiment 112. The compound for use of any one of Embodiments 108 to 111, wherein the disease involves cells showing upregulated expression of fibroblast activation protein (FAP), preferably diseased tissue containing cells showing upregulated expression of fibroblast activation protein (FAP), more preferably disease involving tumor associated fibroblasts.
Embodiment 113. The compound for use of any one of Embodiments 108 to 112, wherein the disease is a neoplasm, preferably a cancer or tumor.
Embodiment 114. The compound for use of Embodiment 113, wherein the neoplasm, cancer, and tumor are each and individually selected from the group comprising a solid tumor, an epithelial tumor, bladder cancer, breast cancer, cervical cancer, colorectal cancer, cholangiocarcinoma, endometrial cancer, esophageal cancer, gastric cancer, gastrointestinal stromal tumors, head and neck cancer, liver cancer, lung cancer, melanoma, mesothelioma, neuroendocrine tumors and carcinomas, ovarian cancer, pancreatic cancer, prostate cancer, renal cell carcinoma, salivary carcinoma, sarcoma, squamous cell carcinoma, and thyroid cancer.
Embodiment 115. The compound for use of Embodiment 114, wherein the neoplasm, cancer, and tumor are each and individually selected from the group comprising breast cancer, colorectal cancer, cholangiocarcinoma, head and neck cancer, lung cancer, mesothelioma, neuroendocrine tumors and carcinomas, ovarian cancer, pancreatic cancer, prostate cancer, sarcoma, and squamous cell carcinoma.
Embodiment 116. The compound for use of any one of Embodiments 108 to 112, wherein the disease is selected from the groups comprising inflammatory disease, cardiovascular disease, autoimmune disease, and fibrotic disease.
Embodiment 117. The compound for use of Embodiment 116, wherein the disease is an inflammatory disease.
Embodiment 118. The compound for use of Embodiment 117, wherein the disease is atherosclerosis, arthritis or rheumatoid arthritis.
Embodiment 119. The compound for use of Embodiment 116, wherein the disease is a cardiovascular disease.
Embodiment 120. The compound for use of Embodiment 119, wherein the disease is a cardiovascular disease involving atherosclerotic plaques.
Embodiment 121. The compound for use of Embodiment 120, wherein the disease is an atherosclerotic pathology caused by rupture of plaques, acute coronary syndrome, myocardial infarction, thrombosis, or vessel occlusion.
Embodiment 122. The compound for use of Embodiment 116, wherein the disease is a fibrotic disease.
Embodiment 123. The compound for use of Embodiment 122, wherein the disease is selected from the group comprising idiopathic pulmonary fibrosis, Crohn's disease, and liver fibrosis.
Embodiment 124. The compound for use of any one of Embodiments 108 to 123, wherein the method of diagnosis is an imaging method.
Embodiment 125. The compound for use of Embodiment 124, wherein the imaging method is selected from the group comprising scintigraphy, Single Photon Emission Computed Tomography (SPECT) and Positron Emission Tomography (PET).
Embodiment 126. The compound for use of any one of Embodiments 108 to 125, wherein the compound comprises a diagnostically active nuclide, preferably a diagnostically active radionuclide.
Embodiment 127. The compound for use of Embodiment 126, wherein the diagnostically active nuclide is selected from the group comprising ⁴³Sc, ⁴⁴SC, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ²⁰¹Tl, ²⁰³Pb, ¹⁸F, ⁷⁶Br, ⁷⁷Br, ¹²³I, ¹²⁴I, ¹²⁵I, preferably ⁴³Sc, ⁴⁴Sc, ⁶⁴Cu, ⁶⁷Ga, ⁶⁸Ga, ⁸⁶Y, ⁸⁹Zr, ^{99m}Tc, ¹¹¹In, ¹⁵²Tb, ¹⁵⁵Tb, ²⁰³Pb, ¹⁸F, ⁷⁶Br, ⁷⁷Br, ¹²³I, ¹²⁴I, ¹²⁵I and most preferably ⁶⁴Cu, ⁶⁸Ga, ⁸⁹Zr, ^{99m}Tc, ¹¹¹In, ¹⁸F, ¹²³I, and ¹²⁴I.
Embodiment 128. The compound of any one of Embodiments 1 to 71, for use in a method for delivering an effector to fibroblast activation protein (FAP), preferably human fibroblast activation protein (FAP), wherein the effector is selected from the group comprising a diagnostically active agent and a therapeutically active agent.
Embodiment 129. The compound for use of Embodiment 128, wherein the effector is selected from the group comprising a diagnostically active nuclide and a therapeutically active nuclide.
Embodiment 130. The compound for use of Embodiment 129, wherein the diagnostically active nuclide is a diagnostically active radionuclide.
Embodiment 131. The compound for use of Embodiment 130, wherein the diagnostically active radionuclide is selected from the group consisting of ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ²⁰¹Tl, ²⁰³Pb, ¹⁸F, ⁷⁶Br, ⁷⁷Br, ¹²³I, ¹²⁴I, ¹²⁵I, preferably ⁴³Sc, ⁴⁴SC, ⁶⁴Cu, ⁶⁷Ga, ⁶⁸Ga, ⁸⁶Y, ⁸⁹Zr, ^{99m}Tc, ¹¹¹In, ¹⁵²Tb, ¹⁵⁵Tb, ²⁰³Pb, ¹⁸F, ⁷⁶Br, ⁷⁷Br, ¹²³I, ¹²⁴I, ¹²⁵I and most preferably ⁶⁴Cu, ⁶⁸Ga, ⁸⁹Zr, ^{99m}Tc, ¹¹¹In, ¹⁸F, ¹²³I, and ¹²⁴I.
Embodiment 132. The compound for use of any one of Embodiments 128 to 131, wherein the fibroblast activation protein (FAP) is expressed by a cell, preferably a fibroblast, a mesenchymal stem cell, smooth muscle cell, a cell of epithelial origin, or an endothelial cell, more preferably a human fibroblast, mesenchymal stem cell, smooth muscle cell, cell of epithelial origin, or endothelial cell, most preferably a human fibroblast, mesenchymal stem cell, smooth muscle cell, cell of epithelial origin, or endothelial cell each showing upregulated expression of fibroblast activation protein (FAP).
Embodiment 133. The compound for use of Embodiment 132, wherein the cell is contained in or part of a tissue, preferably a diseased tissue of a subject suffering from a disease.
Embodiment 134. The compound for use of Embodiment 133, wherein the disease involves cells showing upregulated expression of fibroblast activation protein (FAP), preferably diseased tissue containing cells showing upregulated expression of fibroblast activation protein (FAP), more preferably disease involving tumor associated fibroblasts. Embodiment 135. The compound for use of any one of Embodiments 133 to 134, wherein the disease is a neoplasm, preferably a cancer or tumor.
Embodiment 136. The compound for use of Embodiment 135, wherein the neoplasm, cancer, and tumor are each and individually selected from the group comprising a solid tumor, an epithelial tumor, bladder cancer, breast cancer, cervical cancer, colorectal cancer, cholangiocarcinoma, endometrial cancer, esophageal cancer, gastric cancer, gastrointestinal stromal tumors, head and neck cancer, liver cancer, lung cancer, melanoma, mesothelioma, neuroendocrine tumors and carcinomas, ovarian cancer, pancreatic cancer, prostate cancer, renal cell carcinoma, salivary carcinoma, sarcoma, squamous cell carcinoma, and thyroid cancer.
Embodiment 137. The compound for use of Embodiment 136, wherein the neoplasm, cancer, and tumor are each and individually selected from the group comprising breast cancer, colorectal cancer, cholangiocarcinoma, head and neck cancer, lung cancer, mesothelioma, neuroendocrine tumors and carcinomas, ovarian cancer, pancreatic cancer, prostate cancer, sarcoma, and squamous cell carcinoma.
Embodiment 138. The compound for use of any one of Embodiments 133 to 134, wherein the disease is selected from the groups comprising inflammatory disease, cardiovascular disease, autoimmune disease, and fibrotic disease.
Embodiment 139. The compound for use of Embodiment 138, wherein the disease is an inflammatory disease.
Embodiment 140. The compound for use of Embodiment 139, wherein the disease is atherosclerosis, arthritis or rheumatoid arthritis.
Embodiment 141. The compound for use of Embodiment 138, wherein the disease is a cardiovascular disease.
Embodiment 142. The compound for use of Embodiment 141, wherein the diseases is a cardiovascular disease involving atherosclerotic plaques.
Embodiment 143. The compound for use of Embodiment 142, wherein the disease is an atherosclerotic pathology caused by rupture of plaques, acute coronary syndrome, myocardial infarction, thrombosis, or vessel occlusion.
Embodiment 144. The compound for use of Embodiment 138, wherein the disease is a fibrotic disease.
Embodiment 145. The compound for use of Embodiment 144, wherein the disease is selected form the group comprising idiopathic pulmonary fibrosis, Crohn's disease, and liver fibrosis.
Embodiment 146. The compound for use of Embodiment 129, wherein the therapeutically active nuclide is a therapeutically active radionuclide.
Embodiment 147. The compound for use of Embodiment 146, wherein the therapeutically active radionuclide is selected from the group consisting of ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac , ²²⁶Th, ²²⁷Th, ¹³¹I, ²¹¹At, preferably ⁴⁷Sc, ⁶⁷Cu, ⁹⁰Y, ¹⁷⁷Lu, ¹⁸⁸Re, ²¹²Pb , ²¹³Bi, ²²⁵Ac, ²²⁷Th, ¹³¹I, ²¹¹At and most preferably ⁹⁰Y, ¹⁷⁷Lu, ²²⁵Ac, ²²⁷Th, ¹³¹I and ²¹¹At.
Embodiment 148. The compound for use of any one of Embodiment 146 to 147, wherein the fibroblast activation protein (FAP) is expressed by a cell, preferably a fibroblast, a mesenchymal stem cell, smooth muscle cell, a cell of epithelial origin, or an endothelial cell, more preferably a human fibroblast, mesenchymal stem cell, smooth muscle cell, cell of epithelial origin, or endothelial cell, most preferably a human fibroblast, mesenchymal stem cell, smooth muscle cell, cell of epithelial origin, or endothelial cell showing upregulated expression of fibroblast activation protein (FAP).
Embodiment 149. The compound for use of Embodiment 148, wherein the cell is contained in or part of a tissue, preferably a diseased tissue of a subject suffering from a disease.
Embodiment 150. The compound for use of Embodiment 149, wherein the disease involves cells showing upregulated expression of fibroblast activation protein (FAP), preferably diseased tissue containing cells showing upregulated expression of fibroblast activation protein (FAP), more preferably disease involving tumor associated fibroblasts.
Embodiment 151. The compound for use of any one of Embodiments 148 to 150, wherein the disease is a neoplasm, preferably a cancer or tumor.
Embodiment 152. The compound for use of Embodiment 151, wherein the neoplasm, cancer, and tumor are each and individually selected from the group comprising a solid tumor, an epithelial tumor, bladder cancer, breast cancer, cervical cancer, colorectal cancer, cholangiocarcinoma, endometrial cancer, esophageal cancer, gastric cancer, gastrointestinal stromal tumors, head and neck cancer, liver cancer, lung cancer, melanoma, mesothelioma, neuroendocrine tumors and carcinomas, ovarian cancer, pancreatic cancer, prostate cancer, renal cell carcinoma, salivary carcinoma, sarcoma, squamous cell carcinoma, and thyroid cancer.
Embodiment 153. A composition, preferably a pharmaceutical composition, wherein the composition comprises a compound according to any one of Embodiment 1 to 71 and a pharmaceutically acceptable excipient.
Embodiment 154. The composition of Embodiment 153 for use in any method as defined in any of the preceding claims.
Embodiment 155. A method for the diagnosis of a disease in a subject, wherein the method comprises administering to the subject a diagnostically effective amount of a compound according to any one of Embodiments 1 to 71.
Embodiment 156. The method of Embodiment 155, wherein the compound comprises a diagnostically active agent, whereby the agent is preferably a radionuclide.
Embodiment 157. A method for the treatment of a disease in a subject, wherein the method comprises administering to the subject a therapeutically effective amount of a compound according to any one of Embodiment 1 to 71.
Embodiment 158. The method of Embodiment 157, wherein the compound comprises a therapeutically active agent, whereby the agent is preferably a radionuclide.
Embodiment 159. The method of any one of Embodiments 155 to 158, wherein the disease is a disease involving fibroblast activation protein (FAP), preferably upregulated expression of fibroblast activation protein (FAP).
Embodiment 160. The method of any one of Embodiments 155 to 159, wherein the disease involves cells showing upregulated expression of fibroblast activation protein (FAP), preferably diseased tissue containing cells showing upregulated expression of fibroblast activation protein (FAP), more preferably disease involving tumor associated fibroblasts.
Embodiment 161. The method of any one of Embodiments 155 to 160, wherein the disease is selected from the groups comprising neoplasms, preferably cancers or tumors, and inflammatory disease, cardiovascular disease, autoimmune disease, and fibrotic disease.
Embodiment 162. A kit comprising a compound according to any one of Embodiments 1 to 71, one or more optional excipient(s) and optionally one or more device(s), whereby the device(s) is/are selected from the group comprising a labeling device, a purification device, a handling device, a radioprotection device, an analytical device or an administration device.
Embodiment 163. The kit of Embodiment 162 for use in any method as defined in any of the preceding claims.

More specifically, the problem underlying the present invention is solved in a first aspect by a compound comprising a cyclic peptide
of formula (I) 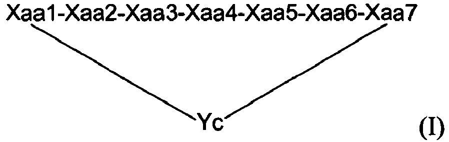 and an N-terminal modification group A attached to Xaal,
wherein
the peptide sequence is drawn from left to right in N to C-terminal direction,
Xaa1 is a residue of an amino acid of formula (II) 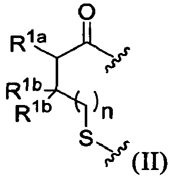 wherein
   R^{1a} is -NH-
   R^{1b} is H or CH₃,
   n = 0 or 1,
   the N-terminal modification group A is covalently attached to the nitrogen atom of Xaa1,
   the carbonyl group of Xaa1 is covalently attached to the nitrogen of Xaa2, and the sulfur atom of Xaa1 is covalently attached as thioether to Yc;
Xaa2 is a residue of an amino acid of formula (III), (IV) or (XX) 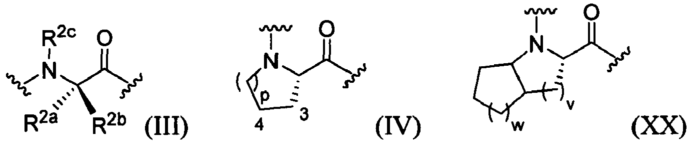 wherein
   R^{2a}, R^{2b} and R^{2c} are each and independently selected from the group consisting of (C₁-C₂)alkyl and H, wherein said (C₁-C₂)alkyl maybe substituted by a substituent selected from the group consisting of OH, NH₂, halogen, (C₅-C₇)cycloalkyl,
   p = 0, 1 or 2
   v = 1 or 2
   w = 1, 2 or 3 and
   the amino acid of formula (IV) maybe substituted by one or two substituents selected from the group consisting of methyl, OH, NH₂ and F, at indicated ring positions 3 and 4;
Xaa3 is a residue of an amino acid of formula (V) or (XX) 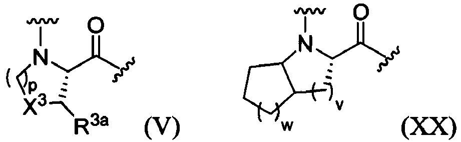 wherein
   X³ is selected from the group consisting of CH₂, CF₂, CH-R^{3b}, S, O and NH,
   p= 1 or2
   v= 1 or2
   w = 1, 2 or 3,
   R^{3a} is H, methyl, OH, NH₂ or F,
   R^{3b} is methyl, OH, NH₂ or F;
Xaa4 is a residue of an amino acid of formula (VI) 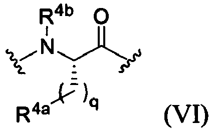 wherein
   R^{4a} is selected from the group consisting of H, OH, COOH, CONH₂, X⁴ and-NH-CO-X⁴, wherein X⁴ is selected from the group consisting of (C₁-C₆)alkyl, (C₅-C₆)aryl and (C₅-C₆)heteroaryl, and X⁴ may be substituted by one or two substituents selected from the group consisting of methyl, CONH₂, halogen, NH₂ and OH;
   q = 1, 2 or 3, wherein optionally one or two hydrogens of these said one, two or three CH₂-groups are each and individually substituted by methyl, ethyl, (C₅-C₆)aryl or (C₅-C₆)heteroaryl,
   R^{4b} is methyl or H;
Xaa5 is a residue of an amino acid of structure (VII) 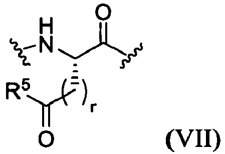 wherein
   R⁵ is selected from the group of OH and NH₂, and
   r = 1, 2 or 3;
Xaa6 is an amino acid selected from the group consisting of an aromatic L-α-amino acid and a heteroaromatic L-α-amino acid;
Xaa7 is a residue of an amino thiol or an amino acid of formula (IX), 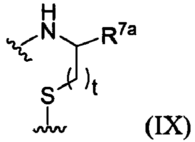 wherein
   R^{7a} is -CO-, -COOH, -CONH₂, -CH₂-OH, -(CO)-NH-R^{7b}, -(CO)-(NR^{7c})-R^{7b} or H, wherein
   R^{7b} and R^{7c} are each and independently (C₁-C₄)alkyl and
   t is 1 or 2;
Yc is a structure of formula (X) 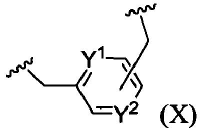 linking the S atom of Xaa1 and the S atom of Xaa7 under the formation of two thioether linkages thus forming a cyclic structure of formula (XXI) 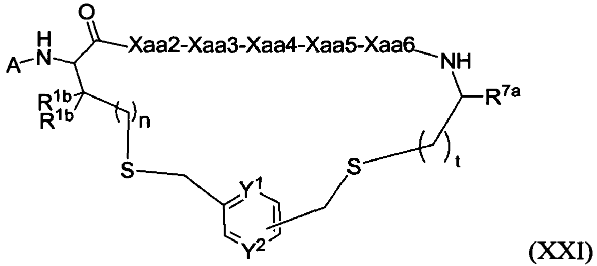 wherein
   the substitution pattern of the aromatic group in formula (X) is *ortho, meta* or *para,*
   n = 0 or 1,
   t = 1 or 2,
   Y¹ is C-H or N,
   Y² is N or C-R^{c1},
   R^{c1} is H or CH₂-R^{c2} and
   R^{c2} is a structure of formula (XI), (XII) or (XXII) 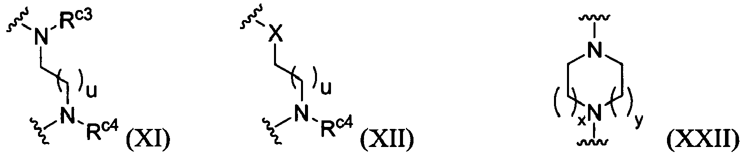
   wherein
   R^{c3} and R^{c4} are each and independently selected from the group consisting of H and (C₁-C₄)alkyl and
      u = 1, 2, 3, 4, 5 or 6,
      x and y are each and independently 1, 2 or 3, and
      X = O or S
      wherein in formulae (XI) and (XXII) one of the nitrogen atoms is attached to -CH₂- of R^{c1} and in formula (XII) -X- is attached to -CH₂- of R^{c1}; and
   wherein the N-terminal modification group A is either a blocking group Abl or an amino acid Aaa.

More specifically, the problem underlying the present invention is solved in a second aspect by the compound according to the first aspect, including any embodiment thereof, for use in a method for the diagnosis of a disease.

More specifically, the problem underlying the present invention is solved in a third aspect by the compound according to the first aspect, including any embodiment thereof, for use in a method for the treatment of a disease.

More specifically, the problem underlying the present invention is solved in a fourth aspect by the compound according to the first aspect, including any embodiment thereof, for use in a method for the identification of a subject, wherein the subject is likely to respond or likely not to respond to a treatment of a disease, wherein the method for the identification of a subject comprises carrying out a method of diagnosis using the compound according to the first aspect including any embodiment thereof.

More specifically, the problem underlying the present invention is solved in a fifth aspect by the compound according to the first aspect, including any embodiment thereof, for use in a method for the selection of a subject from a group of subjects, wherein the subject is likely to respond or likely not to respond to a treatment of a disease, wherein the method for the selection of a subject from a group of subjects comprises carrying out a method of diagnosis using the compound according to the first aspect, including any embodiment thereof.

More specifically, the problem underlying the present invention is solved in a sixth aspect by the compound according to the first aspect, including any embodiment thereof, for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosis using the compound according to the first aspect, including any embodiment thereof.

More specifically, the problem underlying the present invention is solved in a seventh aspect by a composition, preferably a pharmaceutical composition, wherein the composition comprises a compound according to the first aspect including any embodiment thereof and a pharmaceutically acceptable excipient.

More specifically, the problem underlying the present invention is solved in an eighth aspect by a method for the diagnosis of a disease in a subject, wherein the method comprises administering to the subject a diagnostically effective amount of a compound according to the first aspect, including any embodiment thereof.

More specifically, the problem underlying the present invention is solved in a ninth aspect by a method for the treatment of a disease in a subject, wherein the method comprises administering to the subject a therapeutically effective amount of a compound according to the first aspect including any embodiment thereof.

More specifically, the problem underlying the present invention is solved in a tenth aspect by a kit comprising a compound according to the frist aspect, including any embodiment thereof, one or more optional excipient(s) and optionally one or more device(s), whereby the device(s) is/are selected from the group comprising a labeling device, a purification device, a handling device, a radioprotection device, an analytical device or an administration device.

It will be acknowledged by a person skilled in the art that a or the compound of the invention is any compound disclosed herein, including but not limited to any compound described in any of the above embodiments and any of the following embodiments.

It will be acknowledged by a person skilled in the art that a or the method of the invention is any method disclosed herein, including but not limited to any method described in any of the above embodiments and any of the following embodiments.

It will be acknowledged by a person skilled in the art that a or the composition of the invention is any composition disclosed herein, including but not limited to any composition described in any of the above embodiments and any of the following embodiments.

It will be acknowledged by a person skilled in the art that a or the kit of the invention is any kit disclosed herein, including but not limited to any kit described in any of the above embodiments and any of the following embodiments.

The present invention is based on the surprising finding of the present inventors that the compound of the invention and more specifically the cyclic peptide thereof provides for a highly specific binding of a compound comprising such cyclic peptide to fibroblast activation protein (FAP), since FAP-specific cyclic peptide-based inhibitors with nanomolar affinity have not been described so far.

Furthermore, the present invention is based on the surprising finding that a chelator, either directly or indirectly, i.e. using a linker, may be attached to said cyclic peptide at three different positions. The first position is Yc having a structure of formula (X) which links the S atom of Xaa1 and the S atom of Xaa7 thus forming two thioether linkages; the second position is Aaa attached to Xaa1 of the cyclic peptide of formula (I), and the third position is an amino acid or a peptide attached to Xaa7. Surprisingly, the attachment of such chelator does not significantly affect the binding of the compound of the invention to FAP and, respectively, the inhibiting characteristics of the compound of the present invention on FAP. It is also within the present invention that the chelator is attached to the cyclic peptide of formula (I) at any combination of the first, second, and third position as defined above.

Finally, the present inventors have found that the compound of the invention is surprisingly stable in blood plasma.

The expression alkyl as preferably used herein refers each and individually to a saturated, straight-chain or branched hydrocarbon group and is usually accompanied by a qualifier which specifies the number of carbon atoms it may contain. For example the expression (C₁-C₆)alkyl means each and individually any of methyl, ethyl, n-propyl, isopropyl, n-butyl, isobutyl, *sec*-butyl, *tert*-butyl, n-pentyl, 1-methyl-butyl, 1-ethyl-propyl, 3-methyl-butyl, 1,2-dimethyl-propyl, 2-methyl-butyl, 1,1-dimethyl-propyl, 2,2-dimethylpropyl, n-hexyl, 1,1-dimethyl-butyl and any other isoform of alkyl groups containing six saturated carbon atoms.

In an embodiment and as preferably used herein, (C₁-C₂)alkyl means each and individually any of methyl and ethyl.

In an embodiment and as preferably used herein, (C₁-C₃)alkyl means each and individually any of methyl, ethyl, n-propyl and isopropyl.

In an embodiment and as preferably used herein, (C₁-C₄)alkyl means each and individually any of methyl, ethyl, n-propyl, isopropyl, n-butyl, isobutyl, *sec*-butyl and *tert*-butyl.

In an embodiment and as preferably used herein, (C₁-C₆)alkyl means each and individually any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl.

In an embodiment and as preferably used herein, (C₁-C₈)alkyl refers to a saturated or unsaturated, straight-chain or branched hydrocarbon group having from 1 to 8 carbon atoms. Representative (C₁-C₈)alkyl groups include, but are not limited to, any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethyl-butyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methyl-hept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl and 2-ethyl-3-methyl-pentyl. A (C₁-C₈)alkyl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

The expression alkylidene as preferably used herein refers to a saturated straight chain or branched hydrocarbon group wherein two points of substitution are specified. Simple alkyl chains wherein the two points of substitutions are in a maximal distance to each other like methane-1,1-diyl, ethane-1,2-diyl, propane-1,3-diyl, butane-1,4-diyl and pentane-1,5-diyl are also referred to as methylene (which is also referred to as methane-1,1-diyl), ethylene (which is also referred to as ethane-1,2-diyl), propylene (which is also referred to as propane-1,3-diyl), butylene (which is also referred to as butane-1,4-diyl) and pentylene (which is also referred to as pentane-1,5-diyl).

In an embodiment and as preferably used herein, (C₁-C₁₀)alkylidene means each and individually any of methylene, ethane-1,2-diyl, propane-1,3-diyl, propane-1,2-diyl, butane-1,4-diyl, butane-1,3-diyl, butane-1,2-diyl, 2-methyl-propane-1,2-diyl, 2-methyl-propane-1,3-diyl, pentane-1,5-diyl, pentane-1,4-diyl, pentane-1,3-diyl, pentane-1,2-diyl, pentane-2,3-diyl, pentane-2,4-diyl, any other isomer with 5 carbon atoms, hexane-1,6-diyl, any other isomer with 6 carbon atoms, heptane-1,7-diyl, any other isomer with 7 carbon atoms, octane-1,8-diyl, any other isomer with 8 carbon atoms, nonane-1,9-diyl, any other isomer with 9 carbon atoms, decane-1,10-diyl and any other isomer with 10 carbon atoms, preferably (C₁-C₁₀) alkylidene means each and individually any of methylene, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl and decane-1,10-diyl. A (C₁-C₁₀)alkylidene group can be unsubstituted or substituted with one or more groups, including, but not limited to, (C₁-C₈)alkyl, -O-[(C₁-C₈)alkyl], -aryl,-CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "aryl" refers to a carbocyclic aromatic group. Examples of aryl groups include, but are not limited to, phenyl, naphthyl and anthracenyl.

In an embodiment and as preferably used herein, (C₅-C₆)aryl refers to a 5 or 6 carbon atom comprising carbocyclic aromatic group. A carbocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-COR', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "heteroaryl" refers to a heterocyclic aromatic group. Examples of heteroaryl groups include, but are not limited to, furane, thiophene, pyridine, pyrimidine, benzothiophene, benzofurane and quinoline.

In an embodiment and as preferably used herein, (C₅-C₆)heteroaryl refers to a heterocyclic aromatic group consisting of 5 or 6 ring atoms wherein at least one atom is different from carbon, preferably nitrogen, sulfur or oxygen. A heterocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, (C₃-C₈)heterocyclo refers to a (C₃-C₈)heterocycle group defined above wherein one of the carbocycles group hydrogen atoms is replaced with a bond. A (C₃-C₈)heterocyclo can be unsubstituted or substituted with up to six groups including, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂,-NHR', -NR'₂ and -CN; where each R' is independently selected from -(C₁-C₈)alkyl and aryl. In an embodiment and as preferably used herein, arylene refers to an aryl group which has two covalent bonds and can be in the *ortho, meta,* or *para* configurations as shown in the following structures: 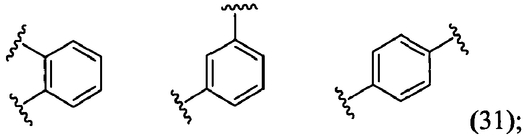 in which the phenyl group can be unsubstituted or substituted with four groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂,-NHR', -NR'₂ and -CN; where each R' is independently selected from -(C₁-C₈)alkyl and aryl.

In an embodiment and as preferably used herein atoms with unspecified atomic mass numbers in any structural formula or in any passage of the instant specification including the claims are either of unspecified isotopic composition, naturally occurring mixtures of isotopes or individual isotopes. This applies in particular to carbon, oxygen, nitrogen, sulfur, phosphorus, halogens and metal atoms, including but not limited to C, O, N, S, F, P, Cl, Br, At, Sc, Cr, Mn, Co, Fe, Cu, Ga, Sr, Zr, Y, Mo, Tc, Ru, Rh, Pd, Pt, Ag, In, Sb, Sn, Te, I, Pr, Pm, Dy, Sm, Gd, Tb, Ho, Dy, Er, Yb, Tm, Lu, Sn, Re, Rd, Os, Ir, Au, Pb, Bi, Po, Fr, Ra, Ac, Th and Fm.

In an embodiment and as preferably used herein, a chelator is a compound which is capable of forming a chelate, whereby a chelate is a compound, preferably a cyclic compound where a metal or a moiety having an electron gap or a lone pair of electrons participates in the formation of the ring. More preferably, a chelator is this kind of compound where a single ligand occupies more than one coordination site at a central atom.

In an embodiment and as preferably used herein, a diagnostically active compound is a compound which is suitable for or useful in the diagnosis of a disease.

In an embodiment and as preferably used herein, a diagnostic agent or a diagnostically active agent is a compound which is suitable for or useful in the diagnosis of a disease.

In an embodiment and as preferably used herein, a therapeutically active compound is a compound which is suitable for or useful in the treatment of a disease.

In an embodiment and as preferably used herein, a therapeutic agent or a therapeutically active agent is a compound which is suitable for or useful in the treatment of a disease.

In an embodiment and as preferably used herein, a theragnostically active compound is a compound which is suitable for or useful in both the diagnosis and therapy of a disease.

In an embodiment and as preferably used herein, a theragnostic agent or a theragnostically active agent is a compound which is suitable for or useful in both the diagnosis and therapy of a disease.

In an embodiment and as preferably used herein, theragonstics is a method for the combined diagnosis and therapy of a disease; preferably, the combined diagnostically and therapeutically active compounds used in theragnostics are radiolabeled.

In an embodiment and as preferably used herein, treatment of a disease is treatment and/or prevention of a disease.

In an embodiment and as preferably used herein, a disease involving FAP is a disease where cells including but not limited to fibroblasts expressing, preferably in an upregulated manner, FAP and tissue either expressing FAP or containing or comprising cells such as fibroblasts, preferably expressing FAP in an upregulated manner respectively, are either a or the cause for the disease and/or the symptoms of the disease, or are part of the pathology underlying the disease. A preferred FAP-expressing cell is a cancer associated fibroblast (CAF). In an embodiment of the disease, preferably when used in connection with the treatment, treating and/or therapy of the disease, affecting the cells, the tissue and pathology, respectively, results in cure, treatment or amelioration of the disease and/or the symptoms of the disease. In an embodiment of the disease, preferably when used in connection with the diagnosis and/or diagnosing of the disease, labeling of the FAP-expressing cells and/or of the FAP-expressing tissue allows discriminating or distinguishing said cells and/or said tissue from healthy or FAP-non-expressing cells and/or healthy or FAP non-expressing tissue. More preferably such discrimination or distinction forms the basis for said diagnosis and diagnosing, respectively. In an embodiment thereof, labeling means the interaction of a detectable label either directly or indirectly with the FAP-expressing cells and/or with the FAP-expressing tissue or tissue containing such FAP-expressing cells; more preferably such interaction involves or is based on the interaction of the label or a compound bearing such label with FAP.

In an embodiment and as preferably used herein, a target cell is a cell which is expressing FAP and is a or the cause for a disease and/or the symptoms of a disease, or is part of the pathology underlying a disease.

In an embodiment and as preferably used herein, a non-target cell is a cell which is either not expressing FAP and/or is not a or the cause for a disease and/or the symptoms of a disease, or is part of the pathology underlying a disease.

In an embodiment and as preferably used herein, a neoplasm is an abnormal new growth of cells. The cells in a neoplasm grow more rapidly than normal cells and will continue to grow if not treated. A neoplasm may be benign or malignant.

In an embodiment and as preferably used herein, a tumor is a mass lesion that may be benign or malignant.

In an embodiment and as preferably used herein, a cancer is a malignant neoplasm.

In an embodiment and as preferably used herein, a linkage is an attachment of two atoms of two independent moieties. A preferred linkage is a chemical bond or a plurality of chemical bonds. More preferably a chemical bond is a covalent bond or a plurality of chemical bonds. Most preferably the linkage is a covalent bond or a coordinate bond. As preferably used herein, an embodiment of a coordinate bond is a bond or group of bonds as realized when a metal is bound by a chelator. Depending on the type of atoms linked and their atomic environment different types of linkages are created. These types of linkage are defined by the type of atom arrangements created by the linkage. For instance, the linking of a moiety comprising an amine with a moiety comprising a carboxylic acid leads to a linkage named amide (which is also referred to as amide linkage, -CO-N-, -N-CO-). It will be acknowledged by a person skilled in the art that this and the following examples of creating linkages are only prototypical examples and are by no means limiting the scope of the instant application. It will be acknowledged by a person in the art that the linking of a moiety comprising an isothiocyanate with a moiety comprising an amine leads to thiourea (which is also referred to as a thiourea linkage, -N-CS-N-), and linking of a moiety comprising a C atom with a moiety comprising a thiol-group (-C-SH) leads to thioether (which is also referred to as a thioether linkage, -C-S-C-). A non-limiting list of linkages as preferably used in connection with the chelator and linker of the invention and their characteristic type of atom arrangement is presented Table 2.

**Table 2:**

| Linkage | Characteristic atom arrangement |
|---|---|
| Amide | 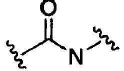 |
| Sulfonamide | 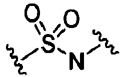 |
| Urea | 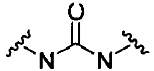 |
| Thioether | 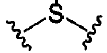 |
| Disulfide | 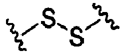 |
| Ether | 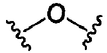 |
| Ester | 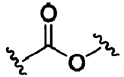 |
| Carbamate | 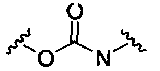 |
| Thiourea | 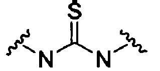 |
| Triazole | 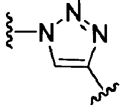 |
| Pyrazine | 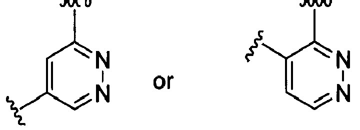 |
| Dihydro-pyrazine | 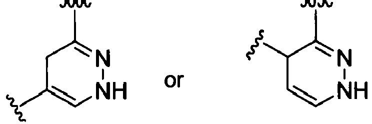 |
| | and isomers |

Examples of reactive groups which, in some embodiments of the invention, are used in the formation of linkages between the chelator and linker or directly between the chelator and the compound of the invention are summarized in Table 3. It will, however, be understood by a person skilled in the art that neither the linkages which may be realized in embodiments for the formation of the conjugates of the invention are limited to the ones of Table 3 nor the reactive groups forming such linkages.

**Table 3:**

| first reactive group | second reactive group | (type of) linkage |
|---|---|---|
| amino | carboxylic acid | amide |
| amino | activated carboxylic acid | amide |
| carboxylic acid | amino | amide |
| sulfhydryl | Michael acceptor (e.g. Maleimide) | thioether |
| bromo | sulfhydryl | thioether |
| isothiocyanate | amino | thiourea |
| hydroxyl | carboxylic acid | ester |
| azide | alkyne | triazole |
| sulfhydryl | sulfhydryl | disulfide |
| sulfhydryl | 2-Pyridine-disulfide | disulfide |
| isocyanate | amino | carbamate |
| bromo | hydroxy | ether |

The following are reactive groups and functionalities which are utilized or amenable of forming linkages between moieties or structures as used in embodiments of the conjugate of the invention:

Primary or secondary amino, carboxylic acid, activated carboxylic acid, chloro, bromo, iodo, sulfhydryl, hydroxyl, sulfonic acid, activated sulfonic acid, sulfonic acid esters like mesylate or tosylate, Michael acceptors, strained alkenes like *trans* cyclooctene, isocyanate, isothiocyanate, azide, alkyne and tetrazine.

As preferably used herein, the term "activated carboxylic acid" refers to a carboxylic acid group with the general formula -CO-X, wherein X is a leaving group. For example, activated forms of a carboxylic acid group may include, but are not limited to, acyl chlorides, symmetrical or unsymmetrical anhydrides, and esters. In some embodiments, the activated carboxylic acid group is an ester with pentafluorophenol, nitrophenol, benzotriazole, azabenzotriazole, thiophenol or N-hydroxysuccinimide (NHS) as leaving group.

As preferably used herein, the term "activated sulfonic acid" refers to a sulfonic acid group with the general formula -SO₂-X, wherein X is a leaving group. For example, activated forms of a sulfonic acid may include, but are not limited to, sulfonyl chlorides or sulfonic acid anhydrides. In some embodiments, the activated sulfonic acid group is sulfonylchloride with chloride as leaving group.

In an embodiment and as preferably used herein the term "mediating a linkage" means that a linkage or a type of linkage is established, preferably a linkage between two moieties. In a preferred embodiment the linkage and the type of linkage is as defined herein.

To the extent it is referred in the instant application to a range indicated by a lower integer and a higher integer such as, for example, 1-4, such range is a representation of the lower integer, the higher integer and any integer between the lower integer and the higher integer. Insofar, the range is actually an individualized disclosure of said integer. In said example, the range of 1-4 thus means 1, 2, 3 and 4.

Compounds of the invention typically contain amino acid sequences as provided herein. Conventional amino acids, also referred to as natural amino acids are identified according to their standard three-letter codes, as set forth in Table 4.

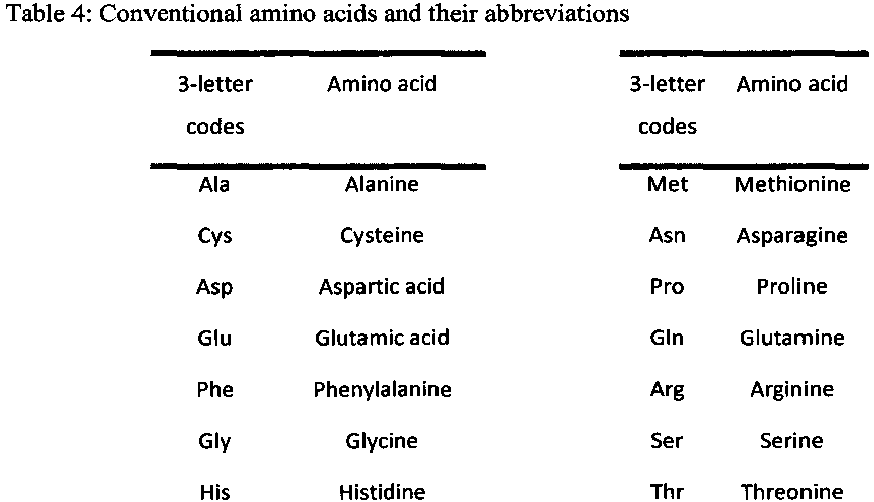

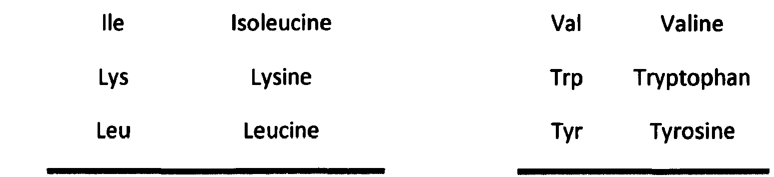

Non-conventional amino acids, also referred to as non-natural amino acids, are any kind of non-oligomeric compound which comprises an amino group and a carboxylic group and is not a conventional amino acid.

Examples of non-conventional amino acids and other building blocks as used for the construction compounds of the invention are identified according to their abbreviation or name found in Table 5. The structures of some building blocks are depicted with an exemplary reagent for introducing the building block into the peptide (e.g., as carboxylic acid like) or these building blocks are shown as residue which is completely attached to another structure like a peptide or amino acid. The structures of the amino acids are shown as explicit amino acids and not as residues of the amino acids how they are presented after implementation in the peptide sequence. Some larger chemical moieties consisting of more than one moiety are also shown for the reason of clarity.

**Table 5: Abbreviation, name and structure of non-natural amino-acid and other building blocks and chemical moieties**

| Abbreviation | Name | Structure |
|---|---|---|
| 2Lut | 2,6-lutidylidene (derived from 2,6-lutidine) | 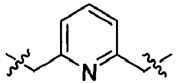 |
| 3Lut | 3,5-lutidylidene (derived from 3,5-lutidine) | 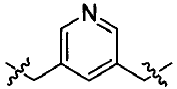 |
| 3MeBn | 3-Methylbenzylidene | 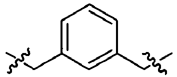 |
| 4Amc | 4-trans-Aminomethylcyclohexane carboxylic acid / Tranexamic acid | 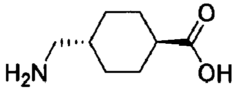 |
| 4Ap | (2S,4S)-4-Amino-pyrrolidine-2-carboxylic acid | 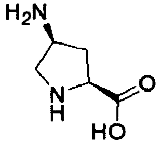 |
| 4Pya | 2-(Pyridin-4-yl)acetic acid | 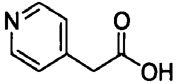 |
| Abu | (S)-2-Amino-butyric acid | 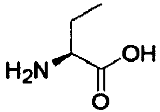 |
| AET | 2-Aminoethanethiol | 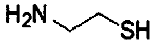 |
| Aic | 2-Aminoindane-2-carboxylic acid | 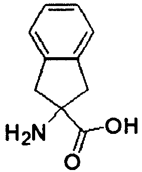 |
| Amf | (S)-α-Methyl-phenylalanine | 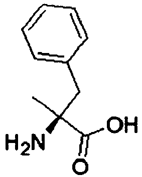 |
| APAc | 2-(4-(Amino)piperidin-1-yl)acetic acid | 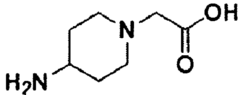 |
| Ape | 1,5-Diaminopentane | 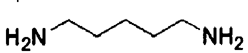 |
| Ape(DOTA) | 4-[[(5-Amino-pentylcarbamoyl)-methyl]-7,10-bis-carboxymethyl-1,4,7,10tetraaza-cyclododec-1-yl]-acetic acid | 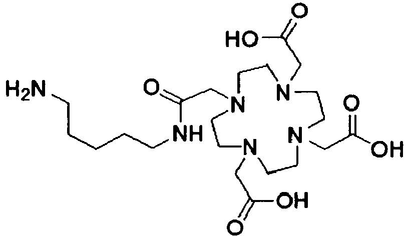 |
| Aze | (S)-Azetidine-2-carboxylic acid | 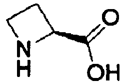 |
| Bal | β-Alanine | 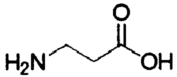 |
| Bhf | (S)-β-Homophenylalanine | 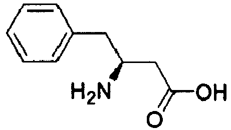 |
| Bhk | (S)-β-Homolysine | 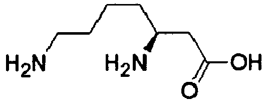 |
| Bio | D(+)-Biotin | 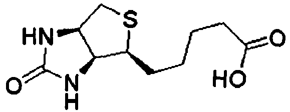 |
| Cfp | (2S,4R)-4-fluoro-pyrrolidine-2-carboxylic acid | 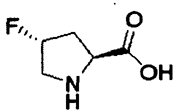 |
| Chg | (S)-Cyclohexylglycine | 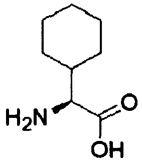 |
| Chy | (2S,4S)-4-Hydroxy-pyrrolidine-2-carboxylic acid | 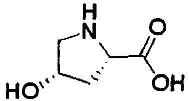 |
| Cit | (S)-Citrulline | 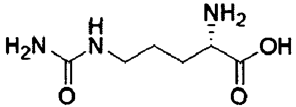 |
| Cmp | 4-Carboxymethyl-piperidine | 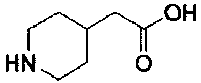 |
| Cpp | trans-3-Azabicyclo[3.1.0]hexane-2-carboxylic acid | 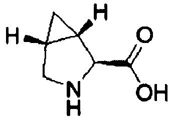 |
| Cya | (R)-Cysteic acid | 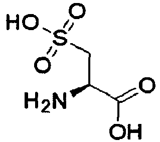 |
| Cys(2Lut) | | 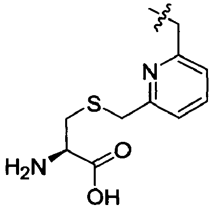 |
| Cys(3Lut) | | 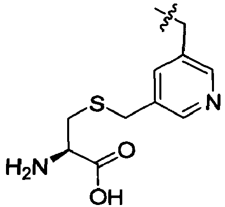 |
| Cys(3MeBn) | | 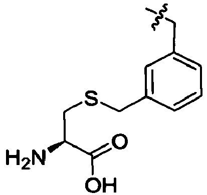 |
| Cys(tMeBn (DOTA-AET)) | | 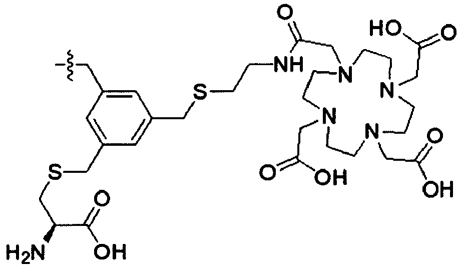 |
| Cys(tMeBn (DOTA-PP)) | | 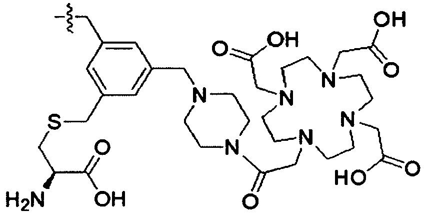 |
| Cys(tMeBn (H-AET)) | | 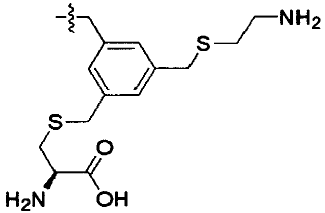 |
| Cys(tMeBn (H-PP)) | | 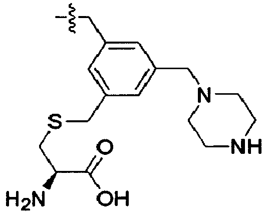 |
| Cysol | (R)-Cysteinol | 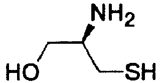 |
| Dab | (S)-2,4-Diaminobutyric acid | 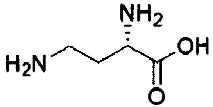 |
| Dap | (S)-2,3-Diaminopropionic acid | 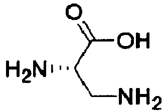 |
| Dmp | (S)-5,5-Dimethyl-proline | 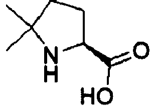 |
| DOTA | 1,4,7,10-Tetraazacyclododecane-1,4,7,10-tetraacetic acid | 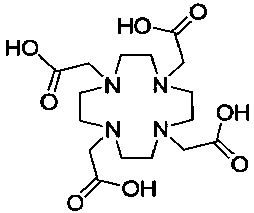 |
| Egd | (S)-ω,ω-Dimethyl-arginine | 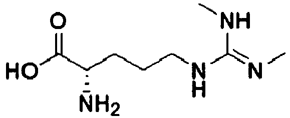 |
| EuDOTA | DOTA complexing Europium | 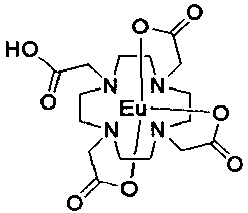 |
| Gab | γ-Aminobutyric acid | 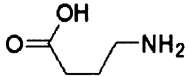 |
| GaDOTA | DOTA complexing Gallium | 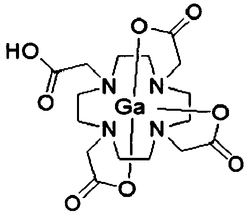 |
| Ghg | (S)-γ-Hydroxy-glutamic acid | 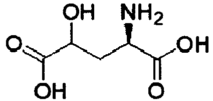 |
| H2NSO2-But | 4-Sulfamoylbutyric acid | 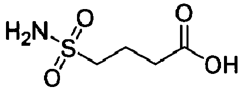 |
| H3p | (2S,3S)-3-hydroxy-pyrrolidine-2-carboxylic acid | 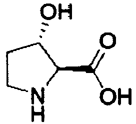 |
| Har | (S)-Homoarginine | 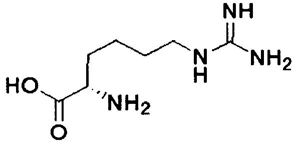 |
| Hci | (S)-Homocitrulline | 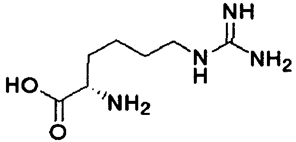 |
| Hcy | (S)-Homocysteine | 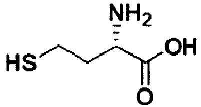 |
| hcy | (R)-Homocysteine | 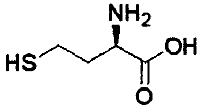 |
| Hex | Hexanoic acid | 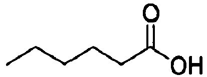 |
| Hex- | hexanoyl | 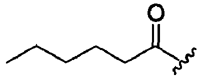 |
| Hfe | (S)-Homophenylalanine | 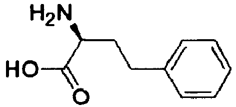 |
| Hga | (S)-Homoglutamic acid | 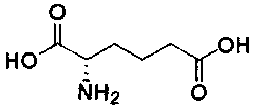 |
| Hle | (S)-Homoleucine | 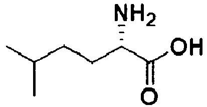 |
| Hth | (S)-Homothreonine | 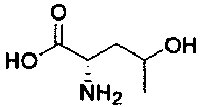 |
| Hym | (2S,4R)-4-Methyoxy-pyrrolidine-2-carboxylic acid | 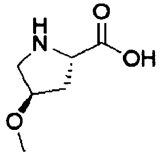 |
| Hyp | (2S,4R)-4-Hydroxy-pyrrolidine-2-carboxylic acid | 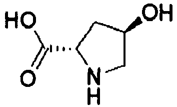 |
| InDOTA | DOTA complexing Indium | 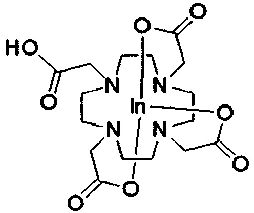 |
| Inp | Isonipecotic acid | 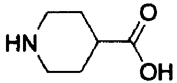 |
| LuDOTA | DOTA complexing Lutetium | 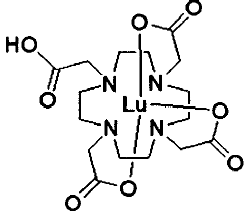 |
| Mamb | 3-Aminomethyl-benzoic acid | 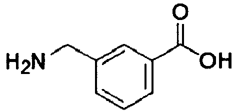 |
| Moo | (S)-Methionine sulfone | 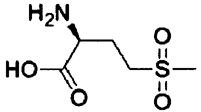 |
| Mpa | 3-Pyridyl-alanine | 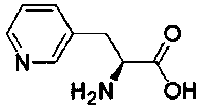 |
| Nle | (S)-Norleucine | 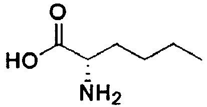 |
| nle | (R)-Norleucine | 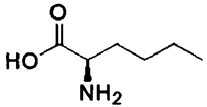 |
| Nma | (S)-N-Methyl-alanine | 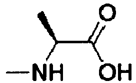 |
| nma | (R)-N-Methyl-alanine | 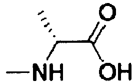 |
| Nmc | (R)-N-Methyl-cysteine ne | 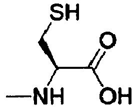 |
| Nmf | (S)-N-Methyl-phenylalanine | 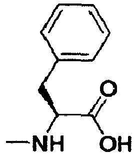 |
| Nmg | N-Methyl-glycine | 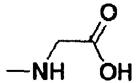 |
| Nva | (S)-Norvaline | 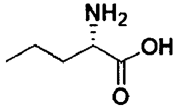 |
| O2Oc | 3,6-Dioxaoctanoic acid | 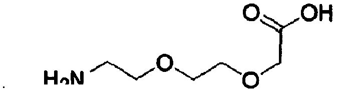 |
| Oct | Octanoic acid | 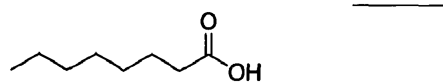 |
| Omr | (S)-ω-Methyl-arginine | 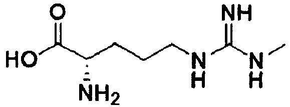 |
| Pamb | 4-Aminomethyl-benzoic acid | 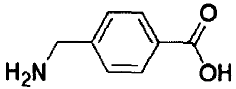 |
| PEG12 | | 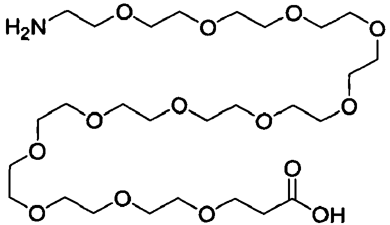 |
| PEG6 | | 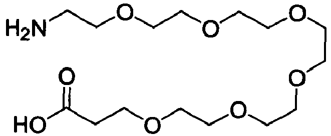 |
| Pen | (R)-Penicillamine | 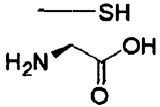 |
| pen | (S)-Penicillamine | 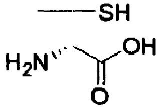 |
| PentylNH-urea | | 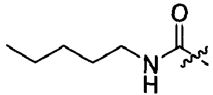 |
| Pentyl-SO2- | Pentyl sulfonyl | 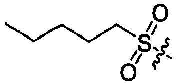 |
| Php | 3-Phenylpropionic acid | 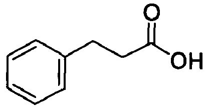 |
| Pip | (S)-Piperidine-2-carboxylic acid | 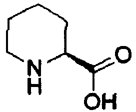 |
| Ppa | (S)-4-Pyridyl-alanine | 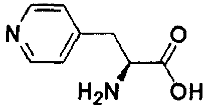 |
| PPAc | 4-Carboxymethyl piperazine | 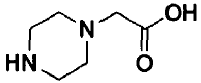 |
| Rni | (R)-Nipecotic acid | 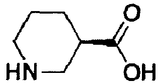 |
| Sni | (S)-Nipecotic acid | 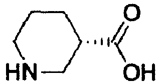 |
| Spa | 3-Mercaptopropionic acid | 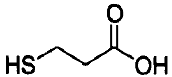 |
| -Succ- | -Succinimide- | 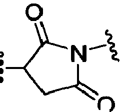 |
| Tap | (2S,4R)-4-Amino-pyrrolidine-2-carboxylic acid | 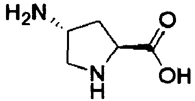 |
| Tfp | (2S,4R)-4-Fluoro-pyrrolidine-2-carboxylic acid | 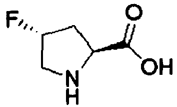 |
| Thi | (S)-β-(2-Thienyl)-alanine | 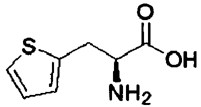 |
| Tic | (S)-1,2,3,4-Tetrahydroisoquinoline-3-carboxylic acid | 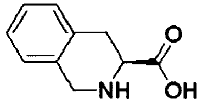 |
| tMeBn | 1,3,5-Trimethylbenzyliden | 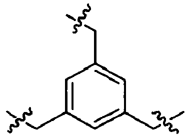 |
| tMeBn(H-AET) | | 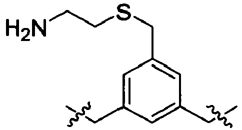 |
| tMeBn(H-PP) | | 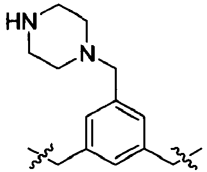 |
| Ttds | 1,13-Diamino-4,7,10-trioxatridecan-succinamic acid | 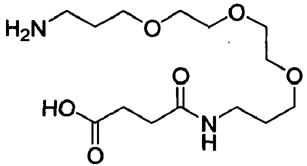 |

The amino acid sequences of the peptides provided herein are depicted in typical peptide sequence format, as would be understood by the ordinary skilled artisan. For example, the three-letter code of a conventional amino acid, or the code for a non-conventional amino acid or the abbreviations for additional building blocks, indicates the presence of the amino acid or building block in a specified position within the peptide sequence. The code for each amino acid or building block is connected to the code for the next and/or previous amino acid or building block in the sequence by a hyphen which (typically represents an amide linkage).

Where an amino acid contains more than one amino and/or carboxy group all orientations of this amino acid are in principle possible, but in α-amino acid the utilization of the α-amino and the α-carboxy group is preferred and otherwise preferred orientations are explicitly specified.

For amino acids, in their abbreviations the first letter indicates the stereochemistry of the C-α-atom if applicable. For example, a capital first letter indicates that the L-form of the amino acid is present in the peptide sequence, while a lower case first letter indicating that the D-form of the correspondent amino acid is present in the peptide sequence.

In an embodiment and as preferably used herein, an aromatic L-α-amino acid is any kind of L-α-amino acid which comprises an aryl group.

In an embodiment and as preferably used herein, a heteroaromatic L-α-amino acid is any kind of L-α-amino acid which comprises a heteroaryl group.

Unless indicated to the contrary, the amino acid sequences are presented herein in N- to C-terminus direction.

Derivatives of the amino acids constituting the peptides of the invention may be as set forth in Table 6. In any embodiment, one or more amino acids of the compounds of the invention are substituted with a derivative of the corresponding preferred amino acids.

**Table 6: Exemplary derivatives of preferred amino acids contained in the compound of the invention**

| **Amino Acid** | **Exemplary derivatives** |
|---|---|
| Ala | Aib, Bal, Abu, Gly, Nva, Nle |
| Cys | Hcy, Nmc |
| Asp | Glu, Asn, Gln, Cya |
| Glu | Asp, Asn, Gln, Cya, Homoglutamic acid, γ-Hydroxy-glutamic acid, |
| Phe | Hfe, Phg, Bhf, Thi, Bta, Bromophenylalanine, Iodophenylalanine, Chlorophenylalanine, Methylphenylalanine, Nitrophenylalanine, Tyr, Trp, Naphthylalanine, Trifluoromethylphenylalanine |
| Gly | Ala, ala, Nmg |
| Nmg | Pro, Ala, ala, Gly, Nma, nma |
| His | 1-Methylhistidine, 3-Methylhistidine, Thi |
| Ile | Leu, Val, Hle, Nva, Nle, Chg |
| Lys | Arg, Dab, Dap, Har, Egd, Omr, Hci, Cit |
| Leu | Ile, Val, Hle, Nle, Nva, Moo |
| Met | Ile, Val, Hle, Nle, Nva, Moo |
| Nle | Ile, Val, Hle, Met, Nva, Moo |
| Asn | Asp, Glu, Gln, Cya, Thr |
| Pro | Aze, Pip, Hyp, Tfp, Cfp, Dmp, Tap, H3p, 4Ap, Cpp, Hym, Chy, Dfp |
| Gln | Asp, Asn, Glu, Cya, Thr, Hse |
| Arg | Arg, Dab, Dap, Har, Egd, Omr, Hci, Cit |
| Ser | Thr, Hse, *allo*-Threonine |
| Thr | Ser, Homothreonine, allo-Threonine |
| Val | Leu, Ile, Hle, Nva, Nle |
| Trp | Hfe, Phg, Bhf, Thi, Bta, Bromophenylalanine, Iodophenylalanine, Chlorophenylalanine, Methylphenylalanine, Nitrophenylalanine, Tyr, Trp, Naphthylalanine, Trifluoromethylphenylalanine |
| Tyr | Hfe, Phg, Bhf, Thi, Bta, Bromophenylalanine, Iodophenylalanine, Chlorophenylalanine, Methylphenylalanine, Nitrophenylalanine, Tyr, Trp, Naphthylalanine, Trifluoromethylphenylalanine |

### Linear peptides

A general linear peptide is typically written from the N-to C-terminal direction as shown below:
NT-Xaa1-Xaa2-Xaa3-Xaa4-.......Xaan-CT;
Therein
1. Xaax is the abbreviation, descriptor or symbol for amino acids or building blocks at specific sequence position x as shown in Table 5,
2. NT is a N-terminal group, e.g. 'H' (Hydrogen for a free N-terminal amino group) or an abbreviation for a specific terminating carboxylic acid like 'Ac' for acetic acid or other chemical group or structural formula of chemical groups linked to the N-terminal amino acid code (Xaal) via a hyphen and
3. CT is a C-terminal group which is typically 'OH' or 'NH₂' (as terminal carboxylic acid or amide) or an abbreviation for a specific terminating amine linked to the C-terminal amino acid code (Xaan) via a hyphen.

### Branched peptides with side chains modified by specific building blocks or peptides

A general linear, branched peptide is written from the N-to C-terminal direction as shown below:
NT*-*Xaa1*-*Xaa2*-*Xaa3(*NT-Xab1-Xab2-........ Xabn*)-........Xaan-CT

Therein the statements 1. - 3. of the description of linear peptides for the specification of Xaax, NT and CT in the main chain of the branched peptide apply.

The position of a branch is specified by parentheses after a Xaax abbreviation. Branches typically occur at lysine (Lys) residues (or similar), which means that the branch is attached to side chain ε-amino function of the lysine via an amide bond.

The content of the parenthesis describes the sequence/structure of the peptide branch *'NT-Xab1-Xab2-........ Xabn'.* Herein
*1. Xabx* is the abbreviation, descriptor or symbol for amino acids or building blocks at specific sequence position x of the branch as shown in Table 3,
2. NT is a N-terminal group, e.g. an abbreviation for a specific terminating carboxylic acid like 'Ac' for acetic acid or other chemical group or structural formula of chemical groups linked to the N-terminal amino acid code (*Xab1*) via a hyphen and
3. the last building block of the branch *Xabn,* which connects the branch with the main chain by forming an amide bond with its own carboxyl function with the side chain amino function of this lysine (or similar residue).

### Cyclic peptides

An exemplaric general cyclic peptide written from the N-to C-terminal direction is shown below:
NT-Xaal-[Xaa2-Xaa3-Xaa4-.......Xaan]-CT;
Therein the statements 1. - 3. of the description of linear peptides for the specification of Xaax, NT and CT in the main chain of the cyclic peptide apply. The characteristics of the peptide cycle are specified by square brackets.
1. The opening square bracket indicates the building block at whose side chain the cycle is initiated (*cycle initiation residue*) and
2. the closing square bracket indicates the building block at whose side chain the cycle is terminated (*cycle termination residue*).

The chemical nature of the connection between these two residues is
1. an amide bond in case that among those indicated residues one residue contains an amino function its side chain (e.g. Lys) while the other contains a carboxyl function in its side chain (e.g. Glu) or
2. a disulphide bond in case that those indicated residues/amino acids contain sulfhydryl moieties (e.g. Cys).

### Cyclic peptides containing an additional cyclization element (Yc)

A general extended cyclic peptide written from the N-to C-terminal direction is shown below:
NT-Xaa1-[Xaa2(Yc)-Xaa3-Xaa4-.......Xaan]-CT;
Therein the statements 1. - 3. of the description of linear peptides for the specification of Xaax, NT and CT in the main chain of the cyclic peptide apply. In addition, Yc is the cyclization element. As in case of cyclic peptides the characteristics of the cycle are specified by square brackets which indicate *cycle initiation residue* and *cycle termination residue.*

The content of the parentheses adjacent to the cycle initiation residue specifies the cyclization element Yc within the extended peptide cycle. The Yc element is linked to the side chain of said residue. Furthermore, the Yc element is linked to the side chain of the *cycle termination residue.* The chemical nature of the linkages between either of these residues the Yc element depend on side chain functionality of the corresponding amino acids Xaan. The linkage is a thioether if the side chain of Xaan contains a sulfhydryl group (e.g., Cys).

As non-limiting example the structure of Ac-[Cys(tMeBn(DOTA-PP))-Pro-Pro-Thr-Gln-Phe-Cys]-OH is depicted below.

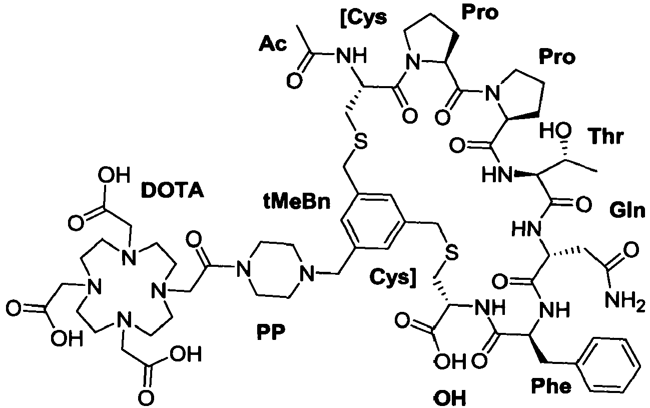

Therein
1. Ac corresponds to NT in the general formula.
2. Cys, Pro, Pro, Thr, Gln, Phe and Cys correspond to Xaa1 to Xaa7 in the general formula.
3. OH corresponds to CT in the general formula.
4. The opening square bracket (,[') adjacent to the N-terminal cysteine in the sequence indicates that at this residue the cycle is initiated (*cycle initiation residue*).
5. The closing square bracket (,]') adjacent to the N-terminal cysteine in the sequence indicates that at this residue the cycle is terminated (*cycle termination residue*).
6. tMeBn within the parentheses adjacent to the Cys indicated as initiation residue specifies the cyclization element Yc. It is further bound to the Cys indicated as cycle termination residue. The Yc element is connected to said residues via thioether linkages.
7. To the remaining connection point of the tMeBn residue a DOTA chelator is attacted via a PP linker. For clarity terms like "Cys(tMeBn(DOTA-PP))" are included in the list of chemical structures in table 2

In an embodiment of the present invention, an amino acid or a peptide is attached to Xaa7, wherein a majority of the amino acids of this peptide are charged or polar and the net charge of the peptide is -2, -1, 0, +1 or +2.

For calculation of peptide net charges negatively charged amino acids are amino acids which bear acidic groups like -COOH or -SO₃H in their side chain and their net charge corresponds to the number of acidic groups, e.g. Asp or Glu with net charge -1.

For this calculation positively charged amino acids are amino acids which bear basic groups like amino or -guanidino in their side chain and their net charge corresponds to the number of basic groups, e.g. Lys or Arg with net charge +1.

Polar amino acids are amino acids which bear polar groups in their side chain. The polar groups are such as CONH2, OH, F, Cl, CN, and heterocycles like for instance imidazole in histidine.

The polar amino acids have a net charge of 0. For some nitrogen containing heterocycles the net charge is considered as 0 for our calculation although it is acknowledged that depending on the pH of the environment it might be protonated in an equilibrium and therefore positively charged to a certain extent.

The majority (50% or more) of the amino acids of this peptide are charged or polar.

Preferably the positive or negative charges are occasionally separated by a polar or non-polar amino acid.

In some embodiments the presence of negative charged amino acid is preferred at Xaa10.

In some embodiments the presence of positively charged amino acid is preferred at Xaa13, preferably Arg and arg.

In accordance with the present invention, the compound of the present invention may comprise a Z group. The Z group comprises a chelator and optionally a linker. As preferably used, a linker is an element, moiety, or structure which separates two parts of a molecule. In the present invention, the linker group forms covalent bonds with both the chelator group and the respective part of the compounds of invention where Z is attached. The linker group may, in principle, be any chemical group which is capable of forming bonds with both the chelator group and the part of the compounds of invention at the specified positions.

An important property or feature of a linker is that it spaces apart the chelator and the cyclic peptide part of the compound of invention. This is especially important in cases where the target binding ability of the cyclic peptide is compromised by the close proximity of the chelator. However, the overall linker length in its most extended conformer should not exceed 200 Å, preferably not more than 150 Å and most preferably not more than 100 Å.

In a preferred embodiment, the linker is -[X]ₐ-, wherein a is an integer from 1 to 10, and each X is an individual building block which is connected independently to its neighbors in the sequence by a functional group selected from comprising an amide linkage, a urea linkage, a carbamate linkage, an ester linkage, an ether linkage, a thioether linkage, a sulfonamide, a triazole and a disulfide linkage.

X₁ is connected to the chelator- and, if present to X₂ or to the compounds of invention at the specified positions. Xₐ is connected, if present to Xₐ₋₁ and to the compounds of invention at the specified positions.

A more preferred class of linker groups is represented by is -[X]ₐ-, wherein a is an integer from 1 to 10, preferably, a is an integer from 1 to 8, 1 to 6, 1 to 5, 1 to 4 or 1 to 3, and each X is an individual building block which is connected independently to its neighbors in the sequence by a functional group selected from a group comprising an amide linkage, a urea linkage, a carbamate linkage, an ester linkage, an ether linkage, a thioether linkage, a sulfonamide linkage, a triazole linkage and a disulfide linkage.

In an embodiment the building block X is of general formula (8)

⁅ Lin²-R⁹-Lin³⁆ (8)

wherein,
fragment Lin², if present, and fragment Lin³, if present, are each individually and independently selected from the group comprising -CO-, -NR.¹⁰-, -S-, -CO-NR¹⁰-, -CS-NR¹⁰-, -O-, -succinimide- and -CH₂-CO-NR¹⁰-; under the proviso that at least one of Lin² or Lin³ is linked to R⁹ with a carbon atom and the nitrogen atom of all nitrogen containing fragments is linked to R⁹_{;}
wherein R¹⁰ is selected from the group consisting of hydrogen and (C₁-C₄)alkyl;
and wherein R⁹ is selected from -(C₁-C₁₀)alkylidene-, -(C₃-C₈)carbocyclo-, -arylene-, -(C₁-C₁₀)alkylidene-arylene-, -arylene-(C₁-C₁₀)alkylidene-, -(C₁-C₁₀)alkylidene-arylene-(C₁-C₁₀)alkylidene-, -(C₁-C₁₀)alkylidene-(C₃-C₈)carbocyclo-, -(C₃-C₈)carbocyclo-(C₁-C₁₀)alkylidene-, -(C₁-C₁₀alkylidene-(C₃-C₈)carbocyclo-(C1-C₁₀)alkylidene-, -(C₃-C₈)heterocyclo-, (C₁-C₁₀)alkylidene-(C₃-C₈)heterocyclo-, -(C₃-C₈)heterocyclo-(C₁-C₁₀)alkylidene-, -(C₁-C₁₀)alkylidene-(C₃-C₈)heterocyclo-(C₁-C₁₀)alkylidene-, -(CH₂CH₂O)ᵣ-, and -(CH₂)ₛ,-(CH₂CH₂O)ᵣ-(CH₂)ₜ-;
and wherein
r is any integer from 1, 2, 3, 4, 5, 6, 7, 8, 9 and 10;
s is any integer from 0, 1, 2, 3 and 4; and
t is any integer from 0, 1, 2, 3 and 4.

Preferably, apart from the linkage between X₁ and the chelator, the linkage is an amide linkage. More preferably building block X₂ to Xₐ are independently selected from the group of comprising an amino acid, a dicarboxylic acid and a diamine and the respective linkages are amides.

In an embodiment the building block X₂ to Xₐ is preferably an amino acid, wherein the amino acid is selected from the group comprising conventional and unconventional amino acids. In an embodiment an amino acid is one selected from the group comprising β-amino acids, γ-amino acids, δ-amino acids, ε-amino acids and ω-amino acids. In a further embodiment an amino acid is a cyclic amino acid or a linear amino acid. It will be appreciated by a person skilled in the art that in case of an amino acid with stereogenic centers all stereoisomeric forms may be used in the building block X.

In an embodiment the building block X₂ to Xₐ is preferably an amino acid, wherein the amino acid is selected from a group comprising amino acids which differ as to the spacing of the amino group from the carboxylic group. This kind of amino acid can be generically represented as follows:

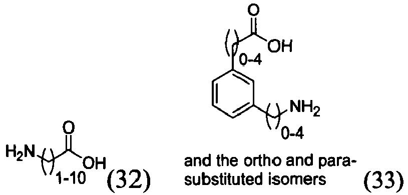

It is within the present invention that such amino acid is not further substituted. It is, however, also within the present invention that such amino acid is further substituted; preferably such substitution is CO-NH₂ and/or Ac-NH-.

Representative of this kind of amino acid (structure 32) which can be used as a building block X are glycine (Gly), β-alanine (Bal), y-aminobutyric acid (GABA), aminopentanoic acid, aminohexanoic acid and homologs with up to 10 CH₂ groups.

Representative of this kind of amino acid (structure 33) which are more preferably used as a building block X are 3-aminomethyl-benzoic acid, 4-aminomethyl-benzoic acid, anthranilic acid, 3-amino benzoic acid and 4-amino benzoic acid.

Relevant building blocks are diamines which are derived from amino acids (structure 32 + 33) by replacing NH₂ with COOH, which are preferably used as a building block X are diamino ethane, 1,3-diamino propane, 1,4-diamino butane, 1,5-diamino pentane, 3-aminomethyl-aniline, 4-aminomethyl-aniline, 1,2-diamino benzene, 1,3-diamino benzene and 1,4-diamino benzene.

Relevant building blocks are dicarboxylic acids which are derived from amino acids (structure 32 + 33) by replacing COOH with NH₂, which are more preferably used as a building block X are malonic acid, succinic acid, glutaric acid, adipic acid, phthalic acid, terephthalic acid, isophthalic acid and 2, 3 or 4 carboxy-phenyl acetic acid.

In a further embodiment, the amino acid is an amino acid which contains, preferably as a backbone, a polyether. Preferably such polyether is polyethylene glycol and consists of up to 30 monomer units. Preferably, an amino acid comprising such polyether shows an increase in hydrophilicity compared to an amino acid not comprising such polyether. If incorporated into a building block X and, ultimately, into a linker group [X]ₐ, the result is typically an increase in hydrophilicity. A preferred embodiment of this kind of amino acid is depicted in the following, wherein it will be acknowledged that such amino acid may comprise 0, 1, 2, 3, 4, 5, 6, 7, 8, 9, 10, 11 or 12 ethylene oxide moieties:

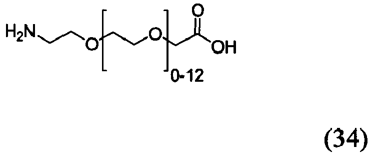

Preferred ethylene glycol containing amino acids are Ttds (N-(3-{2-[2-(3-Amino-propoxy)-ethoxy]-ethoxy}-propyl)-succinamic acid) and O2Oc ([2-(2-Amino-ethoxy)-ethoxy]-acetic acid) the formula of which is as follows:

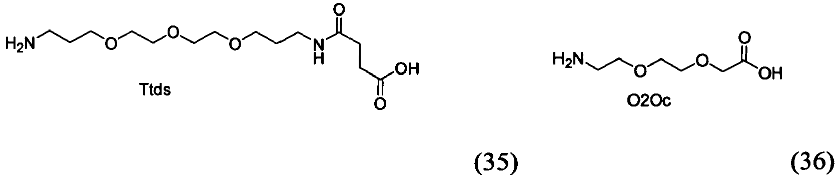

In preferred embodiments, the linker comprises an oligomer or a monomer of only one specific amino acid selected from the group of Ttds, O2Oc, Apac, Gly, Bal, Gab, Mamb, Pamb, Ppac, 4Amc, Inp, Sni, Rni, Nmg, Cmp, PEG6, PEG12, PEG-amino acids and more preferably the linker is monomeric.

In another preferred embodiment, the linker comprises one building block X₂ selected from the group of Ttds, O2Oc, Apac, Gly, Bal, Gab, Mamb Pamb, PEG6, PEG12 and PEG-amino acids and a second building block X₁ which is directly bound to the amino-nitrogen of X₂ and is directly attached to a chelator by a linkage selected from the group consisting of an amide linkage, a urea linkage, a carbamate linkage, an ester linkage, an ether linkage, a thioether linkage, a sulfonamide, a triazole and a disulfide linkage. X₁ serves in this case as adapter to mediate the linkage of the different kind of attachment functionalities provided by a chelator to the nitrogen-atom of the amino acid X₂ in the sense that X₁ provides relevant complementary functionalities for the linkage of the chelator.

However, the use of linkers usually follows a purpose. In some circumstances it is necessary to space a larger moiety apart from a bioactive molecule in order to retain high bioactivity. In other circumstances introduction of a linker opens the chance to tune physicochemical properties of the molecule by introduction of polarity or multiple charges. In certain circumstances it might be a strength and achievement if one can combine the chelator with a bioactive compound without the need for such linkers. Especially in those compounds of the present invention where the chelator is attached to Yc of formula (X) linking the S atom of Xaa1 and the S atom of Xaa7 under the formation of two thioether linkages typically perform excellently without the use of any dedicated linkers.

In an embodiment, the compound of the invention comprises a chelator. Preferably, the chelator is part of the compound of the invention, whereby the chelator is either directly or indirectly such as by a linker attached to the compound of the invention. A preferred chelator is a chelator which forms metal chelates preferably comprising at least one radioactive metal. The at least one radioactive metal is preferably useful in or suitable for diagnostic and/or therapeutic and/or theraognostic use and is more preferably useful in or suitable for imaging and/or radiotherapy.

Chelators in principle useful in and/or suitable for the practicing of the instant invention including diagnosis and/or therapy of a disease are known to the person skilled in the art. A wide variety of respective chelators is available and has been reviewed, e.g. by Banerjee et al. (Banerjee, et al., Dalton Trans, 2005, 24: 3886), and references therein (Price, et al., Chem Soc Rev, 2014, 43: 260; Wadas, et al., Chem Rev, 2010, 110: 2858). Such chelators include, but are not limited to linear, cyclic, macrocyclic, tetrapyridine, N3S, N2S2 and N4 chelators as disclosed in US 5,367,080 A, US 5,364,613 A, US, 5,021,556 A, US 5,075,099 A and US 5,886,142 A.

Representative chelating agents and their derivatives include, but are not limited to AAZTA, BAT, CDTA, DTA, DTPA, CY-DTA, DTCBP, CTA, cyclam, cyclen, TETA, Sarcophagine, CPTA, TEAMA, Cyclen, DO3A, DO2A, TRITA, DATA, DFO, DATA(M), DATA(P), DATA(Ph), DATA(PPh), DEDPA, H₄octapa, H2dedpa, H₅decapa, H₂azapa, H2CHX DEDPA, DFO-Chx-MAL, DFO-p-SCN, DFO-1AC, DFO-BAC, p-SCN-Bn-DFO, DFO-pPhe-NCS, DFO-HOPO, DFC, Diphosphine, DOTA, DOTAGA, , DOTA-MFCO, DOTAM-mono-acid, nitro-DOTA, nitro-PA-DOTA, p-NCS-Bz-DOTA, PA-DOTA, DOTA-NCS, DOTA-NHS, CB-DO2A, PCTA, p-NH₂-Bn-PCTA, p-SCN-Bn-PCTA, p-SCN-Bn-DOTA, DOTMA, NB-DOTA, H4NB-DOTA, H4TCE-DOTA, 3,4,3-(Li-1,2-HOPO), TREN(Me-3,2-HOPO), TCE-DOTA, DOTP, DOXP, p-NCS-DOTA, p-NCS-TRITA, TRITA, TETA, 3p-C-DEPA, 3p-C-DEPA-NCS, p-NH2-BN-OXO-DO3A, p-SCN-BN-TCMC, TCMC, 4-Aminobutyl-DOTA, Azido-mono-amide-DOTA, BCN-DOTA, Butyne-DOTA, BCN-DOTA-GA, DOA3P, DO2a2p, DO2A(trans-H2do2a), DO3A, DO3A-Thiol, DO3AtBu-N-(2-aminoethyl)ethanamide, DO2AP, CB-DO2A, C3B-DO2A, HP-DO3A, DOTA-NHS-ester, Maleimide-DOTA-GA, Maleimido-mono-aminde-DOTA, Maleimide-DOTA, NH2-DOTA-GA, NH2-PEG4-DOTA-GA, GA, p-NH₂-Bn-DOTA, p-NO2-Bn-DOTA, p-SCN-Bn-DOTA, p-SCN-Bz-DOTA, TA-DOTA, TA-DOTA-GA, OTTA, DOXP, TSC, DTC, DTCBP, PTSM, ATSM, H2ATSM, H2PTSM, Dp44mT, DpC, Bp44mT, QT, hybrid thiosemicarbazone-benzothiazole, thiosemicarbazone-styrylpyridine tetradentate ligands H₂L²⁻⁴, HBED, HBED-CC, dmHBED, dmEHPG, HBED-nn, SHBED, Br-Me2HBED, BPCA, HEHA, BF-HEHA, Deferiprone, THP, HYNIC (2-hydrazino nicotinamide), NHS-HYNIC, HYNIC-Kp-DPPB, HYNIC-Ko-DPPB, (HYNIC)(tricine)2, (HYNIC)(EDDA)C1, p-EDDHA, AIM, AIM A,IAM B, MAMA, MAMA-DGal, MAMA-MGal, MAMA-DA, MAMA-HAD, Macropa, Macropaquin, Macroquin-SO3, NₓS₄₋ₓ, N2S2, N3S, N4, MAG3B, NOTA, NODAGA, SCN-Bz-NOTA-R, NOT-P (NOTMP), NOTAM, p-NCS-NOTA, TACN, TACN-TM, NETA, NETA-monoamine, p-SCN-PhPr-NE3TA, C-NE3TA-NCS, C-NETA-NCS, 3p-C-NETA, NODASA, NOPO, NODA, NO2A, N-Benzyl-NODA, C-NOTA, BCNOT-Monoamine, Maleimido-mono-amide-NOTA, NO2A-Azide, NO2A-Butyne, NO2AP, NO3AP, N-NOTA, Oxo-DO3A, p-NH2-Bn-NOTA, p-NH₂-Bn-oxo-DO3A, p-NO2-Bn-Cyclen, p-SCN-Bn-NOTA, p-SCN-Bn-oxo-DO3A, TRAP, PEPA, BF-PEPA, Pycup, Pycup2A, pycup1A1Bn, pycup2Bn, SarAr-R, Diamsar, AmBaSar-R, siamSar, Sar, Tachpyr, tachpyr-(6-Me), TAM A, TAM B, TAME, TAME-Hex, THP-Ph-NCS, THP-NCS, THP-TATE, NTP, H3THP, THPN, CB-TE2A, PCB-TE1A1P,_TETA-NHS, CPTA, CPTA-NHS, CB-TE1K1P, CB-TE2A, TE2A, H2CB-TE2A, TE2P, CB-TE2P, MM-TE2A, DM-TE2A, , 2C-TETA, 6C-TETA, BAT, BAT-6, NHS-BAT ester, SSBAT, SCN-CHX-A-DTPA-P, SCN-TETA, TMT-amine, p-BZ-HTCPP.

HYNIC, DTPA, EDTA, DOTA, TETA, bisamino bisthiol (BAT) based chelators as disclosed in US 5,720,934; Desferrioxamin (DFO) as disclosed in (Doulias, et al., Free Radic Biol Med, 2003, 35: 719), tetrapyridine and N₃S, N₂S₂ and N₄ chelators as disclosed in US 5,367,080 A, US 5,364,613 A, US 5,021,556 A, US 5,075,099 A, US 5,886,142 A, whereby all of the references are included herein by reference in their entirety. 6-Amino-6-methylperhydro-1,4-diazepine-*N,N',N",N"* -tetraacetic acid (AAZTA) is disclosed in Pfister et al., (Pfister, et al., EJNMMI Res, 2015, 5: 74), Deferiprone, a 1,2-dimethyl-3,4-hydroxypyridinone and Hexadentate tris(3,4-hydroxypyridinone) THP) are disclosed in Cusnir et al. (Cusnir, et al., Int J Mol Sci, 2017, 18), monoamine-monoamide dithiol (MAMA)-based chelators are disclosed in Demoin et al. (Demoin, et al., Nucl Med Biol, 2016, 43: 802), MACROPA and analogues are disclosed in Thiele et al. (Thiele, et al., Angew Chem Int Ed Engl, 2017, 56: 14712), 1,4,7,10,13,16-hexaazacyclohexadecane-N,N',N",N"',N"",N""'-hexaacetic acid (HEHA) and PEPA analogues are disclosed in Price and Orvig (Price, et al., Chem Soc Rev, 2014, 43: 260), Pycup and analogous are disclosed in Boros et al. (Boros, et al., Mol Pharm, 2014, 11: 617), N, N-bis(2-hydroxybenzyl)ethylenediamine-N,N-diacetic acid (HBED), 1,4,7,10-tetrakis (carbamoylmethyl) -1,4,7,10-tetraazacyclododecane (TCM), 2-[(carboxymethyl)]-[5-(4-nitrophenyl-1-1-[4,7,10-tris-(carboxymethyl)-1,4,7,10-tetraazacyclododecan-1-yl]pentan-2-yl)-amino]acetic acid (3p-C-DEPA), CB-TE2A, TE2A, TE1A1P, Diamsar, I-N-(4-Aminobenzyl)-3,6,10,13,16,19-hexaazabicyclo[6.6.6]-eicosane-1,8-diamine (SarAr), NETA, N,N0,N00, tris(2-mercaptoethyl)-1,4,7-triazacyclononane (TACN-TM), {4-[2-(Bis-carboxymethyl-amino)-ethyl]-7-carboxymethyl-[1,4,7]triazonan-1-yl}-acetic acid (NETA), diethylenetriaminepentaacetic acid (DTP), 3-({4,7-Bis-[(2-carboxy-ethyl)-hydroxy-phosphinoylmethyl]-[1,4,7]triazonan-1-ylmethyl}-hydroxy-phosphinoyl)-propionic acid (TRAP), NOPO, H4octapa, SHBED, BPCA, 3,6,9,15-tetraazabicyclo[9.3.1]-pentadeca-1(15),11,13-triene-3,6,9,-triacetic acid (PCTA), and 1,4,7,10,13-pentaazacyclopentadecane-N,N',N",N"',N""-pentaacetic acid (PEPA) are disclosed in Price and Orvig (Price, et al., Chem Soc Rev, 2014, 43: 260), 1-hydroxy-2-pyridone ligand (HOPO) is disclosed in Allott et al. (Allott, et al., Chem Commun (Camb), 2017, 53: 8529), [4-Carboxymethyl-6-(carboxymethyl-methyl-amino)-6-methyl-[1,4] diazepan-1-yl]-acetic acid (DATA) is disclosed in Tornesello et al. (Tornesello, et al., Molecules, 2017, 22: 1282), tetrakis(aminomethyl)methane (TAM) and analogues are disclosed in McAuley 1988 (McAuley, et al., Canadian Journal of Chemistry, 1989, 67: 1657), Hexadentate tris(3,4-hydroxypyridinone) (THP) and analogues are disclosed in Ma et al. (Ma, et al., Dalton Trans, 2015, 44: 4884).

The diagnostic and/or therapeutic use of some of the above chelators is described in the prior art. For example, 2-hydrazino nicotinamide (HYNIC) has been widely used in the presence of a coligand for incorporation of ^{99m}Tc and ^{186,188}Re (Schwartz, et al., Bioconjug Chem, 1991, 2: 333; Babich, et al., J Nucl Med, 1993, 34: 1964; Babich, et al., Nucl Med Biol, 1995, 22: 25); DTPA is used in Octreoscan® for complexing ¹¹¹In and several modifications are described in the literature (Li, et al., Nucl Med Biol, 2001, 28: 145; Brechbiel, et al., Bioconjug Chem, 1991, 2: 187); DOTA type chelators for radiotherapy applications are described by Tweedle *et al.* (US Pat 4,885,363); other polyaza macrocycles for chelating trivalent isotopes metals are described by Eisenwiener *et al.* (Eisenwiener, et al., Bioconjug Chem, 2002, 13: 530); and N₄-chelators such as a ^{99m}Tc-N₄-chelator have been used for peptide labeling in the case of minigastrin for targeting CCK-2 receptors (Nock, et al., J Nucl Med, 2005, 46: 1727).

In an embodiment the metal chelator is selected from the group, but not limited to, comprising DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, NₓS₄₋ₓ (N4, N2S2, N3S), Hynic, ^{99m}Tc(CO)₃-Chelators and their analogs, wherein
DOTA stands for 1,4,7,10-tetrazacyclododecane-1,4,7,10-tetraacetic acid,
DOTAGA stand for 1,4,7,10-tetraazacyclodocecane,1-(glutaric acid)-4,7,10-triacetic acid,
NOTA stands for 1,4,7-triazacyclononanetriacetic acid,
NODAGA stands for 1,4,7-triazacyclononane-N-glutaric acid-N',N"-diacetic acid,
NODA-MPAA stands for 1,4,7-triazacyclononane-1,4-diacetate-methyl phenylacetic acid,
HBED stands for bis(2-hydroxybenzyl) ethylenediaminediacetic acid,
TETA stands for 1,4,8,11-tetraazacyclododecane-1,4,8,11-tetraacetic acid,
CB-TE2A stands for 4,11-bis-(carboxymethyl)-1,4,8,11-tetraazabicyclo[6.6.2]-hexadecane,
DTPA stands for diethylenetriaminepentaacetic acid,
DFO stands for the Desferal or Desferrioxamine type group of chelators, the chemical name of the non-limiting example is N-[5-({3-[5-(Acetyl-hydroxy-amino)-pentylcarbamoyl]-propionyl}-hydroxy-amino)-pentyl]-N'-(5-amino-pentyl)-N'-hydroxy-succinamide,
Macropa stands for N,N'-bis[(6-carboxy-2-pyridyl)methyl]-4,13-diaza-18-crown,
HOPO stands for the octadentate hydroxypyridinone type group of chelators, the structure of a non-limiting example is shown below,
TRAP stands for 3-({4,7-Bis-[(2-carboxy-ethyl)-hydroxy-phosphinoylmethyl]-[1,4,7]triazonan-1-ylmethyl} -hydroxy-phosphinoyl)-propionic acid,
THP stands for Hexadentate tris(3,4-hydroxypyridinone),
DATA stands for [4-Carboxymethyl-6-(carboxymethyl-methyl-amino)-6-methyl-[1,4]diazepan-1-yl]-acetic acid
NOTP stands for 1,4,7-triazacyclononane-N,N'N"-tris(methylene phosphonic)acid),
Sarcophagine stands for 3,6,10,13,16,19-hexaazabicyclo[6.6.6]icosane,
FSC stands for 3,15,27-Triamino-7,19,31-trihydroxy-10,22,34-trimethyl-1,13,25-trioxa-7,19,31-triaza-cyclohexatriaconta-9,21,33-triene-2,8,14,20,26,32-hexaone,
NETA, {4-[2-(Bis-carboxymethyl-amino)-ethyl]-7-carboxymethyl-[1,4,7]triazonan-1-yl}-acetic acid
H4octapa, *N,N'*-(6-carboxy-2-pyridylmethyl)-*N*,*N*'-diacetic acid-1,2-diaminoethane Pycup stands for 1,8-(2,6-Pyridinedimethylene)-1,4,8,11-tetraazacyclo-tetradecane,
NₓS₄₋ₓ(N4, N2S2, N3S) stands for a group of tetradentate chelators with N-atoms (basic amine or non-basic amide) and thiols as donors stabilizing Tc-complexes, especially Tc(V)-oxo complexes. The structure of one representative non-limiting example MAG3 is shown below, and
MAG3 stands for {2-[2-(3-Mercapto-propionylamino)-acetylamino]-acetylamino}-acetic acid,
HYNIC stands for 6-Hydrazino-nicotinic acid,
^{99m}Tc(CO)₃-Chelators stands for bi- or tridendate chelators capable of forming stable complexes with technetium tricarbonyl fragments,
and with the chemical structures thereof being as follows:

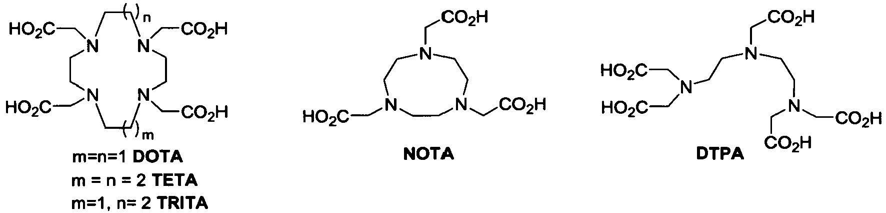

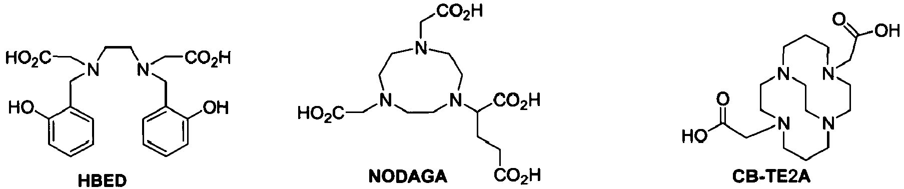

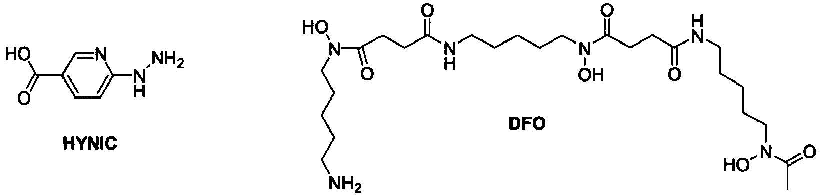

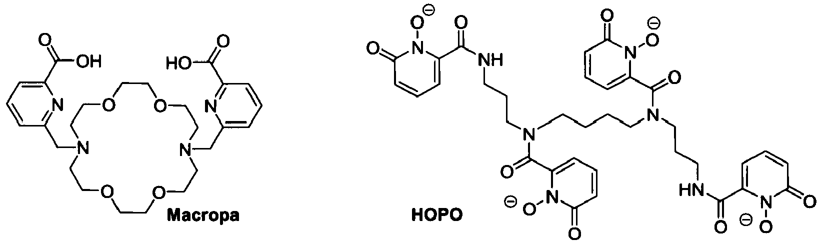

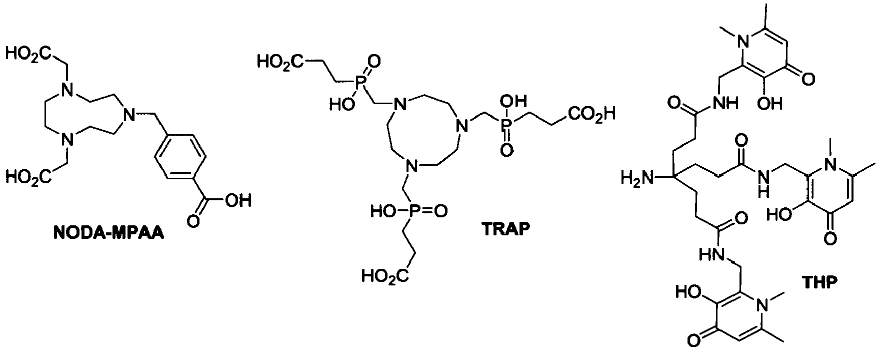

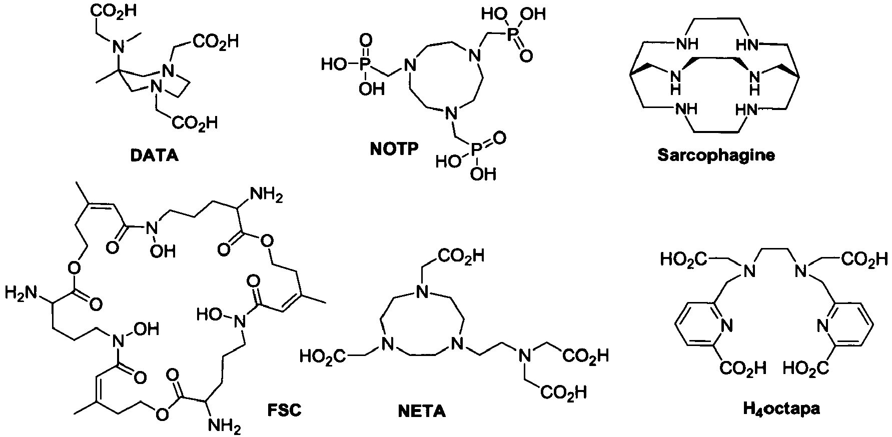

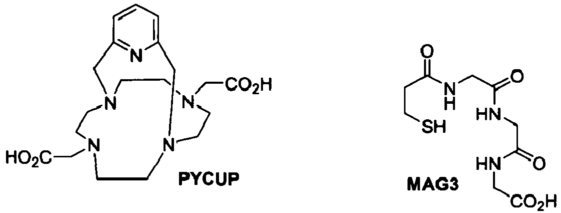

In a preferred embodiment, the metal chelator is selected from the group consisting of DOTA, DOTAGA, NOTA, NODAGA, NODA-MPAA, HBED, CB-TE2A, DFO, THP, N4 and analogs thereof.

In a more preferred embodiment, the metal chelator is selected from the group consisting of DOTA, DOTAGA, NOTA and NODAGA and their analogs thereof.

It will be acknowledged by the persons skilled in the art that the chelator, in principle, may be used regardless whether the compound of the invention is used in or suitable for diagnosis or therapy. Such principle is, among others, outlined in international patent application WO 2009/109332 A1.

It will be further acknowledged by the persons skilled in the art that the presence of a chelator in the compound of the invention includes, if not stated otherwise, the possibility that the chelator is complexed to any metal complex partner, i.e. any metal which, in principle, can be complexed by the chelator. An explicitly mentioned chelator of a compound of the invention or the general term chelator in connection with the compound of the invention refers either to the uncomplexed chelator as such or to the chelator to which any metal complex partner is bound, wherein the metal complex partner is any radioactive or non-radioactive metal complex partner. Preferably the chelator metal complex, i.e. the chelator to which the metal complex partner is bound, is a stable chelator metal complex.

Non-radioactive chelator metal complexes have several applications, e.g. for assessing properties like stability or activity which are otherwise difficult to determine. One aspect is that cold variants of the radioactive versions of the metal complex partner (e.g. non-radioactive Gallium, Lutetium or Indium complexes es described in the examples) can act as surrogates of the radioactive compounds. Furthermore, they are valuable tools for identifying metabolites *in vitro* or *in vivo,* as well as for assessing toxicity properties of the compounds of invention. Additionally, chelator metal complexes can be used in binding assays utilizing the fluorescence properties of some metal complexes with distinct ligands (e.g. Europium salts).

Chelators can be synthesized or are commercially available with a wide variety of (possibly already activated) groups for the conjugation to peptides or amino acids. Direct conjugation of a chelator to an amino-nitrogen of the respective compound of invention is well possible for chelators selected from the group consisting of DOTA, DOTAGA, NOTA, NODAGA, NODA-MPAA, HBED, TETA, CB-TE2A, DTPA, DFO, DATA, sarcophagine, N4, MAG3 and Hynic, preferably DOTA, DOTAGA, NOTA, NODAGA, NODA-MPAA, CB-TE2A, and N4. The preferred linkage in this respect is an amide linkage.

Functional groups at a chelator which are ideal precursors for the direct conjugation of a chelator to an amino-nitrogen are known to the person skilled in the art and include but are not limited to carboxylic acid, activated carboxylic acid, e.g. active ester like for instance NHS-ester, pentafluorophenol-ester, HOBt-ester and HOAt-ester, isothiocyanate.

Functional groups at a chelator which are ideal precursors for the direct conjugation of a chelator to a carboxylic group of a peptide are known to the person skilled in the art and include but are not limited to alkylamino and arylamino nitrogens. Respective chelator reagents are for commercially available some chelators, e.g. for DOTA with either alkylamino or arylamino nitrogen.

It will be acknowledged by a person skilled in the art that the radioactive nuclide which is or which is to be attached to the compound of the invention, is selected taking into consideration the disease to be treated and/or the disease to be diagnosed, respectively, and/or the particularities of the patient and patient group, respectively, to be treated and to be diagnosed, respectively.

In an embodiment of the present invention, the radioactive nuclide is also referred to as radionuclide. Radioactive decay is the process by which an atomic nucleus of an unstable atom loses energy by emitting ionizing particles (ionizing radiation). There are different types of radioactive decay. A decay, or loss of energy, results when an atom with one type of nucleus, called the parent radionuclide, transforms to an atom with a nucleus in a different state, or to a different nucleus containing different numbers of protons and neutrons. Either of these products is named the daughter nuclide. In some decays the parent and daughter are different chemical elements, and thus the decay process results in nuclear transmutation (creation of an atom of a new element). For example, the radioactive decay can be alpha decay, beta decay, and gamma decay. Alpha decay occurs when the nucleus ejects an alpha particle (helium nucleus). This is the most common process of emitting nucleons, but in rarer types of decays, nuclei can eject protons, or specific nuclei of other elements (in the process called cluster decay). Beta decay occurs when the nucleus emits an electron (β⁻-decay) or positron (β⁺-decay) and a type of neutrino, in a process that changes a proton to a neutron or the other way around. By contrast, there exist radioactive decay processes that do not result in transmutation. The energy of an excited nucleus may be emitted as a gamma ray in gamma decay, or used to eject an orbital electron by interaction with the excited nucleus in a process called internal conversion, or used to absorb an inner atomic electron from the electron shell whereby the change of a nuclear proton to neutron causes the emission of an electron neutrino in a process called electron capture (EC), or may be emitted without changing its number of proton and neutrons in a process called isomeric transition (IT). Another form of radioactive decay, the spontaneous fission (SF), is found only in very heavy chemical elements resulting in a spontaneous breakdown into smaller nuclei and a few isolated nuclear particles.

In a preferred embodiment of the present invention, the radionuclide can be used for labeling of the compound of the invention.

In an embodiment of the present invention, the radionuclide is suitable for complexing with a chelator, leading to a radionuclide chelate complex.

In a further embodiment one or more atoms of the compound of the invention are of non-natural isotopic composition, preferably these atoms are radionuclides; more preferably radionuclides of carbon, oxygen, nitrogen, sulfur, phosphorus and halogens: These radioactive atoms are typically part of amino acids, in some case halogen containing amino acids, and/or building blocks and in some cases halogenated building blocks each of the compound of the invention.

In a preferred embodiment of the present invention, the radionuclide has a half-life that allows for diagnostic and/or therapeutic medical use. Specifically, the half-life is between 1 min and 100 days.

In a preferred embodiment of the present invention, the radionuclide has a decay energy that allows for diagnostic and/or therapeutic medical use. Specifically, for γ-emitting isotopes, the decay energy is between 0.004 and 10 MeV, preferably between 0.05 and 4 MeV, for diagnostic use. For positron-emitting isotopes, the decay energy is between 0.6 and 13.2 MeV, preferably between 1 and 6 MeV, for diagnostic use. For particle-emitting isotopes, the decay energy is between 0.039 and 10 MeV, preferably between 0.4 and 6.5 MeV, for therapeutic use.

In a preferred embodiment of the present invention, the radionuclide is industrially produced for medical use. Specifically, the radionuclide is available in GMP quality.

In a preferred embodiment of the present invention, the daughter nuclide(s) after radioactive decay of the radionuclide are compatible with the diagnostic and/or therapeutic medical use. Furthermore, the daughter nuclides are either stable or further decay in a way that does not interfere with or even support the diagnostic and/or therapeutic medical use. Representative radionuclides which may be used in connection with the present invention are summarized in Table 13.

**Table 13: Key properties of relevant radionuclides - half life, decay types and decay energies**

| Radionuclide | Half-life (min) | Half-life (hours) | Half-life (days) | Decay | Energy (MeV) | Additional decays (energy [MeV]) |
|---|---|---|---|---|---|---|
| **Carbon** | | | | | | |
| C-11 | 20.4 | 0.34 | | ECβ⁺ | 1.982 | |

| **Nitrogen** | | | | | | |
|---|---|---|---|---|---|---|
| N-13 | 9.97 | 0.17 | | ECβ+ | 2.220 | |

| Oxygen | | | | | | |
|---|---|---|---|---|---|---|
| O-15 | 2.00 | | | ECβ+ | 2.754 | |

| **Fluorine** | | | | | | |
|---|---|---|---|---|---|---|
| F-18 | 110 | 1.83 | | β+ | 1.656 | |
| Mg-28 | | 20.9 | | β- | 1.832 | |

| **Aluminum** | | | | | | |
|---|---|---|---|---|---|---|
| Al-28 | 2.24 | 0.04 | | β- | 4.642 | |
| Al-29 | 6.56 | | | β- | 3.690 | |

| **Silicon** | | | | | | |
|---|---|---|---|---|---|---|
| Si-31 | 157 | 2.62 | | β- | 1.492 | |

| **Phosphorus** | | | | | | |
|---|---|---|---|---|---|---|
| P-30 | 2.50 | 0.04 | | β+ | 4.232 | |
| P-32 | | | 14.3 | β- | 1.170 | |
| P-33 | | | 25.4 | β- | 0.077 | |

| **Sulphur** | | | | | | |
|---|---|---|---|---|---|---|
| S-35 | | | 87.4 | β- | 0.167 | |
| S-37 | 5.00 | 0.08 | | | | |
| S-38 | | 2.80 | | β- | 2.937 | |

| **Chlorine** | | | | | | |
|---|---|---|---|---|---|---|
| Cl-34m1 | 32.0 | 0.53 | | EC | 5.693 | |
| Cl-38 | 37.2 | 0.62 | | β- | 4.917 | |
| Cl-39 | 55.6 | 0.93 | | β- | 3.422 | |

| **Scandium** | | | | | | |
|---|---|---|---|---|---|---|
| Sc-43 | | 3.89 | | EC | 2.221 | |
| Sc-44 | | 3.97 | | β+ | 0.632 | |
| Sc-44m1 | | 58.6 | 2.44 | IT | 0.271 | 98.8% IT (0.27086), 1.2% EC (3.924) |
| Sc-46 | | | 83.8 | β- | 2.367 | |
| Sc-47 | | 80.4 | 3.35 | β- | 0.601 | |
| Sc-48 | | 43.7 | 1.82 | β- | 3.988 | |
| Sc-49 | 57.4 | 0.96 | | β- | 2.002 | |

| **Titanium** | | | | | | |
|---|---|---|---|---|---|---|
| Ti-45 | 185 | 3.08 | | EC | 2.062 | |
| Ti-51 | 5.76 | | | β- | 2.472 | |

| **Vanadium** | | | | | | |
|---|---|---|---|---|---|---|
| V-47 | 32.6 | 0.54 | | β+ | 2.931 | |
| V-48 | | | 16.2 | EC | 4.013 | |
| V-49 | | | 330 | EC | 0.602 | |
| V-52 | 3.74 | | | β- | 3.975 | |

| **Chromium** | | | | | | |
|---|---|---|---|---|---|---|
| Cr-48 | | 23.0 | | EC | 1.655 | |
| Cr-49 | 42.1 | 0.70 | | β+ | 2.628 | |
| Cr-51 | | | 27.7 | EC | 0.753 | |
| Cr-55 | 3.50 | | | β- | 2.603 | |
| Cr-56 | 5.94 | | | β- | 1.630 | |

| **Manganese** | | | | | | |
|---|---|---|---|---|---|---|
| Mn-51 | 46.2 | 0.77 | | β+ | 2.185 | |
| Mn-52m1 | 21.1 | 0.35 | | EC | 5.091 | 98.25% EC (5.091), 1.75% IT (0.3796) |
| Mn-52 | | | 5.59 | β+ | 3.689 | |
| Mn-54 | | | 312 | EC | 1.377 | |
| Mn-56 | | 2.58 | | β- | 3.696 | |
| **Iron** | | | | | | |
| Fe-52 | | 8.28 | | EC | 2.375 | |
| Fe-53m1 | | 2.54 | | IT | 3.042 | |
| Fe-53 | | 8.51 | | EC | 3.742 | |
| Fe-59 | | | 44.5 | β- | 1.565 | |
| Fe-61 | | 5.98 | | β- | 3.977 | |

| **Cobalt** | | | | | | |
|---|---|---|---|---|---|---|
| Co-55 | | 17.5 | | EC | 3.451 | |
| Co-56 | | | 78.8 | EC | 4.567 | |
| Co-57 | | | 271 | EC | 0.836 | |
| Co-58m1 | | 9.15 | | IT | 0.026 | |
| Co-58 | | | 70.8 | EC | 2.308 | |
| Co-60m1 | 10.5 | 0.17 | | IT | 0.059 | 99.76% IT (0.05932), 0.24% β- (2.882) |
| Co-61 | | 1.65 | | β- | 1.324 | |
| Co-62m1 | 13.9 | 0.23 | | β- | 5.337 | |

| **Nickel** | | | | | | |
|---|---|---|---|---|---|---|
| Ni-56 | | 146 | 6.10 | EC | 2.133 | |
| Ni-57 | | 36.1 | 1.50 | β+ | 3.262 | |
| Ni-63 | | | | β- | 0.067 | |
| Ni-65 | | 2.52 | | β- | 2.138 | |
| Ni-66 | | 54.6 | 2.28 | β- | 0.252 | |

| **Copper** | | | | | | |
|---|---|---|---|---|---|---|
| Cu-60 | 23.2 | 0.39 | | EC | 6.128 | |
| Cu-61 | | 3.41 | | EC | 2.238 | |
| Cu-62 | 9.74 | 0.16 | | EC | 3.959 | |
| Cu-64 | | 12.7 | | β+ | 0.653 | 61.5% EC (1.674), 38.5% β- (0.5797) |
| Cu-66 | 5.10 | 0.09 | | β- | 2.641 | |
| Cu-67 | | | 2.58 | β- | 0.580 | |
| Cu-68m1 | 3.75 | | | IT | 0.722 | 84 % IT (0.72163), 16% β- (5.162) |
| Cu-69 | 2.85 | | | β- | 2.681 | |

| **Zinc** | | | | | | |
|---|---|---|---|---|---|---|
| Zn-60 | 2.38 | | | EC | 4.171 | |
| Zn-62 | | 9.26 | | EC | 1.620 | |
| Zn-63 | 38.1 | 0.64 | | EC | 3.366 | |
| Zn-65 | | | 244 | EC | 1.352 | |
| Zn-69m1 | | 13.8 | | IT | 0.438 | 99.997% IT (0.43818), 0.003% β- (1.348) |
| Zn-69 | 57.0 | 0.95 | | β- | 0.910 | |
| Zn-71m1 | | 3.92 | | β- | 2.970 | 99.95% β- (2.97), 0.05% IT (0.15986) |
| Zn-71 | 2.45 | | | β- | 2.810 | |
| Zn-72 | | 46.5 | 1.94 | β- | 0.443 | |

| **Gallium** | | | | | | |
|---|---|---|---|---|---|---|
| Ga-65 | 15.2 | 0.25 | | EC | 3.255 | |
| Ga-66 | | 9.40 | | EC | 5.175 | |
| Ga-67 | | 78.2 | 3.26 | EC | 1.001 | |
| Ga-68 | 68.0 | 1.13 | | β+ | 2.921 | |
| Ga-70 | 21.1 | 0.35 | | β- | 1.652 | 99.59% β- (1.652), 0.41% EC (0.65456) |
| Ga-72 | | 14.1 | | β- | 3.998 | |
| Ga-73 | | 4.91 | | β- | 1.598 | |
| Ga-74 | 8.12 | 0.14 | | β- | 5.373 | |

| **Selenium** | | | | | | |
|---|---|---|---|---|---|---|
| Se-70 | 41.0 | 0.68 | | β+ | 2.412 | |
| Se-72 | 504 | 8.40 | | EC | 0.362 | |
| Se-73m | 39.0 | 0.65 | | IT | 2.761 | 27.4% EC (2.761), 72.6 % IT (0.03608) |
| Se-73 | 429 | 7.15 | | EC | 2.725 | |
| Se-75 | | | 120 | EC | 0.865 | |
| Se-79m1 | 3.92 | | | IT | 0.096 | 99.94% IT (0.09622), 0.06% (0.247) |
| Se-81m1 | 57.2 | 0.95 | | IT | 0.103 | 99.95% IT (0.10253), 0.05% β- (1.689) |
| Se-81 | 18.5 | 0.31 | | β- | 1.587 | |
| Se-83 | 22.3 | 0.37 | | β- | 3.673 | |
| Se-84 | 3.26 | | | β- | 1.836 | |

| **Bromine** | | | | | | |
|---|---|---|---|---|---|---|
| Br-73 | 3.40 | | | EC | 4.580 | |
| Br-74m1 | 41.5 | 0.69 | | EC | 9.921 | |
| Br-74 | 25.3 | 0.42 | | EC | 6.925 | |
| Br-75 | 98.0 | 1.63 | | EC | 3.062 | |
| Br-76 | | 16.2 | | β+ | 3.941 | |
| Br-77 | | 57.0 | 2.38 | β+ | 0.342 | |
| Br-78 | 6.64 | 0.11 | | EC | 3.574 | 99.99% EC (3.574), 0.01% β- (0.72746) |
| Br-80m1 | 265.20 | 4.42 | | IT | 0.085 | |
| Br-80 | 17.40 | 0.29 | | EC | 1.870 | 1,87 (EC), 2,004 (β-), EC = 91,7, β- = 8,3 |
| Br-82 | | 35.30 | 1.47 | β- | 3.090 | |
| Br-83 | 143.40 | 2.39 | | β- | 0.972 | |
| Br-84 | 31.80 | 0.53 | | β- | 4.656 | |
| Br-84m1 | 6.00 | | | β- | 4.960 | |
| Br-85 | 2.90 | | | β- | 2.905 | |

| **Yttrium** | | | | | | |
|---|---|---|---|---|---|---|
| Y-83 | 7.08 | | | EC | 4.470 | |
| Y-83m1 | 2.85 | | | EC | 4.532 | 4.532 (ECβ+), 0.062 (IT), ECβ+ = 60, IT = 40 |
| Y-84 | | | | | | |
| Y-84m1 | 39.50 | 0.66 | | EC | 6.490 | |
| Y-85 | 160.80 | 2.68 | | EC | 3.250 | |
| Y-85m1 | 291.60 | 4.86 | | EC | 3.270 | |
| Y-86m1 | 48.00 | 0.80 | | IT | 0.218 | |
| Y-86 | | 14.74 | | ECβ+ | 4.22 | |
| Y-87m1 | | 13.37 | | IT | 0.381 | 0.381 (IT), 2.243 (ECβ+), IT = 98,43, ECβ+ = 1,57 |
| Y-87 | | 80.30 | 3.35 | ECβ+ | 1.862 | |
| Y-88 | | | 106.64 | ECβ+ | 3.623 | |
| Y-90m1 | | 3.19 | | IT | 0.682 | |
| Y-90 | | 64.08 | 2.67 | β- | 2.280 | |
| Y-91m1 | 49.71 | 0.83 | | IT | | |
| Y-91 | | | 58.51 | β- | | |
| Y-92 | | 3.54 | | β- | 3.639 | |
| Y-93 | | 10.10 | | β- | 2.893 | |
| Y-94 | 19.10 | 0.32 | | β- | 4.919 | |
| Y-95 | 10.70 | 0.18 | | β- | 4.420 | |

| **Zirconium** | | | | | | |
|---|---|---|---|---|---|---|
| Zr-84 | 25.90 | | | ECβ+ | | |
| Zr-85 | 7.86 | | | ECβ+ | 4.690 | |
| Zr-86 | | 16.50 | | ECβ+ | 1.480 | |
| Zr-87 | 100.80 | 1.68 | | ECβ+ | 3.665 | |
| Zr-88 | | | 83.40 | EC | 0.670 | |
| Zr-89m1 | 4.18 | | | IT | 0.588 | 3.420 (ECβ+), 0.588 (IT), ECβ+ = 6,23, IT = 93,77 |
| Zr-89 | | 78.43 | 3.27 | β+ | 0.9 | |
| Zr-95 | | | 63.98 | β- | 1.125 | |
| Zr-97 | | 16.90 | | β- | 2.658 | |

| **Niobium** | | | | | | |
|---|---|---|---|---|---|---|
| Nb-87 | 2.60 | | | ECβ+ | 5.170 | |
| Nb-87m1 | 3.70 | | | ECβ+ | 5.170 | |
| Nb-88 | 14.50 | 0.24 | | ECβ+ | 7.200 | |
| Nb-88m1 | 7.80 | | | ECβ+ | 7.200 | |
| Nb-89 | 114.00 | 1.90 | | ECβ+ | 4.290 | |
| Nb-89m1 | 70.80 | 1.18 | | ECβ+ | 4.290 | |
| Nb-90 | | 14.60 | | ECβ+ | 6.111 | |
| Nb-91m1 | | | 60.86 | IT | 0.104 | 0.104 (IT), 1.357 (ECβ+), IT = 93, ECβ+ = 7 |
| Nb-95m1 | | 86.60 | 3.61 | IT | 0.236 | |
| Nb-95 | | | 35.15 | β- | 0.926 | |
| Nb-96 | | 23.35 | | β- | 3.187 | |
| Nb-97 | 72.10 | 1.20 | | β- | 1.934 | |
| Nb-98m1 | 51.50 | 0.86 | | β- | 4.586 | |

| **Molybdenum** | | | | | | |
|---|---|---|---|---|---|---|
| Mo-88 | 8.00 | | | ECβ+ | 3.720 | |
| Mo-89 | 2.04 | | | ECβ+ | 5.580 | |
| Mo-90 | | 5.67 | | ECβ+ | 2.489 | |
| Mo-91 | 15.49 | | | ECβ+ | 4.434 | |
| Mo-93m1 | | 6.85 | | IT. ECβ+ | 2.830 | IT = 99.88, ECβ+ = 0.12 |
| Mo-99 | | 66.00 | 2.75 | β- | 1.375 | |
| Mo-101 | 14.62 | 0.24 | | β- | 2.824 | |
| Mo-102 | 11.30 | | | β- | 1.010 | |

| **Technetium** | | | | | | |
|---|---|---|---|---|---|---|
| Tc-91 | 3.14 | 0.05 | | ECβ+ | 6.220 | |
| Tc-91m1 | 3.30 | 0.06 | | ECβ+ | 6.570 | 6.57 (ECβ+), 0.35 (IT); ECβ+ ≈ 100, IT < 1 |
| Tc-92 | 4.23 | 0.07 | | ECβ+ | 7.870 | |
| Tc-93m1 | 43.50 | 0.73 | | IT | 0.392 | 3.593 (ECβ+), 0.392 (IT), IT = 76.6, ECβ+ = 23.4 |
| Tc-93 | | 2.75 | | EC | 3.201 | |
| Tc-94m1 | 52.00 | 0.87 | | β+ | 2.36 | 1.730 (ECβ+), 0.075 (IT); ECβ+ = 100, IT < 0.1 |
| Tc-94 | | 4.90 | | ECβ+ | 4.256 | |
| Tc-95m1 | | | 61.00 | ECβ+ | 1.730 | 1.730 (ECβ+), 0.039 (IT); ECβ+ = 96.12, IT = 3.88 |
| Tc-95 | | 20.00 | | EC | 1.691 | |
| Tc-96m1 | 51.50 | 0.86 | | IT | 0.034 | 3.007 (ECβ+), 0.034 (IT), IT = 98.0, ECβ+ = 2.0 |
| Tc-96 | | 102.72 | 4.28 | EC | 2.973 | |
| Tc-97m1 | | | 87.00 | IT | 0.097 | |
| Tc-99m1 | | 6.02 | | IT | 0.143 | |
| Tc-101 | 14.20 | 0.24 | | β- | 1.614 | |
| Tc-102m1 | 4.35 | | | β- | 4.530 | 4.53 (β-), 0.0 (IT), β- = 98, IT = 2 |
| Tc-104 | 18.20 | 0.30 | | β- | 5.600 | |
| Tc-105 | 7.60 | 0.13 | | β- | 3.640 | |

| **Ruthenium** | | | | | | |
|---|---|---|---|---|---|---|
| Ru-92 | 3.65 | | | ECβ+ | 4.500 | |
| Ru-94 | 51.80 | 0.86 | | EC | 1.593 | |
| Ru-95 | | 1.64 | | ECβ+ | 2.572 | |
| Ru-97 | | 69.60 | 2.90 | EC | 1.115 | |
| Ru-103 | | | 39.28 | β- | 0.763 | |
| Ru-105 | | 4.44 | | β- | 1.917 | |
| Ru-106 | | | 368.20 | β- | 0.039 | |
| Ru-107 | 3.76 | 0.06 | | β- | 2.940 | |
| Ru-108 | 4.55 | 0.08 | | β- | 1.360 | |

| **Rhodium** | | | | | | |
|---|---|---|---|---|---|---|
| Rh-95 | 5.02 | 0.08 | | ECβ+ | 5.110 | |
| Rh-95m1 | 1.96 | 0.03 | | IT | 0.543 | 5.653 (ECβ+), 0.543 (IT); % ECβ+ = 12, IT = 88 |
| Rh-96 | 9.90 | 0.17 | | ECβ+ | 6.446 | |
| Rh-97 | 30.70 | 0.51 | | ECβ+ | 3.520 | |
| Rh-97m1 | 46.20 | 0.77 | | ECβ+ | 3.779 | 3.779 (ECβ+), 0.259 (IT); ECβ+ = 94.4, IT = 5.6 |
| Rh-98 | 8.70 | 0.15 | | ECβ+ | 5.057 | |
| Rh-98m1 | 3.50 | 0.06 | | ECβ+ | 5.057 | 5.057 (ECβ+), 0.0 (IT); ECβ+ > 0 |
| Rh-99m1 | | 4.70 | | ECβ+ | 2.167 | 2.167 (ECβ+), 0.064 (IT), ECβ+ > 99.84, IT < 0.16 |
| Rh-99 | | | 16.00 | ECβ+ | 2.130 | |
| Rh-100 | | 20.80 | | ECβ+ | 3.630 | |
| Rh-101m1 | | 104.16 | 4.34 | EC | 0.699 | 0.699 (EC), 0.157 (IT), EC = 92.8, IT = 7.2 |
| Rh-102 | | | 207.00 | ECβ+ | 2.323 | 2.323 (ECβ+), 1.150 (β-), ECβ+ = 80, β- = 20 |
| Rh-103m1 | 56.12 | 0.94 | | IT | 0.040 | |
| Rh-104m1 | 4.34 | | | IT | 0.129 | 0.129 (IT), 2.570 (β-), IT = 99.87, β- = 0.13 |
| Rh-105 | | 35.36 | 1.47 | β- | 0.567 | |
| Rh-106m1 | 132.00 | 2.20 | | β- | 3.678 | |
| Rh-107 | 21.70 | 0.36 | | β- | 1.511 | |
| Rh-108m1 | 6.00 | | | β- | 4.510 | |

| **Palladium** | | | | | | |
|---|---|---|---|---|---|---|
| Pd-97 | 3.10 | | | ECβ+ | 4.800 | |
| Pd-98 | 17.70 | | | ECβ+ | 1.873 | |
| Pd-99 | 21.40 | | | ECβ+ | 3.365 | |
| Pd-100 | | 87.12 | 3.63 | EC | 0.361 | |
| Pd-101 | | 8.27 | | ECβ+ | 1.980 | |
| Pd-103 | | | 16.96 | EC | 0.543 | |
| Pd-109 | | 13.43 | | β- | 1.116 | |
| Pd-109m1 | 4.70 | | | IT | 0.189 | |
| Pd-111 | 23.40 | 0.39 | | β- | 2.190 | |
| Pd-111m1 | | 5.50 | | IT | 0.172 | 0.172 (IT), 2.362 (β-); IT = 73, β- = 27 |
| Pd-112 | | 21.03 | | β- | 0.288 | |
| Pd-114 | 2.42 | 0.04 | | β- | 1.451 | |

| **Silver** | | | | | | |
|---|---|---|---|---|---|---|
| Ag-100 | 2.01 | | | ECβ+ | 7.050 | |
| Ag-100m1 | 2.24 | | | ECβ+ | 7.066 | 7.066 (ECβ+), 0.015 (IT) |
| Ag-101 | 11.10 | | | ECβ+ | 4.200 | |
| Ag-102 | 12.90 | 0.22 | | ECβ+ | 5.920 | |
| Ag-102m1 | 7.70 | | | ECβ+ | 5.929 | 5.929 (ECβ+), 0.009 (IT), ECβ+ = 51, IT = 49 |
| Ag-103 | 65.70 | 1.10 | | ECβ+ | 2.688 | |
| Ag-104m1 | 33.50 | 0.56 | | ECβ+ | 4.286 | 4.286 (ECβ+), 0.007 (IT), ECβ+ ≈ 100, IT < 0,07 |
| Ag-104 | 69.20 | 1.15 | | ECβ+ | 4.279 | |
| Ag-105 | | | 41.00 | ECβ+ | 1.346 | |
| Ag-106m1 | | 201.84 | 8.41 | EC | 3.055 | |
| Ag-106 | 23.96 | 0.40 | | ECβ+ | 2.965 | 2.965 (ECβ+), 0.195 (β-), ECβ+ = 99.5, β- < 1 |
| Ag-108 | 2.37 | 0.04 | | β- | 1.649 | 1.649 (β-), 1.918 (ECβ+), β- = 97.15, ECβ+ = 2.85 |
| Ag-110m1 | | | 249.90 | β- | 3.010 | 3.010 (β-), 0.188 (IT), β- = 98.64, IT = 1.,36 |
| Ag-111 | | 178.80 | 7.45 | β- | 0.810 | |
| Ag-112 | 187.20 | 3.12 | | β- | 3.956 | |
| Ag-113 | 322.20 | 5.37 | | β- | 2.016 | |
| Ag-115 | 20.00 | 0.33 | | β- | 3.100 | |
| Ag-116 | 2.68 | | | β- | 6.160 | |

| **Cadmium** | | | | | | |
|---|---|---|---|---|---|---|
| Cd-102 | 5.50 | | | ECβ+ | 2.587 | |
| Cd-103 | 7.30 | | | ECβ+ | 4.142 | |
| Cd-104 | 57.70 | 0.96 | | ECβ+ | 1.136 | |
| Cd-105 | 55.50 | | | ECβ+ | 2.739 | |
| Cd-107 | | 6.49 | | ECβ+ | 1.417 | |
| Cd-111 | 48.54 | | | IT | 0.396 | |
| Cd-115m1 | | | 44.60 | β- | 1.627 | |
| Cd-115 | | 53.46 | 2.23 | β- | 1.446 | |
| Cd-117m1 | 201.60 | 3.36 | | β- | 2.653 | |
| Cd-117 | 149.40 | 2.49 | | β- | 2.517 | |
| Cd-118 | 50.30 | | | β- | 0.520 | |
| Cd-119 | 2.69 | | | β- | 3.800 | |
| Cd-119m1 | 2.20 | | | β- | 3.947 | |

| **Indium** | | | | | | |
|---|---|---|---|---|---|---|
| In-105 | 5.07 | | | ECβ+ | 4.85 | |
| In-106 | 6.20 | | | ECβ+ | 6.52 | |
| In-106m1 | 5.20 | | | ECβ+ | 6.55 | |
| In-107 | 32.40 | | | ECβ+ | 3.43 | |
| In-108 | 58.00 | | | ECβ+ | 5.15 | |
| In-108m1 | 39.60 | | | ECβ+ | 5.18 | |
| In-109 | | 4.20 | | ECβ+ | 2.020 | |
| In-110 | | 4.9 | | ECβ+ | 3.878 | |
| In-110m1 | 69.10 | 1.15 | | ECβ+ | 3.940 | |
| In-111 | | 67.92 | 2.83 | EC | 0.245 | |
| In-112 | 14.40 | 0.24 | | ECβ+ | 2.586 | 2.586 (ECβ+), 0.664 (β-); ECβ+ = 56, β- = 44 |
| In-113m1 | | 1.66 | | IT | 0.392 | |
| In-114m1 | | | 49.51 | IT | 0.190 | 0.190 (IT), 1.642 (ECβ+), IT = 96.75, ECβ+ = 3.25 |
| In-115m1 | | 4.49 | | IT | 0.336 | 0.336 (IT), 0.831 (β-), IT = 95.0, β⁻ = 5.0 |
| In-116m1 | 54.15 | 0.90 | | β- | 3.401 | |
| In-117m1 | 116.50 | 1.94 | | β- | 1.770 | 1.770 (β⁻), 0.315 (IT); β-= 52.9, IT = 47.1 |
| In-117 | 43.80 | 0.73 | | β- | 1.455 | |
| In-118m1 | 4.45 | | | β- | 4.483 | |
| In-119m1 | 18.00 | 0.30 | | β- | 2.675 | 2.675 (β⁻), 0.311 (IT); β-= 94.4, IT = 5.6 |
| In-119 | 2.40 | 0.04 | | β- | 2.364 | |
| In-121m1 | 3.88 | 0.06 | | β- | 3.674 | 3.674 (β-), 0.314 (IT), β⁻ = 98.8, IT = 1.2 |

| **Tin** | | | | | | |
|---|---|---|---|---|---|---|
| Sn-107 | 2.90 | | | ECβ+ | 5.01 | |
| Sn-108 | 10.30 | | | ECβ+ | 2.092 | |
| Sn-109 | 18.00 | | | ECβ+ | 3.85 | |
| Sn-110 | | 4.11 | | EC | 0.638 | |
| Sn-111 | 35.30 | 0.59 | | ECβ+ | 2.445 | |
| Sn-113m1 | | 21.40 | | ECβ+ | 1.113 | 0.077 (IT), 1.113 (ECβ+), IT = 91.1, ECβ+ = 8.9 |
| Sn-113 | | | 115.09 | ECβ+ | 1.036 | |
| Sn-117m1 | | | 13.61 | IT | 0.135 | |
| Sn-119m1 | | | 293.00 | IT | 0.090 | |
| Sn-121 | | 27.06 | 1.13 | β- | 0.388 | |
| Sn-123m1 | 40.08 | 0.67 | | β- | 1.429 | |
| Sn-123 | | | 129.20 | β- | 1.404 | |
| Sn-125 | | 231.36 | 9.64 | β- | 2.364 | |
| Sn-125m1 | 9.52 | | | β⁻ | 2.364 | |
| Sn-127 | | 2.10 | | β- | 3.20 | |
| Sn-127m1 | 4.13 | | | β⁻ | 3.21 | |
| Sn-128 | 59.10 | 0.99 | | β- | 1.27 | |
| Sn-129 | 2.23 | | | β⁻ | 4.00 | |
| Sn-129m1 | 6.90 | | | β⁻ | 4.04 | 4.035 (β-), 0.035 (IT), β⁻ ≈ 100, IT ≈ 2·10⁻⁴ |
| Sn-130 | 3.72 | | | β⁻ | 2.15 | |

| **Antimony** | | | | | | |
|---|---|---|---|---|---|---|
| Sb-113 | 6.67 | 0.11 | | β+ | 3.905 | |
| Sb-114 | 3.49 | 0.06 | | β+ | 5.880 | |
| Sb-155 | 32.10 | 0.54 | | β+ | 3.030 | |
| Sb-116 | 15.80 | 0.26 | | β+ | 4.707 | |
| Sb-116m1 | 60.30 | 1.01 | | β+ | 5.090 | |
| Sb -117 | 62.80 | 2.80 | | β+ | 1.757 | |
| Sb-118 | 3.60 | 0.06 | | β+ | 3.657 | |
| Sb-18m1 | | 5.00 | | β+ | 3.907 | |
| Sb-119 | | 38.19 | 1.59 | EC | 0.594 | |
| Sb-120m1 | | 138.24 | 5.76 | EC | 2.681 | |
| Sb-120 | 15.89 | 0.26 | | ECβ+ | 2.681 | |
| Sb-122 | | 65.28 | 2.72 | β- | 1.979 | 1.979 (β-), 1.620 (ECβ+), β- = 97.59, ECβ+ = 2.41 |
| Sb-122m2 | 4.19 | 0.07 | | IT | 0.164 | |
| Sb-124m2 | 20.20 | 0.34 | | IT | 0.037 | |
| Sb-124 | | | 60.20 | β- | 2.905 | |
| Sb-126m1 | 19.15 | 0.32 | | β- | 3.688 | 3.688 (β-), 0.016 (IT), β-= 86, IT = 14 |
| Sb-126 | | | 12.40 | β- | 3.670 | |
| Sb-127 | | 92.40 | 3.85 | β- | 1.581 | |
| Sb-128 | | 9.01 | | β- | 4.380 | |
| Sb-128m1 | 10.40 | 0.17 | | β- | 4.380 | 4.380 (β-), 0.0 (IT), β- = 96.4, IT = 3.6 |
| Sb-129 | 259.20 | 4.32 | | β- | 2.380 | |
| Sb-129m1 | 17.70 | 0.30 | | β- | 4.231 | 4.231 (β-), 1.851 (IT), β-= 85, IT = 15 |
| Sb-130 | 40.00 | 0.67 | | β- | 4.960 | |
| Sb-130m1 | 6.30 | 0.11 | | β⁻ | 4.960 | |
| Sb-131 | 23.00 | 0.38 | | β- | 3.190 | |
| Sb-132 | 2.79 | | | β- | 5.290 | |
| Sb-132m1 | 4.15 | 0.07 | | β⁻ | 5.290 | |
| Sb-133 | 2.50 | 0.04 | | β⁻ | 4.003 | |

| **Tellurium** | | | | | | |
|---|---|---|---|---|---|---|
| Te-112 | 2.00 | | | ECβ+ | 4.35 | |
| Te-114 | 15.20 | | | ECβ+ | 2.8 | |
| Te-115 | 5.80 | | | ECβ+ | 4.64 | |
| Te-115m1 | 6.70 | | | ECβ+ | 4.66 | 4.66 (ECβ+), 0.02 (IT), ECβ+ < 100 |
| Te-116 | | 2.49 | | EC | 1.510 | |
| Te-117 | 62.00 | 1.00 | | ECβ+ | 3.535 | |
| Te-118 | 360.00 | 6 | | EC | 0.278 | |
| Te-119 | 961.80 | 16.03 | | ECβ+ | 2.293 | |
| Te-119m1 | 282.00 | 4.7 | | ECβ+ | 2.554 | 2.554 (ECβ+), 0.261 (IT), ECβ+ ≈ 100, IT < 0.008 |
| Te-121m1 | | | 154.00 | IT | 0.294 | 0.294 (IT), 1.334 (ECβ+), IT = 88.6, ECβ+ = 11.4 |
| Te-121 | | | 17.00 | EC | 1.040 | |
| Te-123m1 | | | 119.70 | IT | 0.248 | |
| Te-125m1 | | | 58.00 | IT | 0.145 | |
| Te-127m1 | | | 109.00 | IT | 0.088 | 0.088 (IT), 0.786 (β-), IT = 97.6, β⁻ = 2.4 |
| Te-127 | | 9.35 | | β- | 0.698 | |
| Te-129m1 | | | 33.60 | IT | 0.105 | 0.105 (IT), 1.604 (β-), IT = 63, β⁻ = 37 |
| Te-129 | 69.60 | 1.16 | | β- | 1.498 | |
| Te-131m1 | | 30.00 | 1.25 | β- | 2.415 | |
| Te-131 | 25.00 | 0.42 | | β- | 2.233 | |
| Te-132 | | 78.20 | 3.26 | β- | 0.493 | |
| Te-133m1 | 55.40 | 0.92 | | β- | 3.254 | 3.254 (β-), 0.334 (IT), β⁻ = 82.5, IT = 17.5 |
| Te-133 | 12.45 | 0.21 | | β- | 2.920 | |
| Te-134 | 41.80 | 0.70 | | β- | 1.560 | |

| **Iodine** | | | | | | |
|---|---|---|---|---|---|---|
| I-117 | 2.22 | | | ECβ+ | 4.67 | |
| I-118 | 13.70 | | | ECβ+ | 7.04 | |
| I-118m1 | 8.50 | | | ECβ+ | 7.14 | 7.144 (ECβ+), 0.104 (IT), ECβ+ < 100, IT > 0 |
| I-119 | 19.10 | | | ECβ+ | 3.51 | |
| I-120m1 | 53.00 | 0.88 | | ECβ+ | 5.615 | |
| I-120 | 81.00 | 1.35 | | ECβ+ | 5.615 | |
| I-121 | 127.20 | 2.12 | | ECβ+ | 2.270 | |
| I-122 | 3.62 | 0.06 | | ECβ+ | 4.234 | |
| I-123 | | 13.20 | | EC | 0.159 | |
| I-124 | | 100.32 | 4.18 | β+ | 2.14 | |
| I-125 | | | 59.408 | EC | 0.035 | |
| I-126 | | | 13.02 | ECβ+ | 2.155 | 2.155 (ECβ+), 1.258 (β-), ECβ+ = 56.3, β- = 43.7 |
| I-128 | 24.99 | 0.42 | | β- | 2.118 | 2118 (β-), 1.251 (ECβ+), β- = 93.1, ECβ+ = 6.9 |
| I-130 | | 12.36 | | β- | 2.949 | |
| I-130m1 | 9.00 | | | IT | 0.040 | 0.040 (IT), 2.989 (β⁻), IT = 84, β- = 16 |
| I-131 | | 192.96 | 8.04 | β- | 0.806 | |
| I-132m1 | 83.60 | 1.39 | | IT | 0.120 | 0.120 (IT), 3.697 (β⁻), IT = 86, β- = 14 |
| I-132 | | 2.30 | | β- | 3.577 | |
| I-133 | | 20.80 | | β- | 1.770 | |
| I-134 | 52.60 | 0.88 | | β- | 4.170 | |
| I-134m1 | 3.60 | | | IT | 0.316 | 0.316 (IT), 4.486 (β⁻), IT = 97.7, β- = 2.3 |
| I-135 | | 6.61 | | β- | 2.648 | |

| **Lanthanum** | | | | | | |
|---|---|---|---|---|---|---|
| La-127 | 5.10 | | | ECβ+ | 4.69 | |
| La-127m1 | 3.70 | | | ECβ+ | 4.705 | |
| La-827 | 5.00 | | | ECβ+ | 6.7 | |
| La-129 | 11.60 | | | ECβ+ | 3.72 | |
| La-130 | 8.70 | | | ECβ+ | 5.6 | |
| La-131 | 59.00 | 0.98 | | ECβ+ | 2.960 | |
| La-132 | | 4.80 | | ECβ+ | 4.710 | |
| La-132m1 | 24.30 | | | IT | 0.188 | 0.188 (IT), 4.898 (ECβ+), IT = 76, ECβ+ = 24 |
| La-133 | 234.72 | 3.912 | | ECβ+ | 2.23 | |
| La-134 | 6.67 | 0.11 | | ECβ+ | 6.450 | |
| La-135 | | 19.50 | | ECβ+ | 1.200 | |
| La-136 | 9.87 | | | ECβ+ | 2.87 | |
| La-140 | | 40.27 | 1.68 | β- | 3.762 | |
| La-141 | | 3.93 | | β- | 2.502 | |
| La-142 | 92.50 | 1.54 | | β- | 4.505 | |
| La-143 | 14.23 | 0.24 | | β- | 3.425 | |

| **Cerium** | | | | | | |
|---|---|---|---|---|---|---|
| Ce-129 | 3.50 | 0.06 | | ECβ+ | 5.05 | |
| Ce-130 | 25.00 | 0.42 | | ECβ+ | 2.2 | |
| Ce-131 | 10.20 | 0.17 | | ECβ+ | 4 | |
| Ce-131m1 | 5.00 | | | ECβ+ | 4 | |
| Ce-132 | 210.60 | 3.51 | | ECβ+ | 1.29 | 1.29 (ECβ+), 2.341 (IT) |
| Ce-133 | 97.00 | 1.62 | | ECβ+ | 2.9 | |
| Ce-133m1 | 294.00 | 4.9 | | ECβ+ | 2.937 | |
| Ce-134 | | 72.00 | 3.00 | EC | 0.500 | |
| Ce-135 | | 17.60 | | ECβ+ | 2.026 | |
| Ce-137m1 | | 34.40 | 1.43 | IT | 0.254 | 0.254 (IT), 1.476 (ECβ+), IT = 99.22, ECβ+ = 0.78 |
| Ce-137 | 540.00 | 9.00 | | EC | 1.222 | |
| Ce-139 | | | 137.66 | EC | 0.278 | |
| Ce-141 | | | 32.50 | β- | 0.581 | |
| Ce-143 | | 33.00 | 1.38 | β- | 1.462 | |
| Ce-144 | | | 284.30 | β- | 0.319 | |
| Ce-145 | 3.01 | | | β⁻ | 2.54 | |
| Ce-146 | 13.52 | | | β⁻ | 1.04 | |

| **Praseodymium** | | | | | | |
|---|---|---|---|---|---|---|
| Pr-133 | 6.50 | | | ECβ+ | 4.3 | |
| Pr-134 | 17.00 | | | ECβ+ | 6.2 | |
| Pr-134m1 | 11.00 | | | ECβ+ | 6.2 | |
| Pr-135 | 24.00 | | | ECβ+ | 3.72 | |
| Pr-136 | 13.10 | 0.22 | | ECβ+ | 5.126 | |
| Pr-137 | 76.60 | 1.28 | | ECβ+ | 2.702 | |
| Pr-138m1 | | 2.10 | | ECβ+ | 4.801 | |
| Pr-139 | | 4.51 | | ECβ+ | 2.129 | |
| Pr-140 | 3.39 | | | ECβ+ | 3.388 | |
| Pr-142m1 | 14.60 | 0.24 | | IT | 0.004 | |
| Pr-142 | | 19.12 | | β- | 2.162 | β- ≈ 100, EC = 0.0164 |
| Pr-143 | | | 13.56 | β- | 0.934 | |
| Pr-144m1 | 7.20 | 0.12 | | IT | 0.059 | IT ≈ 100, β⁻ = 0.07 |
| Pr-144 | 17.28 | 0.29 | | β- | 2.997 | |
| Pr-145 | | 5.98 | | β- | 1.805 | |
| Pr-146 | 24.15 | | | β⁻ | 4.2 | |
| Pr-147 | 13.60 | 0.23 | | β- | 2.69 | |
| Pr-148 | 2.27 | | | β⁻ | 4.93 | |
| Pr-148m1 | 2.00 | | | β⁻ | 5.02 | |
| Pr-149 | 2.26 | | | β⁻ | 3.397 | |

| **Neodymium** | | | | | | |
|---|---|---|---|---|---|---|
| Nd-134 | 8.50 | | | ECβ+ | 2.77 | |
| Nd-135 | 12.40 | | | ECβ+ | 4.8 | |
| Nd-135m1 | 5.50 | | | ECβ+ | 4.856 | |
| Nd-136 | 50.65 | 0.84 | | ECβ+ | 2.210 | |
| Nd-137 | 38.50 | | | ECβ+ | 3.69 | |
| Nd-138 | 302.40 | 5.04 | | EC | 1.1 | |
| Nd-139m1 | 330.00 | 5.50 | | ECβ+ | 3.021 | 3.021 (ECβ+), 0.231 (IT), ECβ+ = 88.2, IT = 11.8 |
| Nd-139 | 29.70 | 0.50 | | ECβ+ | 2.79 | |
| Nd-140 | 202.20 | 3.37 | | EC | 0.222 | |
| Nd-141 | | 2.49 | | ECβ+ | 1.823 | |
| Nd-147 | | | 10.98 | β- | 0.896 | |
| Nd-149 | | 1.73 | | β- | 1.691 | |
| Nd-151 | 12.44 | 0.21 | | β- | 2.442 | |
| Nd-152 | 11.40 | | | β⁻ | 1.11 | |

| **Promethium** | | | | | | |
|---|---|---|---|---|---|---|
| Pm-137 | 2.40 | | | ECβ+ | | |
| Pm-138m1 | 3.24 | | | ECβ+, IT | 6.9 | |
| Pm-139 | 4.15 | | | ECβ+ | 4.52 | |
| Pm-140m1 | 5.95 | | | ECβ+ | 6.09 | |
| Pm-140m2 | 5.95 | | | ECβ+ | | |
| Pm-141 | 20.90 | 0.35 | | ECβ+ | 3.715 | |
| Pm-143 | | | 265.00 | EC | 1.041 | |
| Pm-148m1 | | | 41.30 | β- | 2.606 | 2.606 (β-), 0.138 (IT), β- = 95.0, IT = 5.0 |
| Pm-148 | | 128.88 | 5.37 | β- | 2.468 | |
| Pm-149 | | 53.08 | 2.21 | β- | 1.071 | |
| Pm-150 | | 2.68 | | β- | 3.454 | |
| Pm-151 | | 28.40 | 1.18 | β- | 1.187 | |
| Pm-152 | 4.12 | | | β⁻ | 3.5 | |
| Pm-152m1 | 7.52 | | | β⁻ | 3.56 | |
| Pm-152m2 | 13.80 | | | β⁻ | | β⁻ < 100, IT > 0 |
| Pm-153 | 5.25 | | | β⁻ | 1.9 | |
| Pm-154m1 | 2.68 | | | β⁻ | 4.05 | |

| **Samarium** | | | | | | |
|---|---|---|---|---|---|---|
| Sm-138 | 3.10 | 0.05 | | ECβ+ | 3.900 | |
| Sm-139 | 2.57 | 0.04 | | ECβ+ | 5.460 | |
| Sm-140 | 14.80 | 0.25 | | ECβ+ | 3.020 | |
| Sm-141m1 | 22.60 | 0.38 | | ECβ+ | 4.719 | 4.719 (ECβ+), 0.176 (IT); ECβ+ = 99.69, IT = 0.31 |
| Sm-141 | 10.20 | 0.17 | | ECβ+ | 4.543 | |
| Sm-142 | 72.49 | 1.21 | | ECβ+ | 2.090 | |
| Sm-143 | 8.83 | | | ECβ+ | 3.443 | |
| Sm-145 | | | 340.00 | EC | 0.617 | |
| Sm-153 | | 46.80 | 1.95 | β- | 0.810 | |
| Sm-155 | 22.30 | 0.37 | | β- | 1.627 | |
| Sm-156 | | 9.40 | | β- | 0.722 | |
| Sm-158 | 5.30 | 0.09 | | β- | 1.999 | |

| **Europium** | | | | | | |
|---|---|---|---|---|---|---|
| Eu-143 | 2.63 | | | ECβ+ | 5.275 | |
| Eu-145 | | 142.56 | 5.94 | ECβ+ | 2.660 | |
| Eu-146 | | 110.64 | 4.61 | ECβ+ | 3.878 | |
| Eu-147 | | | 24.10 | ECβ+ | 1.722 | |
| Eu-148 | | | 54.50 | ECβ+ | 3.107 | |
| Eu-149 | | | 93.10 | EC | 0.692 | |
| Eu-150 | | 12.62 | | β- | 1.013 | ECβ+ = 11, β- = 89 , IT < 5-10-8 |
| Eu-152m1 | | 9.32 | | β- | 1.865 | ECβ+ = 28 , β- = 72, 1.920 (ECβ+), 1.865 (β-) |
| Eu-152m2 | 96.00 | 1.6 | | IT | 0.148 | |
| Eu-154m1 | 46.30 | 0.77 | | IT | 0.145 | |
| Eu-156 | | | 15.19 | β- | 2.451 | |
| Eu-157 | | 15.15 | | β- | 1.363 | |
| Eu-158 | 45.90 | 0.77 | | β- | 3.490 | |
| Eu-159 | 18.10 | | | β⁻ | 2.514 | |

| **Gadolinium** | | | | | | |
|---|---|---|---|---|---|---|
| Gd-144 | 4.50 | | | ECβ+ | 3.74 | |
| Gd-145 | 22.90 | 0.38 | | ECβ+ | 5.050 | |
| Gd-146 | | | 48.30 | EC | 1.030 | |
| Gd-147 | | 38.10 | 1.59 | ECβ+ | 2.187 | |
| Gd-149 | | 225.60 | 9.40 | ECβ+ | 1.314 | |
| Gd-151 | | | 120.00 | EC | 0.464 | |
| Gd-153 | | | 242.00 | EC | 0.485 | |
| Gd-159 | | 18.49 | | β- | 0.971 | |
| Gd-161 | 3.66 | | | β⁻ | 1.956 | |
| Gd-162 | 8.40 | | | β⁻ | 1.39 | |

| **Terbium** | | | | | | |
|---|---|---|---|---|---|---|
| Tb-147 | | 1.65 | | ECβ+ | 4.609 | |
| Tb-148 | | 1.00 | | ECβ+ | 5.690 | |
| Tb-148m1 | 2.20 | | | ECβ+ | 5.78 | |
| Tb-149 | | 4.15 | | β+ | 2.62 | 3.636 (ECβ+), 4.113 (α); ECβ+ = 83.3 , α = 16.7 |
| Tb-149m1 | 4.16 | | | ECβ+ | 3.672 | 3.672 (ECβ+), 4.077 (α), ECβ+ = 99.978, α = 0.022 |
| Tb-150 | | 3.27 | | ECβ+ | 4.656 | |
| Tb-150m1 | 5.80 | | | ECβ+ | 5.13 | |
| Tb-151 | | 17.60 | | β+ | 1.54 | 2.565 (ECβ+), 3.497 (α); ECβ+ ≈ 100, α = 9.510-3 |
| Tb-152m1 | 4.20 | | | IT | 0.052 | 0.502 (IT), 4.492 (ECβ+), IT = 78.8, ECβ+ = 21.2 |
| Tb-152 | | 17.50 | | ECβ+ | 3.990 | 3,.990 (ECβ+), 3.090 (α); ECβ+ ≈ 100, α < 7·10-7 |
| Tb-153 | | 56.16 | 2.34 | ECβ+ | 1.570 | |
| Tb-154 | | 21.40 | | ECβ+ | 3.560 | 3.56 (ECβ+), 0.25 (β-), ECβ+ ≈ 100, β- < 0.1 |
| Tb-154m1 | | 9.4 | | ECβ+ | 3.560 | 3.56 (ECβ+), 0.0 (IT), 0.25 (β-), ECβ+ = 78.2, IT = 21.8, β- < 0.1 |
| Tb-154m2 | | 22.7 | | ECβ+ | 3.560 | 3.56 (ECβ+), 0.0 (IT), ECβ+ = 98.2, IT = 1.8 |
| Tb-155 | | 127.68 | 5.32 | EC | 0.821 | |
| Tb-156m1 | | 24.40 | | IT | 0.050 | |
| Tb-156m2 | | 5.00 | | IT | 0.088 | 0.088 (IT), 2.532 (ECβ+) |
| Tb-156 | | 128.40 | 5.35 | ECβ+ | 2.444 | 2.444 (ECβ+), 0.434 (β-); ECβ+ ≈ 100, β- = ? |
| Tb-160 | | | 72.30 | β- | 1.835 | |
| Tb-161 | | 165.84 | 6.91 | β- | 0.593 | |
| Tb-162 | 7.60 | 0.13 | | β- | 2.510 | |
| Tb-163 | 19.50 | 0.33 | | β- | 1.785 | |
| Tb-164 | 3.00 | | | β⁻ | 3.89 | |
| Tb-165 | 2.11 | | | β⁻ | 3 | |

| **Dysprosium** | | | | | | |
|---|---|---|---|---|---|---|
| Dy-148 | 3.10 | 186 | | ECβ+ | 2.678 | |
| Dy-149 | 4.20 | 252 | | ECβ+ | 3.812 | |
| Dy-150 | 7.17 | 430.2 | | ECβ+ | 1.794 | 4.351 (α), 1.794 (ECβ+), α = 36, ECβ+ = 64 |
| Dy-151 | 17.90 | | | ECβ+ | 2.870 | 2.87 (ECβ+), 4.180 (α), ECβ+ = 94.4, α = 5.6 |
| Dy-152 | | 2.38 | | ECβ+ | 0.600 | 0.60 (ECβ+), 3.727 (α), EC(?) = 99.900, α = 0.100 |
| Dy-153 | | 6.4 | | ECβ+ | 2.170 | 2.17 (ECβ+), 3.559 (α), ECβ+ ≈ 100, α = 0.0094 |
| Dy-155 | | 9.90 | | ECβ+ | 2.095 | |
| Dy-157 | | 8.14 | | ECβ+ | 1.341 | |
| Dy-159 | | | 144.40 | EC | 0.366 | |
| Dy-165 | | 2.33 | | β- | 1.290 | |
| Dy-166 | | 81.60 | 3.40 | β- | 0.486 | |
| Dy-167 | 6.20 | | | β⁻ | 2.35 | |
| Dy-168 | 8.70 | | | β⁻ | 1.6 | |

| **Holmium** | | | | | | |
|---|---|---|---|---|---|---|
| Ho-153 | 2.01 | | | ECβ+ | 4.129 | 4.129 (ECβ+), 4.015 (α), ECβ+ = 99.949, α = 0.051 |
| Ho-153m1 | 9.30 | | | ECβ+ | 4.179 | 4.179 (ECβ+), 4.119 (α), ECβ+ = 99.82, α = 0.18 |
| Ho-154 | 11.76 | | | ECβ+ | 5.751 | 5.751 (ECβ+), 4.042 (α), ECβ+ = 99.981, α = 0.019 |
| Ho-154m1 | 3.10 | | | ECβ+ | 6.071 | 6.071 (ECβ+), 4.362 (α), 0.320 (IT), ECβ+ ≈ 100, α < 0.001, IT ≈ 0 |
| Ho-155 | 48.00 | 0.80 | | ECβ+ | 3.102 | |
| Ho-156 | 56.00 | 0.93 | | ECβ+ | 5.060 | |
| Ho-157 | 12.60 | 0.21 | | ECβ+ | 2.540 | |
| Ho-158 | 11.30 | | | ECβ+ | 4.23 | |
| Ho-158m1 | 28.00 | | | IT | 0.067 | 4.297 (ECβ+), 0.067 (IT), ECβ+ < 19, IT > 81 |
| Ho-158m2 | 21.30 | | | ECβ+ | 4.410 | 4.41 (ECβ+), 0.18 (IT), ECβ+ > 93, IT < 7 |
| Ho-159 | 33.00 | 0.55 | | ECβ+ | 1.838 | |
| Ho-160 | 25.60 | | | ECβ+ | 3.29 | |
| Ho-160m1 | 301.20 | 5.02 | | IT | 0.060 | 0.06 (IT), 3.35 (ECβ+), IT = 65, ECβ+ = 35 |
| Ho-161 | 150.00 | 2.50 | | EC | 0.895 | |
| Ho-162m1 | 67.00 | 1.12 | | IT | 0.106 | 0.106 (IT), 2.246 (ECβ+), IT = 62, ECβ+ = 38 |
| Ho-162 | 15.00 | 0.25 | | ECβ+ | 2.140 | |
| Ho-164m1 | 37.50 | 0.63 | | IT | 0.140 | |
| Ho-164 | 29.00 | 0.48 | | EC | 0.987 | 0.987 (EC), 0.962 (β-); EC = 60 , β- = 40 |
| Ho-166 | | 26.80 | 1.12 | β- | 1.855 | |
| Ho-167 | | 3.10 | | β- | 1.007 | |
| Ho-168 | 2.99 | | | β- | 2.91 | |
| Ho-169 | 4.70 | | | β- | 2.124 | |
| Ho-170 | 2.76 | | | β- | 3.87 | |

| **Erbium** | | | | | | |
|---|---|---|---|---|---|---|
| Er-154 | 3.73 | | | ECβ+ | 2.032 | 2.032 (ECβ+), 4.280 (α), ECβ+ = 99.53, α = 0.47 |
| Er-155 | 5.30 | | | ECβ+ | 3.84 | 3.84 (ECβ+), 4.12 (α), ECβ+ = 99.978, α = 0.022 |
| Er-156 | 19.50 | | | ECβ+ | 1.37 | |
| Er-157 | 18.65 | | | ECβ+ | 3.5 | 3.50 (ECβ+), 3.30 (α), ECβ+ ≈ 100, α < 0.02 |
| Er-158 | 137.40 | 2.29 | | EC | 0.9 | |
| Er-159 | 36.00 | | | ECβ+ | 2.769 | |
| Er-160 | | 28.58 | | EC | 0.33 | |
| Er-161 | 192.60 | 3.21 | | ECβ+ | | |
| Er-163 | 75.00 | 1.25 | | ECβ+ | 1.21 | |
| Er-165 | 621.60 | 10.36 | | EC | 0.376 | |
| Er-169 | | 223.20 | 9.30 | β- | 0.340 | |
| Er-171 | 451.20 | 7.52 | | β- | 1.490 | |
| Er-172 | | 49.30 | 2.05 | β- | 0.891 | |
| Er-174 | 3.30 | | | β- | 1.8 | |

| **Thulium** | | | | | | |
|---|---|---|---|---|---|---|
| Tm-157 | 3.63 | | | ECβ+ | 4.48 | |
| Tm-158 | 3.98 | | | ECβ+ | 6.6 | |
| Tm-159 | 9.13 | | | ECβ+ | 3.85 | |
| Tm-160 | 9.40 | | | ECβ+ | 5.6 | |
| Tm-161 | 33.00 | | | ECβ+ | 3.16 | |
| Tm-162 | 21.70 | 0.36 | | ECβ+ | 4.810 | |
| Tm-163 | 108.60 | 1.81 | | ECβ+ | 2.439 | |
| Tm-164 | 2.00 | | | ECβ+ | 3.962 | |
| Tm-164m1 | 5.10 | | | ECβ+ | 3.962 | |
| Tm-165 | | 30.06 | | ECβ+ | 1.592 | |
| Tm-166 | 462.00 | 7.70 | | ECβ+ | 3.040 | |
| Tm-167 | | 221.76 | 9.24 | EC | 0.748 | |
| Tm-168 | | | 93.10 | ECβ+ | 1.679 | 1.679 (ECβ+), 0.257 (β-), ECβ+ = 99.990, β- = 0.010 |
| Tm-170 | | | 128.60 | β- | 0.968 | 0.314 (ECβ+), 0.968 (β-), EC, β-(99%) |
| Tm-172 | | 63.60 | 2.65 | β- | 1.880 | |
| Tm-173 | | 8.24 | | β- | 1.298 | |
| Tm-174 | 5.40 | | | β- | 3.08 | |
| Tm-175 | 15.20 | 0.25 | | β- | 2.39 | |
| Tm-176 | 1.90 | | | β- | 3.88 | |

| **Ytterbium** | | | | | | |
|---|---|---|---|---|---|---|
| Yb-160 | 4.80 | | | ECβ+ | 2.3 | |
| Yb-161 | 4.20 | | | ECβ+ | 4.15 | |
| Yb-162 | 18.90 | 0.32 | | EC | 1.660 | |
| Yb-163 | 11.05 | | | ECβ+ | 3.37 | |
| Yb-164 | 75.80 | | | EC | 1 | |
| Yb-165 | 9.90 | | | ECβ+ | 2.762 | |
| Yb-166 | | 56.70 | 2.36 | EC | 0.304 | |
| Yb-167 | 17.50 | 0.29 | | ECβ+ | 1.954 | |
| Yb-169 | | | 32.01 | EC | 0.909 | |
| Yb-175 | | 100.56 | 4.19 | β- | 0.47 | |
| Yb-177 | | 1.90 | | β- | 1.399 | |
| Yb-178 | 74.00 | 1.23 | | β- | 0.645 | |
| Yb-179 | 8.00 | | | β- | 2.4 | |
| Yb-180 | 2.40 | | | β- | | |

| **Lutetium** | | | | | | |
|---|---|---|---|---|---|---|
| Lu-162m2 | 1.90 | | | ECβ+ | | |
| Lu-164 | 3.14 | | | ECβ+ | 6.25 | |
| Lu-165 | 10.74 | | | ECβ+ | 3.92 | |
| Lu-166 | 2.65 | | | ECβ+ | 5.48 | |
| Lu-166m2 | 2.12 | | | ECβ+ | 5.523 | 5.523 (ECβ+), 0.043 (IT),ECβ+ > 80, IT < 20 |
| Lu-167 | 51.50 | 0.86 | | ECβ+ | 3.130 | |
| Lu-168 | 5.50 | | | ECβ+ | 4.48 | |
| Lu-168m1 | 6.70 | | | ECβ+ | 4.700 | 4.70 (ECβ+), 0.220 (IT), ECβ+ > 95, IT < 5 |
| Lu-169 | | 34.06 | 1.42 | ECβ+ | 2.293 | |
| Lu-170 | | 48.00 | 2.00 | ECβ+ | 3.459 | |
| Lu-171 | | 197.28 | 8.22 | ECβ+ | 1.479 | |
| Lu-172 | | 160.80 | 6.70 | ECβ+ | 2.519 | |
| Lu-174m1 | | | 142.00 | IT | 0.171 | 0.171 (IT), 1.545 (EC), IT = 99.38, EC = 0.62 |
| Lu-176m1 | | 3.68 | | β- | 1.316 | 1.316 (β-), 0.229 (EC), β-= 99.905, EC = 0.095 |
| Lu-177m1 | | | 160.90 | β- | 1.468 | 1.468 (β-), 0.970 (IT), β- = 78.3, IT = 21.7 |
| Lu-177 | | | 6.71 | β- | 0.490 | |
| Lu-178m1 | 22.70 | 0.38 | | β- | 2.219 | |
| Lu-178 | 28.40 | 0.47 | | β- | 2.099 | |
| Lu-179 | | 4.59 | | β- | 1.405 | |
| Lu-180 | 5.70 | | | β⁻ | 3.1 | |
| Lu-181 | 3.50 | | | β⁻ | 2.5 | |
| Lu-182 | 2.00 | | | β⁻ | | |

| **Hafnium** | | | | | | |
|---|---|---|---|---|---|---|
| Hf-166 | 6.77 | | | ECβ+ | 2.3 | |
| Hf-167 | 2.05 | | | ECβ+ | 4 | |
| Hf-168 | 25.95 | | | ECβ+ | 1.8 | |
| Hf-169 | 3.24 | | | ECβ+ | 3.27 | |
| Hf-170 | | 16.01 | | EC | 1.1 | |
| Hf-171 | | 12.1 | | ECβ+ | 2.4 | |
| Hf-173 | | 23.60 | 0.98 | ECβ+ | 1.610 | |
| Hf-175 | | | 70.00 | EC | 0.686 | |
| Hf-177m1 | 51.40 | 0.86 | | IT | 2.740 | |
| Hf-179m2 | | | 25.10 | IT | 1.106 | |
| Hf-180m1 | | 5.50 | | IT | 1.141 | 1.141 (IT), 1.287 (β-), IT = 99,.7, β- = 0.3 |
| Hf-181 | | | 42.40 | β- | 1.027 | |
| Hf-182m1 | 61.50 | 1.03 | | β- | 1.546 | 1.546 (β-), 1.173 (IT), β- = 58, IT = 42 |
| Hf-183 | 64.00 | 1.07 | | β- | 2.010 | |
| Hf-184 | | 4.12 | | β- | 1.340 | |
| Hf-185 | 3.50 | | | β⁻ | | |

| **Tantalum** | | | | | | |
|---|---|---|---|---|---|---|
| Ta-168 | 2.00 | | | ECβ+ | 6.7 | |
| Ta-169 | 4.90 | | | ECβ+ | 4.44 | |
| Ta-170 | 6.76 | | | ECβ+ | 6 | |
| Ta-171 | 23.30 | | | ECβ+ | 3.7 | |
| Ta-172 | 36.80 | 0.61 | | ECβ+ | 4.920 | |
| Ta-173 | | 3.65 | | ECβ+ | 2.790 | |
| Ta-174 | | 1.20 | | ECβ+ | 3.850 | |
| Ta-175 | | 10.50 | | ECβ+ | 2.000 | |
| Ta-176 | | 8.08 | | ECβ+ | 3.110 | |
| Ta-177 | | 56.60 | 2.36 | EC | 1.166 | |
| Ta-178m1 | | 2.36 | | EC | 1.910 | |
| Ta-178 | 9.31 | 0.16 | | EC | 1.910 | |
| Ta-180 | | 8.15 | | EC | 0.854 | 0.854 (EC), 0.708 (β-), EC = 86, β- = 14 |
| Ta-182m1 | 15.84 | 0.26 | | IT | 0.52 | |
| Ta-182 | | | 115.00 | β- | 1814.000 | |
| Ta-183 | | 122.40 | 5.10 | β- | 1.070 | |
| Ta-184 | | 8.70 | | β- | 2.870 | |
| Ta-185 | 49.00 | 0.82 | | β- | 1.992 | |
| Ta-186 | 10.50 | 0.18 | | β- | 3.000 | |

| **Tungsten** | | | | | | |
|---|---|---|---|---|---|---|
| W-170 | 2.42 | | | ECβ+ | 3 | |
| W-171 | 2.38 | | | ECβ+ | 4.6 | |
| W-172 | 6.60 | | | ECβ+ | 2.5 | |
| W-173 | 7.60 | | | ECβ+ | 4 | |
| W-174 | 31.00 | | | ECβ+ | 1.9 | |
| W-175 | 35.20 | | | ECβ+ | 2.91 | |
| W-176 | | 2.50 | | EC | 0.790 | |
| W-177 | 135.00 | 2.25 | | ECβ+ | 2.000 | |
| W-178 | | | 21.70 | EC | 0.091 | |
| W-179m1 | 6.40 | | | IT | 0.222 | 0.222 (IT), 1.282 (ECβ+), IT = 99.72, ECβ+ = 0.28 |
| W-181 | | | 121.20 | EC | 0.188 | |
| W-185 | | | 75.10 | β- | 0.433 | |
| W-187 | | 23.72 | 0.99 | β- | 1.311 | |
| W-188 | | | 69.40 | β- | 0.349 | |
| W-189 | 11.50 | | | β⁻ | 2.5 | |
| W-190 | 30.00 | | | β⁻ | 1.27 | |

| **Rhenium** | | | | | | |
|---|---|---|---|---|---|---|
| Re-173 | 1.98 | | | ECβ+ | 4.8 | |
| Re-174 | 2.40 | | | ECβ+ | 6.5 | |
| Re-175 | 5.89 | | | ECβ+ | 4.3 | |
| Re-176 | 5.30 | | | ECβ+ | 5.6 | |
| Re-177 | 14.00 | 0.23 | | ECβ+ | 3.400 | |
| Re-178 | 13.20 | 0.22 | | ECβ+ | 13.200 | |
| Re-179 | 19.50 | | | ECβ+ | 2.71 | |
| Re-180 | 2.43 | 0.04 | | ECβ+ | 3.800 | |
| Re-181 | | 20.00 | | ECβ+ | 1.739 | |
| Re-182 | | 64.00 | | EC | 2.800 | |
| Re-182m1 | | 12.70 | | ECβ+ | 2.800 | |
| Re-183 | | | 70.00 | EC | 0.556 | |
| Re-184m1 | | | 169.00 | IT | 0.188 | 0.188 (IT), 1.671 (EC), IT = 75.4, EC = 24.6 |
| Re-184 | | | 38.00 | ECβ+ | 1.483 | |
| Re-186 | | 90.48 | 3.72 | β- | 1.07 | 0.582 (EC), 1.069 (β-); EC = 7.47, β- = 92.53 |
| Re-188m1 | 18.60 | 0.31 | | IT | 0.172 | |
| Re-188 | | 16.98 | | β- | 2.120 | |
| Re-189 | | 24.30 | 1.01 | β- | 1.009 | |
| Re-190 | 3.10 | | | β⁻ | 3.15 | |
| Re-190m1 | 192.00 | 3.2 | | β⁻ | 3.269 | 3.269 (β-), 0.119 (IT), β⁻ = 54.4, IT = 45.6 |
| Re-191 | 9.80 | | | β⁻ | 2.045 | |

| **Osmium** | | | | | | |
|---|---|---|---|---|---|---|
| Os-176 | 3.60 | | | ECβ+ | 3.2 | |
| Os-177 | 2.80 | | | ECβ+ | 4.5 | |
| Os-178 | 5.00 | | | ECβ+ | 2.3 | |
| Os-179 | 6.50 | | | ECβ+ | 3.68 | |
| Os-180 | 22.00 | 0.37 | | ECβ+ | 1.470 | |
| Os-181 | 105.00 | 1.75 | | ECβ+ | 2.930 | |
| Os-181m1 | 2.70 | | | ECβ+ | 2.979 | |
| Os-182 | | 22.00 | | EC | 0.91 | |
| Os-183 | 13.00 | | | ECβ+ | 2.13 | |
| Os-183m1 | 9.90 | | | ECβ+ | 2.301 | 2.301 (ECβ+), 0.171 (IT), ECβ+ = 85, IT = 15 |
| Os-185 | | | 94.00 | EC | 1.013 | |
| Os-189m1 | | 6.00 | | IT | 0.031 | |
| Os-190m1 | 9.90 | 0.17 | | IT | 1.705 | |
| Os-191m1 | | 13.03 | | IT | 0.074 | |
| Os-191 | | | 15.40 | β- | 0.314 | |
| Os-193 | | 30.00 | 1.25 | β- | 1.140 | |
| Os-195 | 6.50 | | | β⁻ | 2 | |
| Os-196 | 34.90 | | | β⁻ | 1.16 | |

| **Iridium** | | | | | | |
|---|---|---|---|---|---|---|
| Ir-181 | 4.90 | | | ECβ+ | 4.07 | |
| Ir-182 | 15.00 | 0.25 | | ECβ+ | 5.61 | |
| Ir-183 | 58.00 | | | ECβ+ | 3.45 | |
| Ir-184 | | 3.02 | | ECβ+ | 4.600 | |
| Ir-185 | | 14.00 | | ECβ+ | 2.370 | |
| Ir-186 | | 15.80 | | ECβ+ | 3.831 | |
| Ir-186m1 | | 1.90 | | ECβ+ | 3.831 | 3.831 (ECβ+), 0 (IT), ECβ+ ≈ 75, IT ≈ 25 |
| Ir-187 | | 10.50 | | EC | 1.502 | |
| Ir-188 | | 41.50 | 1.73 | ECβ+ | 2.809 | |
| Ir-189 | | | 13.30 | EC | 0.532 | |
| Ir-190m2 | | 3.25 | | ECβ+ | 2.149 | 2.149 (ECβ+), 0.140 (IT), ECβ+ = 94.4, IT = 5.6 |
| Ir-190m1 | | 1.20 | | IT | 0.026 | |
| Ir-190 | | | 12.10 | ECβ+ | 2.000 | |
| Ir-192 | | | 73.83 | β- | 1.460 | 1.46 (β-), 1.046 (EC), β- = 95.24, EC = 4.76 |
| Ir-193m1 | | | 10.53 | IT | 0.08 | |
| Ir-194m1 | | | 171.00 | β- | 2.437 | |
| Ir-194 | | 19.15 | | β- | 2.247 | |
| Ir-195m1 | | 3.80 | | β- | 1.220 | 1.22 (β-), 0.10 (IT), β- = 95, IT = 5 |
| Ir-195 | | 2.50 | | β- | 1.120 | |
| Ir-196m1 | 84.00 | 1.4 | | β- | 3.620 | |
| Ir-197 | 5.80 | | | β- | 2.155 | |
| Ir-197m1 | 8.90 | | | β- | 2.270 | 2.27 (β-), 0.115 (IT), β⁻ = 99.75, IT = 0.25 |

| **Platinum** | | | | | | |
|---|---|---|---|---|---|---|
| Pt-182 | 3.00 | | | ECβ+ | 2.850 | 2.85 (ECβ+), 4.943 (α), ECβ+ = 99.969, α = 0.031 |
| Pt-183 | 6.50 | | | ECβ+ | 4.600 | |
| Pt-184 | 17.30 | | | ECβ+ | 2.300 | |
| Pt-185 | 70.90 | 1.1817 | | ECβ+ | 3.800 | |
| Pt-185m1 | 33.00 | | | ECβ+ | 3.903 | 3.903 (ECβ+), 0.103 (IT), 4.643 (α), ECβ+ = 99, IT < 2 |
| Pt-186 | | 2.00 | | ECβ+ | 1.380 | |
| Pt-188 | | | 10.20 | EC | 0.507 | |
| Pt-187 | | 2.35 | | ECβ+ | 3.11 | |
| Pt-189 | | 10.87 | | ECβ+ | 1.971 | |
| Pt-191 | | 67.20 | 2.80 | EC | 1.019 | |
| Pt-193m1 | | 103.92 | 4.33 | IT | 0.150 | |
| Pt-195m1 | | 96.48 | 4.02 | IT | 0.259 | |
| Pt-197m1 | 95.41 | 1.59 | | IT | 0.399 | 0.399 (IT) 1.119 (β-), IT = 96.7, β- = 3.3 |
| Pt-197 | | 18.30 | | β- | 0.719 | |
| Pt-199 | 30.80 | 0.51 | | β- | 1.702 | |
| Pt-200 | | 12.50 | | β- | 0.660 | |
| Pt-201 | 2.50 | | | β⁻ | 2.66 | |
| Pt-202 | | 44 | | β⁻ | | |

| **Gold** | | | | | | |
|---|---|---|---|---|---|---|
| Au-185 | 4.25 | | | ECβ+ | 4.71 | 4.71 (ECβ+), 5.18 (α), ECβ+ = 99.74, α = 0.26 |
| Au-185m1 | 6.80 | | | ECβ+ | 4.71 | |
| Au-186 | 10.70 | | | ECβ+ | 6.04 | |
| Au-187 | 8.40 | | | ECβ+ | 3.6 | 3,6 (ECβ+), 4,79 (α), ECβ+ = 99,997, α = 0,003 |
| Au-188 | 8.84 | | | ECβ+ | 5.3 | |
| Au-189 | 28.70 | | | ECβ+ | 2.85 | ECβ+ ≈ 100, α < 3.10-5 |
| Au-189m1 | 4.59 | | | ECβ+ | 3.097 | ECβ+ ≈ 100, IT > 0 |
| Au-190 | 42.80 | | | ECβ+ | 4.442 | ECβ+ ≈ 100, α < 1-10-6 |
| Au-191 | 3.18 | | | ECβ+ | 1.83 | |
| Au-192 | 4.94 | | | ECβ+ | 3.516 | |
| Au-193 | | 17.65 | | EC | 1.069 | |
| Au-194 | | 398.02 | 16.58 | ECβ+ | 2.492 | |
| Au-195 | | | 183.00 | EC | 0.227 | |
| Au-196 | | 148.39 | 6.18 | ECβ+ | 1.500 | 1.506 (ECβ+), 0.686 (β-), ECβ+ = 92.80, β- = 7.20 |
| Au-196m2 | | 9.60 | | IT | 0.596 | |
| Au-198m1 | | 55.20 | 2.30 | IT | 0.812 | |
| Au-198 | | 64.70 | 2.70 | β- | 1.372 | |
| Au-199 | | 75.34 | 3.14 | β- | 0.453 | |
| Au-200m1 | | 18.70 | | β- | 3.202 | 3.202 (β-), 0.962 (IT), β- = 82, IT = 18 |
| Au-200 | 48.40 | 0.81 | | β- | 2.240 | |
| Au-201 | 26.40 | 0.44 | | β- | 1.275 | |

| **Thallium** | | | | | | |
|---|---|---|---|---|---|---|
| TI-189 | 2.30 | | | ECβ+ | 5.18 | |
| TI-190 | 2.60 | | | ECβ+ | 7 | |
| TI-190m1 | 3.70 | | | ECβ+ | 7 | |
| TI-191 | | | | ECβ+ | 4.49 | |
| TI-191m1 | 5.22 | | | ECβ+ | 4.789 | |
| TI-192 | 9.60 | | | ECβ+ | 6.12 | |
| TI-192m1 | 10.80 | | | ECβ+ | 6.12 | |
| TI-193 | 21.60 | | | ECβ+ | 3.64 | |
| TI-193m1 | 2.11 | | | IT | 0.365 | 0.365 (IT), 4.005 (ECβ+), IT = 75, ECβ+ = 25 |
| TI-194m1 | 32.80 | 0.55 | | ECβ+ | 5.280 | |
| TI-194 | 33.00 | 0.55 | | EC | 5.280 | |
| TI-195 | | 1.16 | | ECβ+ | 2.810 | |
| TI-196 | | 1.84 | | ECβ+ | 4.38 | |
| TI-196m1 | | 1.41 | | ECβ+ | 4.774 | 4.774 (ECβ+) 0.394 (IT), ECβ+ = 95.5, IT = 4.5 |
| TI-197 | | 2.84 | | ECβ+ | 2.180 | |
| TI-198m1 | | 1.87 | | ECβ+ | 4.004 | 4.004 (ECβ+), 0.544 (IT), ECβ+ = 54, IT = 46 |
| TI-198 | | 5.30 | | ECβ+ | 3.460 | |
| TI-199 | | 7.42 | | ECβ+ | 1.440 | |
| TI-200 | | 26.10 | 1.09 | ECβ+ | 2.456 | |
| TI-201 | | | 3.04 | EC | 0.483 | |
| TI-202 | | | 12.23 | ECβ+ | 1.365 | |
| TI-206 | 4.20 | 0.07 | | β- | 1.533 | |
| TI-206m1 | 3.74 | | | IT | 2.643 | |
| TI-207 | 4.77 | 0.08 | | β- | 1.423 | |
| TI-208 | 3.07 | 0.05 | | β- | 5.001 | |
| TI-209 | 2.20 | 0.04 | | β- | 3.980 | |

| **Lead** | | | | | | |
|---|---|---|---|---|---|---|
| Pb-191m1 | 2.10 | | | ECβ+ | 6.038 | |
| Pb-192 | 3.50 | | | ECβ+ | 3.400 | 3.4 (ECβ+), 5.221 (a), ECβ+ = 99.9941, α = 0.0059 |
| Pb-193 | 2.00 | | | ECβ+ | | |
| Pb-193m1 | 5.80 | | | ECβ+ | 5.200 | |
| Pb-194 | 12.00 | | | ECβ+ | 2.700 | |
| Pb-195 | 15.00 | | | ECβ+ | 4.500 | |
| Pb-195m1 | 15.80 | 0.26 | | ECβ+ | 4.500 | |
| Pb-196 | 37.00 | | | ECβ+ | 2.050 | 2.05 (ECβ+), 4.2 (a), ECβ+ ≈ 100, α < 3.10-5 |
| Pb-197 | 8.00 | | | ECβ+ | 3.58 | |
| Pb-197m1 | 43.00 | | | ECβ+ | 3.889 | 3.889 (ECβ+), 0.319 (IT), ECβ+ = 81, IT = 19 |
| Pb-198 | | 2.40 | | ECβ+ | 1.410 | |
| Pb-199 | 90.00 | 1.50 | | ECβ+ | 2.880 | |
| Pb-199m1 | 12.20 | | | IT | 0.425 | 0.425 (IT), 3.305 (ECβ+), IT = 93, ECβ+ = 7 |
| Pb-200 | | 21.50 | | EC | 0.811 | |
| Pb-201 | | 9.33 | | ECβ+ | 1.900 | |
| Pb-202m1 | | 3.53 | | IT | 2.710 | |
| Pb-203 | | 51.87 | 2.16 | EC | 0.975 | |
| Pb-204m1 | 67.20 | 1.12 | | IT | 2.186 | |
| Pb-209 | | 3.25 | | β- | 0.644 | |
| Pb-211 | 36.10 | 0.60 | | β- | 1.373 | |
| Pb-212 | | 10.64 | | β- | 0.574 | |
| Pb-213 | 10.20 | 0.17 | | β- | 2.070 | |
| Pb-214 | 26.80 | 0.45 | | β- | 1.024 | |

| **Bismuth** | | | | | | |
|---|---|---|---|---|---|---|
| Bi-197 | 9.33 | | | ECβ+ | 5.200 | 5.2 (ECβ+), 5.39 (α), ECβ+ ≈ 100, α = 1.10-4 |
| Bi-197m1 | 5.04 | | | α | 5.890 | 5.89 (α), 5,7 (ECβ+), 0.50 (IT), α = 55, ECβ+ = 45, IT < 0.3 |
| Bi-198 | 10.30 | | | ECβ+ | 6.56 | |
| Bi-198m1 | 11.60 | | | ECβ+ | 6.56 | |
| Bi-199 | 27.00 | | | ECβ+ | 4.34 | |
| Bi-199m1 | 24.70 | | | ECβ+ | 5.020 | 5.02 (ECβ+), 5.64 (α), 0.68 (IT), ECβ+ = 99, α ≈ 0.01, IT < 2 |
| Bi-200 | 36.40 | | | ECβ+ | 5.89 | |
| Bi-200m1 | 31.00 | | | ECβ+ | 5.89 | ECβ+ > 90, IT < 10 |
| Bi-201 | 108.00 | 1.80 | | EC | 3.84 | |
| Bi-201m1 | 59.10 | 0.99 | | EC | 4.686 | 4.686 (EC), 5.346 (IT), 5.346 (α), EC > 93, IT < 6.8, α ≈ 0.3 |
| Bi-202 | | 1.67 | | ECβ+ | 5.150 | 5.15 (ECβ+), 4.29 (a), ECβ+ ≈ 100, α < 1-10-5 |
| Bi-203 | | 11.76 | | ECβ+ | 3.253 | 3.253 (ECβ+), 4.15 (α), ECP+ = 100, α ≈ 1-10-5 |
| Bi-204 | | 11.22 | | ECβ+ | 4.438 | |
| Bi-205 | | | 15.31 | ECβ+ | 2.708 | |
| Bi-206 | | 149.83 | 6.24 | ECβ+ | 3.758 | |
| Bi-210 | | 120.29 | 5.01 | β- | 1.163 | |
| Bi-211 | 2.14 | 0.04 | | α | 6.751 | 6.751 (α), 0.579 (β-), α = 99.724, β- = 0.276 |
| Bi-212 | 60.55 | 1.01 | | β- | 2.254 | 2.254 (β-), 6.207 (α), 11.208 (β⁻+α); β- = 64.06, α = 35.94 |
| Bi-212m1 | 25.00 | | | α | 6.457 | 6.457 (α), 2.504 (β-), α = 67, β⁻ = 33, β⁻α = 30 |
| Bi-212m2 | 7.00 | | | β- | 4.164 | |
| Bi-213 | 45.6 | 0.76 | | α | 5.98 | 1.464 (β⁻), 5.982 (α); β- = 97.91, α = 2.09 |
| Bi-214 | 19.90 | 0.33 | | β- | 3.272 | 3.272 (β⁻), 5.617 (α); β- = 99.979, α = 0.021 |
| Bi-215 | 7.60 | | | β- | 2.25 | |
| Bi-216 | 3.60 | | | β- | 4 | |

| **Polonium** | | | | | | |
|---|---|---|---|---|---|---|
| Po-199 | 5.48 | | | ECβ+ | 5.600 | 5.6 (ECβ+), 6.074 (α), ECβ+ = 88, α = 12 |
| Po-199m1 | 4.13 | | | ECβ+ | 5.910 | 5.91 (ECβ+), 6.384 (α), 0.310 (IT), ECβ+ = 59, α = 39, IT = 2.1 |
| Po-200 | 11.50 | | | ECβ+ | 3.350 | 3.35 (ECβ+), 5.982 (α), ECβ+ = 88.9, α = 11.1 |
| Po-201 | 15.30 | | | ECβ+ | 4.880 | 4.88 (ECβ+), 5.799 (α), ECβ+ = 98.4, α = 1.6 |
| Po-201m1 | 8.90 | | | IT | 0.424 | 0.424 (IT), 5.304 (ECβ+), 6.223 (α), IT = 56, EC = 41, α ≈ 2.9 |
| Po-202 | 44.70 | 0.75 | | ECβ+ | 2.820 | 2.82 (ECβ+), 5.701 (α), ECβ+ = 98.08, α = 1.92 |
| Po-203 | 36.70 | 0.61 | | ECβ+ | 4.230 | 4.23 (ECβ+), 5.496 (α), ECβ+ = 99.89, α = 0.11 |
| Po-204 | | 3.53 | | ECβ+ | 2.340 | 2.34 (ECβ+), 5.485 (α), ECβ+ = 99.34, α = 0.,66 |
| Po-205 | | 1.66 | | ECβ+ | 3.530 | 3.53 (ECβ+), 5.324 (α), ECβ+ = 99.96, α = 0.04 |
| Po-206 | | 8.8 | | ECβ+ | 1.846 | 1.846 (ECβ+), 5.326 (α), ECβ+ = 94.55, α = 5.45 |
| Po-207 | | 5.8 | | ECβ+ | 2.909 | 2.909 (ECβ+), 5.216 (α), ECβ+ = 99.979, α = 0.021 |
| Po-210 | | | 138.38 | α | 5.307 | |
| Po-218 | 3.05 | 0.05 | | α | 6.115 | 6.115 (α), 0.265 (β-), α = 99.980 , β⁻ = 0.020 |

| **Astatine** | | | | | | |
|---|---|---|---|---|---|---|
| At-203 | 7.40 | | | ECβ+ | 5.060 | 5.06 (ECβ+), 6.21 (α), ECβ+ = 69, α = 31 |
| At-204 | 9.20 | | | ECβ+ | 6.480 | 6.48 (ECβ+), 6.07 (α), ECβ+ = 96.2, α = 3.8 |
| At-205 | 26.20 | 0.44 | | ECβ+ | 4.540 | 4.54 (ECβ+), 6.02 (α), ECβ+ = 90, α = 10 |
| At-206 | 30.00 | 0.50 | | ECβ+ | 5.720 | 5.72 (ECβ+), 5.888 (α), ECβ+ = 99.11, α = 0.89 |
| At-207 | | 1.80 | | ECβ+ | 3.910 | 3.91 (ECβ+), 5.873 (α), ECβ+ = 91.4, α = 8.6 |
| At-208 | | 1.63 | | ECβ+ | 4.973 | 4.973 (ECβ+), 5.751 (α), ECβ+ = 99.45, α = 0.55 |
| At-209 | | 5.41 | | ECβ+ | 3.486 | 3.486 (ECβ+), 5.757 (α), ECβ+ = 95.9, α = 4.1 |
| At-210 | | 8.1 | | ECβ+ | 3.981 | 3.981 (ECβ+), 5.631 (α), ECβ+ = 99.825, α = 0.175 |
| At-211 | | 7.21 | | α+ | 5.98 | 0.786 (ECβ+), 5.982 (α), EC = 58.2, α = 41.8 |
| At-220 | 3.71 | | | β- | | α = 8, β- = 92, 3.65 (ECβ+), 6.05 (α) |
| At-221 | 2.30 | | | β- | | |

| **Radon** | | | | | | |
|---|---|---|---|---|---|---|
| Rn-205 | 2.80 | | | ECβ+ | 5.240 | 5.24 (ECβ+), 6.39 (α), ECβ+ = 77, α = 23 |
| Rn-206 | 5.67 | | | α | 6.384 | 6.384 (α), 3.,31 (ECβ+), α = 63, ECβ+ = 37 |
| Rn-207 | 9.25 | | | ECβ+ | 4.610 | 4.61 (ECβ+), 6.251 (α), ECβ+ = 79, α = 21 |
| Rn-208 | 24.35 | 0.41 | | α | 6.260 | 6.26 (α), 2.85 (ECβ+), α = 62, ECβ+ = 38 |
| Rn-209 | 28.50 | 0.48 | | ECβ+ | 3.930 | 3.93 (ECβ+), 6.155 (α), ECβ+ = 83, α = 17 |
| Rn-210 | | 2.40 | | α | 6.159 | 6.159 (α), 2.374 (ECβ+), α = 96, ECβ+ = 4 |
| Rn-211 | | 14.60 | | EC | 2.892 | 2.892 (ECβ+), 5.965 (α), EC = 72.6, α = 27.4 |
| Rn-212 | 23.90 | 0.40 | | α | 6.385 | |
| Rn-221 | 25.00 | | | β- | 1.220 | 1.22 (β-), 6.146 (α), β⁻ = 78, α = 22 |
| Rn-222 | | | 3.82 | α | 5.590 | |
| Rn-223 | 23.20 | | | β- | | β⁻ ≈ 100, α = 0.0004 |
| Rn-224 | 107.00 | | | β⁻ | 0.8 | |
| Rn-225 | 4.50 | | | β⁻ | | |
| Rn-226 | 7.40 | | | β⁻ | 1.4 | |

| **Francium** | | | | | | |
|---|---|---|---|---|---|---|
| Fr-210 | 3.18 | | | α | 6.700 | 6.7 (α), 6.262 (ECβ+), α = 60, ECβ+ = 40 |
| Fr-211 | 3.10 | | | α | 6.660 | 6.66 (α), 4.605 (ECβ+), α > 80, EC < 20 |
| Fr-212 | 20.00 | 0.33 | | ECβ+ | 5.117 | 5.117 (ECβ+), 6.529 (α), ECβ+ = 57, α = 43 |
| Fr-221 | 4.80 | 0.08 | | α | 6.458 | α ≈ 100, β⁻ = ?, ¹⁴C = 8.8·10 |
| Fr-222 | 14.40 | 0.24 | | β- | 2.033 | |
| Fr-223 | 21.80 | 0.36 | | β- | 1.149 | |
| Fr-224 | 3.33 | | | β⁻ | 2.83 | |
| Fr-225 | 4.00 | | | β⁻ | 1.866 | |
| Fr-227 | 2.47 | | | β⁻ | 2.49 | |

| **Radium** | | | | | | |
|---|---|---|---|---|---|---|
| Ra-213 | 2.74 | | | α | 6.859 | 6.859 (α), 3.88 (ECβ+), α = 80, ECβ+ = 20 |
| Ra-223 | | | 11.43 | α | 5.979 | |
| Ra-224 | | 87.84 | 3.66 | α | 5.789 | |
| Ra-225 | | | 14.80 | β- | 0.357 | |
| Ra-227 | 42.20 | 0.70 | | β- | 1.325 | |
| Ra-229 | 4.00 | | | β⁻ | 1.76 | |
| Ra-230 | 93.00 | 1.55 | | β- | 0.990 | |

| **Actinium** | | | | | | |
|---|---|---|---|---|---|---|
| Ac-223 | 2.10 | 0.04 | | α | 6.783 | |
| Ac-224 | | 2.90 | | α | 1.403 | 1.403 (EC), 6.327 (α), 0.232 (β-), EC = 90.9, α = 9.1, β- < 1.6 |
| Ac-225 | | | 10.00 | α | 5.935 | |
| Ac-226 | | 29.00 | 1.21 | β- | 1.117 | 1.117 (β⁻), 0.64 (EC), 5.563 (α), β- ≈ 83, EC = 17, α = 6·10-3 |
| Ac-228 | | 6.13 | | β- | 2.127 | |
| Ac-229 | 62.70 | | | β⁻ | 1.1 | |
| Ac-231 | 7.50 | | | β⁻ | 2.1 | |

| **Thorium** | | | | | | |
|---|---|---|---|---|---|---|
| Th-225 | 8.72 | | | α | 6.922 | 6.922 (α), 0.675 (EC), α ≈ 90, EC ≈ 10 |
| Th-226 | 30.90 | 0.52 | | α | 6.451 | |
| Th-227 | | | 18.72 | α | 6.051 | |
| Th-231 | | 25.52 | 1.06 | β- | 0.389 | |
| Th-233 | 22.30 | | | β⁻ | 1.245 | |
| Th-234 | | | 24.10 | β- | 0.273 | |
| Th-235 | 7.10 | | | β⁻ | 1.93 | |
| Th-236 | 37.00 | 0.62 | | β⁻ | | |
| Th-237 | 5.00 | | | β⁻ | | |

| **Protactinium** | | | | | | |
|---|---|---|---|---|---|---|
| Pa-227 | 38.30 | 0.64 | | α | 6.580 | 6.580 (α), 1.019 (EC), α = 85, EC = 15 |
| Pa-228 | | 22.00 | | ECβ+ | 2.148 | 2.148 (ECβ+), 6.265 (α), ECβ+ = 98.0, α = 2.0 |
| Pa-229 | | 36.00 | 1.50 | EC | 0.316 | |
| Pa-230 | | | 17.40 | ECβ+ | 1.310 | 1.310 (ECβ+), 0.563 (β-), 5.439 (α), ECβ+ = 91.6, β- = 8.4, α = 0.0032 |
| Pa-232 | | 31.44 | 1.31 | β- | 1337.000 | |
| Pa-233 | | | 27.00 | β- | 0.571 | |
| Pa-234 | | 6.70 | | β- | 2.197 | |
| Pa-235 | 24.50 | | | β⁻ | 1.41 | |
| Pa-236 | 9.10 | | | β⁻ | 2.9 | |
| Pa-237 | 8.70 | | | β⁻ | 2.25 | |
| Pa-238 | 2.30 | | | β⁻ | 3.46 | |

| **Uranium** | | | | | | |
|---|---|---|---|---|---|---|
| U-228 | 9.10 | | | α | 6.801 | 6.804 (α), 0.307 (EC), α > 95, EC < 5 |
| U-229 | 58.00 | 0.97 | | ECβ+ | 1.309 | 1.309 (ECβ+), 6.475 (α), ECβ+ ≈ 80, α ≈ 20 |
| U-230 | | | 20.80 | α | 5.993 | |
| U-231 | | 100.80 | 4.20 | EC | 0.360 | |
| U-235m1 | 25.00 | | | IT | | |
| U-237 | | 162.00 | 6.75 | β- | 0.519 | |
| U-239 | 23.54 | 0.39 | | β- | 1.265 | |
| U-240 | | 14.10 | | β- | 0.338 | |
| U-242 | 16.80 | | | β⁻ | | |

| **Neptunium** | | | | | | |
|---|---|---|---|---|---|---|
| Np-229 | 4.00 | | | α | 2.560 | 7.01 (α), 2.56 (EC), α > 50, EC < 50 |
| Np-230 | 4.60 | | | ECβ+ | 3.610 | 3.,61 (ECβ+), 6.78 (α), ECβ+ < 97, α > 3 |
| Np-231 | 48.80 | | | ECβ+ | 1.840 | 1.84 (EC), 6.37 (α), EC = 98, α = 2 |
| Np-232 | 14.70 | 0.25 | | ECβ+ | 2.700 | |
| Np-233 | 36.20 | 0.60 | | EC | 1.230 | |
| Np-234 | | 105.60 | 4.40 | ECβ+ | 1.810 | |
| Np-236m1 | | 22.50 | | EC | 1.000 | 1.00 (EC), 0.55 (β-), EC = 52, β- = 48 |
| Np-238 | | 50.81 | 2.12 | β- | 1.292 | |
| Np-239 | | 56.52 | 2.36 | β- | 0.722 | |
| Np-240m1 | 7.40 | 0.12 | | β- | 2.200 | |
| Np-240 | 65.00 | 1.08 | | β- | 2.200 | |
| Np-241 | 13.90 | | | β⁻ | 1.31 | |
| Np-242 | 5.50 | | | β⁻ | 2.7 | |
| Np242m1 | 2.20 | | | β⁻ | 2.7 | |
| Np-244 | 2.29 | | | β⁻ | | |

| **Plutonium** | | | | | | |
|---|---|---|---|---|---|---|
| Pu-231 | 8.60 | | | ECβ+, α | | |
| Pu-232 | 34.10 | | | ECβ+ | 1.06 | 1.06 (ECβ+), 6.716 (α), EC = 77, α = 23 |
| Pu-233 | 20.90 | 0.35 | | ECβ+ | 1.900 | |
| Pu-234 | | 8.80 | | EC | 0.388 | 0.388 (ECβ+), 6.31 (α), EC ≈ 94, α ≈ 6 |
| Pu-235 | 25.30 | 0.42 | | ECβ+ | 1.170 | |
| Pu-237 | | | 45.30 | EC | 0.220 | |
| Pu-243 | | 4.96 | | β- | 0.528 | |
| Pu-245 | | 10.50 | | β- | 1.205 | |
| Pu-246 | | | 10.85 | β- | 0.401 | |
| Pu-247 | | | 2.27 | β⁻ | | |

| **Americium** | | | | | | |
|---|---|---|---|---|---|---|
| Am-234 | 2.32 | | | | | EC ≈ 100, α = 0.039, ECSF = 0.0066 |
| Am-235 | 15.00 | | | | | |
| Am-237 | 73.00 | 1.22 | | EC | 1.730 | |
| Am-238 | 98.00 | 1.63 | | EC | 2.260 | |
| Am-239 | | 11.90 | | EC | 0.803 | |
| Am-240 | | 50.80 | 2.12 | EC | 1.379 | |
| Am-242 | | 16.02 | | β- | 0.665 | 0.665 (β-), 0.751 (EC), β- = 82.7, EC = 17.3 |
| Am-244m1 | 26.00 | 0.43 | | β- | 1.516 | |
| Am-244 | | 10.10 | | β- | 1.428 | |
| Am-245 | | 2.05 | | β- | 0.894 | |
| Am-246m1 | 25.00 | 0.42 | | β- | 2.376 | |
| Am-246 | 39.00 | 0.65 | | β- | 2.376 | |
| Am-247 | 23.00 | | | β⁻ | 1.7 | |
| Am-248 | | | | β⁻ | 3.1 | |

| **Curium** | | | | | | |
|---|---|---|---|---|---|---|
| Cm-236 | 10.00 | | | ECβ+ | 1.710 | |
| Cm-237 | 20.00 | | | | | |
| Cm-238 | | 2.40 | | EC | 0.970 | 0.97 (EC), 6.62 (α), EC = 96.16, α = 3.84 |
| Cm-239 | | 2.90 | | EC | 1.700 | |
| Cm-240 | | | 27.00 | α | 6.397 | |
| Cm-241 | | | 32.80 | EC | 0.767 | |
| Cm-242 | | | 162.80 | α | 6.216 | |
| Cm-249 | 64.15 | 1.07 | | β- | | |
| Cm-251 | 16.80 | | | β⁻ | 1.42 | |
| Cm-252 | | | 2 | β⁻ | | |

| **Berkelium** | | | | | | |
|---|---|---|---|---|---|---|
| Bk-240 | 4.80 | | | ECβ+ | 3.94 | |
| Bk-242 | 7.00 | 0.12 | | ECβ+ | 3.000 | |
| Bk-243 | | 4.50 | | EC | 1.508 | |
| Bk-244 | | 4.35 | | EC | 2.260 | |
| Bk-245 | | 118.56 | 4.94 | EC | 0.810 | |
| Bk-246 | | 43.92 | 1.83 | EC | 1.350 | 1.35 (EC), 6.07 (α), EC = 100, α < 0,2 |
| Bk-248m1 | | 23.7 | | β- | 0.870 | β- = 70, EC = 30, α < 0.001, 0,87 (β-), 0.717 (EC), 5.803 (a) |
| Bk-249 | | | 320.00 | β- | 0.125 | |
| Bk-250 | | 3.22 | | β- | 1.780 | |
| Bk-251 | 55.60 | | | β- | 1.093 | β⁻ ≈ 100, α ≈ 1·10⁻⁵ |

| **Californium** | | | | | | |
|---|---|---|---|---|---|---|
| Cf-241 | 3.78 | | | EC | 3.300 | EC ≈ 75, α ≈ 25, 3.3 (EC), 7.66 (a) |
| Cf-242 | 3.49 | | | α | 7.516 | α = 65, SF < 1,4·10⁻² |
| Cf-243 | 10.70 | | | EC | 2.220 | EC ≈ 86, α ≈ 14, 2.22 (EC), 7.39 (a) |
| Cf-244 | 19.40 | 0.32 | | α | 7.329 | |
| Cf-245 | 45.00 | 0.75 | | EC | 1.569 | EC = 64, α = 36, 1.569 (EC), 7.256 (α) |
| Cf-246 | | 35.70 | 1.49 | α | 6.862 | α ≈ 100, SF = 2,3·10-4, EC < 5·10-4 |
| Cf-247 | | 3.11 | | EC | 0.646 | EC ≈ 100, α = 0.035 |
| Cf-248 | | | 333.50 | α | 6.361 | |
| Cf-253 | | | 17.81 | β- | 0.285 | |
| Cf-254 | | | 60.50 | SF | 5.926 | |
| Cf-255 | 85.00 | | | β- | 0.700 | |
| Cf-256 | 12.30 | | | α | 5.600 | SF = 100, β⁻ < 1, α ≈ 1·10⁻⁶ |

| **Einsteinium** | | | | | | |
|---|---|---|---|---|---|---|
| Es-246 | 7.70 | | | EC | 3.880 | EC = 90.1, α = 9,9, ECSF = 0.003 |
| Es-247 | 4.55 | | | EC | 2.480 | 2.48 (EC), 7.49 (α), EC ≈ 93, α ≈ 7 |
| Es-248 | 27.00 | 0.45 | | EC | | |
| Es-249 | 102.00 | 1.70 | | EC | 1.450 | |
| Es-250 | | 8.60 | | EC | 2.100 | |
| Es-250m1 | 132.00 | 2.2 | | EC | 2.100 | 2.10 (EC), 6.88 (α), EC ≈ 100, α < 1 |
| Es-251 | | 33.00 | 1.38 | EC | 0.376 | |
| Es-253 | | | 20.47 | α | 6.739 | |
| Es-254m1 | | 39.30 | 1.64 | α, β- | | |
| Es-254 | | | 275.70 | α | 6.618 | |
| Es-255 | | | 39.80 | β- | 0.288 | |
| Es-256 | 25.40 | | | β- | 1.67 | |
| Es-256m1 | 456.00 | 7.6 | | β- | 1.67 | β⁻ ≈ 100, SF = 0.002 |
| Es-257 | | | 7.8 | | | |

| **Fermium** | | | | | | |
|---|---|---|---|---|---|---|
| FM-249 | 2.60 | | | EC | 2.440 | EC ≈ 85, α ≈ 15, 2.44 (EC), 7.81 (α) |
| Fm-250 | 30.00 | 0.50 | | α | 7.557 | 7.557 (α), 0.8 (EC), α > 90, EC < 10, SF = 0.0069 |
| Fm-251 | | 5.30 | | EC | 1.474 | 1.474 (EC), 7.425 (α), EC = 98.20, α = 1.80 |
| Fm-252 | | 22.70 | | α | 7.425 | |
| Fm-253 | | 72.00 | 3.00 | EC | 0.333 | 0.333 (EC), 7.197 (α), EC = 88, α = 12 |
| Fm-254 | | 3.24 | | α | 7.307 | α ≈ 100, SF = 0.0592 |
| Fm-255 | | 20.07 | | α | 7.241 | |
| Fm-256 | 157.60 | 2.60 | | α | 7.027 | SF = 91.9, α = 8.1 |
| Fm-257 | | | 100.50 | α | 6.864 | |

| **Mendelevium** | | | | | | |
|---|---|---|---|---|---|---|
| Md-251 | 4.00 | | | EC | 3.070 | 3,07 (EC), 8,02 (α), EC > 90, α < 10 |
| Md-252 | 2.30 | | | EC | 3.89 | EC > 50, α < 50 |
| Md-253 | 6.00 | | | ECβ+ | 1.96 | |
| Md-254 | 10.00 | | | EC | 2.68 | EC < 100 |
| Md-254m1 | 28.00 | | | EC | | EC < 100 |
| Md-255 | 27.00 | | | EC | 1.043 | 1.043 (EC), 7.907 (α), EC = 92, α = 8, SF < 1.4 |
| Md-256 | 78.10 | | | EC | 2.130 | 2.13 (EC), 7.897 (α), EC = 90.7, α = 9.3, SF < 2.8 |
| Md-257 | | 5.52 | | EC | 0.406 | 0.406 (EC), 7.271 (α), EC = 85, α = 15, SF < 1 |
| Md-258 | | | 51.50 | α | 7.241 | 7.271 (α), 1.23 (EC), α ≈ 100, SF < 0.003, β- < 0.003, EC < 0.003 |
| Md-258m1 | | 57.00 | | EC | 1.230 | EC > 70, SF < 30, α < 1.2, β- < 30 |
| Md-259 | 96.00 | 1.60 | | α | 7.100 | SF > 73, α < 25, β- < 10, 7.0 (α), 1.0 (β-) |
| Md-260 | | | 27.80 | α | 7.000 | SF > 73, α < 25, β⁻ < 10 |

| **Nobelium** | | | | | | |
|---|---|---|---|---|---|---|
| No-255 | 3.10 | | | α | 8.445 | α = 61.4, EC = 38.w6 |
| No-259 | 58.00 | | | α | 7.910 | α ≈ 100, EC = 25, SF < 10 |

| **Lawrencium** | | | | | | |
|---|---|---|---|---|---|---|
| Lr-261 | 39.00 | | | SF | | SF < 100 |
| Lr-262 | 216.00 | | | EC | 2.1 | EC > 10, SF < 10 |

| **Rutherfordium** | | | | | | |
|---|---|---|---|---|---|---|
| Rf-263 | 15.00 | | | SF | | |

| **Seaborgium** | | | | | | |
|---|---|---|---|---|---|---|
| Sg-271 | 2.40 | | | α, SF | | α > 50, SF < 50 |

| **Hassium** | | | | | | |
|---|---|---|---|---|---|---|
| Hs-278 | 11.00 | | | SF | | |

| **Meitnerium** | | | | | | |
|---|---|---|---|---|---|---|
| Mt-278 | 30.00 | | | α | 9.1 | |

| **Roentgenium** | | | | | | |
|---|---|---|---|---|---|---|
| Rg-282 | 4.00 | | | α, SF | 9.4 | |

| **Nithonium** | | | | | | |
|---|---|---|---|---|---|---|
| Nh-285 | 2.00 | | | α, SF | 10 | |
| Nh-286 | 5.00 | | | α | 9.7 | |
| Nh-287 | 20.00 | | | α, SF | 9.3 | |

In an embodiment of the present invention, the radionuclide is used for diagnosis. Preferably, the radioactive isotope is selected from the group, but not limited to, comprising ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ¹⁷⁷Lu, ²⁰¹Tl, ²⁰³Pb, ¹⁸F, ⁷⁶Br, ⁷⁷Br, ¹²³I, ¹²⁴I, ¹²⁵I. More preferably, the radionuclide is selected from the group comprising ⁴³Sc, ⁴⁴Sc, ⁶⁴Cu, ⁶⁷Ga, ⁶⁸Ga, ⁸⁶Y,⁸⁹Zr, ^{99m}Tc, ¹¹¹In, ¹⁵²Tb, ¹⁵⁵Tb, ²⁰³Pb, ¹⁸F, ⁷⁶Br, ⁷⁷Br, ¹²³I, ¹²⁴I, ¹²⁵I. Even more preferably, the radionuclide is ⁶⁴Cu, ⁶⁸Ga, ⁸⁹Zr, ^{99m}Tc, ¹¹¹In, ¹⁸F, ¹²³I, and ¹²⁴I. It will however, also be acknowledged by a person skilled in the art that the use of said radionuclide is not limited to diagnostic purposes, but encompasses their use in therapy and theragnostics when conjugated to the compound of the invention.

In an embodiment of the present invention, the radionuclide is used for therapy. Preferably, the radioactive isotope is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I, ²¹¹At. More preferably, the radioactive isotope is selected from the group comprising ⁴⁷Sc, ⁶⁷Cu, ⁹⁰Y, ¹⁷⁷Lu, ¹⁸⁸Re, ²¹²Pb, ²¹³Bi, ²²⁵Ac, ²²⁷Th, ¹³¹I, ²¹¹At. Even more preferably, the radionuclide is selected from the group comprising ⁹⁰Y, ¹⁷⁷Lu, ²²⁵Ac, ²²⁷Th, ¹³¹I and ²¹¹At. It will however, also be acknowledged by a person skilled in the art that the use of said radionuclide is not limited to therapeutic purposes, but encompasses their use in diagnostic and theragnostics when conjugated to the compound of the invention.

In an embodiment the compound of the invention is present as a pharmaceutically acceptable salt.

A "pharmaceutically acceptable salt" of the compound of the present invention is preferably an acid salt or a base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Compounds of the invention are capable of forming internal salts which are also pharmaceutically acceptable salts.

Suitable pharmaceutically acceptable salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, *i.e.,* 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

A "pharmaceutically acceptable solvate" of the compound of the invention is preferably a solvate of the compound of the invention formed by association of one or more solvent molecules to one or more molecules of a compound of the invention. Preferably, the solvent is one which is generally considered in the art to be suitable for use in contact with the tissues of human beings or animals without excessive toxicity or carcinogenicity, and preferably without irritation, allergic response, or other problem or complication. Such solvent includes an organic solvent such as alcohols, ethers, esters and amines.

A "hydrate" of the compound of the invention is formed by association of one or more water molecules to one or more molecules of a compound of the invention. Such hydrate includes but is not limited to a hemi-hydrate, mono-hydrate, dihydrate, trihydrate and tetrahydrate. Independent of the hydrate composition all hydrates are generally considered as pharmaceutically acceptable.

The compound of the invention has a high binding affinity to FAP and a high inhibitory activity on FAP. Because of this high binding affinity, the compound of the invention is effective as, useful as and/or suitable as a targeting agent and, if conjugated to another moiety, as a targeting moiety. As preferably used herein a targeting agent is an agent which interacts with the target molecule which is in the instant case said FAP. In terms of cells and tissues thus targeted by the compound of the invention any cell and tissue, respectively, expressing said FAP is or may be targeted.

In an embodiment, the compound interacts with a fibroblast activation protein (FAP), preferably with human FAP having an amino acid sequence of SEQ ID NO: 1 or a homolog thereof, wherein the amino acid sequence of the homolog has an identity of FAP that is at least 85% to the amino acid sequence of SEQ ID NO: 1. In preferred embodiments, the identity is 90%, preferably 95 %, 96 %, 97 %, 98 % or 99%.

The identity between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or protein or polypeptide which is said to be identical or to be tested whether it is identical, and if so, to what extent, to a different protein or polypeptide, whereby such different protein or polypepetide is also referred to as the reference sequence and is preferably the protein or polypeptide of wild type, more preferably the human FAP of SEQ ID NO: 1.

Optimal alignment of sequences for comparison can be conducted, e.g., by the local homology algorithm of Smith & Waterman (Smith, et al., Advances in Applied Mathematics, 1981, 2: 482), by the homology alignment algorithm of Needleman & Wunsch (Needleman, et al., J Mol Biol, 1970, 48: 443), by the search for similarity method of Pearson & Lipman (Pearson, et al., Proc Natl Acad Sci U S A, 1988, 85: 2444), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al., 1990 (Altschul, et al., J Mol Biol, 1990, 215: 403) and Altschul et al., 1997 (Altschul, et al., Nucleic Acids Res, 1997, 25: 3389). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al. (McGinnis, et al., Nucleic Acids Res, 2004, 32: W20).

It is within the present invention that the compound of the invention is used or is for use in a method for the treatment of a disease as disclosed herein. Such method, preferably, comprises the step of administering to a subject in need thereof a therapeutically effective amount of the compound of the invention. Such method includes, but is not limited to, curative or adjuvant cancer treatment. It is used as palliative treatment where cure is not possible and the aim is for local disease control or symptomatic relief or as therapeutic treatment where the therapy has survival benefit and it can be curative.

The method for the treatment of a disease as disclosed herein includes the treatment of the disease disclosed herein, including tumors and cancer, and may be used either as the primary therapy or as second, third, fourth or last line therapy. It is also within the present invention to combine the compound of the invention with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

In an embodiment of the present invention, the disease is selected from the group comprising neoplasm nos, neoplasm, benign, neoplasm, uncertain whether benign or malignant, neoplasm, malignant, neoplasm, metastatic, neoplasm, malignant, uncertain whether primary or metastatic, tumor cells, benign, tumor cells, uncertain whether benign or malignant, tumor cells, malignant, malignant tumor, small cell type, malignant tumor, giant cell type, malignant tumor, fusiform cell type, epithelial neoplasms nos, epithelial tumor, benign, carcinoma in situ nos, carcinoma nos, carcinoma, metastatic nos, carcinomatosis, epithelioma, benign, epithelioma, malignant, large cell carcinoma nos, carcinoma, undifferentiated type nos, carcinoma, anaplastic type nos, pleomorphic carcinoma, giant cell and spindle cell carcinoma, giant cell carcinoma, spindle cell carcinoma, pseudosarcomatous carcinoma, polygonal cell carcinoma, spheroidal cell carcinoma, tumorlet, small cell carcinoma nos, oat cell carcinoma, small cell carcinoma, fusiform cell type, papillary and squamous cell neoplasms, papilloma nos, papillary carcinoma in situ, papillary carcinoma nos, verrucous papilloma, verrucous carcinoma nos, squamous cell papilloma, papillary squamous cell carcinoma, inverted papilloma, papillomatosis nos, squamous cell carcinoma in situ nos, squamous cell carcinoma nos, squamous cell carcinoma, metastatic nos, squamous cell carcinoma, keratinizing type nos, squamous cell carcinoma, large cell, nonkeratinizing type, squamous cell carcinoma, small cell, nonkeratinizing type, squamous cell carcinoma, spindle cell type, adenoid squamous cell carcinoma, squamous cell carcinoma in situ with questionable stromal invasion, squamous cell carcinoma, microinvasive, queyrat's erythroplasia, bowen's disease, lymphoepithelial carcinoma, basal cell neoplasms, basal cell tumor, basal cell carcinoma nos, multicentric basal cell carcinoma, basal cell carcinoma, morphea type, basal cell carcinoma, fibroepithelial type, basosquamous carcinoma, metatypical carcinoma, intraepidermal epithelioma of jadassohn, trichoepithelioma, trichofolliculoma, tricholemmoma, pilomatrixoma, transitional cell papillomas and carcinomas, transitional cell papilloma nos, urothelial papilloma, transitional cell carcinoma in situ, transitional cell carcinoma nos, schneiderian papilloma, transitional cell papilloma, inverted type, schneiderian carcinoma, transitional cell carcinoma, spindle cell type, basaloid carcinoma, cloacogenic carcinoma, papillary transitional cell carcinoma, adenomas and adenocarcinomas, adenoma nos, bronchial adenoma nos, adenocarcinoma in situ, adenocarcinoma nos, adenocarcinoma, metastatic nos, scirrhous adenocarcinoma, linitis plastica, superficial spreading adenocarcinoma, adenocarcinoma, intestinal type, carcinoma, diffuse type, monomorphic adenoma, basal cell adenoma, islet cell adenoma, islet cell carcinoma, insulinoma nos, insulinoma, malignant, glucagonoma nos, glucagonoma, malignant, gastrinoma nos, gastrinoma, malignant, mixed islet cell and exocrine adenocarcinoma, bile duct adenoma, cholangiocarcinoma, bile duct cystadenoma, bile duct cystadenocarcinoma, liver cell adenoma, hepatocellular carcinoma nos, hepatocholangioma, benign, combined hepatocellular carcinoma and cholangiocarcinoma, trabecular adenoma, trabecular adenocarcinoma, embryonal adenoma, eccrine dermal cylindroma, adenoid cystic carcinoma, cribriform carcinoma, adenomatous polyp nos, adenocarcinoma in adenomatous polyp, tubular adenoma nos, tubular adenocarcinoma, adenomatous polyposis coli, adenocarcinoma in adenomatous polyposis coli, multiple adenomatous polyps, solid carcinoma nos, carcinoma simplex, carcinoid tumor nos, carcinoid tumor, malignant, carcinoid tumor, argentaffin nos, carcinoid tumor, argentaffin, malignant, carcinoid tumor, nonargentaffin nos, carcinoid tumor, nonargentaffin, malignant, mucocarcinoid tumor, malignant, composite carcinoid, pulmonary adenomatosis, bronchiolo-alveolar adenocarcinoma, alveolar adenoma, alveolar adenocarcinoma, papillary adenoma nos, papillary adenocarcinoma nos, villous adenoma nos, adenocarcinoma in villous adenoma, villous adenocarcinoma, tubulovillous adenoma, chromophobe adenoma, chromophobe carcinoma, acidophil adenoma, acidophil carcinoma, mixed acidophil-basophil adenoma, mixed acidophil-basophil carcinoma, oxyphilic adenoma, oxyphilic adenocarcinoma, basophil adenoma, basophil carcinoma, clear cell adenoma, clear cell adenocarcinoma nos, hypernephroid tumor, renal cell carcinoma, clear cell adenofibroma, granular cell carcinoma, chief cell adenoma, water-clear cell adenoma, water-clear cell adenocarcinoma, mixed cell adenoma, mixed cell adenocarcinoma, lipoadenoma, follicular adenoma, follicular adenocarcinoma nos, follicular adenocarcinoma, well differentiated type, follicular adenocarcinoma, trabecular type, microfollicular adenoma, macrofollicular adenoma, papillary and follicular adenocarcinoma, nonencapsulated sclerosing carcinoma, multiple endocrine adenomas, juxtaglomerular tumor, adrenal cortical adenoma nos, adrenal cortical carcinoma, adrenal cortical adenoma, compact cell type, adrenal cortical adenoma, heavily pigmented variant, adrenal cortical adenoma, clear cell type, adrenal cortical adenoma, glomerulosa cell type, adrenal cortical adenoma, mixed cell type, endometrioid adenoma nos, endometrioid adenoma, borderline malignancy, endometrioid carcinoma, endometrioid adenofibroma nos, endometrioid adenofibroma, borderline malignancy, endometrioid adenofibroma, malignant, adnexal and skin appendage neoplasms, skin appendage adenoma, skin appendage carcinoma, sweat gland adenoma, sweat gland tumor nos, sweat gland adenocarcinoma, apocrine adenoma, apocrine adenocarcinoma, eccrine acrospiroma, eccrine spiradenoma, hidrocystoma, papillary hydradenoma, papillary syringadenoma, syringoma nos, sebaceous adenoma, sebaceous adenocarcinoma, ceruminous adenoma, ceruminous adenocarcinoma, mucoepidermoid neoplasms, mucoepidermoid tumor, mucoepidermoid carcinoma, cystic, mucinous, and serous neoplasms, cystadenoma nos, cystadenocarcinoma nos, serous cystadenoma nos, serous cystadenoma, borderline malignancy, serous cystadenocarcinoma nos, papillary cystadenoma nos, papillary cystadenoma, borderline malignancy, papillary cystadenocarcinoma nos, papillary serous cystadenoma nos, papillary serous cystadenoma, borderline malignancy, papillary serous cystadenocarcinoma, serous surface papilloma nos, serous surface papilloma, borderline malignancy, serous surface papillary carcinoma, mucinous cystadenoma nos, mucinous cystadenoma, borderline malignancy, mucinous cystadenocarcinoma nos, papillary mucinous cystadenoma nos, papillary mucinous cystadenoma, borderline malignancy, papillary mucinous cystadenocarcinoma, mucinous adenoma, mucinous adenocarcinoma, pseudomyxoma peritonei, mucin-producing adenocarcinoma, signet ring cell carcinoma, metastatic signet ring cell carcinoma, ductal, lobular, and medullary neoplasms, intraductal carcinoma, noninfiltrating nos, infiltrating duct carcinoma, comedocarcinoma, noninfiltrating, comedocarcinoma nos, juvenile carcinoma of the breast, intraductal papilloma, noninfiltrating intraductal papillary adenocarcinoma, intracystic papillary adenoma, noninfiltrating intracystic carcinoma, intraductal papillomatosis nos, subareolar duct papillomatosis, medullary carcinoma nos, medullary carcinoma with amyloid stroma, medullary carcinoma with lymphoid stroma, lobular carcinoma in situ, lobular carcinoma nos, infiltrating ductular carcinoma, inflammatory carcinoma, paget's disease, mammary, paget's disease and infiltrating duct carcinoma of breast, paget's disease, extramammary, acinar cell neoplasms, acinar cell adenoma, acinar cell tumor, acinar cell carcinoma, complex epithelial neoplasms, adenosquamous carcinoma, adenolymphoma, adenocarcinoma with squamous metaplasia, adenocarcinoma with cartilaginous and osseous metaplasia, adenocarcinoma with spindle cell metaplasia, adenocarcinoma with apocrine metaplasia, thymoma, benign, thymoma, malignant, specialized gonadal neoplasms, sex cord-stromal tumor, thecoma nos, theca cell carcinoma, luteoma nos, granulosa cell tumor nos, granulosa cell tumor, malignant, granulosa cell-theca cell tumor, androblastoma, benign, androblastoma nos, androblastoma, malignant, sertoli-leydig cell tumor, gynandroblastoma, tubular androblastoma nos, sertoli cell carcinoma, tubular androblastoma with lipid storage, leydig cell tumor, benign, leydig cell tumor nos, leydig cell tumor, malignant, hilar cell tumor, lipid cell tumor of ovary, adrenal rest tumor, paragangliomas and glomus tumors, paraganglioma nos, paraganglioma, malignant, sympathetic paraganglioma, parasympathetic paraganglioma, glomus jugulare tumor, aortic body tumor, carotid body tumor, extra-adrenal paraganglioma nos, extra-adrenal paraganglioma, malignant, pheochromocytoma nos, pheochromocytoma, malignant, glomangiosarcoma, glomus tumor, glomangioma, nevi and melanomas, pigmented nevus nos, malignant melanoma nos, nodular melanoma, balloon cell nevus, balloon cell melanoma, halo nevus, fibrous papule of the nose, neuronevus, magnocellular nevus, nonpigmented nevus, amelanotic melanoma, junctional nevus, malignant melanoma in junctional nevus, precancerous melanosis nos, malignant melanoma in precancerous melanosis, hutchinson's melanotic freckle, malignant melanoma in hutchinson's melanotic freckle, superficial spreading melanoma, intradermal nevus, compound nevus, giant pigmented nevus, malignant melanoma in giant pigmented nevus, epithelioid and spindle cell nevus, epithelioid cell melanoma, spindle cell melanoma nos, spindle cell melanoma, type a, spindle cell melanoma, type b, mixed epithelioid and spindle cell melanoma, blue nevus nos, blue nevus, malignant, cellular blue nevus, soft tissue tumors and sarcomas nos, soft tissue tumor, benign, sarcoma nos, sarcomatosis nos, spindle cell sarcoma, giant cell sarcoma, small cell sarcoma, epithelioid cell sarcoma, fibromatous neoplasms, fibroma nos, fibrosarcoma nos, fibromyxoma, fibromyxosarcoma, periosteal fibroma, periosteal fibrosarcoma, fascial fibroma, fascial fibrosarcoma, infantile fibrosarcoma, elastofibroma, aggressive fibromatosis, abdominal fibromatosis, desmoplastic fibroma, fibrous histiocytoma nos, atypical fibrous histiocytoma, fibrous histiocytoma, malignant, fibroxanthoma nos, atypical fibroxanthoma, fibroxanthoma, malignant, dermatofibroma nos, dermatofibroma protuberans, dermatofibrosarcoma nos, myxomatous neoplasms, myxoma nos, myxosarcoma, lipomatous neoplasms, lipoma nos, liposarcoma nos, fibrolipoma, liposarcoma, well differentiated type, fibromyxolipoma, myxoid liposarcoma, round cell liposarcoma, pleomorphic liposarcoma, mixed type liposarcoma, intramuscular lipoma, spindle cell lipoma, angiomyolipoma, angiomyoliposarcoma, angiolipoma nos, angiolipoma, infiltrating, myelolipoma, hibemoma, lipoblastomatosis, myomatous neoplasms, leiomyoma nos, intravascular leiomyomatosis, leiomyosarcoma nos, epithelioid leiomyoma, epithelioid leiomyosarcoma, cellular leiomyoma, bizarre leiomyoma, angiomyoma, angiomyosarcoma, myoma, myosarcoma, rhabdomyoma nos, rhabdomyosarcoma nos, pleomorphic rhabdomyosarcoma, mixed type rhabdomyosarcoma, fetal rhabdomyoma, adult rhabdomyoma, embryonal rhabdomyosarcoma, alveolar rhabdomyosarcoma, complex mixed and stromal neoplasms, endometrial stromal sarcoma, endolymphatic stromal myosis, adenomyoma, pleomorphic adenoma, mixed tumor, malignant nos, mullerian mixed tumor, mesodermal mixed tumor, mesoblastic nephroma, nephroblastoma nos, epithelial nephroblastoma, mesenchymal nephroblastoma, hepatoblastoma, carcinosarcoma nos, carcinosarcoma, embryonal type, myoepithelioma, mesenchymoma, benign, mesenchymoma nos, mesenchymoma, malignant, embryonal sarcoma, fibroepithelial neoplasms, brenner tumor nos, brenner tumor, borderline malignancy, brenner tumor, malignant, fibroadenoma nos, intracanalicular fibroadenoma nos, pericanalicular fibroadenoma, adenofibroma nos, serous adenofibroma, mucinous adenofibroma, cellular intracanalicular fibroadenoma, cystosarcoma phyllodes nos, cystosarcoma phyllodes, malignant, juvenile fibroadenoma, synovial neoplasms, synovioma, benign, synovial sarcoma nos, synovial sarcoma, spindle cell type, synovial sarcoma, epithelioid cell type, synovial sarcoma, biphasic type, clear cell sarcoma of tendons and aponeuroses, mesothelial neoplasms, mesothelioma, benign, mesothelioma, malignant, fibrous mesothelioma, benign, fibrous mesothelioma, malignant, epithelioid mesothelioma, benign, epithelioid mesothelioma, malignant, mesothelioma, biphasic type, benign, mesothelioma, biphasic type, malignant, adenomatoid tumor nos, germ cell neoplasms, dysgerminoma, seminoma nos, seminoma, anaplastic type, spermatocytic seminoma, germinoma, embryonal carcinoma nos, endodermal sinus tumor, polyembryoma, gonadoblastoma, teratoma, benign, teratoma nos, teratoma, malignant nos, teratocarcinoma, malignant teratoma, undifferentiated type, malignant teratoma, intermediate type, dermoid cyst, dermoid cyst with malignant transformation, struma ovarii nos, struma ovarii, malignant, strumal carcinoid, trophoblastic neoplasms, hydatidiform mole nos, invasive hydatidiform mole, choriocarcinoma, choriocarcinoma combined with teratoma, malignant teratoma, trophoblastic, mesonephromas, mesonephroma, benign, mesonephric tumor, mesonephroma, malignant, endosalpingioma, blood vessel tumors, hemangioma nos, hemangiosarcoma, cavernous hemangioma, venous hemangioma, racemose hemangioma, kupffer cell sarcoma, hemangioendothelioma, benign, hemangioendothelioma nos, hemangioendothelioma, malignant, capillary hemangioma, intramuscular hemangioma, kaposi's sarcoma, angiokeratoma, verrucous keratotic hemangioma, hemangiopericytoma, benign, hemangiopericytoma nos, hemangiopericytoma, malignant, angiofibroma nos, hemangioblastoma, lymphatic vessel tumors, lymphangioma nos, lymphangiosarcoma, capillary lymphangioma, cavernous lymphangioma, cystic lymphangioma, lymphangiomyoma, lymphangiomyomatosis, hemolymphangioma, osteomas and osteosarcomas, osteoma nos, osteosarcoma nos, chondroblastic osteosarcoma, fibroblastic osteosarcoma, telangiectatic osteosarcoma, osteosarcoma in paget's disease of bone, juxtacortical osteosarcoma, osteoid osteoma nos, osteoblastoma, chondromatous neoplasms, osteochondroma, osteochondromatosis nos, chondroma nos, chondromatosis nos, chondrosarcoma nos, juxtacortical chondroma, juxtacortical chondrosarcoma, chondroblastoma nos, chondroblastoma, malignant, mesenchymal chondrosarcoma, chondromyxoid fibroma, giant cell tumors, giant cell tumor of bone nos, giant cell tumor of bone, malignant, giant cell tumor of soft parts nos, malignant giant cell tumor of soft parts, miscellaneous bone tumors, ewing's sarcoma, adamantinoma of long bones, ossifying fibroma, odontogenic tumors, odontogenic tumor, benign, odontogenic tumor nos, odontogenic tumor, malignant, dentinoma, cementoma nos, cementoblastoma, benign, cementifying fibroma, gigantiform cementoma, odontoma nos, compound odontoma, complex odontoma, ameloblastic fibro-odontoma, ameloblastic odontosarcoma, adenomatoid odontogenic tumor, calcifying odontogenic cyst, ameloblastoma nos, ameloblastoma, malignant, odontoameloblastoma, squamous odontogenic tumor, odontogenic myxoma, odontogenic fibroma nos, ameloblastic fibroma, ameloblastic fibrosarcoma, calcifying epithelial odontogenic tumor, miscellaneous tumors, craniopharyngioma, pinealoma, pineocytoma, pineoblastoma, melanotic neuroectodermal tumor, chordoma, gliomas, glioma, malignant, gliomatosis cerebri, mixed glioma, subependymal glioma, subependymal giant cell astrocytoma, choroid plexus papilloma nos, choroid plexus papilloma, malignant, ependymoma nos, ependymoma, anaplastic type, papillary ependymoma, myxopapillary ependymoma, astrocytoma nos, astrocytoma, anaplastic type, protoplasmic astrocytoma, gemistocytic astrocytoma, fibrillary astrocytoma, pilocytic astrocytoma, spongioblastoma nos, spongioblastoma polare, astroblastoma, glioblastoma nos, giant cell glioblastoma, glioblastoma with sarcomatous component, primitive polar spongioblastoma, oligodendroglioma nos, oligodendroglioma, anaplastic type, oligodendroblastoma, medulloblastoma nos, desmoplastic medulloblastoma, medullomyoblastoma, cerebellar sarcoma nos, monstrocellular sarcoma, neuroepitheliomatous neoplasms, ganglioneuroma, ganglioneuroblastoma, ganglioneuromatosis, neuroblastoma nos, medulloepithelioma nos, teratoid medulloepithelioma, neuroepithelioma nos, spongioneuroblastoma, ganglioglioma, neurocytoma, pacinian tumor, retinoblastoma nos, retinoblastoma, differentiated type, retinoblastoma, undifferentiated type, olfactory neurogenic tumor, esthesioneurocytoma, esthesioneuroblastoma, esthesioneuroepithelioma, meningiomas, meningioma nos, meningiomatosis nos, meningioma, malignant, meningotheliomatous meningioma, fibrous meningioma, psammomatous meningioma, angiomatous meningioma, hemangioblastic meningioma, hemangiopericytic meningioma, transitional meningioma, papillary meningioma, meningeal sarcomatosis, nerve sheath tumor, neurofibroma nos, neurofibromatosis nos, neurofibrosarcoma, melanotic neurofibroma, plexiform neurofibroma, neurilemmoma nos, neurinomatosis, neurilemmoma, malignant, neuroma nos, granular cell tumors and alveolar soft part sarcoma, granular cell tumor nos, granular cell tumor, malignant, alveolar soft part sarcoma, lymphomas, nos or diffuse, lymphomatous tumor, benign, malignant lymphoma nos, malignant lymphoma, non hodgkin's type, malignant lymphoma, undifferentiated cell type nos, malignant lymphoma, stem cell type, malignant lymphoma, convoluted cell type nos, lymphosarcoma nos, malignant lymphoma, lymphoplasmacytoid type, malignant lymphoma, immunoblastic type, malignant lymphoma, mixed lymphocytic-histiocytic nos, malignant lymphoma, centroblastic-centrocytic, diffuse, malignant lymphoma, follicular center cell nos, malignant lymphoma, lymphocytic, well differentiated nos, malignant lymphoma, lymphocytic, intermediate differentiation nos, malignant lymphoma, centrocytic, malignant lymphoma, follicular center cell, cleaved nos, malignant lymphoma, lymphocytic, poorly differentiated nos, prolymphocytic lymphosarcoma, malignant lymphoma, centroblastic type nos, malignant lymphoma, follicular center cell, noncleaved nos, reticulosarcomas, reticulosarcoma nos, reticulosarcoma, pleomorphic cell type, reticulosarcoma, nodular, hodgkin's disease, hodgkin's disease nos, hodgkin's disease, lymphocytic predominance, hodgkin's disease, mixed cellularity, hodgkin's disease, lymphocytic depletion nos, hodgkin's disease, lymphocytic depletion, diffuse fibrosis, hodgkin's disease, lymphocytic depletion, reticular type, hodgkin's disease, nodular sclerosis nos, hodgkin's disease, nodular sclerosis, cellular phase, hodgkin's paragranuloma, hodgkin's granuloma, hodgkin's sarcoma, lymphomas, nodular or follicular, malignant lymphoma, nodular nos, malignant lymphoma, mixed lymphocytic-histiocytic, nodular, malignant lymphoma, centroblastic-centrocytic, follicular, malignant lymphoma, lymphocytic, well differentiated, nodular, malignant lymphoma, lymphocytic, intermediate differentiation, nodular, malignant lymphoma, follicular center cell, cleaved, follicular, malignant lymphoma, lymphocytic, poorly differentiated, nodular, malignant lymphoma, centroblastic type, follicular, malignant lymphoma, follicular center cell, noncleaved, follicular, mycosis fungoides, mycosis fungoides, sezary's disease, miscellaneous reticuloendothelial neoplasms, microglioma, malignant histiocytosis, histiocytic medullary reticulosis, letterer-siwe's disease, plasma cell tumors, plasma cell myeloma, plasma cell tumor, benign, plasmacytoma nos, plasma cell tumor, malignant, mast cell tumors, mastocytoma nos, mast cell sarcoma, malignant mastocytosis, burkitt's tumor, burkitt's tumor, leukemias, leukemias nos, leukemia nos, acute leukemia nos, subacute leukemia nos, chronic leukemia nos, aleukemic leukemia nos, compound leukemias, compound leukemia, lymphoid leukemias, lymphoid leukemia nos, acute lymphoid leukemia, subacute lymphoid leukemia, chronic lymphoid leukemia, aleukemic lymphoid leukemia, prolymphocytic leukemia, plasma cell leukemias, plasma cell leukemia, erythroleukemias, erythroleukemia, acute erythremia, chronic erythremia, lymphosarcoma cell leukemias, lymphosarcoma cell leukemia, myeloid leukemias, myeloid leukemia nos, acute myeloid leukemia, subacute myeloid leukemia, chronic myeloid leukemia, aleukemic myeloid leukemia, neutrophilic leukemia, acute promyelocytic leukemia, basophilic leukemias, basophilic leukemia, eosinophilic leukemias, eosinophilic leukemia, monocytic leukemias, monocytic leukemia nos, acute monocytic leukemia, subacute monocytic leukemia, chronic monocytic leukemia, aleukemic monocytic leukemia, miscellaneous leukemias, mast cell leukemia, megakaryocytic leukemia, megakaryocytic myelosis, myeloid sarcoma, hairy cell leukemia, miscellaneous myeloproliferative and lymphoproliferative disorders, polycythemia vera, acute panmyelosis, chronic myeloproliferative disease, myelosclerosis with myeloid metaplasia, idiopathic thrombocythemia, chronic lymphoproliferative disease.

in an embodiment of the present invention, the disease is selected from the group comprising tumors of pancreas, pancreatic adenocarcinoma, tumors of head of pancreas, of body of pancreas, of tail of pancreas, of pancreatic duct, of islets of langerhans, neck of pancreas, tumor of prostate, prostate adenocarcinoma, prostate gland, neuroendocrine tumors, breast cancer, tumor of central portion of breast, upper inner quadrant of breast, lower inner quadrant of breast, upper outer quadrant of breast, lower outer quadrant of breast, axillary tail of breast, overlapping lesion of breast, juvenile carcinoma of the breast, tumors of parathyroid gland, myeloma, lung cancer, small cell lung cancer, non-small cell lung cancer, tumor of main bronchus, of upper lobe lung, of middle lobe lung, of lower lobe lung, colorectal carcinoma, tumor of ascending colon, of hepatic flexure of colon, of transverse colon, of splenic flexure of colon, of descending colon, of sigmoid colon, of overlapping lesion of colon, of small intestine, tumors of liver, liver cell adenoma, hepatocellular carcinoma, hepatocholangioma, ombined hepatocellular carcinoma and cholangiocarcinoma, hepatoblastoma, ovarian carcinoma,sarcoma, osteosarcoma, fibrosarcoma, gastrointestinal stroma tumors, gastrointestinal tract, gastric carcinoma, thyroid carcinoma, medullary thyroid carcinoma, thyroid gland, renal cell carcinoma, renal pelvis, tumors of bladder, bladder carcinoma, tumors of trigone bladder, of dome bladder, of lateral wall bladder, of posterior wall bladder, of ureteric orifice, of urachus, overlapping lesion of bladder, basal cell carcinoma, basal cell neoplasms, basal cell tumor, basal cell carcinoma, multicentric basal cell carcinoma, basaloid carcinoma, basal cell adenoma, squamous cell carcinoma, oral squamous cell carcinoma, squamous cell carcinoma of the larynx, cervical carcinoma, tumors of exocervix, of overlapping lesion of cervix uteri, of cervix uteri, of isthmus uteri, tumors of uterus, tumors of ovary, tumors of cervical esophagus, of thoracic esophagus, of abdominal esophagus, of upper third of esophagus, of esophagus middle third, of esophagus lower third, of overlapping lesion of esophagus, endometrial carcinoma, head and neck cancer, lymphoma, malignant mesothelioma, mesothelial neoplasms, mesothelioma, fibrous mesothelioma, fibrous mesothelioma, epithelioid mesothelioma, epithelioid mesothelioma, duodenal carcinoma, neuroendocrine tumors, neuroendocrine tumors of the lung, neuroendocrine tumors of the pancreas, neuroendocrine tumors of the foregut, neuroendocrine tumors of the midgut, neuroendocrine tumors of the hindgut, gastroenteropancreatic neuroendocrine tumors, neuroendocrine carcinomas, neuroendocrine tumors of the breast, neuroendocrine tumors o the ovaries, testicular cancer, thymic carcinoma, tumors of stomach, fundus stomach, body stomach, gastric antrum, pylorus, lesser curvature of stomach, greater curvature of stomach, overlapping lesion of stomach, paragangliomas, ganglioma, melanomas, malignant melanoma, nodular melanoma, amelanotic melanoma, superficial spreading melanoma, epithelioid cell melanoma, spindle cell melanoma, mixed epithelioid and spindle cell melanoma.

In a still further embodiment, the aforementioned indications may occur in organs and tissues selected from the group comprising external upper lip, external lower lip, external lip nos, upper lip mucosa, lower lip mucosa, mucosa lip nos, commissure lip, overlapping lesion of lip, base of tongue nos, dorsal surface tongue nos, border of tongue, ventral surface of tongue nos, anterior 2/3 of tongue nos, lingual tonsil, overlapping lesion of tongue, tongue nos, upper gum, lower gum, gum nos, anterior floor of mouth, lateral floor of mouth, overlapping lesion of floor of mouth, floor of mouth nos, hard palate, soft palate nos, uvula, overlapping lesion of palate, palate nos, cheek mucosa, vestibule of mouth, retromolar area, overlapping lesion of other and unspecified parts of mouth, mouth nos, parotid gland, submaxillary gland, sublingual gland, overlapping lesion of major salivary glands, major salivary gland nos, tonsillar fossa, tonsillar pillar, overlapping lesion of tonsil, tonsil nos, vallecula, anterior surface of epiglottis, lateral wall oropharynx, posterior wall oropharynx, branchial cleft, overlapping lesion of oropharynx, oropharynx nos, superior wall of nasopharynx, posterior wall nasopharynx, lateral wall nasopharynx, anterior wall nasopharynx, overlapping lesion of nasopharynx, nasopharynx nos, pyriform sinus, postcricoid region, hypopharyngeal aspect of aryepiglottic fold, posterior wall hypopharynx, overlapping lesion of hypopharynx, hypopharynx nos, pharynx nos, laryngopharynx, waldeyer's ring, overlapping lesion of lip oral cavity and pharynx, cervical esophagus, thoracic esophagus, abdominal esophagus, upper third of esophagus, middle third of esophagus, esophagus lower third, overlapping lesion of esophagus, esophagus nos, cardia nos, fundus stomach, body stomach, gastric antrum, pylorus, lesser curvature of stomach nos, greater curvature of stomach nos, overlapping lesion of stomach, stomach nos, duodenum, jejunum, ileum, meckel's diverticulum, overlapping lesion of small intestine, small intestine nos, cecum, appendix, ascending colon, hepatic flexure of colon, transverse colon, splenic flexure of colon, descending colon, sigmoid colon, overlapping lesion of colon, colon nos, rectosigmoid junction, rectum nos, anus nos, anal canal, cloacogenic zone, overlapping lesion of rectum anus and anal canal, liver, intrahepatic bile duct, gallbladder, extrahepatic bile duct, ampulla of vater, overlapping lesion of biliary tract, biliary tract nos, head of pancreas, body pancreas, tail pancreas, pancreatic duct, islets of langerhans, neck of pancreas, overlapping lesion of pancreas, pancreas nos, intestinal tract nos, overlapping lesion of digestive system, gastrointestinal tract nos, nasal cavity, middle ear, maxillary sinus, ethmoid sinus, frontal sinus, sphenoid sinus, overlapping lesion of accessory sinuses, accessory sinus nos, glottis, supraglottis, subglottis, laryngeal cartilage, overlapping lesion of larynx, larynx nos, trachea, main bronchus, upper lobe lung, middle lobe lung, lower lobe lung, overlapping lesion of lung, lung nos, thymus, heart, anterior mediastinum, posterior mediastinum, mediastinum nos, pleura nos, overlapping lesion of heart mediastinum and pleura, upper respiratory tract nos, overlapping lesion of respiratory system and intrathoracic organs, respiratory tract nos, upper limb long bones joints, upper limb short bones joints, lower limb long bones joints, lower limb short bones joints, overlapping lesion of bones joints and articular cartilage of limbs, bone limb nos, skull and facial bone, mandible, vertebral column, rib sternum clavicle, pelvic bone, overlapping lesion of bones joints and articular cartilage, bone nos, blood, bone marrow, spleen, reticuloendothelial system nos, hematopoietic system nos, skin lip nos, eyelid nos, external ear, skin face, skin scalp neck, skin trunk, skin limb upper, skin limb lower, peripheral nerve head neck, peripheral nerve shoulder arm, peripheral nerve leg, peripheral nerve thorax, peripheral nerve abdomen, peripheral nerve pelvis, peripheral nerve trunk, overlapping lesion of peripheral nerves and autonomic nervous system, autonomic nervous system nos, retroperitoneum, peritoneum, peritoneum nos, overlapping lesion of retroperitoneum and peritoneum, connective tissue head, connective tissue arm, connective tissue leg, connective tissue thorax, connective tissue abdomen, connective tissue pelvis, connective tissue trunk nos, overlapping lesion of connective subcutaneous and other soft tissues, connective tissue nos, nipple, central portion of breast, upper inner quadrant of breast, lower inner quadrant of breast, upper outer quadrant of breast, lower outer quadrant of breast, axillary tail of breast, overlapping lesion of breast, breast nos, labium majus, labium minus, clitoris, overlapping lesion of vulva, vulva nos, vagina nos, endocervix, exocervix, overlapping lesion of cervix uteri, cervix uteri, isthmus uteri, endometrium, myometrium, fundus uteri, overlapping lesion of corpus uteri, corpus uteri, uterus nos, ovary, fallopian tube, broad ligament, round ligament, parametrium, uterine adnexa, wolffian body, overlapping lesion of female genital organs, female genital tract nos, prepuce, glans penis, body penis, overlapping lesion of penis, penis nos, prostate gland, undescended testis, descended testis, testis nos, epididymis, spermatic cord, scrotum nos, tunica vaginalis, overlapping lesion of male genital organs, male genital organs nos, kidney nos, renal pelvis, ureter, trigone bladder, dome bladder, lateral wall bladder, posterior wall bladder, ureteric orifice, urachus, overlapping lesion of bladder, bladder nos, urethra, paraurethral gland, overlapping lesion of urinary organs, urinary system nos, conjunctiva, cornea nos, retina, choroid, ciliary body, lacrimal gland, orbit nos, overlapping lesion of eye and adnexa, eye nos, cerebral meninges, spinal meninges, meninges nos, cerebrum, frontal lobe, temporal lobe, parietal lobe, occipital lobe, ventricle nos, cerebellum nos, brain stem, overlapping lesion of brain, brain nos, spinal cord, cauda equina, olfactory nerve, optic nerve, acoustic nerve, cranial nerve nos, overlapping lesion of brain and central nervous system, nervous system nos, thyroid gland, adrenal gland cortex, adrenal gland medulla, adrenal gland nos, parathyroid gland, pituitary gland, craniopharyngeal duct, pineal gland, carotid body, aortic body, overlapping lesion of endocrine glands and related structures, endocrine gland nos, head face or neck nos, thorax nos, abdomen nos, pelvis nos, upper limb nos, lower limb nos, other illdefined sites, overlapping lesion of ill-defined sites, lymph node face head neck, intrathoracic lymph node, intra-abdominal lymph nodes, lymph node axilla arm, lymph node inguinal region leg, lymph node pelvic, lymph nodes of multiple regions, lymph node nos, unknown primary site,

The subjects treated with the presently disclosed and claimed compounds may be treated in combination with other non-surgical anti-proliferative (e.g., anti-cancer) drug therapy. In one embodiment, the compounds may be administered in combination with an anti-cancer compound such as a cytostatic compound. A cytostatic compound is a compound (e.g., a small molecule, a nucleic acid, or a protein) that suppresses cell growth and/or proliferation. In some embodiments, the cytostatic compound is directed towards the malignant cells of a tumor. In yet other embodiments, the cytostatic compound is one which inhibits the growth and/or proliferation of vascular smooth muscle cells or fibroblasts.

Suitable anti-proliferative drugs or cytostatic compounds to be used in combination with the presently disclosed and claimed compounds include anti-cancer drugs. Numerous anti-cancer drugs which may be used are well known and include, but are not limited to: Acivicin; Aclarubicin; Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Fluorocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-nl; Interferon Alfa-n3; Interferon Beta-I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Niraparib; Nocodazole; Nogalamycin; Olaparib; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Rucaparib; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talazoparib; Talisomycin; Taxol; Taxotere; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Tubulozole Hydrochloride; Uracil Mustard; Uredepa; Vapreotide; Velaparib; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; and Zorubicin Hydrochloride.

Other anti-cancer drugs include, but are not limited to: 20-epi-1,25 dihydroxyvitamin D3; 5-ethynyluracil; abiraterone; acylfulvene; adecypenol; adozelesin; ALL-TK antagonists; ambamustine; amidox; amifostine; aminolevulinic acid; amrubicin; anagrelide; andrographolide; angiogenesis inhibitors; antagonist D; antagonist G; antarelix; anti-dorsalizing morphogenetic protein-1; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bisaziridinylspermine; bisnafide; bistratene A; breflate; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; casein kinase inhibitors (ICOS); castanospermine; cecropin B; cetrorelix; chlorins; chloroquinoxaline sulfonamide; cicaprost; cis-porphyrin; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; dehydrodidemnin B; deslorelin; dexifosfamide; dexrazoxane; dexverapamil; didemnin B; didox; diethylnorspermine; dihydro-5-azacytidine; dihydrotaxol, 9-; dioxamycin; diphenyl spiromustine; docosanol; dolasetron; doxifluridine; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; filgrastim; finasteride; flavopiridol; flezelastine; fluasterone; fludarabine; fluorodaunorunicin hydrochloride; forfenimex; formestane; fotemustine; gadolinium texaphyrin; gallium nitrate; galocitabine; ganirelix; gelatinase inhibitors; glutathione inhibitors; hepsulfam; heregulin; hexamethylene bisacetamide; hypericin; ibandronic acid; idoxifene; idramantone; ilmofosine; ilomastat; imidazoacridones; imiquimod; immunostimulant peptides; insulin-like growth factor-I receptor inhibitor; interferon agonists; interferons; interleukins; iobenguane; iododoxorubicin; ipomeanol, 4-; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anti cancer compound; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; saintopin; SarCNU; sarcophytol A; sargramostim; Sdi 1 mimetics; senescence derived inhibitor 1; sense oligonucleotides; signal transduction inhibitors; signal transduction modulators; single chain antigen binding protein; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temozolomide; tetrachlorodecaoxide; tetrazomine; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; titanocene dichloride; topsentin; toremifene; totipotent stem cell factor; translation inhibitors; tretinoin; triacetyluridine; triciribine; tropisetron; turosteride; tyrosine kinase inhibitors; tyrphostins; UBC inhibitors; ubenimex; urogenital sinus-derived growth inhibitory factor; urokinase receptor antagonists; variolin B; vector system, erythrocyte gene therapy; velaresol; veramine; verdins; vinorelbine; vinxaltine; vitaxin; zanoterone; zilascorb; and zinostatin stimalamer.

The presently disclosed and claimed compounds can also be used in combination with any of the following treatments:
Therapy in combination with inhibitors of Poly(ADP-ribose) polymerases (PARP), a class of chemotherapeutic agents directed at targeting cancers with defective DNA-damage repair (Yuan, et al., Expert Opin Ther Pat, 2017, 27: 363). Such PARP inhibitors include but are not limited to olaparib, rupacarib, velaparib, niraparib, talazoparib, pamiparib, iniparib, E7449, and A-966492.

Therapy in combination with inhibitors of signaling pathways and mechanisms leading to repair of DNA single and double strand breaks as e.g. nuclear factor-kappaB signaling (Pilie, et al., Nat Rev Clin Oncol, 2019, 16: 81; Zhang, et al., Chin J Cancer, 2012, 31: 359). Such inhibitors include but are not limited to inhibitors of ATM and ATR kinases, checkpoint kinase 1 and 2, DNA-dependen protein kinase, and WEE1 kinase (Pilie, et al., Nat Rev Clin Oncol, 2019, 16: 81).

Therapy in combination with an immunomodulator (Khalil, et al., Nat Rev Clin Oncol, 2016, 13: 394), a cancer vaccine (Hollingsworth, et al., NPJ Vaccines, 2019, 4: 7), an immune checkpoint inhibitor (e.g. PD-1, PD-L1, CTLA-4-inhibitor) (Wei, et al., Cancer Discov, 2018, 8: 1069), a Cyclin-D-Kinase 4/6 inhibitor (Goel, et al., Trends Cell Biol, 2018, 28: 911), an antibody being capable of binding to a tumor cell and/or metastases and being capable of inducing antibody-dependent cellular cytotoxicity (ADCC) (Kellner, et al., Transfus Med Hemother, 2017, 44: 327), a T cell- or NK cell engager (e.g. bispecific antibodies) (Yu, et al., J Cancer Res Clin Oncol, 2019, 145: 941), a cellular therapy using expanded autologous or allogeneic immune cells (e.g. chimeric antigen receptor T (CAR-T) cells) (Khalil, et al., Nat Rev Clin Oncol, 2016, 13: 394). Immune checkpoint inhibitors incluce but are not limited to nivolumab, ipilimumab, pembrolizumab, atezolizumab, avelumab, durvalumab, and cemiplimab.

According to the present invention, the compounds may be administered prior to, concurrent with, or following other anti-cancer compounds. The administration schedule may involve administering the different agents in an alternating fashion. In other embodiments, the compounds may be delivered before and during, or during and after, or before and after treatment with other therapies. In some cases, the compound is administered more than 24 hours before the administration of the other anti-proliferative treatment. In other embodiments, more than one anti-proliferative therapy may be administered to a subject. For example, the subject may receive the present compounds, in combination with both surgery and at least one other anti-proliferative compound. Alternatively, the compound may be administered in combination with more than one anti-cancer drug.

In an embodiment, the compounds of the present invention are used to detect cells and tissues overexpressing FAP, whereby such detection is achieved by conjugating a detectable label to the compounds of the invention, preferably a detectable radionuclide. In a preferred embodiment, the cells and tissues detected are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) or a non-oncology indication (e.g. inflammatory disease, cardiovascular disease, autoimmune disease, and fibrotic disease).

In another embodiment, the compounds of the present invention are used to treat cells and tissues overexpressing FAP. In a preferred embodiment, the cells and tissues treated are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) and the therapeutic activity is achieved by conjugating therapeutically active effector to the compounds of the present invention, preferably a therapeutically active radionuclide. In a further preferred embodiment, the diseased cells and tissues are causing and/or are part of a non-oncology indication (e.g. inflammatory disease, cardiovascular disease, autoimmune disease, and fibrotic disease) and the therapeutic activity is achieved by inhibition of the enzymatic activity of FAP.

In a further embodiemt, particularly if the disease is a non-oncology disease or a non-oncology indication (e.g. inflammatory disease, cardiovascular disease, autoimmune disease, and fibrotic disease), the compounds of the present invention are administered in therapeutically effective amounts; preferably the compound of the present invention does not comprise a therapeutifally active nuclide such as a therapeucially active nuclide An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In other embodiments, a therapeutically effective amount will be an amount necessary to extend the dormancy of micrometastases or to stabilize any residual primary tumor cells following surgical or drug therapy.

Generally, when using an unconjugated compound without a therapeutically active radionuclide, a therapeutically effective amount will vary with the subject's age, condition, and sex, as well as the nature and extent of the disease in the subject, all of which can be determined by one of ordinary skill in the art. The dosage may be adjusted by the individual physician or veterinarian, particularly in the event of any complication. A therapeutically effective amount is typically, but not limited to, an amount in a range from 0.1 µg/kg to about 2000 mg/kg, or from 1.0 µg/kg to about 1000 mg/kg, or from about 0.1 mg/kg to about 500 mg/kg, or from about 1.0 mg/kg to about 100 mg/kg, in one or more dose administrations daily, for one or more days. If desired, the effective daily dose of the active compound may be administered as two, three, four, five, six, or more sub-doses for example administered separately at appropriate intervals throughout the day, optionally, in unit dosage forms. In some embodiments, the compounds are administered for more than 7 days, more than 10 days, more than 14 days and more than 20 days. In still other embodiments, the compound is administered over a period of weeks, or months. In still other embodiments, the compound is delivered on alternate days. For example, the agent is delivered every two days, or every three days, or every four days, or every five days, or every six days, or every week, or every month. In a preferred embodiment, the compound of the present invention is for use in the treatment and/or prevention of a disease, whereby such treatment is radionuclide therapy.

Preferably, radionuclide therapy makes use of or is based on different forms of radiation emitted by a radionuclide. Such radiation can, for example, be any one of radiation of photons, radiation of electrons including but not limited to β⁻-particles and Auger-electrons, radiation of protons, radiation of neutrons, radiation of positrons, radiation of α-particles or an ion beam. Depending on the kind of particle or radiation emitted by said radionuclide, radionuclide therapy can, for example, be distinguished as photon radionuclide therapy, electron radionuclide therapy, proton radionuclide therapy, neutron radionuclide therapy, positron radionuclide therapy, α-particle radionuclide therapy or ion beam radionuclide therapy. All of these forms of radionuclide therapy are encompassed by the present invention, and all of these forms of radionuclide therapy can be realized by the compound of the invention, preferably under the proviso that the radionuclide attached to the compound of the invention, more preferably as an effector, is providing for this kind of radiation.

Radionuclide therapy preferably works by damaging the DNA of cells. The damage is caused by a photon, electron, proton, neutron, positron, α-particle or ion beam directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high pressure oxygen tanks, blood substitutes that carry increased oxygen, hypoxic cell radiosensitizers such as misonidazole and metronidazole, and hypoxic cytotoxins, such as tirapazamine may be applied.

Other factors that are considered when selecting a radioactive dose include whether the patient is receiving chemotherapy, whether radiation therapy is being administered before or after surgery, and the degree of success of surgery.

The total radioactive dose may be fractionated, i.e. spread out over time in one or more treatments for several important reasons. Fractionation allows normal cells time to recover, while tumor cells are generally less efficient in repair between fractions. Fractionation also allows tumor cells that were in a relatively radio-resistant phase of the cell cycle during one treatment to cycle into a sensitive phase of the cycle before the next fraction is given. Similarly, tumor cells that were chronically or acutely hypoxic and, therefore, more radioresistant, may reoxygenate between fractions, improving the tumor cell kill.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

It is important to distinguish radiosensitivity of a particular tumor, which to some extent is a laboratory measure, from "curability" of a cancer by an internally delivered radioactive dose in actual clinical practice. For example, leukemias are not generally curable with radiotherapy, because they are disseminated through the body. Lymphoma may be radically curable if it is localized to one area of the body. Similarly, many of the common, moderately radioresponsive tumors can be treated with curative doses of radioactivity if they are at an early stage. This applies, for example, to non-melanoma skin cancer, head and neck cancer, non-small cell lung cancer, cervical cancer, anal cancer, prostate cancer.

The response of a tumor to radiotherapy is also related to its size. For complex reasons, very large tumors respond less well to radiation than smaller tumors or microscopic disease. Various strategies are used to overcome this effect. The most common technique is surgical resection prior to radiotherapy. This is most commonly seen in the treatment of breast cancer with wide local excision or mastectomy followed by adjuvant radiotherapy. Another method is to shrink the tumor with neoadjuvant chemotherapy prior to radical radionuclide therapy. A third technique is to enhance the radiosensitivity of the cancer by giving certain drugs during a course of radiotherapy. Examples of radiosensiting drugs include, but are not limited to Cisplatin, Nimorazole, and Cetuximab.

Introperative radiotherapy is a special type of radiotherapy that is delivered immediately after surgical removal of the cancer. This method has been employed in breast cancer (TARGeted Introperative radioTherapy), brain tumors and rectal cancers.

Radionuclide therapy is in itself painless. Many low-dose palliative treatments cause minimal or no side effects. Treatment to higher doses may cause varying side effects during treatment (acute side effects), in the months or years following treatment (long-term side effects), or after re-treatment (cumulative side effects). The nature, severity, and longevity of side effects depends on the organs that receive the radiation, the treatment itself (type of radionuclide, dose, fractionation, concurrent chemotherapy), and the patient.

It is within the present inventions that the method for the treatment of a disease of the invention may realize each and any of the above strategies which are as such known in the art, and which insofar constitute further embodiments of the invention.

It is also within the present invention that the compound of the invention is used in a method for the diagnosis of a disease as disclosed herein. Such method, preferably, comprises the step of administering to a subject in need thereof a diagnostically effective amount of the compound of the invention.

In accordance with the present invention, an imaging method is selected from the group consisting of scintigraphy, Single Photon Emission Computed Tomography (SPECT) and Positron Emission Tomography (PET).

Scintigraphy is a form of diagnostic test or method used in nuclear medicine, wherein radiopharmaceuticals are internalized by cells, tissues and/or organs, preferably internalized *in vivo,* and radiation emitted by said internalized radiopharmaceuticals is captured by external detectors (gamma cameras) to form and display two-dimensional images. In contrast thereto, SPECT and PET forms and displays three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled chemical emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled chemical. To acquire SPECT images, the gamma camera is rotated around the patient. Projections are acquired at defined points during the rotation, typically every 3 - 6 degrees. In most cases, a full 360 degree rotation is used to obtain an optimal reconstruction. The time taken to obtain each projection is also variable, but 15 - 20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical status or metabolic activity of cells within the human body. PET is unique since it produces images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical processes are also altered by a disease, and may occur before any gross changes in anatomy. PET is an imaging technique that can visualize some of these early biochemical changes. PET scanners rely on radiation emitted from the patient to create the images. Each patient is given a minute amount of a radioactive pharmaceutical that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present inventions that the method for the diagnosis of a disease of the invention may realize each and any of the above strategies which are as such known in the art, and which insofar constitute further embodiments of the invention.

Compounds of the present invention are useful to stratify patients, i.e. to create subsets within a patient population that provide more detailed information about how the patient will respond to a given drug. Stratification can be a critical component to transforming a clinical trial from a negative or neutral outcome to one with a positive outcome by identifying the subset of the population most likely to respond to a novel therapy.

Stratification includes the identification of a group of patients with shared "biological" characteristics to select the optimal management for the patients and achieve the best possible outcome in terms of risk assessment, risk prevention and achievement of the optimal treatment outcome

A compound of the present invention may be used to assess or detect, a specific disease as early as possible (which is a diagnostic use), the risk of developing a disease (which is a susceptibility/risk use), the evolution of a disease including indolent vs. aggressive (which is a prognostic use) and it may be used to predict the response and the toxicity to a given treatment (which is a predictive use).

It is also within the present invention that the compound of the invention is used in a theragnostic method. The concept of theragnostics is to combine a therapeutic agent with a corresponding diagnostic test that can increase the clinical use of the therapeutic drug. The concept of theragnostics is becoming increasingly attractive and is widely considered the key to improving the efficiency of drug treatment by helping doctors identify patients who might profit from a given therapy and hence avoid unnecessary treatments.

The concept of theragnostics is to combine a therapeutic agent with a diagnostic test that allows doctors to identify those patients who will benefit most from a given therapy. In an embodiment and as preferably used herein, a compound of the present invention is used for the diagnosis of a patient, i.e. identification and localization of the primary tumor mass as well as potential local and distant metastases. Furthermore, the tumor volume can be determined, especially utilizing three-dimensional diagnostic modalities such as SPECT or PET. Only those patients having FAP-positive tumor masses and who, therefore, might profit from a given therapy are selected for a particular therapy and hence unnecessary treatments are avoided. Preferably, such therapy is a FAP-targeted therapy using a compound of the present invention. In one particular embodiment, chemically identical tumor-targeted diagnostics, preferably imaging diagnostics for scintigraphy, PET or SPECT and radiotherapeutics are applied. Such compounds only differ in the radionuclide and therefore usually have a very similar if not identical pharmacokinetic profile. This can be realized using a chelator and a diagnostic or therapeutic radiometal. Alternatively, this can be realized using a precursor for radiolabeling and radiolabeling with either a diagnostic or a therapeutic radionuclide. In one embodiment diagnostic imaging is used preferably by means of quantification of the radiation of the diagnostic radionuclide and subsequent dosimetry which is known to those skilled in the art and the prediction of drug concentrations in the tumor compared to vulnerable side effect organs. Thus, a truly individualized drug dosing therapy for the patient is achieved.

In an embodiment and as preferably used herein, the theragnostic method is realized with only one theragnostically active compound such as a compound of the present invention labeled with a radionuclide emitting diagnostically detectable radiation (e.g. positrons or gamma rays) as well as therapeutically effective radiation (e.g. electrons or alpha particles).

The invention also contemplates a method of intraoperatively identifying/disclosing diseased tissues expressing FAP in a subject. Such method uses a compound of the invention, whereby such compound of the invention preferably comprises as Effector a diagnostically active agent.

According to a further embodiment of the invention, the compound of the invention, particularly if complexed with a radionuclide, may be employed as adjunct or adjuvant to any other tumor treatment including, surgery as the primary method of treatment of most isolated solid cancers, radiation therapy involving the use of ionizing radiation in an attempt to either cure or improve the symptoms of cancer using either sealed internal sources in the form of brachytherapy or external sources, chemotherapy such as alkylating agents, antimetabolites, anthracyclines, plant alkaloids, topoisomerase inhibitors, and other antitumor agents, hormone treatments that modulate tumor cell behavior without directly attacking those cells, targeted agents which directly target a molecular abnormality in certain types of cancer including monoclonal antibodies and tyrosine kinase inhibitors, angiogenesis inhibitors, immunotherapy, cancer vaccination, palliative care including actions to reduce the physical, emotional, spiritual, and psycho-social distress to improve the patient's quality of life and alternative treatments including a diverse group of health care systems, practices, and products that are not part of conventional medicine.

In an embodiment of the methods of the invention, the subject is a patient. In an embodiment, a patient is a subject which has been diagnosed as suffering from or which is suspected of suffering from or which is at risk of suffering from or developing a disease, whereby the disease is a disease as described herein and preferably a disease involving FAP.

Dosages employed in practicing the methods for treatment and diagnosis, respectively, where a radionuclide is used and more specifically attached to or part of the compound of the invention will vary depending e.g. on the particular condition to be treated, for example the known radiosensitivity of the tumor type, the volume of the tumor and the therapy desired. In general, the dose is calculated on the basis of radioactivity distribution to each organ and on observed target uptake. A γ-emitting complex may be administered once or at several times for diagnostic imaging. In animals, an indicated dose range may be from 0.1 µg/kg to 5 mg/kg of the compound of the invention complexed e.g. with 1 to 200 MBq of ¹¹¹In or ⁸⁹Zr. A β-emitting complex of the compound of the invention may be administered at several time points e.g. over a period of 1 to 3 weeks or longer. In animals, an indicated dosage range may be of from 0.1 µg/kg to 5 mg/kg of the compound of the invention complexed e.g. with 1 to 200 MBq ⁹⁰Y or ¹⁷⁷Lu. In larger mammals, for example humans, an indicated dosage range is from 0.1 to 100 µg/kg of the compound of the invention complexed with e.g. 10 to 400 MBq ¹¹¹In or ⁸⁹Zr. In larger mammals, for example humans, an indicated dosage range is of from 0.1 to 100 µg/kg of the compound of the invention complexed with e.g. 10 to 5000 MBq ⁹⁰Y or ¹⁷⁷Lu.

In a further aspect, the instant invention is related to a composition and a pharmaceutical composition in particular, comprising the compound of the invention.

The pharmaceutical composition of the present invention comprises at least one compound of the invention and, optionally, one or more carrier substances, excipients and/or adjuvants. The pharmaceutical composition may additionally comprise, for example, one or more of water, buffers such as, *e.g.,* neutral buffered saline or phosphate buffered saline, ethanol, mineral oil, vegetable oil, dimethylsulfoxide, carbohydrates such as *e.g.,* glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical composition of the invention.

The pharmaceutical composition of the invention may be formulated for any appropriate route of administration, including, for example, topical such as, e.g., transdermal or ocular, oral, buccal, nasal, vaginal, rectal or parenteral administration. The term parenteral as used herein includes subcutaneous, intradermal, intravascular such as, e.g., intravenous, intramuscular, intrathecal and intraperitoneal injection, as well as any similar injection or infusion technique. A preferred route of administration is intravenous administration.

In an embodiment of the invention the compound of the invention comprising a radionuclide is administered by any conventional route, in particular intravenously, e.g. in the form of injectable solutions or suspensions. The compound of the invention may also be administered advantageously by infusion, e.g., by an infusion of 30 to 60 min.

Depending on the site of the tumor, the compound of the invention may be administered as close as possible to the tumor site, e.g. by means of a catheter. Such administration may be carried out directly into the tumor tissue or into the surrounding tissue or into the afferent blood vessels. The compound of the invention may also be administered repeatedly in doses, preferably in divided doses.

According to a preferred embodiment of the invention, a pharmaceutical composition of the invention comprises a stabilizer, e.g. a free radical scavenger, which inhibits autoradiolysis of the compound of the invention. Suitable stabilizers include, e.g., serum albumin, ascorbic acid, retinol, gentisic acid or a derivative thereof, or an amino acid infusion solution such, e.g., used for parenteral protein feeding, preferably free from electrolyte and glucose, for example a commercially available amino acid infusion such as Proteinsteril® KE Nephro. Ascorbic acid and gentisic acid are preferred.

A pharmaceutical composition of the invention may comprise further additives, e.g. an agent to adjust the pH between 7.2 and 7.4, e.g. sodium or ammonium acetate or Na₂HP0₄ . Preferably, the stabilizer is added to the non-radioactive compound of the invention and introduction of the radionuclide, for instance the complexation with the radionuclide, is performed in the presence of the stabilizer, either at room temperature or, preferably, at a temperature of from 40 to 120° C. The complexation may conveniently be performed under air free conditions, e.g. under N₂ or Ar. Further stabilizer may be added to the composition after complexation.

Excretion of the compound of the invention, particularly if the Effector is a radionuclide, essentially takes place through the kidneys. Further protection of the kidneys from radioactivity accumulation may be achieved by administration of lysine or arginine or an amino acid solution having a high content of lysine and/or arginine, e.g. a commercially available amino acid solution such as Synthamin®-14 or -10, prior to the injection of or together with the compound of the invention, particularly if the Effector is a radionuclide. Protection of the kidneys may also be achieved by administration of plasma expanders such as e.g. gelofusine, either instead of or in addition to amino acid infusion. Protection of the kidneys may also be achieved by administration of diuretics providing a means of forced diuresis which elevates the rate of urination. Such diuretics include high ceiling loop diuretics, thiazides, carbonic anhydrase inhibitors, potassium-sparing diuretics, calcium-sparing diuretics, osmotic diuretics and low ceiling diuretics. A pharmaceutical composition of the invention may contain, apart from a compound of the invention, at least one of these further compounds intended for or suitable for kidney protection, preferably kidney protection of the subject to which the compound of the invention is administered.

It will be understood by a person skilled in the art that the compound of the invention is disclosed herein for use in various methods. It will be further understood by a person skilled in the art that the composition of the invention and the pharmaceutical composition of the invention can be equally used in said various methods. It will also be understood by a person skilled in the art that the composition of the invention and the pharmaceutical composition are disclosed herein for use in various methods. It will be equally understood by a person skilled in the art that the compound of the invention can be equally used in said various methods.

It will be acknowledged by a person skilled in the art that the composition of the invention and the pharmaceutical composition of the invention contain one or more further compounds in addition to the compound of the invention. To the extent that such one or more further compounds are disclosed herein as being part of the composition of the invention and/or of the pharmaceutical composition of the invention, it will be understood that such one or more further compounds can be administered separately from the compound of the invention to the subject which is exposed to or the subject of a method of the invention. Such administration of the one or more further compounds can be performed prior, concurrently with or after the administration of the compound of the invention. It will also be acknowledged by a person skilled in the art that in a method of the invention, apart from a compound of the invention, one or more further compound may be administered to a subject. Such administration of the one or more further compounds can be performed prior, concurrently with or after the administration of the compound of the invention. To the extent that such one or more further compounds are disclosed herein as being administered as part of a method of the invention, it will be understood that such one or more further compounds are part of a composition of the invention and/or of a pharmaceutical composition of the invention. It is within the present invention that the compound of the invention and the one or more further compounds may be contained in the same or a different formulation. It is also within the present invention that the compound of the invention and the one or more further compounds are not contained in the same formulation, but are contained in the same package containing a first formulation comprising a compound of the invention, and a second formulation comprising the one or more further compounds, whereby the type of formulation may be the same or may be different.

It is within the present invention that more than one type of a compound of the invention is contained in the composition of the invention and/or the pharmaceutical composition of the invention. It is also within the present invention that more than one type of a compound of the invention is used, preferably administered, in a method of the invention.

It will be acknowledged that a composition of the invention and a pharmaceutical composition of the invention may be manufactured in conventional manner.

Radiopharmaceuticals have decreasing content of radioactivity with time, as a consequence of the radioactive decay. The physical half-life of the radionuclide is often short for radiopharmaceutical diagnostics. In these cases, the final preparation has to be done shortly before administration to the patient. This is in particular the case for positron emitting radiopharmaceuticals for tomography (PET radiopharmaceuticals). It often leads to the use of semi-manufactured products such as radionuclide generators, radioactive precursors and kits.

Preferably, a kit of the invention comprises apart from one or more than one compounds of the invention typically at least one of the followings: instructions for use, final preparation and/or quality control, one or more optional excipient(s), one or more optional reagents for the labeling procedure, optionally one or more radionuclide(s) with or without shielded containers, and optionally one or more device(s), whereby the device(s) is/are selected from the group comprising a labeling device, a purification device, an analytical device, a handling device, a radioprotection device or an administration device.

Shielded containers known as "pigs" for general handling and transport of radiopharmaceutical containers come in various configurations for holding radiopharmaceutical containers such as bottles, vials, syringes, etc. One form often includes a removable cover that allows access to the held radiopharmaceutical container. When the pig cover is in place, the radiation exposure is acceptable.

A labeling device is selected from the group of open reactors, closed reactors, microfluidic systems, nanoreactors, cartridges, pressure vessels, vials, temperature controllable reactors, mixing or shaking reactors and combinations thereof.

A purification device is preferably selected from the group of ion exchange chromatography columns or devices, size-exclusion chromatography columns or devices, affinity chromatography columns or devices, gas or liquid chromatography columns or devices, solid phase extraction columns or devices, filtering devices, centrifugations vials columns or devices.

An analytical device is preferably selected from the group of tests or test devices to determine the identity, radiochemical purity, radionuclidic purity, content of radioactivity and specific radioactivity of the radiolabelled compound.

A handling device is preferably selected from the group consisting of devices for mixing, diluting, dispensing, labeling, injecting and administering radiopharmaceuticals to a subject.

A radioprotection device is used in order to protect doctors and other personnel from radiation when using therapeutic or diagnostic radionuclides. The radioprotection device is preferably selected from the group consisting of devices with protective barriers of radiation-absorbing material selected from the group consisting of aluminum, plastics, wood, lead, iron, lead glass, water, rubber, plastic, cloth, devices ensuring adequate distances from the radiation sources, devices reducing exposure time to the radionuclide, devices restricting inhalation, ingestion, or other modes of entry of radioactive material into the body and devices providing combinations of these measures.

An administration device is preferably selected from the group of syringes, shielded syringes, needles, pumps, and infusion devices. Syringe shields are commonly hollow cylindrical structures that accommodate the cylindrical body of the syringe and are constructed of lead or tungsten with a lead glass window that allows the handler to view the syringe plunger and liquid volume within the syringe.

The present invention is now further illustrated by reference to the following figures and examples from which further features, embodiments and advantages, may be taken, wherein
Fig. 1 shows, indicated as %ID/g, quantified organ uptake in kidney, liver, bloodpool and HEK-FAP induced tumor as determined by SPECT-imaging of ¹¹¹In-3BP-3105 (A) and ¹¹¹In-3BP-3168 (B) 1h, 3h, 6h and 24h post injection into the mouse model (see, Example 16);
Figure 2 shows, indicated as %ID/g, quantified organ uptake in kidney, liver, bloodpool and HEK-FAP induced tumor as determined by SPECT-imaging of ¹¹¹In-3BP-3320 (A) and ¹¹¹In-3BP-3321 (B) 1h, 3h, 6h and 24h post injection into the mouse model (see, Example 16);
Figure 3 shows, indicated as %ID/g, quantified organ uptake in kidney, liver, bloodpool and HEK-FAP induced tumor as determined by SPECT-imaging of ¹¹¹In-3BP-3275 (A) and ¹¹¹In-3BP-3397 (B) 1h, 3h, 6h and 24h post injection into the mouse model (see, Example 16);
Figure 4 shows, indicated as %ID/g, quantified organ uptake in kidney, liver, bloodpool and HEK-FAP induced tumor as determined by SPECT-imaging of ¹¹¹In-3BP-3398 (A) and ¹¹¹In-3BP-3407 (B) 1h, 3h, 6h and 24h post injection into the mouse model (see, Example 16);
Figure 5 shows, indicated as %ID/g, quantified organ uptake in kidney, liver, bloodpool and HEK-FAP induced tumor as determined by SPECT-imaging of ¹¹¹In-3BP-3554 (A) and ¹¹¹In-3BP-3652 (B) 1h, 3h, 6h and 24h post injection into the mouse model (see, Example 16);
Figure 6 shows, indicated as %ID/g, quantified organ uptake in kidney, liver, bloodpool and HEK-FAP induced tumor as determined by SPECT-imaging of ¹¹¹In-3BP-3654 (A) and ¹¹¹In-3BP-3656 (B) 1h, 3h, 6h and 24h post injection into the mouse model (see, Example 16);
Figure 7 shows, indicated as %ID/g, quantified organ uptake in kidney, liver, bloodpool and HEK-FAP induced tumor as determined by SPECT-imaging of ¹¹¹In-3BP-3659 (A) and ¹¹¹In-3BP-3678 (B) 1h, 3h, 6h and 24h post injection into the mouse model (see, Example 16);
Figure 8 shows, indicated as %ID/g, quantified organ uptake in kidney, liver, bloodpool and HEK-FAP induced tumor as determined by SPECT-imaging of ¹¹¹In-3BP-3692 (A) and ¹¹¹In-3BP-3767 (B) 1h, 3h, 6h and 24h post injection into the mouse model (see, Example 16);
Figure 9 shows SPECT-images of ¹¹¹In-3BP-3554 1 h, 3 h, 6 h, 24 h and 48 h post injection into the mouse model (see, Example 16); and
Figure 10 shows SPECT-images of ¹¹¹In-3BP-3767 1 h, 3 h, 6 h, 24 h and 48 h post injection into the mouse model (see, Example 16); and

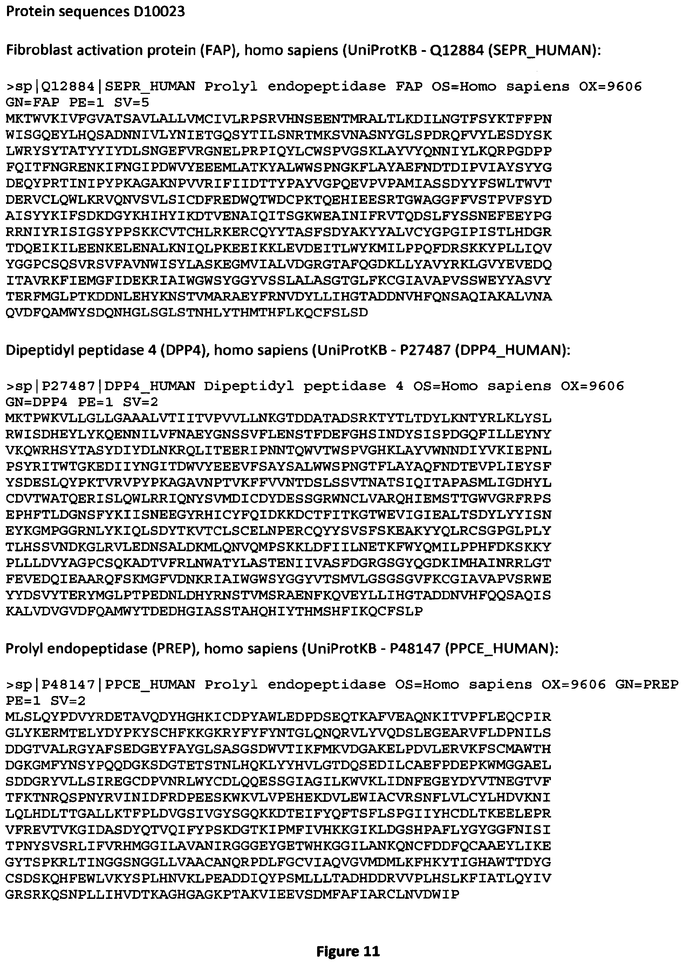

Figure 11 shows the amino acid sequences of human fibroblast activating protein (FAP), human dipeptidyl peptidase 4 (DDP4) and human prolyl endopeptidase (PREP).

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific examples that follow are only intended for the purposes of illustration, and are not to be construed as limiting in any manner to make compounds of the disclosure by other methods.

### EXAMPLES

Abbreviations used in the instant application and the following examples in particular are as follows:
ACN means acetonitrile
Ahx means 6-Aminohexanoic acid
amu means atomic mass unit
AMC means 7-amino-4-methylcoumarin
aq. means aqueous
BSA means bovine serum albumin
CAF means cancer associated fibroblasts
CM means ChemMatrix™
CT means computed tomography
Cy5 means Cyanine-5
DCM means dichloromethane
DAD means Diode Array Detector
Dde means N-(1-(4,4-dimethyl-2,6-dioxocyclohexylidene)ethyl)
DEG means di ethylene glycol dimethacrylate
DIC means N,N'-Diisopropylcarbodiimide
DIPEA means diisopropylethylamine
DMF means N,N-dimethylformamide
DMSO means dimethyl sulfoxide
DOTA means 1,4,7,10-tetraazacyclododecane-1,4,7,10-tetraacetic acid
DOTA(tBu)₃-OH means Tri-*tert*-butyl-1,4,7,10-tetraazacyclo-dodecane-1,4,7,10-tetraacetate
DPP means dipeptidyl peptidase
EC₅₀ means half-maximal excitatory concentration
EC means electron capture
ECACC means European Collection of Authenticated Cell Cultures
EMEM means Eagle's Minimum Essential Medium
ESI means electrospray ionization
Et₂O means Diethylether
EtOAc means ethylacetate
FACS means fluorescence-activated cell sorting
FAP means fibroblast activation protein
FBS means fetal bovine serum
FGF21 means fibroblast growth factor 21
FITC means 5(6)-fluorescein isothiocyanate
Fmoc means 9-Fluorenylmethoxycarbonyl
FRET means Fluorescence Resonance Energy Transfer
Gab means gamma-amino butyric acid
GABA means gamma-amino butyric acid
h means hour(s)
HATU means O-(7-azabenzotriazol-1-yl)-N,N,N',N'-tetramethyluronium hexafluorophosphate
HEK-FAP means human embryonic kidney 293 cells expressing human FAP
HEPES means 4-(2-hydroxyethyl)-1-piperazineethanesulfonic acid
HFIP means hexafluoro-2-isopanol
HOAc means acetic acid
HOAt means 1-Hydroxy-7-azabenzotriazole
HPLC means high performance liquid chromatography
IC₅₀ means half-maximal inhibitory concentration
ID/g means injected dose per gram
IS means isomeric transition
Kᵢ means inhibitory constant
kDa means 1000 Dalton
LC-MS means high performance liquid chromatography coupled with mass spectrometry
LDH means lactate dehydrogenase
LiOH means lithium hydroxide
Leu means leucine
M means molar or mol per Liter
m/z means mass divided by charge
max. means maximum
MeOH means Methanol
MeV means mega electron volt
min means minute(s)
MMP means matrix metalloproteinase
MTBE means Methyl-*tert*-butylether
Mtt means Methyltrityl
NaHCO₃ means sodium hydrogencarbonate
NaCl means sodium chloride
Na₂SO₄ means sodium sulfate
n.d. means not determined
NHS means N-Hydroxysuccinimide
NMP means 1-methyl-2-pyrrolidone
NOS means not otherwise specified
Oic means L-octahydroindol-2-carbonsäure
p.a. means: for analytical purpose (quality grade)
Pbf means 2,2,4,6,7-pentamethyl-2,3-dihydrobenzofuran-5-sulfonyl
PBS means phosphate buffered saline
PET means positron emission tomography
pIC50 means the negative log of the IC50 value when converted to molar
POP means prolyl oligopeptidase
prep. means preparative
PREP means prolyl endopeptidase
PS means polystyrene
RFU means relative fluorescence unit
RLB means radioligand binding assay
RP means reversed phase
RT means room temperature
Rₜ means retention time
sat. means saturated
sec means second
SF means spontaneous fission
SPECT means single photon emission computed tomography
SPPS means Solid Phase Peptide Synthesis
tBu means *tert.* butyl
TFA means trifluoroacetate or trifluoroacetic acid
TIPS means triisopropylsilane
TLC means thin layer chromatography
TME means tumor microenvironment
UHPLC means ultrahigh performance liquid chromatography

### Example 1: Material and Methods

The materials and methods as well as general methods are further illustrated by the following examples.

### Solvents:

Solvents were used in the specified quality without further purification. Acetonitrile (Super Gradient, HPLC, VWR - for analytical purposes; PrepSolv, Merck - for preparative purposes); dichloromethane (synthesis, Roth); ethyl acetate (synthesis grade, Roth); N,N-dimethylformamide (peptide synthesis grade, Biosolve); 1-methyl-2-pyrolidone (peptide grade, IRIS BioTech) 1,4-dioxane (reinst, Roth); methanol (p. a., Merck).

### Water: Milli-Q Plus, Millipore, demineralized.

### Chemicals:

Chemicals were synthesized according to or in analogy to literature procedures or purchased from Sigma-Aldrich-Merck (Deisenhofen, Germany), Bachem (Bubendorf, Switzerland), VWR (Darmstadt, Germany), Novabiochem (Merck Group, Darmstadt, Germany), Acros Organics (distribution company Fisher Scientific GmbH, Schwerte, Germany), Iris Biotech (Marktredwitz, Germany), Amatek Chemical (Jiangsu, China), Roth (Karlsruhe, Deutschland), Molecular Devices (Chicago, USA), Biochrom (Berlin, Germany), Peptech (Cambridge, MA, USA), Synthetech (Albany, OR, USA), Pharmacore (High Point, NC, USA), PCAS Biomatrix Inc (Saint-Jean-sur-Richelieu, Quebec, Canada), Alfa Aesar (Karlsruhe, Germany), Tianjin Nankai Hecheng S&T Co., Ltd (Tianjin, China), CheMatech (Dijon, France) and Anaspec (San Jose, CA, USA) or other companies and used in the assigned quality without further purification.

### Cells:

HT29 (ECACC Cat. No. 91072201) and WI-38 (ECACC Cat. No. 90020107) were purchased from ECACC and HEK293 cells expressing human FAP (Q12884) were produced by InSCREENeX GmbH (Braunschweig, Germany) using recombinase-mediated cassette exchange (RMCE). The RMCE procedure is described by Nehlsen et al. (Nehlsen, et al., BMC Biotechnol, 2009, 9: 100).

### HPLC/MS analyses

HPLC/MS analyses were performed by injection of 5 µl of a solution of the sample, using a 2 step gradient for all chromatograms (5-65% B in 12 min, followed by 65-90% in 0.5 min, A: 0.1% TFA in water and B: 0.1% TFA in ACN). RP columns were from Agilent (Type Poroshell 120, 2.7µm, EC-C18, 50 x 3.00 mm, flow 0.8 ml, HPLC at room temperature); Mass spectrometer: Agilent 6230 LC/TOF-MS, ESI ionization. MassHunter Qualitative Analysis B.07.00 SP2 was used as software. UV detection was done at λ = 230 nm. Retention times (Rt) are indicated in the decimal system (e.g. 1.9 min = 1 min 54 s) and are referring to detection in the UV spectrometer. For the evaluation of observed compound masses the 'Find Compounds by Formula'-feature was used. In particular, the individual 'neutral mass of a compound (in units of Daltons)'-values and the corresponding isotope distribution pattern were used to confirm compound identity. The accuracy of the mass spectrometer was approx. ± 5 ppm.

### Preparative HPLC:

Preparative HPLC separations were done with reversed phase columns (Kinetex 5µ XB-C18 100 Å, 150 x 30 mm from Phenomenex or RLRP-S 8µ, 100 Å, 150 x 25 mm) as stationary phase. As mobile phase 0.1% TFA in water (A) and 0.1% TFA in ACN (B) were used which were mixed in linear binary gradients. The gradients are described as: "10 to 40% B in 30 min", which means a linear gradient from 10% B (and correspondingly 90% A) to 40% B (and correspondingly 60% A) was run within 30min. Flow-rates were within the range of 30 to 50 ml/min. A typical gradient for the purification of the compounds of the invention started at 5-25% B and ended after 30 min at 35-50% B and the difference between the percentage B at end and start was at least 10%. A commonly used gradient was "15 to 40% B in 30 min".

### General procedures for Automated/Semi-automated Solid-Phase Synthesis:

Automated solid-phase of peptides and polyamides was performed on a Tetras Peptide Synthesizer (Advanced ChemTech) in 50 µmol and 100 µmol scales. Manual steps were performed in plastic syringes equipped with frits (material PE, Roland Vetter Laborbedarf OHG, Ammerbuch, Germany). The amount of reagents in the protocols described corresponds to the 100 µmol scale, unless stated otherwise.

Solid-phase synthesis was performed on polystyrene (cross linked with 1,4-divinylbenzene (PS) or di (ethylene glycol) dimethacrylate (DEG)), ChemMatrix (CM) or TentaGel (TG) resin. Resin linkers were trityl, wang and rink amide.

### Resin loading:

In case of the trityl linker the attachment of the first building block (resin loading) was performed as follows. The resin (polystyrene (PS) trityl chloride, initial loading: 1.8 mmol/g) was swollen in DCM (5 ml) for 30 minutes and subsequently washed with DCM (3 ml, 1 minute). Then the resin was treated with a mixture of the corresponding building block (0.5 mmol, 5 eq.) and DIPEA (350 µl, 3.5 mmol, 35 eq.) in DCM (4 ml) for 1 hour. Afterwards the resin was washed with methanol (5 ml, 5 minutes) and DMF (3 ml, 2x 1 minute).

In case of the Wang linker pre-loaded resins (polystyrene (PS) and TentaGel (TG)) were employed.

In case of the rink amide linker the attachment of the first residue the resin (CM, DEG) was performed with the same procedure as for the chain assembly as described below.

### Alloc/Allyl-deprotection:

After swelling in DMF, the resin was washed with DMF and DCM. DCM was de-oxygenated by passing a stream of nitrogen through the stirred solvent. The oxygen-free solvent was used to wash the resin trice. Then 2 ml of a 2 M solution of barbituric acid in oxygen-free DCM and 1 ml of a 25 µM solution of Tetrakis(triphenylphosphine)palladium(0) in oxygen-free DCM were added to the resin. The resin was agitated for 1 hour and then washed with DCM, MeOH, DMF, 5% DIPEA in DMF, 5% dithiocarbamate in DMF, DMF and DCM (each washing step was repeated 3 times with 3 ml, 1 minute).

### Fmoc-deprotection:

After swelling in DMF, the resin was washed with DMF and then treated with piperidine/DMF (1:4, 3 ml, 2 and 20 minutes) and subsequently washed with DMF (3 ml, 5x 1 minute).

### Dde-deprotection:

After swelling in DMF, the resin was washed with DMF and then treated with hydrazine-hydrate/DMF (2/98, 3 ml 2x 10 minutes) and subsequently washed with DMF (3 ml, 5x 1 minute).

### Mtt-deprotection:

After swelling in DCM, the resin was washed with DCM and then treated with HFIP/DCM (7/3, 4 - 6 ml, 4 hours) and subsequently washed with DCM (3 ml, 3x 1 minute), DMF (3 ml, 3x 1ml) and DIPEA (0.9 M in DMF, 3 ml, 1 minute).

### Solutions of reagents:

Building Blocks (0.3 M in DMF or NMP), DIPEA (0.9 M in DMF), HATU (0.4 M in DMF), Acetic anhydride (0.75 M in DMF)

### Coupling: Coupling of building blocks/amino acids (chain assembly):

Unless otherwise stated, coupling of building blocks was performed as follows: After subsequent addition of solutions of the corresponding building block (1.7 ml, 5eq.), DIPEA solution (1.15 ml, 10 eq.) and HATU solution (1.25 ml, 5 eq.) the resin was shaken for 45 min. If necessary, the resin was washed with DMF (3 ml, 1 minute) and the coupling step was repeated.

### Terminal acetylation:

After addition of DIPEA solution (1.75 ml, 16 eq.) and acetic anhydride solution (1.75 ml, 13 eq.) the resin was shaken for 10 minutes. Afterwards the resin was washed with DMF (3 ml, 6x 1 minutes).

### Cleavage method A: Cleavage of protected fragments from hyper-acid labile resin:

After the completion of the assembly of the sequence the resin was finally washed with DCM (3 ml, 4x 1 minute) and then dried in the vacuum. Then the resin was treated with HFIP/DCM (7/1, 4 ml, 4 hours) and the collected solution evaporated to dryness. The residue was purified with preparative HPLC or used without further purification.

### Cleavage method B: Cleavage of unprotected fragments (complete resin cleavage):

After the completion of the assembly of the sequence the resin was finally washed with DCM (3 ml, 4x 1 minute), dried in the vacuum overnight and treated with TFA, EDT, water and TIPS (94/2.5/2.5/1) for 2 h (unless otherwise stated). Afterwards the cleavage solution was poured into a chilled mixture of MTBE and cyclohexane (1/1, 10-fold excess compared to the volume of cleavage solution), centrifuged at 4 °C for 5 minutes and the precipitate collected and dried in the vacuum. The residue was lyophilized from water/acetonitrile prior to purification or further modification.

More relevant Fmoc-solid-phase-peptide synthesis methods are described in detail in "Fmoc Solid Phase Peptide Synthesis" Editors W. Chan, P. White, Oxford University Press, USA, 2000. Compounds were named using MestreNova version 12 Mnova IUPAC Name plugin (Mestrelab Research, S.L.), or AutoNom version 2.2 (Beilstein Informationssysteme Copyright© 1988-1998, Beilstein Institut für Literatur der Organischen Chemie licensed to Beilstein Chemiedaten and Software GmbH, where appropriate.

### Preparation of compounds:

Specific embodiments for the preparation of compounds of the invention are provided in the following examples. Unless otherwise specified, all starting materials and reagents are of standard commercial grade, and are used without further purification, or are readily prepared from such materials by routine methods. Those skilled in the art of organic synthesis will recognize in light of the instant disclosure that starting materials and reaction conditions may be varied including additional steps employed to produce compounds encompassed by the present invention.

One general synthesis route for compounds of the invention comprises
1. Solid Phase Peptide Synthesis (SPPS) of a linear peptide precursor with two thiol moieties.
2. the thiol-site specific cyclization of this linear peptide precursor with
   a. a bis(bromomethyl)benzene derivative or
   b. a tris(bromomethyl)benzene derivative.
3. In case of cyclizations with a tris(bromomethyl)benzene derivative the intermediate formed in the cyclization reaction was further reacted with a linker that enabled the attachment of a chelator.

### Example 2: Synthesis of Ac-Met-[Cys(3MeBn)-Pro-Pro-Thr-Glu-Phe-Cys]-Asp-His-Nmf-Arg-Asp-NH₂ (3BP-3188)

The sequence (Ac-Met-Cys-Pro-Pro-Thr-Glu-Phe-Cys-Asp-His-Nmf-Arg-Asp-NH₂) of the peptide was assembled according to the 'General procedures for Automated/Semi-automated Solid-Phase Synthesis' in a 50 µmol scale on a Rink amide resin. After performing the steps of *'Cleavage method B'* the lyophilized crude peptide residue was dissolved in 60 ml of a 1:1 mixture of ammonium bicarbonate solution (50 mM, pH = 8.5) and acetonitrile. To this mixture a solution of 14.5 mg α,α'-dibromo-m-xylene (55 µmol, 1.1 eq compared to initial resin loading) in 0.5 ml acetonitrile was added. Upon completion of the cyclization reaction 50 µl TFA were added and the solvent removed by lyophilization. The remainder was subjected to HPLC purification (15 to 45% B in 30 min - Kinetex) to yield 8.61 mg of the pure title compound (9.8%). HPLC: t_{R} = 5.87 min. LC/TOF-MS: exact mass 1753.716 (calculated 1753.705). C₇₉H₁₀₇N₁₉O₂₁S₃ (MW = 1755.011).

### Example 3: Synthesis of DOTA-Ttds-Leu-[Cys(3MeBn)-Pro-Pro-Thr-Glu-Phe-Cys]-Asp-His-Phe-Arg-Asp-NH₂ (3BP-3172)

The sequence (DOTA-Ttds-Leu-Cys-Pro-Pro-Thr-Glu-Phe-Cys-Asp-His-Phe-Arg-Asp-NH2) of the peptide was assembled according to the 'General procedures for Automated/Semi-automated Solid-Phase Synthesis' in a 50 µmol scale on a Rink amide resin. After performing the steps of *'Cleavage method B'* the lyophilized crude peptide residue was dissolved in 60 ml of a 1:1 mixture of ammonium bicarbonate solution (50 mM, pH = 8.5) and acetonitrile. To this mixture a solution of 14.5 mg α,α'-dibromo-m-xylene (55 µmol, 1.1 eq compared to initial resin loading) in 0.5 ml acetonitrile was added. Upon completion of the cyclization reaction 50 µl TFA were added and the solvent removed by lyophilization. The remainder was subjected to HPLC purification (20 to 45% B in 30 min - Kinetex) to yield 35.46 mg of the pure title compound (29.8%). HPLC: t_{R} = 5.9 min. LC/TOF-MS: exact mass 2368.091 (calculated 2368.087). C₁₀₇H₁₅₇N₂₅O₃₂S₂ (MW = 2369.676).

### Example 4: Synthesis of Hex-[Cys(3MeBn)-Pro-Pro-Thr-Glu-Phe-Cys]-Asp-His-Ppa-arg-Ttds-Lys(DOTA)-NH₂ (3BP-3277)

The sequence (Hex-Cys-Pro-Pro-Thr-Glu-Phe-Cys-Asp-His-Ppa-arg-Ttds-Lys(Mtt)-NH₂) of the peptide was assembled according to the 'General procedures for Automated/Semi-automated Solid-Phase Synthesis' in a 50 µmol scale on a Rink amide resin. Then a *'Mtt deprotection'* described in the 'General procedures for Automated/Semi-automated Solid-Phase Synthesis' was performed to liberate the ε-amino function of the C-terminal lysine residue of the peptide resin. DOTA(tBu)3-OH (143.3 mg, 250 µmol, 5 eq compared to the intial resin loading) was dissolved in 0.6 ml of a 0.4 M solution of HATU in DMF and 0.65 ml of a 0.9 M of DIPEA in DMF. After leaving the mixture for 1 minute for pre-activation it was added to the resin. An hour later 0.2 ml of a 3.2 M of DIC in DMF was added and the gentle agitation of the resin continued for a further hour. The resin was thoroughly washed and subjected to the *'Cleavage method B'* protocol. The lyophilized remainder (linear, branched peptide Hex-Cys-Pro-Pro-Thr-Glu-Phe-Cys-Asp-His-Ppa-arg-Ttds-Lys(DOTA)-NH₂) was dissolved in 60 ml of a 1:1 mixture of ammonium bicarbonate solution (50 mM, pH = 8.5) and acetonitrile. To this mixture a solution of 14.5 mg α,α'-dibromo-m-xylene (55 µmol, 1.1 eq compared to initial resin loading) in 0.5 ml acetonitrile was added. Upon completion of the cyclization reaction 50 µl TFA were added and the solvent removed by lyophilization. The remainder was subjected to HPLC purification (15 to 40% B in 30 min - Kinetex) to yield 17.18 mg of the pure title compound (14.5%). HPLC: t_{R} = 5.8 min. LC/TOF-MS: exact mass 2367.150 (calculated 2367.139). C₁₀₈H₁₆₂N₂₆O₃₀S₂ (MW = 2368.735).

### Example 5: Synthesis of Pentyl-SO2-[Cys(tMeBn(DOTA-PP))-Pro-Pro-Thr-Gln-Phe-Cys]-Asp-NH₂ (3BP-3692)

The sequence (H-Cys-Pro-Pro-Thr-Gln-Phe-Cys-Asp-NH2) of the peptide was assembled according to the 'General procedures for Automated/Semi-automated Solid-Phase Synthesis' in a 50 µmol scale on a Rink amide resin. The N-terminal sulfonamide was attached by treatment of the resin bound peptide with a solution of n-pentyl sulfonyl chloride (42.7 µl, 300 µmol, 6 eq) and 2,4,6-collidine (29.7 µl, 225 µmοl, 4.5 eq). After overnight agitation the resin was thoroughly washed and subjected to the *'Cleavage method B'* protocol. The lyophilized remainder (linear peptide Pentyl-SO2-Cys-Pro-Pro-Thr-Gln-Phe-Cys-Asp-NH₂) was dissolved in 60 ml of a 1:1 mixture of ammonium bicarbonate solution (50 mM, pH = 8.5) and acetonitrile. To this mixture a solution of 26.8 mg 1,3,5-tris(bromomethyl)benzene (75 µmol, 1.5 eq compared to initial resin loading) in 0.5 ml acetonitrile was added. After stirring the solution for 1 hour 43 mg piperazine (500 µmol, 10 eq compared to intial resin loading) were added. After 2 hours 50 µl TFA were added and the solvent removed by lyophilization. The remainder was subjected to HPLC purification (15 to 45% B in 30 min - Kinetex) to yield 9.15 mg (7.4 µmol) of the peptide intermediate Pentyl-SO2-[Cys(tMeBn(H-PP))-Pro-Pro-Thr-Gln-Phe-Cys]-Asp-NH₂ (14.7%). To the solution of the latter in 150 µl DMSO 2.5 µl DIPEA were added to adjust the pH value to approximately 7.5 - 8. Then 8.4 mg of DOTA-NHS (11 µmol, 1.5 eq compared to the peptide intermediate) in 100 µl DMSO were added. During the course of the LC/TOF-MS monitored reaction 2.5 µl DIPEA was added 3 times to re-adjust the pH value to the starting value. After reaction completion the solution was subjected to HPLC purification (15 to 45% B in 30 min - Kinetex) to yield 7.09 mg of the pure title compound (8.7% overall yield). HPLC: t_{R} = 6.0 min. LC/TOF-MS: exact mass 1628.706 (calculated 1628.704). C₇₂H₁₀₈N₁₆O₂₁S₃ (MW = 1629.924).

### Example 6: Synthesis of Hex-[Cys(tMeBn(DOTA-AET))-Pro-Pro-Thr-Gln-Phe-Cys]-OH (3BP-3554)

Fmoc-Cys(Trt)-OH was loaded onto the trityl resin as described in the 'General procedures for Automated/Semi-automated Solid-Phase Synthesis' in a 50 µmol scale. Onto this resin the sequence (Hex-Cys-Pro-Pro-Thr-Gln-Phe-Cys-OH) of the peptide was assembled according to the 'General procedures for Automated/Semi-automated Solid-Phase Synthesis'. After performing the steps of *'Cleavage method B'* the lyophilized crude peptide residue was dissolved in 60 ml of a 1:1 mixture of ammonium bicarbonate solution (50 mM, pH = 8.5) and acetonitrile. To this mixture a solution of 26.8 mg 1,3,5-tris(bromomethyl)benzene (75 µmol, 1.5 eq compared to initial resin loading) in 0.5 ml acetonitrile was added. The solution was stirred for 1 hour and then 38.6 mg cystamine (500 µmol, 10 eq compared to initial resin loading) was added. After 2 hours 50 µl TFA were added and the solvent removed by lyophilization. The remainder was subjected to HPLC purification (15 to 45% B in 30 min - Kinetex) to yield 19.47 mg (18 µmol) of the intermediate Hex-[Cys(tMeBn(H-AET))-Pro-Pro-Thr-Gln-Phe-Cys]-OH (35.9%). To the solution of the latter in 300 µl DMSO 5 µl DIPEA were added to adjust the pH value to approximately 7.5 - 8. Then 20.5 mg of DOTA-NHS (27 µmol, 1.5 eq compared to the peptide intermediate) in 200 µl DMSO were added. During the course of the LC/TOF-MS monitored reaction 5 µl DIPEA was added 3 times to re-adjust the pH value to the starting value. After reaction completion the solution was subjected to HPLC purification (15 to 45% B in 30 min - Kinetex) to yield 20.44 mg of the pure title compound (27.8% overall yield). HPLC: t_{R} = 5.9 min. LC/TOF-MS: exact mass 1469.640 (calculated 1469.639). C₆₇H₉₉N₁₃O₁₈S₃ (MW = 1470.780).

### Example 7: Synthesis of Hex-[Cys(tMeBn(DOTA-AET))-Pro-Pro-Thr-Gln-Phe-AET] (3BP-3654)

This synthesis was performed as the synthesis of 3BP-3554 described in Example 6 except for the fact that a commercially available pre-loaded aminoethanthiol trityl resin was used for the assembly of the linear peptide precursor Hex-Cys-Pro-Pro-Thr-Gln-Phe-AET. After performing all the steps described in Example 6 HPLC purification (15 to 45% B in 30 min - Kinetex) finally yielded 21.25 mg of the pure title compound (29.8% overall yield). HPLC: t_{R} = 6.2 min. LC/TOF-MS: exact mass 1425.661 (calculated 1425.649). C₆₆H₉₉N₁₃O₁₆S₃ (MW = 1426.771).

### Example 8: Synthesis of Hex-[Cys(tMeBn(DOTA-AET))-Pro-Pro-Thr-Gln-Phe-Cysol] (3BP-3762)

This synthesis was performed as the synthesis of 3BP-3554 described in Example 6 except for the fact that Fmoc-Cysteinol(Trt)-OH was loaded onto the trityl resin. Differing from the description in the 'General procedures for Automated/Semi-automated Solid-Phase Synthesis' this was achieved as follows: 50 µmol of trityl resin were swollen in THF and subsequently washed with dry THF (3 times). Then the resin was treated with a solution of Fmoc-Cysteinol(Trt)-OH (57 mg, 100 µmol, 2 eq) and pyridine (16.1 µl, 200 µmol, 4 eq) in dry THF (1 ml) for 20 hours at 60 °C. After washing the resin thoroughly (THF, MeOH, DCM, DMF, 3 ml, 3x 1 min) the linear peptide precursor Hex-Cys-Pro-Pro-Thr-Gln-Phe-Cysol was assembled as described in the 'General procedures for Automated/Semi-automated Solid-Phase Synthesis'. After performing all the steps described in Example 6 HPLC purification (15 to 45% B in 30 min - Kinetex) finally yielded 7.8 mg of the pure title compound (10.7% overall yield). HPLC: t_{R} = 5.9 min. LC/TOF-MS: exact mass 1455.666 (calculated 1455.660). C₆₇H₁₀₁N₁₃O₁₇S₃ (MW = 1456.797).

### Example 9: Preparation of DOTA-transition metal complexes of compounds of the invention

### A. General procedure for the preparation of a peptide comprising DOTA-transition metal-complexes from corresponding peptides comprising uncomplexed DOTA

A 0.1 mM solution of the peptide comprised by uncomplexed DOTA in
- 0.4 M sodium acetate, pH = 5 (Buffer A) (in case of In(III), Lu(III) or Ga(III) complexes) or
- 0.1 M ammonium acetate, pH = 8 (Buffer B) (in case of Eu(III) complexes)
was diluted with a solution 0.1 mM solution of the corresponding metal salt in water whereby the molar ratio of peptide to metal was adjusted to 1 : 3. The solution was stirred
- at 50 °C for 20 minutes (also referred to herein as Condition A) (in case of In(III), Lu(III) or Ga(III) complexes) or
- at room temperature overnight (also referred to herein as Condition B) (in case of Eu(III) complexes).

The solution was then applied to
- HPLC purification (also referred to herein as Purification A) or
- solid phase extraction (also referred to herein as Purification B).
   In case of solid phase extraction 250 mg Varian Bondesil-ENV was placed in a 15 ml polystyrene syringe, pre-washed with methanol (1 x 5 ml) and water (2 x 5 ml). Then the reaction solution was applied to the column. Thereafter elution was performed with water (2 x 5 ml - to remove excess salt), 5 ml of 50% ACN in water as first fraction and each of the next fractions were eluted with 5 ml of 50% ACN in water containing 0.1% TFA.

In either case (HPLC purification or solid phase extraction) fractions containing the pure product were pooled and freeze dried.

### B. Indium-complex of Hex-[Cys(tMeBn(DOTA-PP))-Pro-Pro-Thr-Gln-Phe-Cys]-Asp-NH₂ (3BP-3590)

The complex was prepared starting from 25 mg peptide 3BP-3407 (15.7 µmol) dissolved in Buffer A, diluted with a solution of InCl₃ x 4 H₂O which was treated with Condition A. In the purification step 'Purification A' was employed (15 to 40% B in 30 min - RLRP-S) to yield 18.24 mg of the pure title compound (68.1% yield). HPLC: t_{R} = 5.6 min. LC/TOF-MS: exact mass 1702.622 (calculated 1702.617). C₇₃H₁₀₅InN₁₆O₂₀S₂ (MW = 1705.663).

### C. Gallium-complex of Hex-[Cys(tMeBn(DOTA-PP))-Pro-Pro-Thr-Gln-Phe-Cys]-Asp-NH₂ (3BP-3592)

The complex was prepared starting from 25 mg peptide 3BP-3407 (15.7 µmol) dissolved in Buffer A, diluted with a solution of Ga(NO₃)₃ x H₂O which was treated with Condition A. In the purification step 'Purification A' was employed (15 to 40% B in 30 min - RLRP-S) to yield 16.78 mg of the pure title compound (69.3% yield). HPLC: t_{R} = 5.7 min. LC/TOF-MS: exact mass 1658.664 (calculated 1658.639). C₇₃H₁₀₅GaN₁₆O₂₀S₂ (MW = 1660.568).

### D. Lutetium-complex of Hex-[Cys(tMeBn(DOTA-PP))-Pro-Pro-Thr-Gln-Phe-Cys]-Asp-NH₂ (3BP-3591)

The complex was prepared starting from 25 mg peptide 3BP-3407 (15.7 µmol) dissolved in Buffer A, diluted with a solution of LuCl3 which was treated with Condition A. In the purification step 'Purification A' was employed (15 to 40% B in 30 min - RLRP-S) to yield 16.66 mg of the pure title compound (60.1% yield). HPLC: t_{R} = 5.6 min. LC/TOF-MS: exact mass 1764.654 (calculated 1764.654). C₇₃H₁₀₅LuN₁₆O₂₀S₂ (MW = 1765.812).

### E. Europium-complex of Hex-[Cys(tMeBn(DOTA-PP))-Pro-Pro-Thr-Gln-Phe-Cys]-Asp-NH₂ (3BP-3661)

The complex was prepared starting from 9.5 mg peptide (6 µmol) 3BP-3407 dissolved in Buffer B, diluted with a solution of EuCl₃ x 6 H₂O which was treated with Condition B. In the purification step 'Purification B' was employed to yield 8.24 mg of the pure title compound (79.3% yield). HPLC: t_{R} = 5.7 min. LC/TOF-MS: exact mass 1740.636 (calculated 1740.633). C₇₃H₁₀₅EuN₁₆O₂₀S₂ (MW = 1742.809).

### Example 10: Plasma stability assay

In order to determine the stability of selected compounds of the invention in human and mouse plasma, a plasma stability assay was carried out. Such plasma stability assay measures degradation of compounds of the present invention in blood plasma. This is an important characteristic of a compound as compounds, with the exception of pro-drugs, which rapidly degrade in plasma, generally show poor *in vivo* efficacy. The results show that those compounds are highly stable in human and mouse plasma. The stability is sufficient for the diagnostic, therapeutic and theragnostic use of these compounds according to the present invention.

The plasma stability samples were prepared by spiking 50 µl plasma aliquots with 1 µl of a 0.5 mM compound stock solution in DMSO. After vortexing the samples were incubated in a Thermomixer at 37°C for 0, 4 and 24 hours. After incubation the samples were stored on ice until further treatment. All samples were prepared in duplicates.

A suitable internal standard was added to each sample (1 µl of a 0.5 mM stock solution in DMSO). Protein precipitation was performed using two different methods depending on the compound conditions as indicated in Table 7.
A) 250 µl of acetonitrile containing 1% trifluoroacetic acid was added. After incubation at room temperature for 30 min the precipitate was separated by centrifugation and 150 µl of the supernatant was diluted with 150 µl of 1% aqueous formic acid.
B) 150 µl of a zinc sulphate precipitation agent containing 78% 0.1 M zinc sulphate and 22% acetonitrile was added. After incubation at room temperature for 30 min the precipitate was separated by centrifugation. To 100 µl of the supernatant 10 µl of 1% formic acid was added followed by further incubation at 60°C for 10 min to complete the formation of the zinc chelate, if the compound contains a free DOTA moiety.

The determination of the analyte in the clean sample solutions was performed on an Agilent 1290 UHPLC system coupled to an Agilent 6530 Q-TOF mass spectrometer. The chromatographic separation was carried out on a Phenomenex BioZen XB-C18 HPLC column (50 x 2 mm, 1.7 µm particle size) with gradient elution using a mixture of 0.1% formic acid in water as eluent A and acetonitrile as eluent B (2% B to 41% in 7 min, 800 µl/min, 40°C). Mass spectrometric detection was performed in positive ion ESI mode by scanning the mass range from m/z 50 to 3000 with a sampling rate of 2 / sec.

From the mass spectrometric raw data the ion currents for the double or triple charged monoisotopic signal was extracted for both, the compound and the internal standard.

Quantitation was performed by external matrix calibration with internal standard using the integrated analyte signals.

Additionally, recovery was determined by spiking a pure plasma sample that only contained the internal standard after treatment with a certain amount of the compound.

Carry-over was evaluated by analysis of a blank sample (20% acetonitrile) after the highest calibration sample.

The results of this assay performed on some of the compounds according to the present invention are given in the following Table 7. The result is stated as "% intact compound remaining after 4 h or 24 h" and means that from the amount of material at the start of the experiment the stated percentage is detected as unchanged material at the end of the experiment by LC-MS quantification. Since all compounds are more than 50% intact after at least 4 h they are considered as stable enough for diagnostic and therapeutic applications

**Table 7: Results of the plasma stability assay**

| | **Protein precipitation method** | **% intact compound remaining after 4/24h incubation** | |
|---|---|---|---|
| **Compound** | | **Human plasma** | **Mouse plasma** |
| 3BP-2974 | A | | 92% (4 h) |
| 3BP-2975 | A | | 100% (4 h) |
| 3BP-2976 | A | | 93% (4 h) |
| 3BP-3086 | A | | 79% (4 h) |
| 3BP-3105 | A | | 55% (4 h) |
| 3BP-3168 | A | | 100% (4 h) |
| 3BP-3177 | A | | 79% (4 h) |
| 3BP-3181 | A | | 100% (4 h) |
| 3BP-3183 | A | | 98% (4 h) |
| 3BP-3187 | A | | 100% (4 h) |
| 3BP-3188 | A | | 97% (4 h) |
| 3BP-3189 | A | | 100% (4 h) |
| 3BP-3190 | A | | 88% (4 h) |
| 3BP-3191 | A | | 100% (4 h) |
| 3BP-3196 | A | | 87% (4 h) |
| 3BP-3202 | A | | 78% (4 h) |
| 3BP-3203 | A | | 100% (4 h) |
| 3BP-3210 | A | | 100% (4 h) |
| 3BP-3211 | A | | 85% (4 h) |
| 3BP-3212 | A | | 80% (4 h) |
| 3BP-3275 | A | | 94% (4 h) |
| 3BP-3319 | A | | 100% (4 h) |
| 3BP-3320 | A | | 75% (4 h) |
| 3BP-3321 | A | | 94% (4 h) |
| 3BP-3397 | A | 100% (24 h) | 92% (24 h) |
| 3BP-3398 | A | 99% (24 h) | 94% (24 h) |
| 3BP-3407 | A | 100% (24 h) | 79% (24 h) |
| 3BP-3426 | B | | 73% (24 h) |
| 3BP-3554 | B | | 85% (24 h) |
| 3BP-3555 | B | | 88% (24 h) |
| 3BP-3590 | B | 94% (24 h) | 97% (24 h) |
| 3BP-3623 | B | | 100% (24 h) |

### Example 11: FACS Binding Assay

In order to determine binding of compounds according to the present invention to FAP-expressing cells, a competitive FACS binding assay was established.

FAP-expressing human WI-38 fibroblasts (ECACC) were cultured in EMEM including 15% fetal bovine serum, 2mM L-Glutamine and 1% Non-essential amino acids. Cells were detached with Accutase (Biolegend, #BLD-423201) and washed in FACS buffer (PBS including 1% FBS). Cells were diluted in FACS buffer to a final concentration of 100.000 cells per ml and 200 µl of the cell suspension are transferred to a u-shaped non-binding 96-well plate (Greiner). Cells were washed in ice-cold FACS buffer and incubated with 3 nM of Cy5-labeled compound (H-Met-[Cys(3MeBn)-Pro-Pro-Thr-Glu-Phe-Cys]-Asp-His-Phe-Arg-Asp-Ttds-Lys(Cy5S03)-NH2) in the presence of increasing concentrations of peptides at 4°C for 1 hour. Cell were washed twice with FACS buffer and resuspended in 200 µl FACS buffer. Cells were analyzed in an Attune NxT flow cytometer. Median fluorescence intensities (Cy5 channel) was calculated by Attune NxT software and plotted against peptide concentrations. Four parameter logistic (4PL) curve fitting and pIC50 calculations were performed using ActivityBase software. The results of this assay as well as the ones of the FAP protease activity assay as subject to Example 12 for each compound according to the present invention are presented in Table 8 (shown in Example 12). pIC50 category A stands for pIC50 values >8.0, category B for pIC50 values between 7.1 and 8.0, category C for pIC50 values between 6.1 and 7.0 and category D for pIC50 values ≤ 6.0.

### Example 12: FAP Protease Activity Assay

In order to determine the inhibitory activity of the peptides of example 11, a FRET-based FAP protease activity assay was established.

Recombinant human FAP (R&D systems, # 3715-SE) was diluted in assay buffer (50 mM Tris, 1 M NaCl, 1 mg/mL BSA, pH 7.5) to a concentration of 3.6 nM. 25 µl of the FAP solution was mixed with 25 µl of a 3-fold serial dilution of the test compounds and incubated for 5 min in a white 96-well ProxiPlate (Perkin Elmer). As specific FAP substrate the FRET-peptide HiLyteFluor™ 488 - VS(D-)P SQG K(QXL® 520) - NH2 was used (Bainbridge, et al., Sci Rep, 2017, 7: 12524). 25 µL of a 30 µM substrate solution, diluted in assay buffer, was added. All solutions were equilibrated at 37°C prior to use. Substrate cleavage and increase in fluorescence (excitation at 485 nm and emission at 538 nm) was measured in a kinetic mode for 5 minutes at 37°C in a SPECTRAmax M5 plate reader. RFU/sec was calculated by SoftMax Pro software and plotted against peptide concentration. Four parameter logistic (4PL) curve fitting and pIC50 calculations were performed using ActivityBase software. The results of this assay for each compound according to the present invention are given in Table 8 (Example 11). pIC50 category A stands for pIC50 values >8.0, category B for pIC50 values between 7.1 and 8.0, category C for pIC50 values between 6.1 and 7.0 and category D for pIC50 values ≤ 6.0.

As evident from Table 8, the compounds of the present invention show surprisingly superior results in both the FACS Binding assay and the FAP protease activity assay.

In addition to this one can easily find SAR-data which demonstrates that compounds with conjugated chelator are of very similar activity to compounds without chelator but similar peptide sequence. For instance, 3BP-3168 and 3BP-3169 posess chelator and linker at the C-terminus (DOTA-Ttds-Nle/Met) and are in the highest activity categories of pIC₅₀ >8. Corresponding compounds without chelator and linker at the N-Terminus (3BP-2974 with N-terminal Hex-, 3BP-2975 with N-terminal Ac-Met and 3BP-2976 with N-terminal H-met) exhibit all similar activity compared to the chelator comprising compounds 3BP-3168 and 3BP-3169.

This means that the activity data from chelator free compounds is predictive for the activity of the chelator comprising compounds. This phenomenon is additionally also observed if the chelator is conjugated to the compounds of invention according to the other two specified possibilities. Examples for chelator attachment to the C-terminus compared to corresponding compounds without chelator show the same trends and are 3BP-3105 vs. 3BP-2974, 3BP-3395 or 3BP-3397 vs. 3BP-3476 and examples for the attachment of the chelator to Y_{c} vs. orresponding compounds without chelator are 3BP-3407 vs. 3B-3476 or 3BP-3426 vs. 3BP-3476.

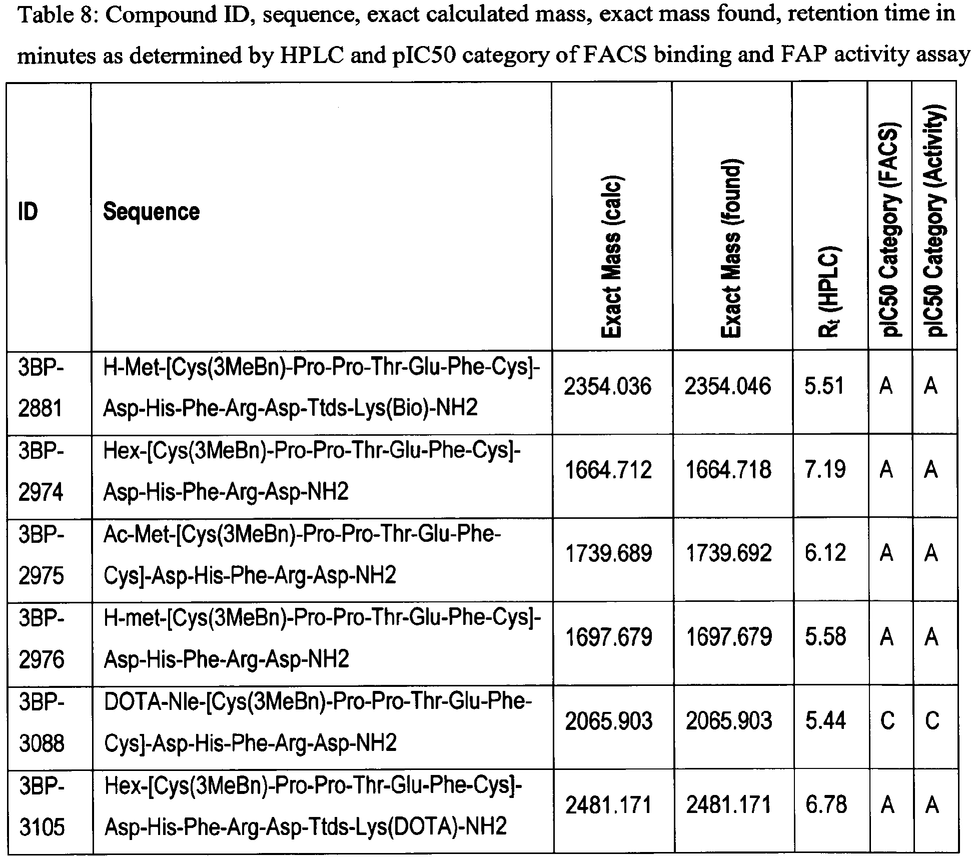

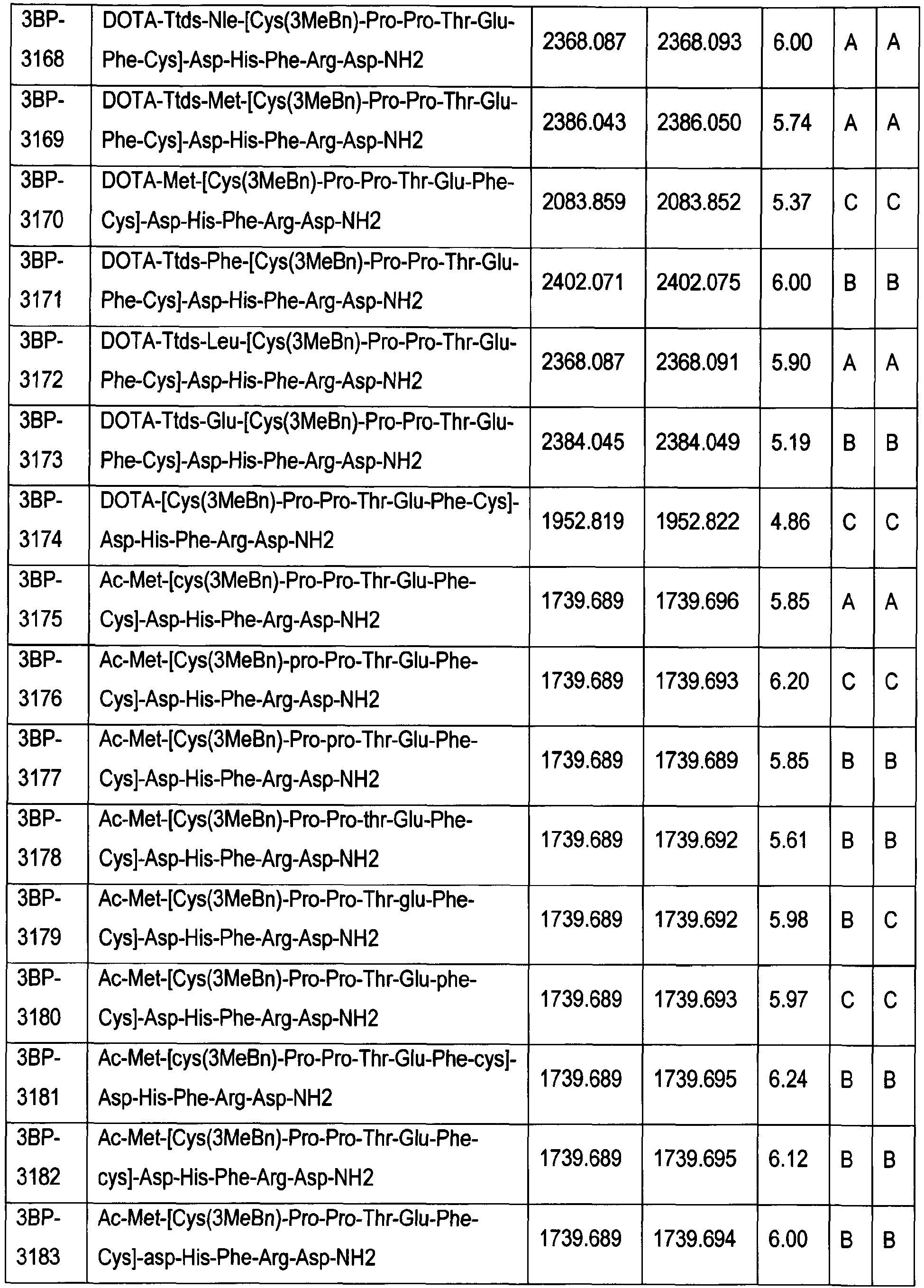

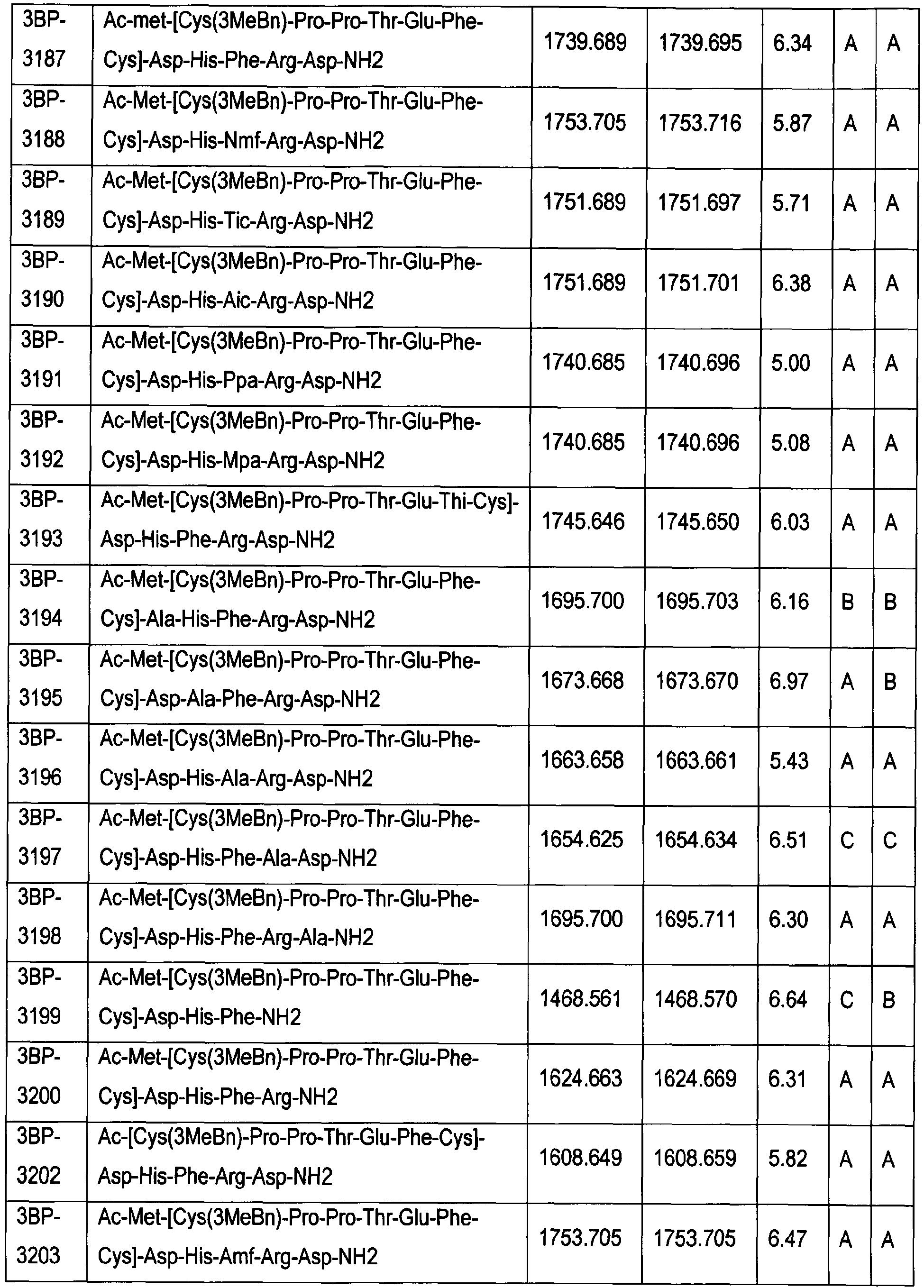

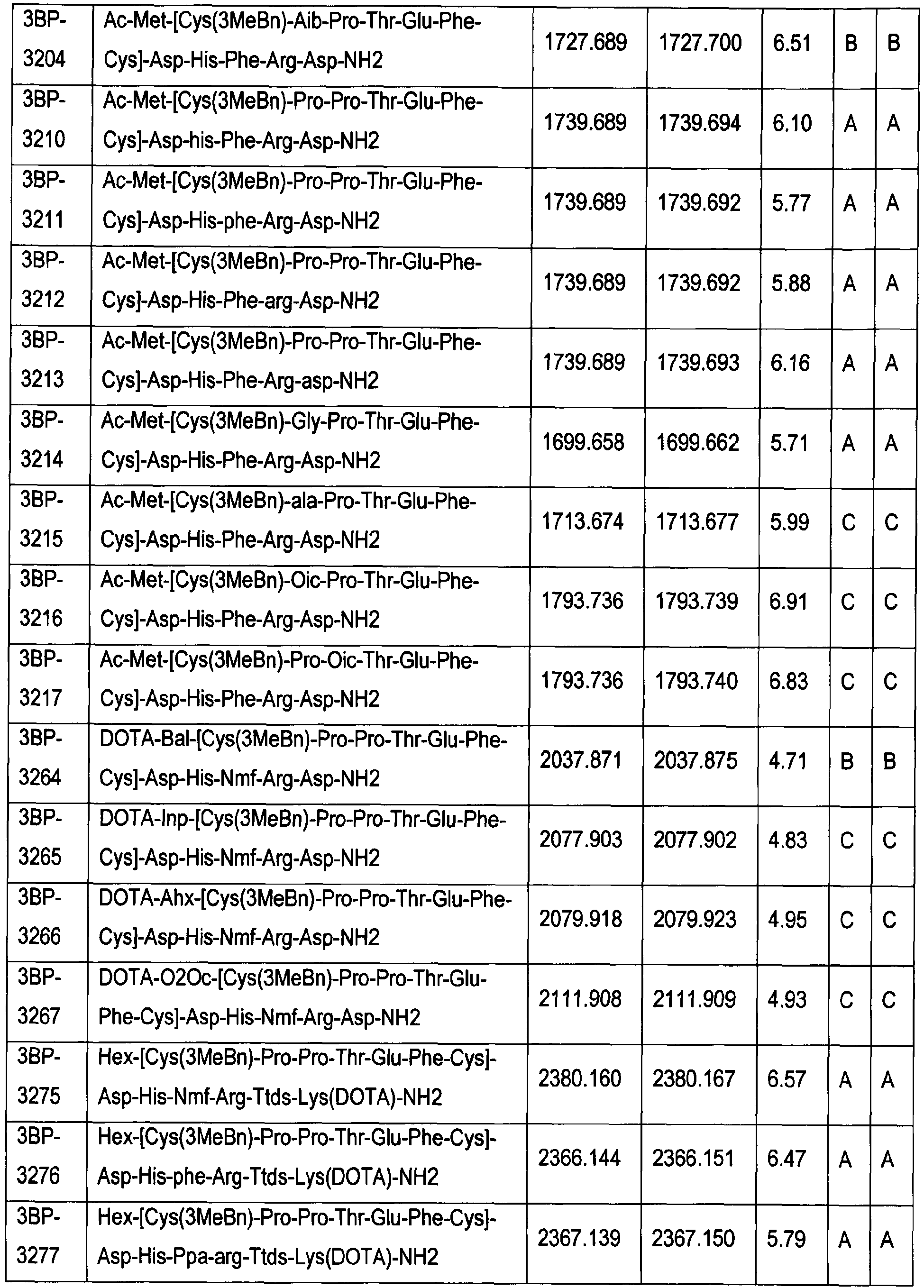

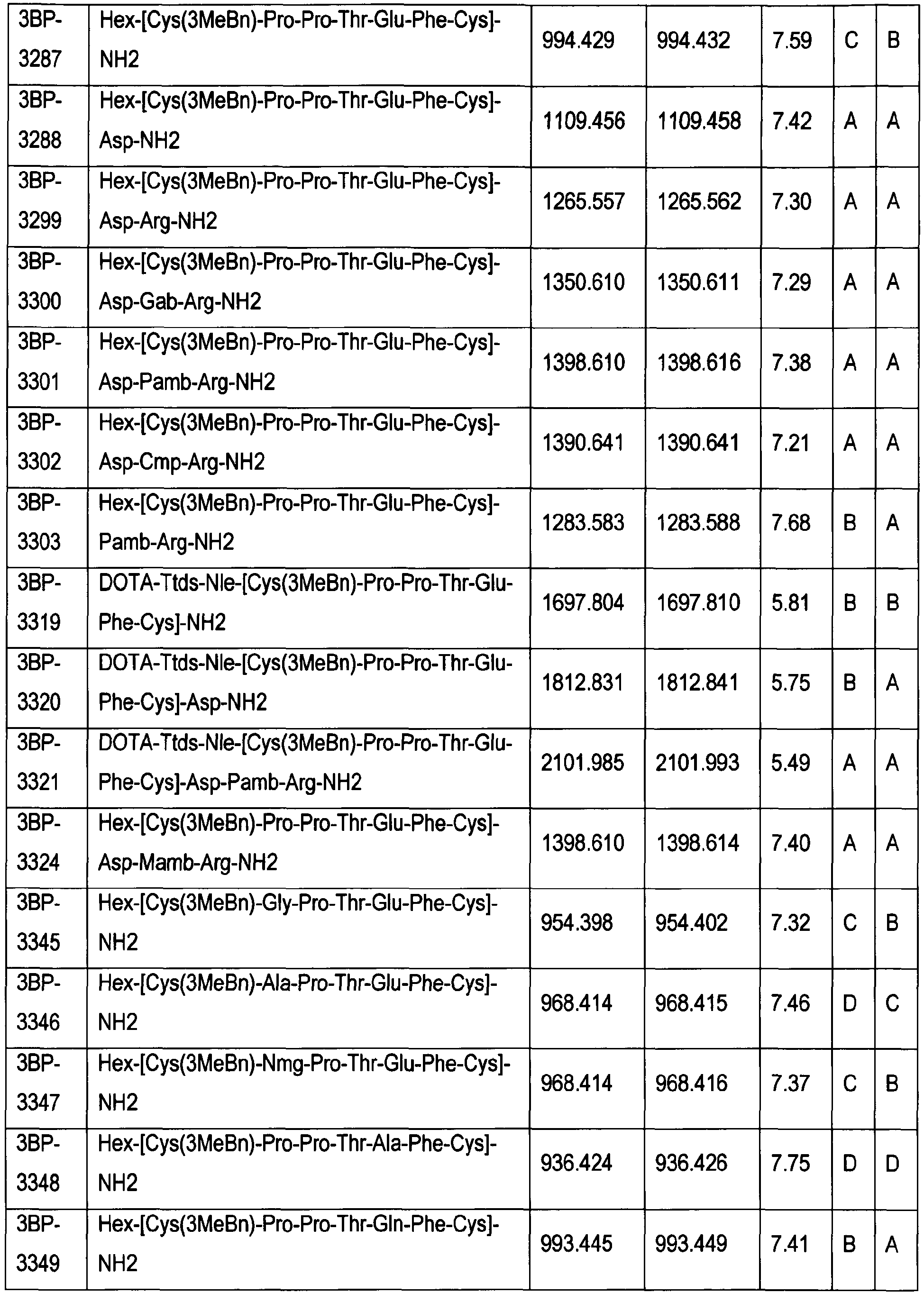

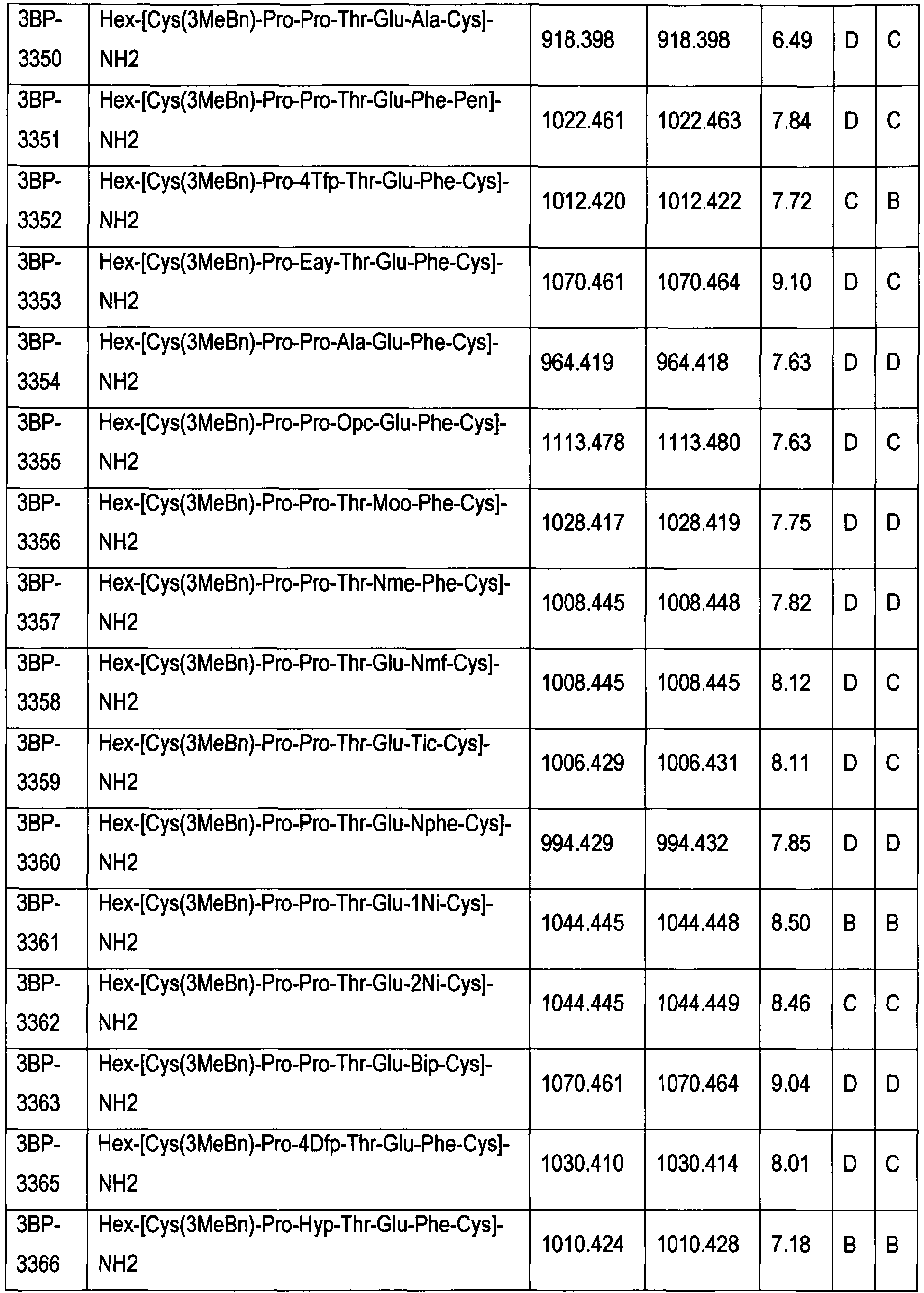

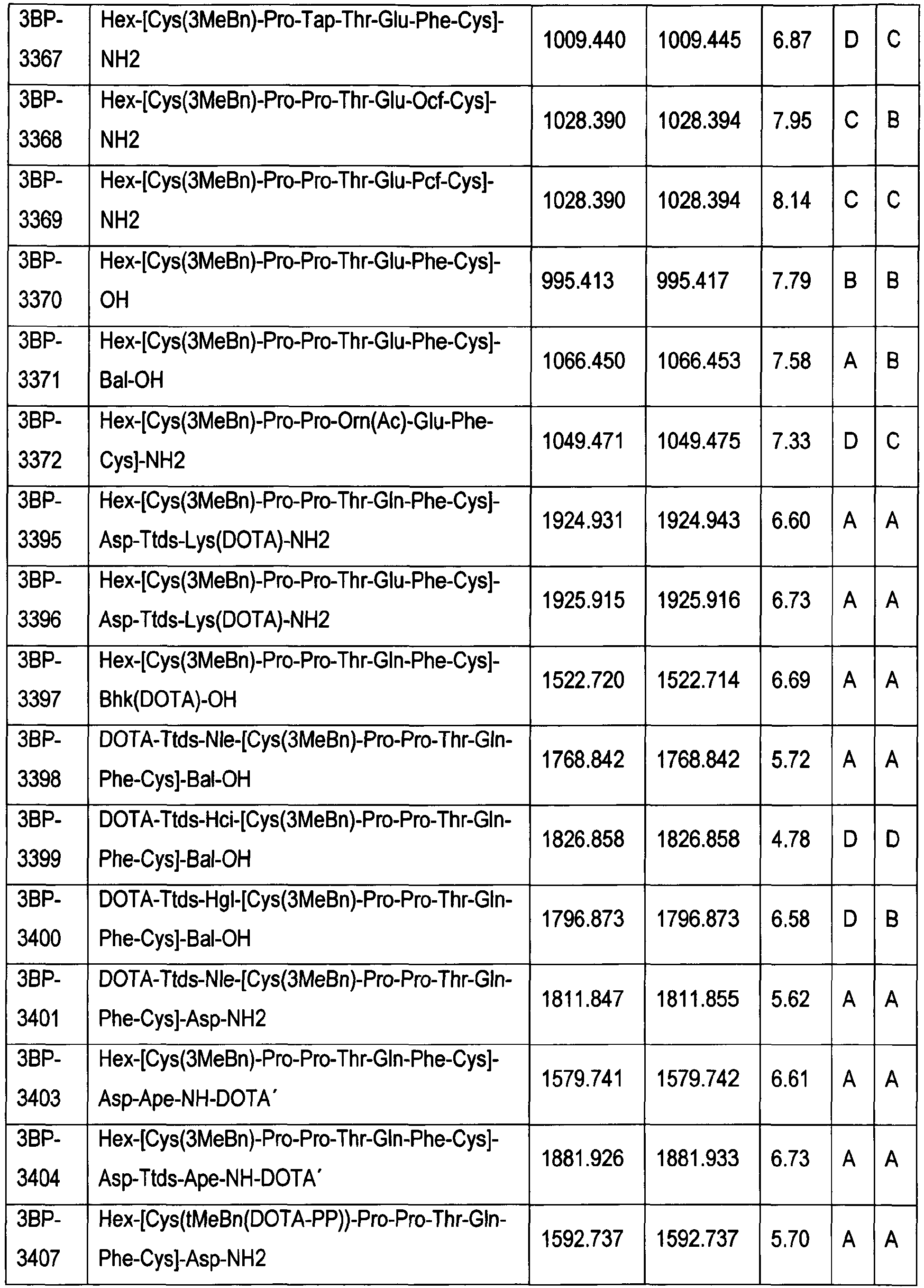

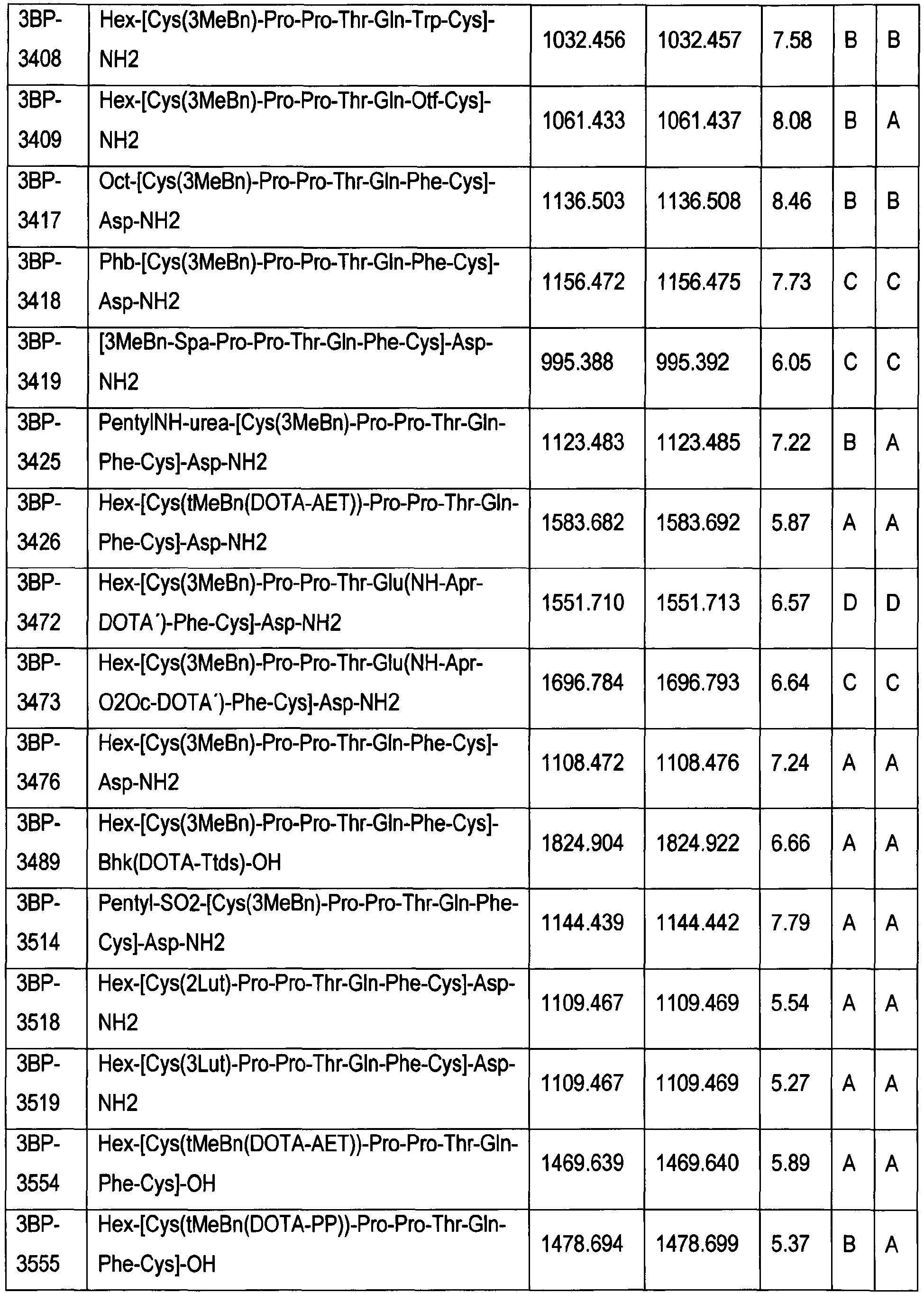

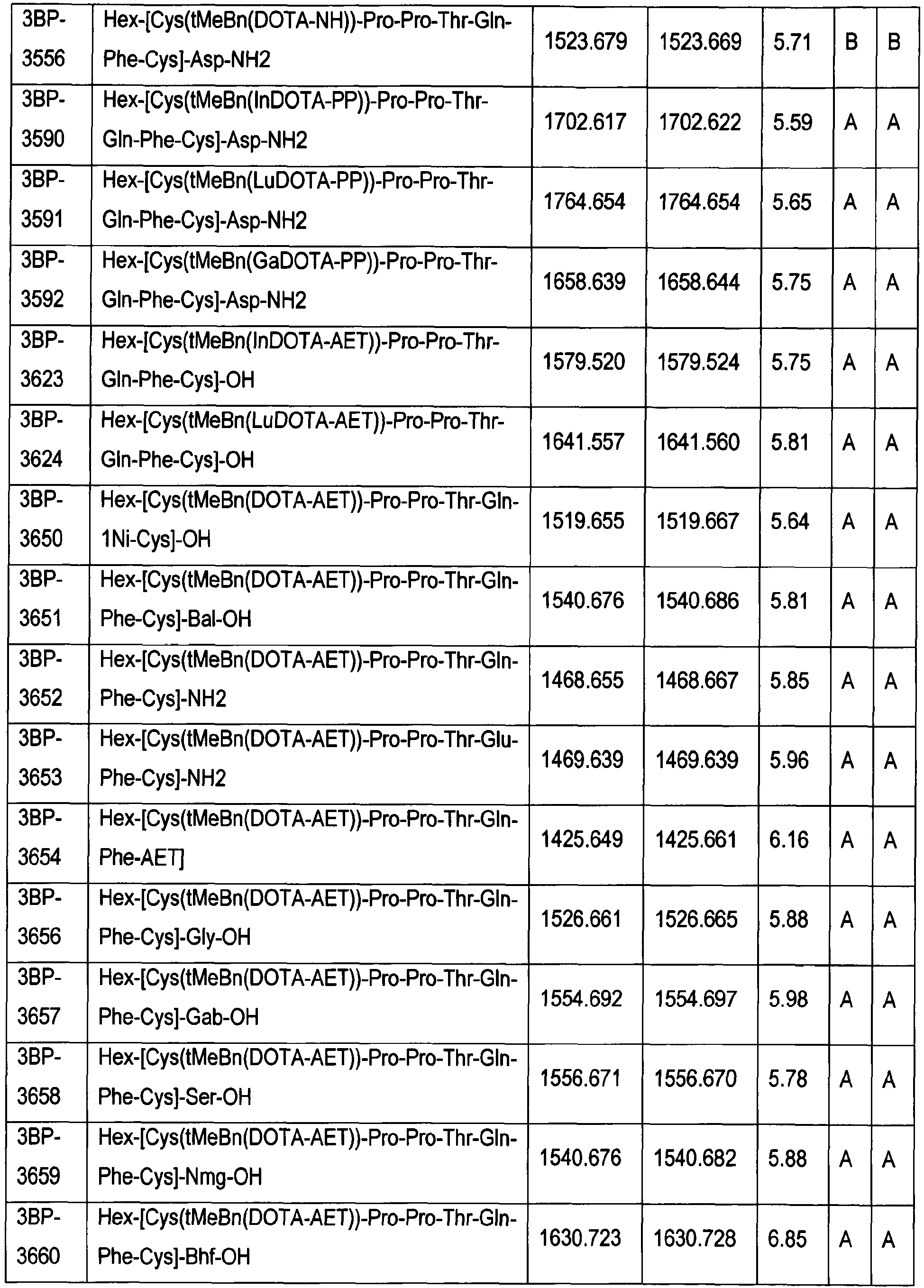

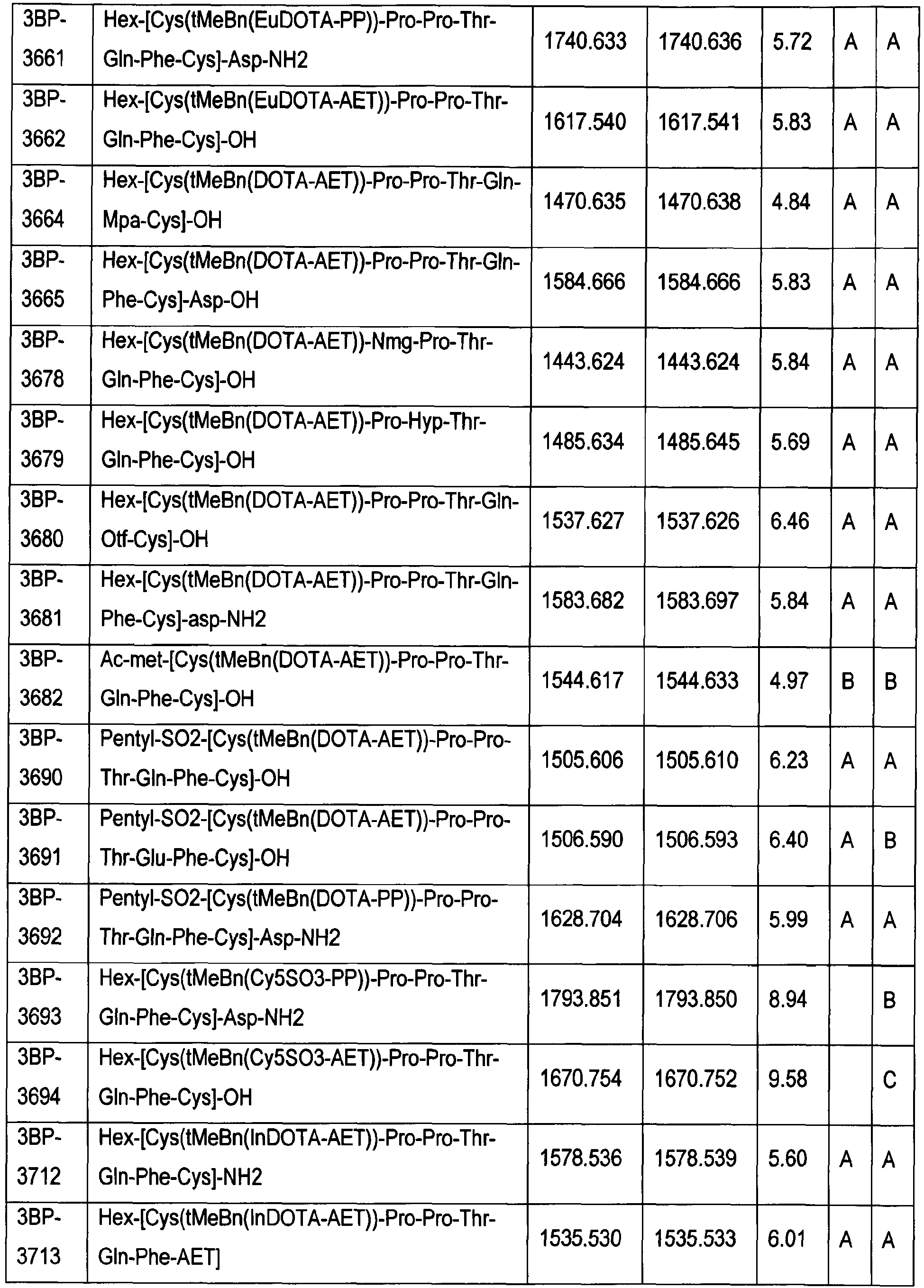

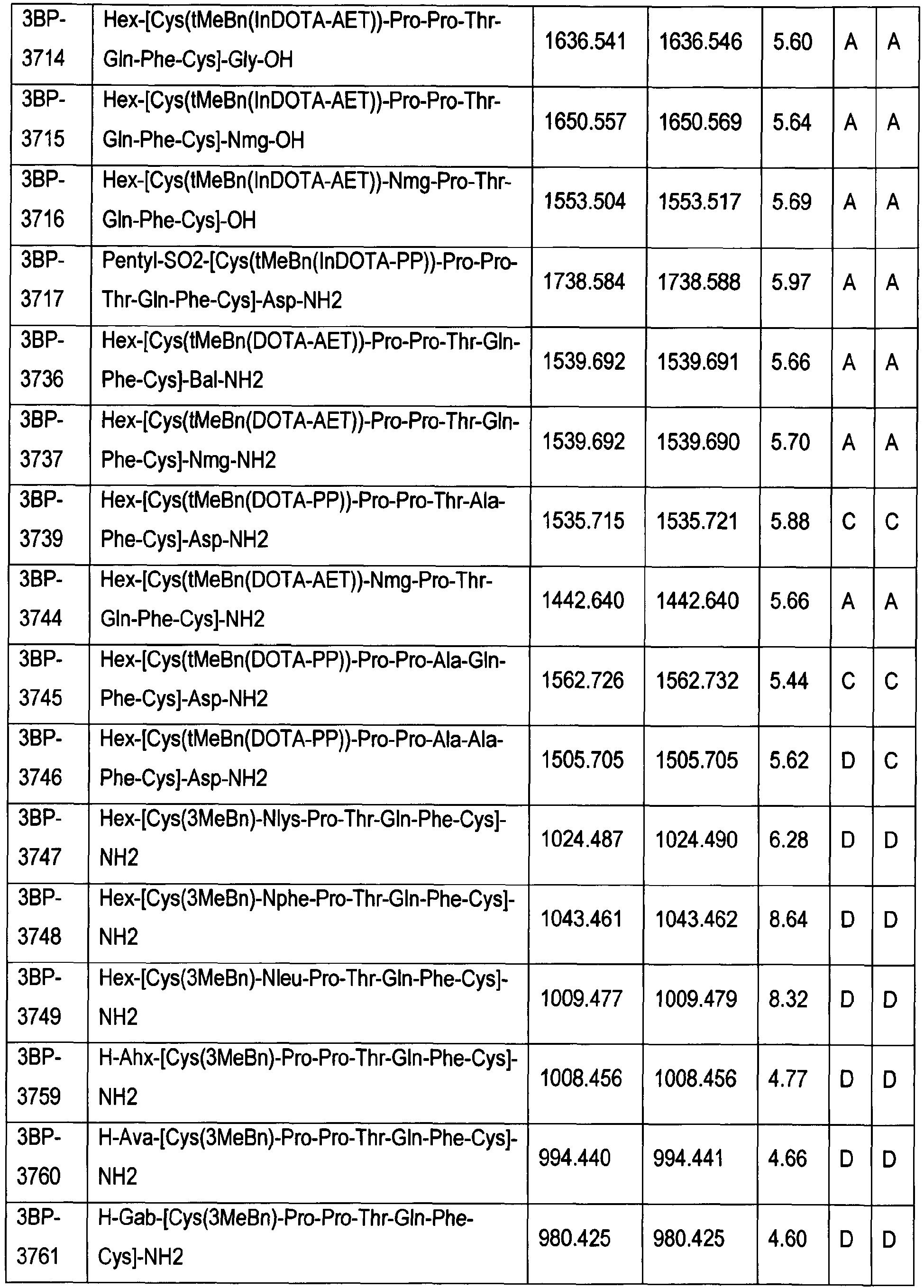

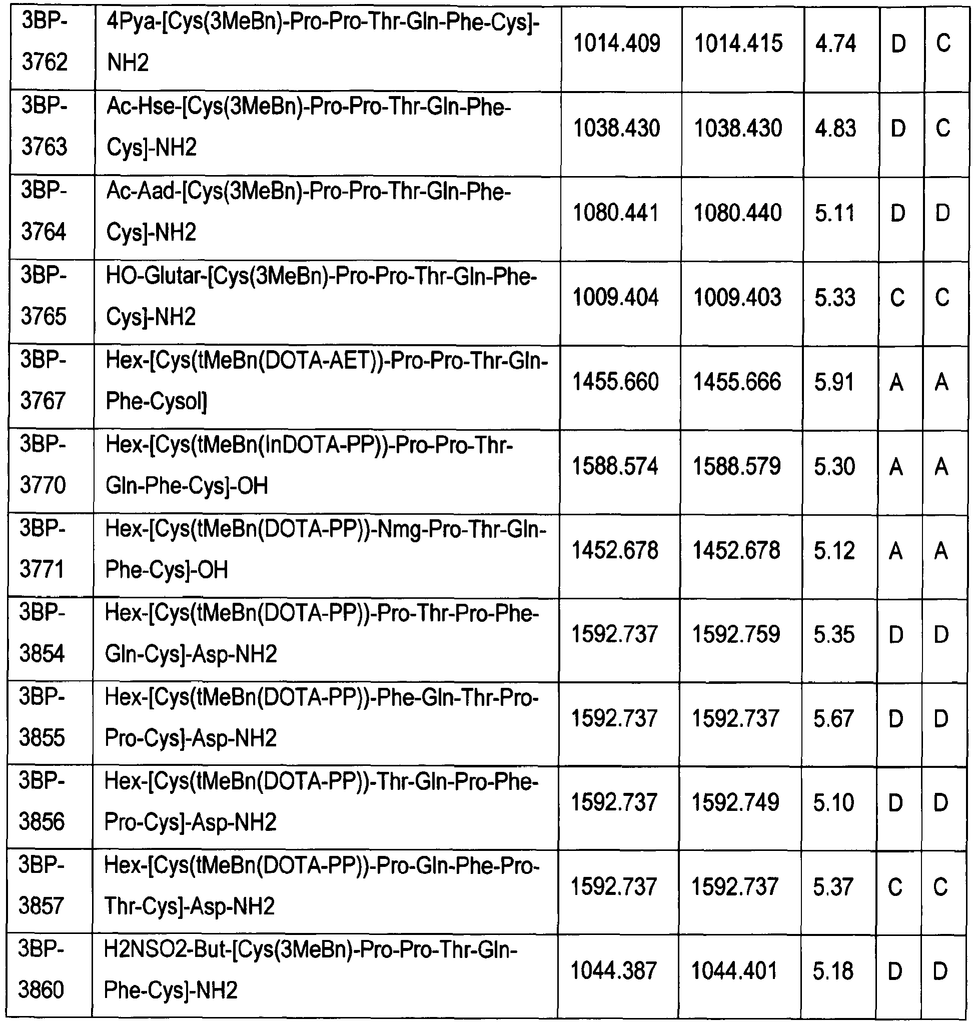

### Example 13: PREP and DPP4 Protease Activity Assay

In order to test selectivity of FAP binding peptides toward both PREP and DPP4, protease activity assays were performed analogues to the FAP activity assay described above with following exceptions.

PREP activity was measured with recombinant human PREP (R&D systems, #4308-SE). As substrate 50 µM Z-GP-AMC (Bachem, # 4002518) was used. The DPP4 activity assay was performed in DPP assay buffer (25 mM Tris, pH 8.0). Recombinant human DPP4 was purchased from R&D systems (#9168-SE). 20 µM of GP-AMC (Santa Cruz Biotechnology, #115035-46-6) was used as substrate.

Fluorescence of AMC (excitation at 380 nm and emission at 460 nm) after cleavage was measured in a kinetic mode for 5 minutes at 37°C in a SPECTRAmax M5 plate reader. RFU/sec was calculated by SoftMax Pro software and plotted against peptide concentration. Four parameter logistic (4PL) curve fitting and pIC50 calculations were performed using ActivityBase software. The results of this assay for some of the compounds according to the present invention are given in the following Table 9.

**Table 9: Results (pIC50 values) of PREP and DPP4 activity assays**

| **ID** | **pIC50 (PREP)** | **pIC50 (DPP4)** |
|---|---|---|
| 3BP-2881 | <6 | <6 |
| 3BP-3105 | <6 | <6 |
| 3BP-3168 | <6 | <6 |
| 3BP-3275 | <6 | <6 |
| 3BP-3287 | <6 | <6 |
| 3BP-3319 | 6.2 | <6 |
| 3BP-3320 | <6 | <6 |
| 3BP-3321 | <6 | <6 |
| 3BP-3349 | <6 | <6 |
| 3BP-3397 | <6 | <6 |
| 3BP-3398 | <6 | <6 |
| 3BP-3407 | <6 | <6 |
| 3BP-3419 | <6 | <6 |
| 3BP-3426 | <6 | <6 |
| 3BP-3476 | <6 | <6 |
| 3BP-3554 | <6 | <6 |

### Example 14: Specificity Screen

The specificity screening was carried out in order to early identify significant off-target interactions of compounds of the present invention. The specificity was tested using a standard battery of assays ("SafetyScreen44™ Panel") comprising 44 selected targets and compounds binding thereto (referred to as "reference compounds", Ref. Compounds), recommended by Bowes et al. (Bowes, et al., Nat Rev Drug Discov, 2012, 11: 909). The reference compounds served as positive controls for the respective assays, therefore inhibition is expected to be detected with these reference compounds. The compounds of the invention, however, were not expected to show inhibition in these assays. These binding and enzyme inhibition assays were performed by Eurofins Cerep SA (Celle l'Evescault, France).

3BP-3407 was tested at 10µM. Compound binding was calculated as % inhibition of the binding of a radioactively labeled ligand specific for each target ("% Inhibition of Specific Binding (3BP-3407)"). Compound enzyme inhibition effect was calculated as % inhibition of control enzyme activity.

Results showing an inhibition or stimulation higher than 50% are considered to represent significant effects of the test compounds. Such effects were not observed at any of the receptors studied which are listed in the following Table 10. The results of this assay are summarized in the following Table 10.

**Table 10: Results of the specificity screening (SafetyScreen44™ Panel) for 10 µM 3BP-3407**

| **Assay** | **% Inhibition of Specific Binding (3BP-3407)** | **Ref Compound** | **Ki Ref [M]** | **Cerep Catalog Ref** | **Literature Reference** |
|---|---|---|---|---|---|
| A2A (h) (agonist radioligand) | -4 | NECA | 2.90E-08 | 4 | (Luthin, et al., Mol Pharmacol, 1995, 47: 307) |
| alpha 1A (h) (antagonist radioligand) | 2 | WB 4101 | 2.40E-10 | 2338 | (Schwinn, et al., J Biol Chem, 1990, 265: 8183) |
| alpha 2A (h) (antagonist radioligand) | -9 | yohimbine | 2.40E-09 | 13 | (Langin, et al., Eur J Pharmacol, 1989, 167: 95) |
| beta 1 (h) (agonist radioligand) | 4 | atenolol | 3.40E-07 | 18 | (Levin, et al., J Biol Chem, 2002, 277: 30429) |
| beta 2 (h) (antagonist radioligand) | 4 | ICI 118551 | 1.60E-10 | 20 | (Joseph, et al., Naunyn Schmiedebergs Arch Pharmacol, 2004, 369: 525) |
| BZD (central) (agonist radioligand) | -9 | diazepam | 8.10E-09 | 28 | (Speth, et al., Life Sci, 1979, 24:351) |
| CB1 (h) (agonist radioligand) | 5 | CP 55940 | 2.10E-09 | 36 | (Rinaldi-Carmona, et al., J Pharmacol Exp Ther, 1996, 278: 871) |
| CB2 (h) (agonist radioligand) | 2 | WIN 55212-2 | 1.60E-09 | 37 | (Munro, et al., Nature, 1993, 365: 61) |
| CCK1 (CCKA) (h) (agonist radioligand) | 24 | CCK-8s | 4.90E-11 | 39 | (Bignon, et al., J Pharmacol Exp Ther, 1999, 289: 742) |
| D1 (h) (antagonist radioligand) | 0 | SCH 23390 | 2.00E-10 | 44 | (Zhou, et al., Nature, 1990, 347: 76) |
| D2S (h) (agonist radioligand) | 15 | 7-OH-DPAT | 1.30E-09 | 1322 | (Grandy, et al., Proc Natl Acad Sci U S A, 1989, 86: 9762) |
| ETA (h) (agonist radioligand) | -18 | endothelin-1 | 1.50E-11 | 54 | (Buchan, et al., Br J Pharmacol, 1994, 112: 1251) |
| NMDA (antagonist radioligand) | 9 | CGS 19755 | 1.40E-07 | 66 | (Sills, et al., Eur J Pharmacol, 1991, 192: 19) |
| H1 (h) (antagonist radioligand) | 11 | pyrilamine | 1.10E-09 | 870 | (Smit, et al., Br J Pharmacol, 1996, 117: 1071) |
| H2 (h) (antagonist radioligand) | -5 | cimetidine | 4.30E-07 | 1208 | (Leurs, et al., Br J Pharmacol, 1994, 112: 847) |
| MAO-A (antagonist radioligand) | -5 | clorgyline | 7.30E-10 | 443 | (Cesura, et al., Mol Pharmacol, 1990, 37: 358) |
| M1 (h) (antagonist radioligand) | 6 | pirenzepine | 2.90E-08 | 91 | (Dorje, et al., J Pharmacol Exp Ther, 1991, 256: 727) |
| M2 (h) (antagonist radioligand) | -4 | Methoctramine | 4.80E-08 | 93 | (Dorje, et al., J Pharmacol Exp Ther, 1991, 256: 727) |
| M3 (h) (antagonist radioligand) | 10 | 4-DAMP | 8.00E-10 | 95 | (Peralta, et al., Embo J, 1987, 6: 3923) |
| N neuronal alpha 4beta 2 (h) (agonist radioligand) | -8 | nicotine | 1.20E-09 | 3029 | (Gopalakrishnan, et al., J Pharmacol Exp Ther, 1996, 276: 289) |
| delta (DOP) (h) (agonist radioligand) | 0 | DPDPE | 1.20E-09 | 114 | (Simonin, et al., Mol Pharmacol, 1994, 46: 1015) |
| kappa (h) (KOP) (agonist radioligand) | 7 | U50488 | 4.50E-10 | 4461 | (Simonin, et al., Proc Natl Acad Sci USA, 1995, 92: 7006) |
| mu (MOP) (h) (agonist radioligand) | 2 | DAMGO | 3.70E-10 | 118 | (Wang, et al., FEBS Lett, 1994, 338: 217) |
| 5-HT1A (h) (agonist radioligand) | -3 | 8-OH-DPAT | 2.20E-10 | 131 | (Mulheron, et al., J Biol Chem, 1994, 269: 12954) |
| 5-HT1 B(h) (antagonist radioligand) | -11 | Serotonine | 6.60E-08 | 4376 | (Maier, et al., J Pharmacol Exp Ther, 2009, 330: 342) |
| 5-HT2A (h) (agonist radioligand) | -2 | (±)DOI | 2.10E-10 | 471 | (Bryant, et al., Life Sci, 1996, 59: 1259) |
| 5-HT2B (h) (agonist radioligand) | 2 | (±)DOI | 4.20E-09 | 1333 | (Choi, et al., FEBS Lett, 1994, 352: 393) |
| 5-HT3 (h) (antagonist radioligand) | 2 | MDL 72222 | 6.50E-09 | 411 | (Hope, et al., Br J Pharmacol, 1996, 118: 1237) |
| GR (h) (agonist radioligand) | -2 | Dexamethasone | 1.90E-09 | 469 | (Clark, et al., Invest Ophthalmol Vis Sci, 1996, 37: 805) |
| AR (h) (agonist radioligand) | 3 | Testosterone | 2.00E-09 | 933 | (Zava, et al., Endocrinology, 1979, 104: 1007) |
| V1a (h) (agonist radioligand) | 16 | [d(CH2)51, Tyr(Me)2]-AVP | 1.10E-09 | 159 | (Tahara, et al., Br J Pharmacol, 1998, 125: 1463) |
| Ca2+ channel (L, dihydropyridine site) (antagonist radioligand) | 42 | nitrendipine | 1.40E-10 | 161 | (Gould, et al., Proc Natl Acad Sci USA, 1982, 79: 3656) |
| Potassium Channel hERG (human)- [3H] Dofetilide | 2 | Terfenadine | 4.40E-08 | 4094 | (Huang, et al., Assay Drug Dev Technol, 2010, 8: 727) |
| KV channel (antagonist radioligand) | -5 | alpha - dendrotoxin | 9.70E-11 | 166 | (Sorensen, et al., Mol Pharmacol, 1989, 36: 689) |
| Na+ channel (site 2) (antagonist radioligand) | -7 | veratridine | 1.20E-05 | 169 | (Brown, J Neurosci, 1986, 6: 2064) |
| norepinephrine transporter (h) (antagonist radioligand) | -8 | protriptyline | 2.30E-09 | 355 | (Pacholczyk, et al., Nature, 1991, 350: 350) |
| dopamine transporter (h) (antagonist radioligand) | 12 | BTCP | 6.80E-09 | 52 | (Pristupa, et al., Mol Pharmacol, 1994, 45: 125) |
| 5-HT transporter (h) (antagonist radioligand) | -3 | imipramine | 1.40E-09 | 439 | (Tatsumi, et al., Eur J Pharmacol, 1999, 368: 277) |
| COX1(h) | 10 | Diclofenac | 1.30E-08 | 4173 | (Vanachayangkul, et al., Enzyme Res, 2012, 2012: 416062) |
| COX2(h) | -14 | NS398 | 5.40E-08 | 4186 | (Vanachayangkul, et al., Enzyme Res, 2012, 2012: 416062) |
| PDE3A (h) | -3 | milrinone | 1.00E-06 | 4072 | (Maurice, et al., Nat Rev Drug Discov, 2014, 13: 290) |
| PDE4D2 (h) | -5 | Ro 20-1724 | 2.30E-07 | 4077 | (Maurice, et al., Nat Rev Drug Discov, 2014, 13: 290) |
| Lck kinase (h) | 10 | Stauro-sporine | 2.30E-08 | 2906 | (Park, et al., Anal Biochem, 1999, 269: 94) |
| acetylcholinesterase (h) | -6 | Galanthamine | 7.00E-07 | 363 | (Ellman, et al., Biochem Pharmacol, 1961, 7: 88) |

Additionally, a specificity screen for proteases was performed by BPS Biosciences to further determine the specificity of the compounds of the invention (Turk, Nat Rev Drug Discov, 2006, 5: 785; Overall, et al., Nat Rev Cancer, 2006, 6: 227; Anderson, et al., Handb Exp Pharmacol, 2009, 189: 85).

3BP-3407 was tested at 1 µM and 10 µM in duplicates. In the absence of the compound, the fluorescent intensity (Ft) in each data set was defined as 100% activity. In the absence of the enzyme, the background fluorescent intensity (Fb) in each data set was defined as 0% activity. The percent activity in the presence of each compound was calculated according to the following equation: %activity = (F-Fb)/(Ft-Fb), where F= the fluorescent intensity in the presence of the compound. Percentage inhibition was calculated according to the following formula: %inhibition = 100% - %activity. Results showing an inhibition higher than 50% are considered to represent significant effects of the tested compound. The results of this assay are given in the following Table 11.

**Table 11: Results of the specificity protease screening for 1 µM and 10 µM 3BP-3407**

| **Enzyme** | **Percentage inhibition (%)** | | |
|---|---|---|---|
| | **3BP-34 07** | | **Reference** |
| | **1µM** | **10µM** | |
| Activated Protein C | 5 | 8 | 74 |
| | | | (20µM Dabigatran) |
| Beta secretase | -8 | -5 | 84 |
| | | | (150nM Verubecestat) |
| Caspase-3 | 1 | -2 | 89 |
| | | | (100nM Caspase 3/7 Inhibitor I) |
| Caspase-6 | 1 | -1 | 94 |
| | | | (1µM Caspase 8 Inhibitor I) |
| Caspase-7 | -3 | -3 | 92 |
| | | | (1µM Caspase 3/7 Inhibitor I) |
| Caspase-8 | 0 | 0 | 87 |
| | | | (100nM Caspase 8 Inhibitor I) |
| Caspase-9 | 5 | 8 | N/A |
| Cathepsin B | 26 | 36 | 97 |
| | | | (100nM E-64) |
| Cathepsin F | -3 | -24 | 74 |
| | | | (1µM Cystatin C) |
| Cathepsin L | 3 | 6 | 97 |
| | | | (1µM E-64) |
| Cathepsin S | 3 | 18 | 91 |
| | | | (100nM E-64) |
| Cathepsin V | 1 | -18 | 83 |
| | | | (100nM E-64) |
| A20 | 2 | -4 | 99 |
| | | | (1µM Ub-Aldehyde) |
| Ataxin3 | 1 | 10 | 77 |
| | | | (10µM Ub-Aldehyde) |
| Deubiquitinase OTUD6B | 2 | 15 | 97 |
| | | | (1µM Ub-Aldehyde) |
| Ubiquitin carboxy-terminal hydrolase L1 | -2 | 4 | 92 |
| | | | (100nM Ub-Aldehyde) |
| Ubiquitin carboxy-terminal hydrolase L3 | -1 | 14 | 95 |
| | | | (10nM Ub-Aldehyde) |
| Ubiquitin carboxyl-terminal hydrolase 2 | 3 | 7 | 91 |
| | | | (1µM Ub-Aldehyde) |
| Ubiquitin carboxyl-terminal hydrolase 5 | 3 | 46 | 84 |
| | | | (1µM Ub-Aldehyde) |
| Ubiquitin carboxyl-terminal hydrolase 7 | 5 | 5 | 95 |
| | | | (1 µM Ub-Aldehyde) |
| Ubiquitin carboxyl-terminal hydrolase 8 | -3 | 6 | 73 |
| | | | (1µM Ub-Aldehyde) |
| Ubiquitin carboxyl-terminal hydrolase 10 | -2 | 5 | 82 |
| | | | (1µM Ub-Aldehyde) |
| Ubiquitin carboxyl-terminal hydrolase 14 | -1 | 5 | 96 |
| | | | (100nM Ub-Aldehyde) |
| DPP7 | 2 | -3 | 83 |
| | | | (200µM KR62436) |
| DPP8 | 1 | 5 | 96 |
| | | | (200µM KR62436) |
| DPP9 | -1 | 0 | 99 |
| | | | (200µM KR62436) |
| FAP | 98 | 99 | 100 |
| | | | (100nM SP-13786) |
| serine protease NS3 (a.a. 3-181) from Hepatitis C virus genotype 1a (mutant D168V) | 1 | -68 | 94 |
| | | | (100nM Denoprevir) |
| serine protease NS3 (a.a. 3-181) from Hepatitis C virus genotype 1b | 1 | 5 | 100 |
| | | | (100nM Denoprevir) |
| serine protease NS3 (a.a. 3-181) from Hepatitis C virus genotype 1b (mutant D168V) | 1 | -6 | 99 |
| | | | (100nM Denoprevir) |
| serine protease NS3 (a.a. 3-181) from Hepatitis C virus genotype 1b (mutant R155K) | -2 | 5 | 90 |
| | | | (100nM Denoprevir) |
| serine protease NS3 (a.a. 3-181) from Hepatitis C virus genotype 1b (mutant R155Q) | 0 | 2 | 99 |
| | | | (1µM Denoprevir) |
| serine protease NS3 (a.a. 3-181) from Hepatitis C virus genotype 2a | 0 | -2 | 98 |
| | | | (100nM Denoprevir) |
| Matrix metalloprotease1 | -1 | 2 | 87 |
| | | | (1µM NNGH) |
| Matrix metalloprotease 2 | 3 | 3 | 95 |
| | | | (100nM NNGH) |
| Matrix metalloprotease 9 (mutant Q279R) | 3 | 2 | 92 |
| | | | (100nM NNGH) |
| Renin | -1 | 3 | 99 |
| | | | (30nM Aliskiren) |

### Example 15: ¹¹¹In- and ¹⁷⁷Lu-labeling of selected compounds

In order to serve as a diagnostically, therapeutically, or theragnostically active agent, a compound needs to be labeled with a radioactive isotope. The labeling procedure needs to be appropriate to ensure a high radiochemical yield and purity of the radiolabeled compound of the invention. This example shows that the compounds of the present invention are appropriate for radiolabeling and can be labeled in high radiochemical yield and purity.

30-100 MBq of ¹¹¹InCl₃ (in 0.02 M HCl) were mixed with 1 nmol of compound (200 µM stock solution in 0.1 M HEPES pH 7) per 30 MBq and buffer (1 M sodium acetate buffer pH 5 or 1 M sodium acetate / ascorbic acid buffer pH 5 containing 25 mg/ml methionine) at a final buffer concentration of 0.1 - 0.2 M. The mixture was heated to 80 °C for 20-30 min. After cooling down, DTPA and TWEEN-20 were added at a final concentration of 0.2 mM and 0.1%, respectively.

0.2 - 2.0 GBq ¹⁷⁷LuCl₃ (in 0.04 M HCl) were mixed with 1 nmol of compound (200 µM stock solution in 0.1 M HEPES pH 7) per 45 MBq and buffer (1 M sodium acetate / ascorbic acid buffer pH 5 containing 25 mg/ml methionine) at a final buffer concentration of ∼0.4 M. The mixture was heated to 90 °C for 20 min.

The labeling efficiency was analyzed by thin layer chromatography (TLC) and HPLC. For TLC analysis, 1-2 µl of diluted labeling solution was applied to a strip of iTLC-SG chromatography paper (Agilent, 7.6 x 2.3 mm) and developed in citrate-dextrose solution (Sigma). The iTLC strip was then cut into 3 pieces and associated radioactivity was measured with a gamma-counter. The radioactivity measured at the solvent front represents free radionuclide and colloids, whereas the radioactivity at the origin represents radiolabeled compound. For HPLC, 5 µl of diluted labeling solution was analyzed with a Poroshell SB-C18 2.7 µm (Agilent). Eluent A: MeCN, eluent B: H₂O, 0.1 % TFA, gradient from 5% B to 70% B within 15 min, flow rate 0.5 ml/min; detector: NaI (Raytest), DAD 230 nm. The peak eluting with the dead volume represents free radionuclide, the peak eluting with the peptide-specific retention time as determined with an unlabeled sample represents radiolabeled compound. Radionuclidic incorporation yield was ≥ 95% and radiochemical purity ≥ 90%.

### Example 16: Imaging and biodistribution studies

Radioactively labeled compounds can be detected by imaging methods such as SPECT and PET. Furthermore, the data acquired by such techniques can be confirmed by direct measurement of radioactivity contained in the individual organs prepared from an animal injected with a radioactively labeled compound of the invention. Thus, the biodistribution of a radioactively labeled compound can be determined and analyzed. This example shows that the compounds of the present invention show a biodistribution appropriate for diagnostic imaging and therapeutic treatment of tumors.

All animal experiments were conducted in compliance with the German animal protection laws. Male SCID beige mice (6- to 8-week-old, Charles River, Sulzfeld, Germany) were inoculated with 5 × 10⁶ HEK-FAP cells in one shoulder. When tumors reached a size of > 150 mm³ mice received ∼30 MBq ¹¹¹In-labelled compounds of the invention (diluted to 100 µL with PBS) administered intravenously via the tail vein. Images were obtained on a NanoSPECT/CT system (Mediso Medical Imaging Systems, Budapest, Hungary) using exemplarily the following acquisition and reconstruction parameters (Table 12).

**Table 12: Acquisition and reconstruction parameters of NanoSPECT/CT imaging**

| **Acquistion parameters SPECT** | |
|---|---|
| System | NanoSPECT/CT ™ |
| Scan range | whole body, 3-bed holder (mouse hotel) |
| Time per projection | 60s |
| Aperture model, pinhole diameter | Aperture #2, 1,5 mm |

| **Reconstruction parameters** | |
|---|---|
| Method | HiSPECT (Scivis), iterative reconstruction |
| Smoothin | 35% |
| Iterations | 9 |
| Voxel size | 0.15 mm x 0.15 mm x 0.15 mm |

| **Acquisition parameters CT** | |
|---|---|
| System | NanoSPECT/CT ™ |
| Scan range | whole body, 3-bed holder (mouse hotel) |
| Scan duration | 7 minutes |
| Tube voltage | 45 kVp |
| Exposure time | 500 ms |
| Number of projections | 240 |

Imaging data were saved as DICOM files and analysed using VivoQuant™ software (Invicro, Boston, USA). Results are expressed as a percentage of injected dose per gram of tissue (%ID/g). For biodistribution studies, animals were sacrificed by cervical dislocation at 24h or 48h post injection and then dissected. Different organs and tissues were collected and weighed, and the radioactivity was determined by γ-counting. Two animals were used per time point. Results are expressed as a percentage of injected dose per gram of tissue (%ID/g).

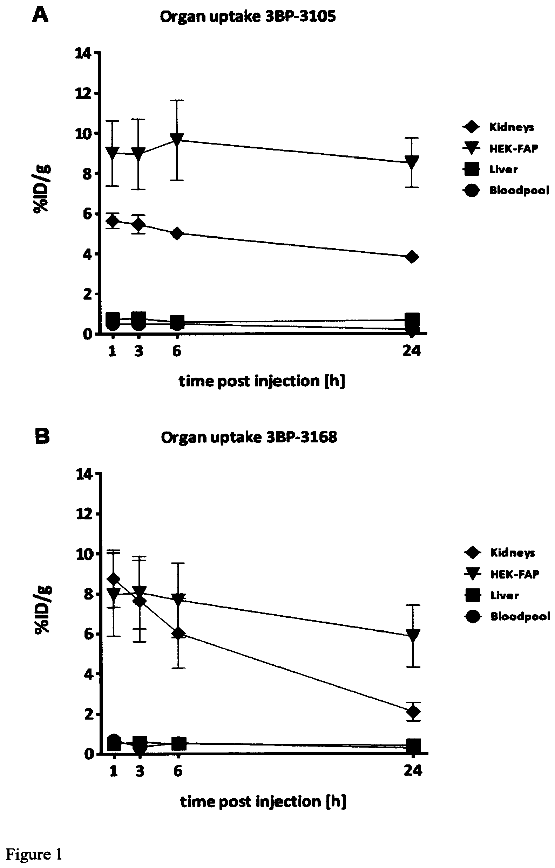

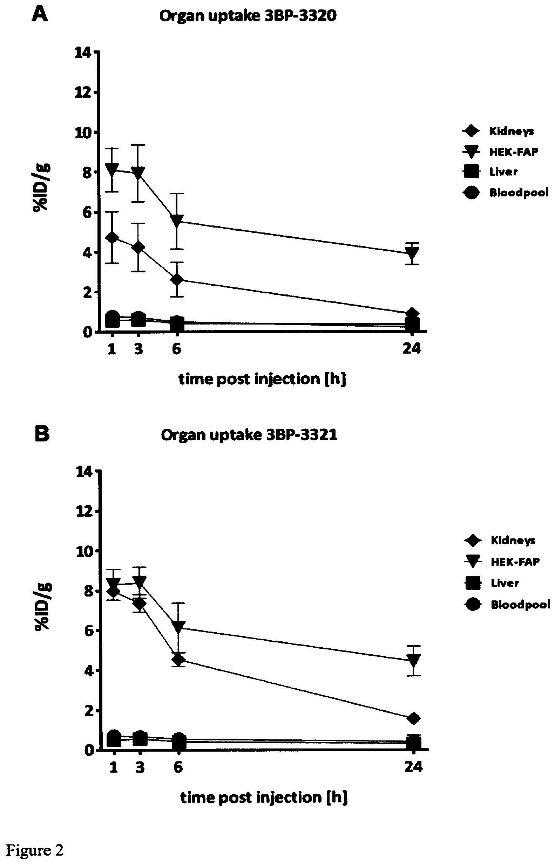

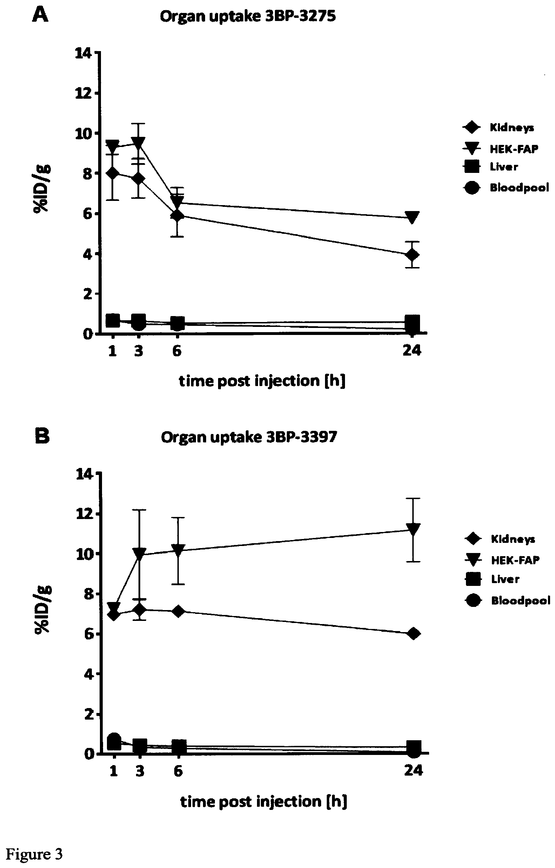

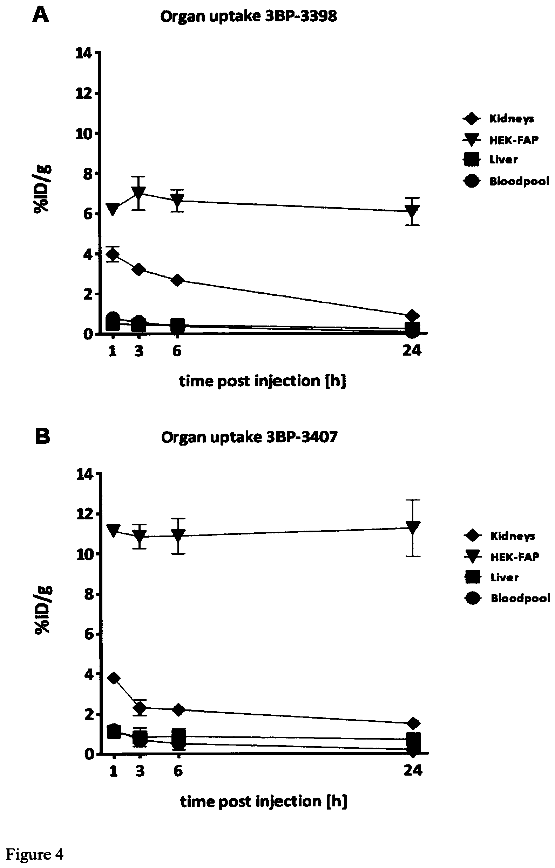

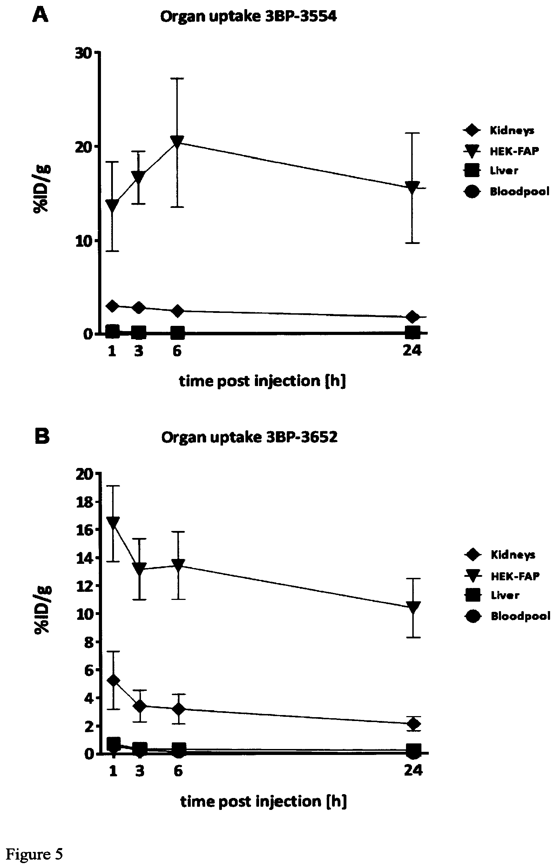

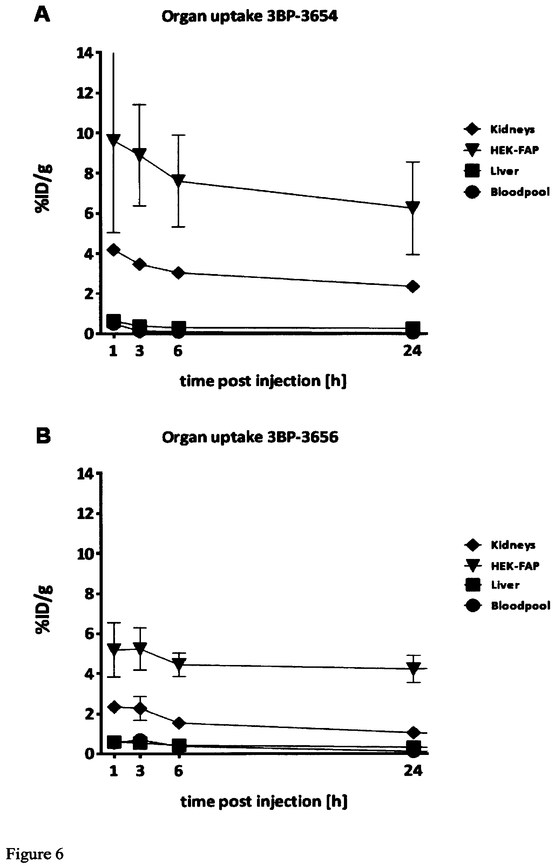

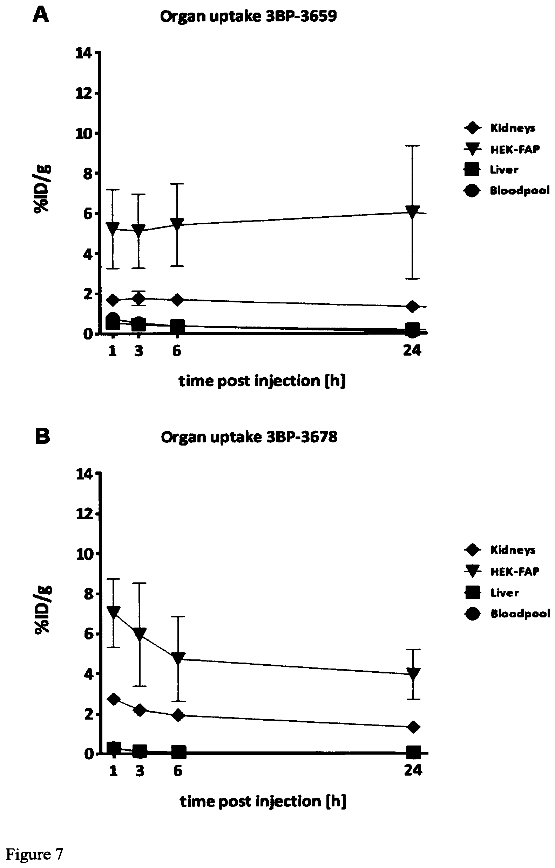

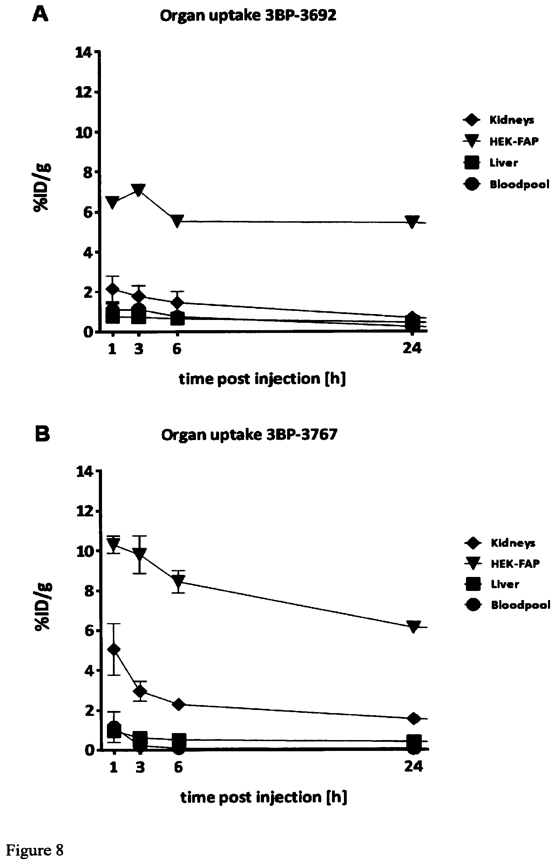

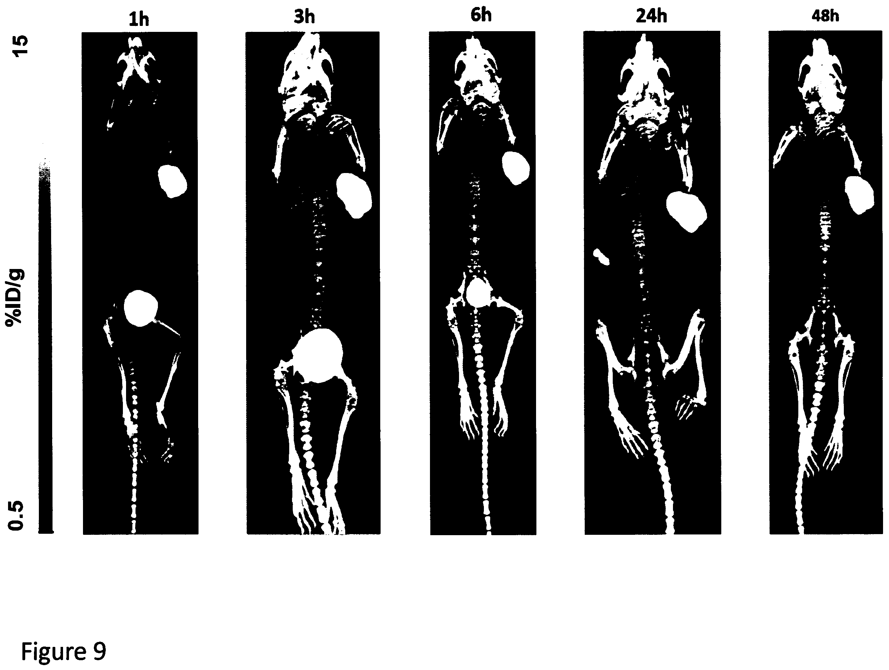

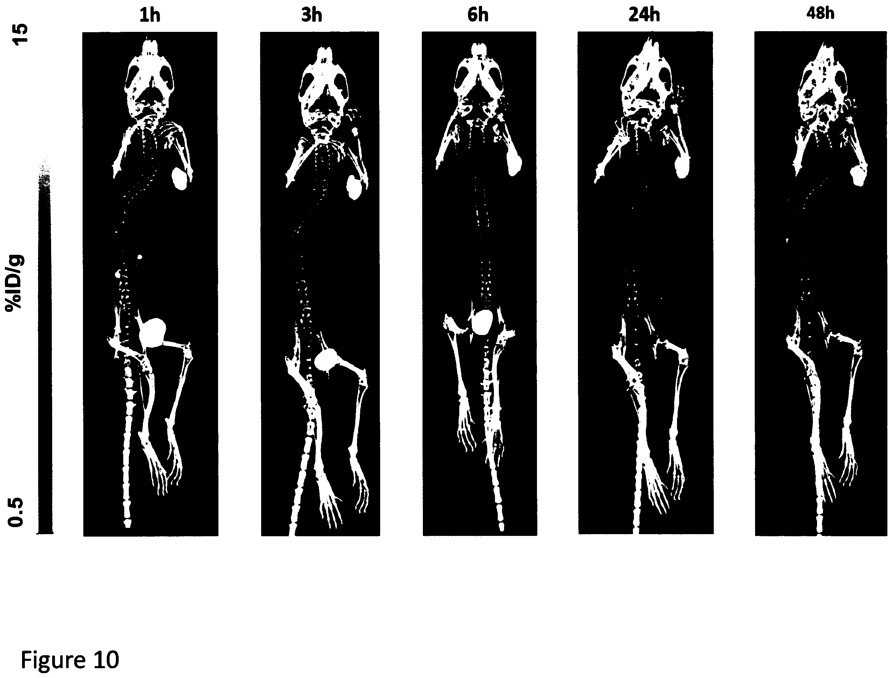

The results of the imaging and biodistribution studies for selected compounds are shown in Figs. 1-10.

The features of the present invention disclosed in the specification, the claims, the sequence listing and/or the drawings may both separately and in any combination thereof be material for realizing the invention in various forms thereof.

### References

The disclosure of each and any document recited herein is incorporated by reference.

## Claims

1. A compound comprising a cyclic peptide
of formula (I) 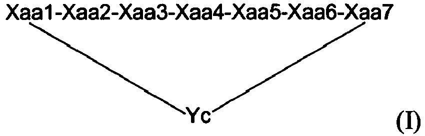 and an N-terminal modification group A attached to Xaa1,
wherein
the peptide sequence is drawn from left to right in N to C-terminal direction,
Xaa1 is a residue of an amino acid of formula (II) 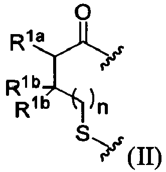 wherein
R^{1a} is -NH-
R^{1b} is H or CH₃,
n = 0 or 1,
the N-terminal modification group A is covalently attached to the nitrogen atom of Xaa1,
the carbonyl group of Xaa1 is covalently attached to the nitrogen of Xaa2,
and the sulfur atom of Xaa1 is covalently attached as thioether to Yc;
Xaa2 is a residue of an amino acid of formula (III), (IV) or (XX) 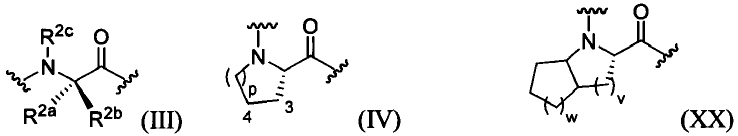 wherein
R^{2a}, R^{2b}, R^{2c} are each and independently selected from the group consisting of (C₁-C₂)alkyl and H, wherein said (C₁-C₂)alkyl maybe substituted by a substituent selected from the group consisting of OH, NH₂, halogen, (C₅-C₇)cycloalkyl,
p = 0, 1 or 2
v = 1 or 2
w = 1, 2 or3 and
the amino acid of formula (IV) maybe substituted by one or two substituents selected from the group consisting of methyl, OH, NH₂ and F at indicated ring positions 3 and 4;
Xaa3 is a residue of an amino acid of formula (V) or (XX) 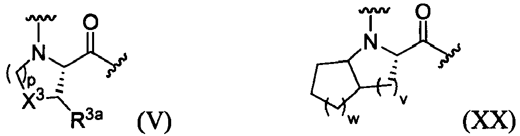 wherein
X³ is selected from the group consisting of CH₂, CF₂, CH-R^{3b}, S, O and NH,
p= 1 or 2
v = 1 or 2
w = 1, 2 or 3,
R^{3a} is H, methyl, OH, NH₂ or F,
R^{3b} is methyl, OH, NH₂ or F;
Xaa4 is a residue of an amino acid of formula (VI) 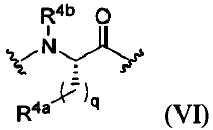 wherein
R^{4a} is selected from the group consisting of H, OH, COOH, CONH₂, X⁴ and-NH-CO-X⁴, wherein X⁴ is selected from the group consisting of (C₁-C₆)alkyl, (C₅-C₆)aryl and (C₅-C₆)heteroaryl, and X⁴ may be substituted by one or two substituents selected from the group consisting of methyl, CONH₂, halogen, NH₂ and OH;
q = 1, 2 or 3, wherein optionally one or two hydrogens of said one, two or three CH₂-groups are each and individually substituted by methyl, ethyl, (C₅-C₆)aryl or (C₅-C₆)heteroaryl,
R^{4b} is methyl or H;
Xaa5 is a residue of an amino acid of structure (VII) 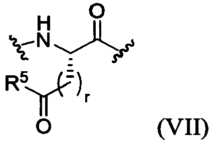 wherein
R⁵ is selected from the group of OH and NH₂, and
r = 1, 2 or 3;
Xaa6 is an amino acid selected from the group consisting of an aromatic L-α-amino acid and a heteroaromatic L-α-amino acid;
Xaa7 is a residue of an amino thiol or an amino acid of formula (IX), 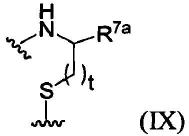 wherein
R^{7a} is -CO-, -COOH, -CONH₂, -CH₂-OH, -(CO)-NH-R^{7b}, -(CO)-(NR^{7C})-R^{7b} or H, wherein
R^{7b} and R^{7c} are each and independently (C₁-C₄)alkyl and
t is 1 or 2;
Yc is a structure of formula (X) 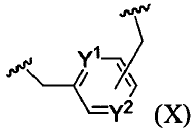 linking the S atom of Xaa1 and the S atom of Xaa7 under the formation of two thioether linkages thus forming a cyclic structure of formula (XXI) 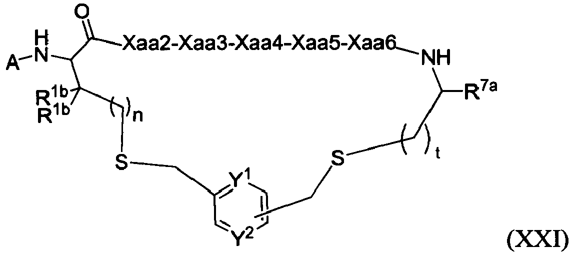 wherein
the substitution pattern of the aromatic group in formula (X) is *ortho, meta* or *para,*
n = 0 or 1,
t = 1 or 2,
Y¹ is C-H or N,
Y² is N or C-R^{c1},
R^{c1} is H or CH₂-R^{c2} and
R^{c2} is a structure of formula (XI), (XII) or (XXII) 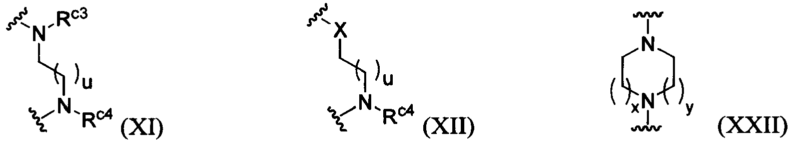 wherein
R^{c3} and R^{c4} are each and independently selected from the group consisting of H and (C₁-C₄)alkyl and
u = 1, 2, 3, 4, 5 or 6,
x and y are each and independently 1, 2 or 3, and
X = O or S
wherein in formulae (XI) and (XXII) one of the nitrogen atoms is attached to -CH₂- of R^{c1} and in formula (XII) -X- is attached to -CH₂- of R^{c1}; and
wherein the N-terminal modification group A is either a blocking group Abl or an amino acid Aaa.

2. The compound of claim 1, where the blocking group Abl is selected from the group consisting of R^{a1}-C(O)-, R^{a1}-S(O₂)-, R^{a1}-NH-C(O)- and R^{a1}-O-C(O)-; wherein R^{a1} is (C₁-C₈)alkyl optionally substituted by up to two substituents each and independently selected from the group consisting of OH, F, COOH, (C₃-C₈)cycloalkyl, aryl, heteroaryl and (C₃-C₈)heterocycle, and wherein in (C₁-C₈)alkyl one of the -CH₂-groups is optionally replaced by -S- or -O- .

3. The compound of claim 2, wherein the blocking group Abl is hexanoyl or pentyl sulfonyl, preferably blocking group Abl is hexanoyl.

4. The compound of claim 1, wherein the amino acid Aaa is a D-amino acid residue or an L- amino acid residue each of structure (XIV): 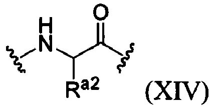 wherein
R^{a2} is selected from the group consisting of (C₁-C₆)alkyl, modified (C₁-C₆)alkyl,
(C₁-C₃)alkyl, modified (C₁-C₃), (C₃-C₈)carbocycle, aryl, heteroaryl and (C₃-C₈)heterocycle, wherein in modified (C₁-C₆)alkyl one -CH₂- group is replaced by -S- or -O-, and in modified (C₁-C₃)alkyl one of the H is substituted by OH, F or COOH, or two of the H are substituted by F.

5. The compound of claim 4, wherein Aaa is selected from the group consisting of the amino acid residues of Nle, nle, Met and met, and their derivatives.

6. The compound of any one of claim 1 to 5, wherein Xaa1 is a D-amino acid residue selected from the group consisting of cys, hcy and pen, or Xaa1 is an L-amino acid residue selected from the group consisting of Cys, Hcy and Pen.

7. The compound of any one of claims 1 to 6, wherein Xaa2 is an amino acid residue selected from the group consisting of Pro, Gly, Nmg and their derivatives, wherein Xaa3 is an amino acid residue selected from the group consisting of Pro, Hyp, Tfp, Cfp, Dmp, Aze and Pip, and their derivatives, wherein Xaa4 is an amino acid residue selected from the group consisting of Thr, Hse, Asn, Gln and Ser, and their derivatives, wherein Xaa5 is an amino acid residue selected from the group consisting of Gln and Glu, and their derivatives, wherein Xaa6 is an amino acid residue of any one of formulae (VIIIa), (VIIIb), (VIIIc) and (VIIId): 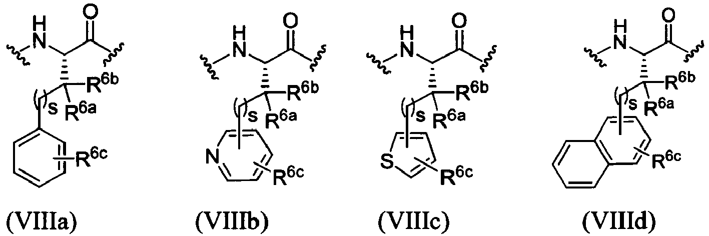 wherein
R^{6a} and R^{6b} are each and independently selected from the group consisting of H, methyl, ethyl, propyl and isopropyl,
R^{6c} represents from 0 to 3 substituents, each such substituent being each and independently selected from the group consisting of Cl, F, Br, NO₂, NH₂, CN, CF₃, OH, OR^{6d} and C₁-C₄ alkyl,
R^{6d} is selected from the group consisting of methyl, ethyl, propyl, and isopropyl, and
s is 0 or 1,
preferably Xaa6 is an amino acid residue of any one of formulae (VIIIa), (VIIIb), (VIIIc) and (VIIId): 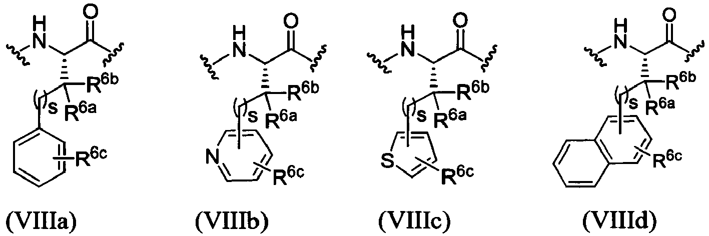 wherein
R^{6a} and R^{6b} are each H
R^{6c} represents from 0 to 2 substituents, each such substituent being each and independently selected from the group consisting of Cl, F, Br, NO₂, NH₂, CN, CF₃, OH, OR^{6d} and methyl,
R^{6d} is selected from the group consisting of methyl, ethyl, propyl, and isopropyl, and
s is 0, and/or
wherein Xaa7 is an amino thiol residue selected from the group consisting of Cys, Cysol, AET, Hcy, cys and hcy.

8. The compound of any one of claims 1 to 7, wherein the compound is a compound of formula (LI), (LII), (LIII) or (LIV):

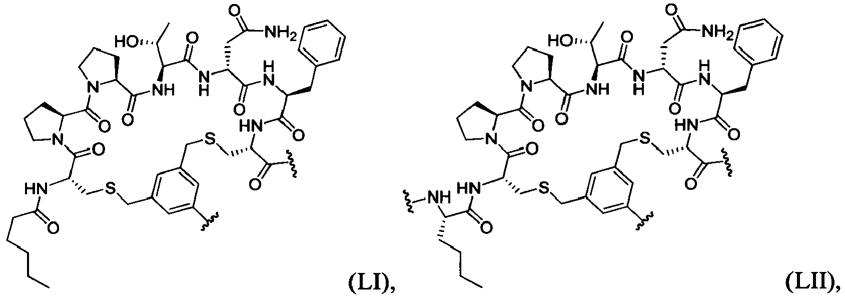

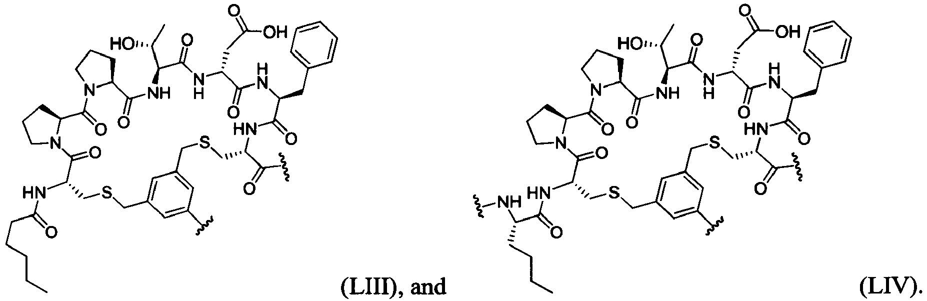

9. The compound of any one of claims 1 to 8, wherein Yc is a structure of formula (XIII): 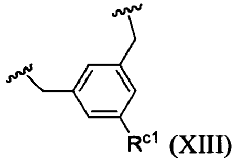 preferably Yc comprises a NH group, preferably a reactive NH group, wherein the NH group allows conjugation of a moiety to Yc, preferably, the NH group is provided by the structure R^{c2}, wherein R^{c2} is selected from the group consisting of a structure of any one of formulae (XXIb), (XIc) and (XIIb): 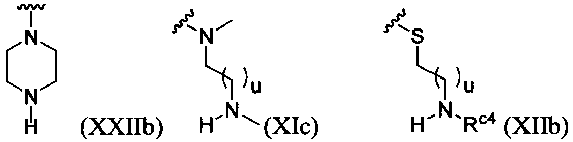
wherein R^{c4} is H or methyl and
u = 1, 2, 3, 4 or 5.

10. The compound of claim 9, wherein the compound comprises a Z group, wherein the Z group is covalently attached to Yc, preferably to the structure of formula (X), wherein the Z group comprises a chelator and optionally a linker, preferably the Z group is covalently attached to R^{c2}, forming a structure of any one of formulae (XXIIc), (XId) and (XIId): 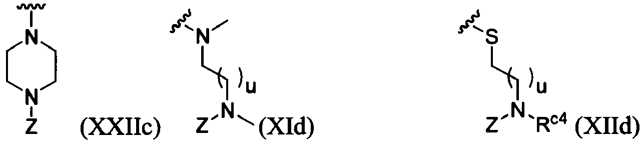
wherein R^{c4} is H or methyl and
u = 1, 2, 3, 4 or 5.

11. The compound of any one of claims 1 to 10, wherein the N-terminal modification group A is the amino acid Aaa and wherein the compound comprises a Z group covalently attached to the amino acid Aaa, wherein the Z group comprises a chelator and optionally a linker, wherein, if the linker is present, the linker covalently links the chelator to the amino acid Aaa, preferably to the α-nitrogen of the amino acid Aaa, preferably the covalent linkage between the linker and the α-nitrogen of the amino acid Aaa is an amide.

12. The compound of claim 11, wherein the linker is selected from the group comprising Ttds, 020c, Apac, Gly, Bal, Gab, Mamb, Pamb, Ppac, 4Amc, Inp, Sni, Rni, Nmg, Cmp, PEG6, PEG12 and other PEG-amino acids, and most preferably Ttds, 020c, Apac, 4Amc, PEG6 and PEG12, preferably the linker amino acid is selected from the group consisting of Ttds, 020c and PEG6.

13. The compound of any one of claims 1 to 12, wherein an amino acid or a peptide is attached to Xaa7, wherein a majority of the amino acids of this peptide are charged or polar and the net charge of the peptide is -2, -1, 0, +1 or +2, preferably the peptide is selected from the group consisting of peptides of formula (XXXa-f)
Xaa10-Xaa11-Xaa12-Xaa13-Xaa14-Xaa15-Xaa16 (XXXa)
Xaa10-Xaa11-Xaa12-Xaa13-Xaa14-Xaa15 (XXXb)
Xaa10-Xaa11-Xaa12-Xaa13-Xaa14 (XXXc)
Xaa1 O-Xaa11-Xaa12-Xaa13 (XXXd)
Xaa10-Xaa11-Xaa12 (XXXe)
Xaa10-Xaa11 (XXXf)
wherein
Xaa10 is Asp, asp, Bal, Gly, Gab, Ser, Nmg, Bhf. Lys, Ttds or Bhk
Xaa11 is His, his, Lys, Ttds, Arg, Ape or Ala,
Xaa12 is Phe, Nmf, Tic, Aic, Ppa, Mpa, Amf, Nmf, phe, Lys, Ape, Ttds and Ppa
Xaa13 is Arg, Lys, Ape, Ttds or arg,
Xaa14 is Asp, Ala, asp, Lys, Ape or Ttds,
Xaa15 is Ttds, Ape or Lys, and
Xaa16 is Lys or Ape,
wherein, optionally,
Xaa11 and Xaa12 together form a single amino acid selected from the group consisting of Gab, Pamb, Cmp, Pamb, Mamb, and, optionally,
Xaa10, Xaa11 and Xaa12 form together a single amino acid selected from the group consisting of Gab, Pamb, Cmp, Pamb, and Mamb,
under the proviso that in the peptides of formulae (XXXa-f) Ape, if present, is the C-terminal building block.

14. The compound of claim 13, wherein a Z-group is covalently attached to the peptide, wherein the Z group comprises a chelator and optionally a linker or
wherein a Z-group is covalently attached to the amino acid attached to Xaa7, wherein the Z group comprises a chelator and optionally a linker.

15. The compound of any one of claims 13 to 14, wherein the chelator is covalently linked to the amino acid attached to Xaa7 or the chelator is covalently linked to the C-terminal amino acid of the peptide, preferably the C-terminal amino acid of any one of peptide of formulae (LI), (LII), (LIII) and (LIV).

16. The compound of any one of claims 1 to 15, wherein the chelator is selected from the group consisting of DOTA, DOTAGA, NOTA, NODAGA, NODA-MPAA, HBED, TETA, CB-TE2A, DTPA, DFO, Macropa, HOPO, TRAP, THP, DATA, NOTP, sarcophagine, FSC, NETA, H4octapa, Pycup, NₓS₄₋ₓ (N4, N2S2, N3S), Hynic, ^{99m}Tc(CO)₃-Chelators, more preferably DOTA, DOTAGA, NOTA, NODAGA, NODA-MPAA, HBED, CB-TE2A, DFO, THP, N4 and most preferred DOTA, DOTAGA, NOTA and NODAGA.

17. The compound of any one of claims 1 to 16, wherein the compound is selected from the group consisting of
compound
H-Met-[Cys(3MeBn)-Pro-Pro-Thr-Glu-Phe-Cys]-Asp-His-Phe-Arg-Asp-Ttds-Lys(Bio)-NH2 (3BP-2881) of the following formula 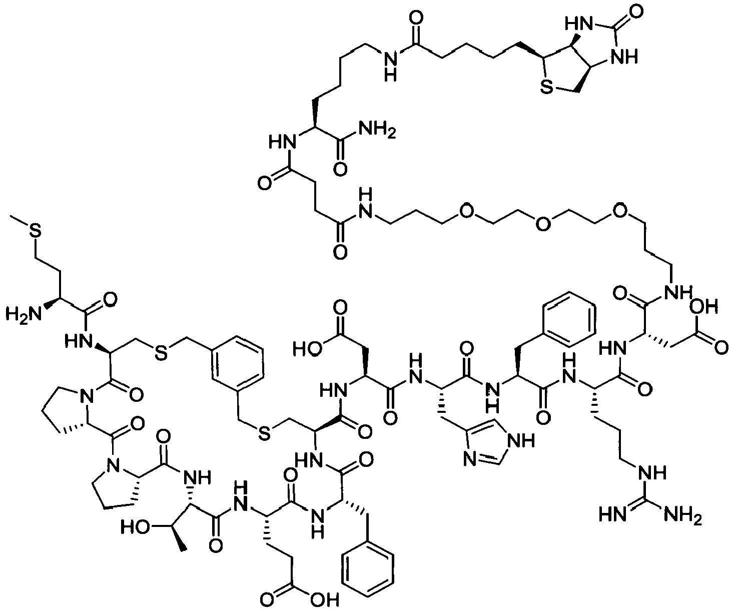
compound Hex-[Cys(3MeBn)-Pro-Pro-Thr-Glu-Phe-Cys]-Asp-His-Phe-Arg-Asp-NH2 (3BP-2974) of the following formula 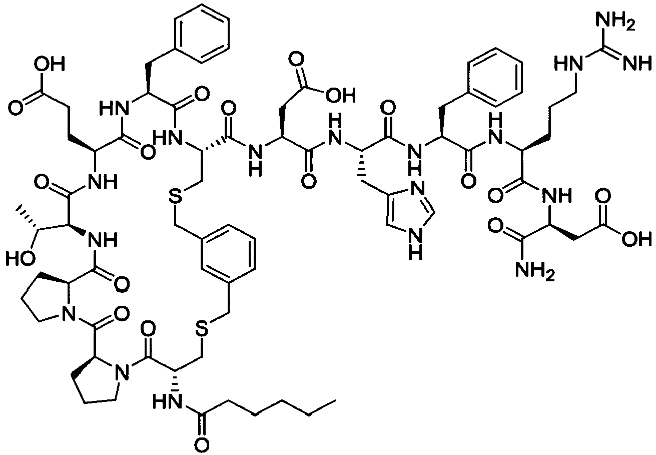
compound Ac-Met-[Cys(3MeBn)-Pro-Pro-Thr-Glu-Phe-Cys]-Asp-His-Phe-Arg-Asp-NH2 (3BP-2975) of the following formula 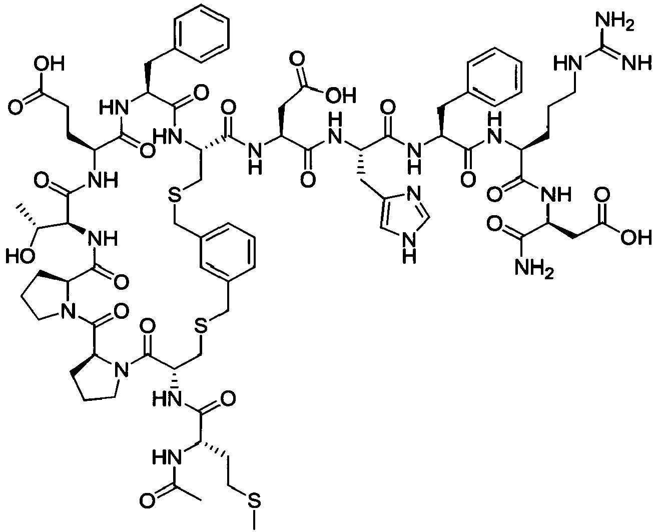
compound H-met-[Cys(3MeBn)-Pro-Pro-Thr-Glu-Phe-Cys]-Asp-His-Phe-Arg-Asp-NH2 (3BP-2976) of the following formula 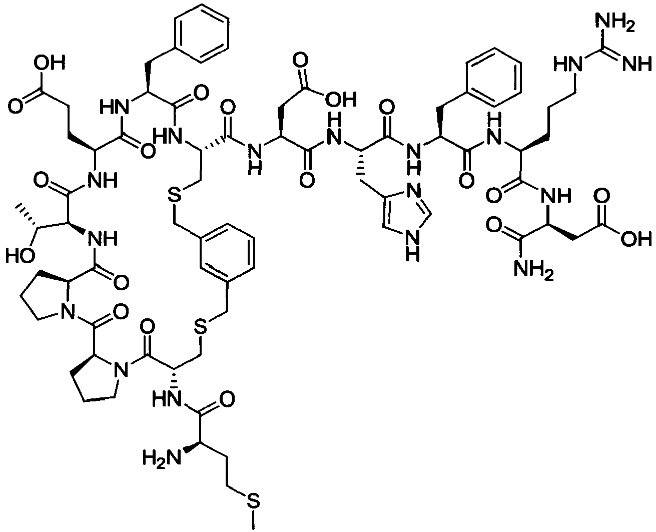
compound Hex-[Cys(3MeBn)-Pro-Pro-Thr-Glu-Phe-Cys]-Asp-His-Phe-Arg-Asp-Ttds-Lys(DOTA)-NH2 (3BP-3105) of the following formula 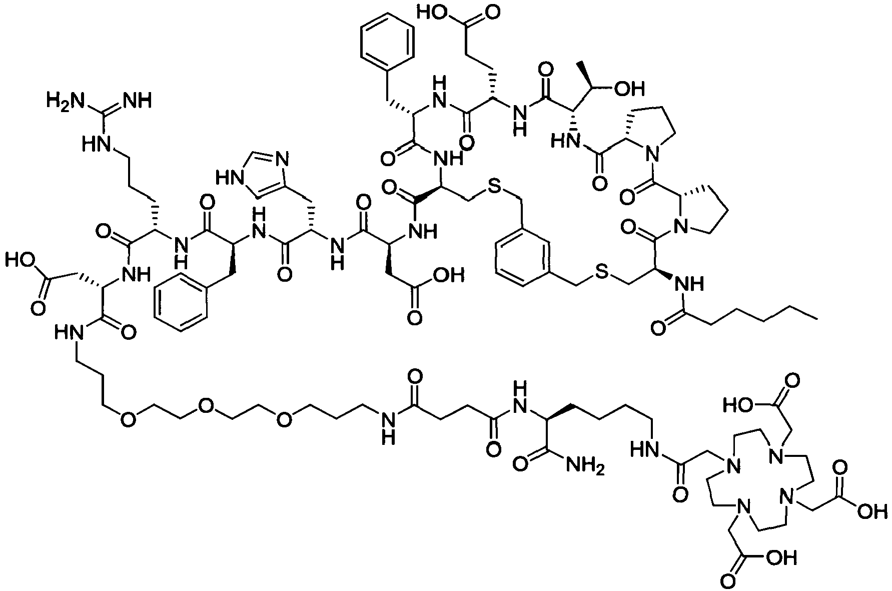
compound DOTA-Ttds-Nle-[Cys(3MeBn)-Pro-Pro-Thr-Glu-Phe-Cys]-Asp-His-Phe-Arg-Asp-NH2 (3BP-3168) of the following formula 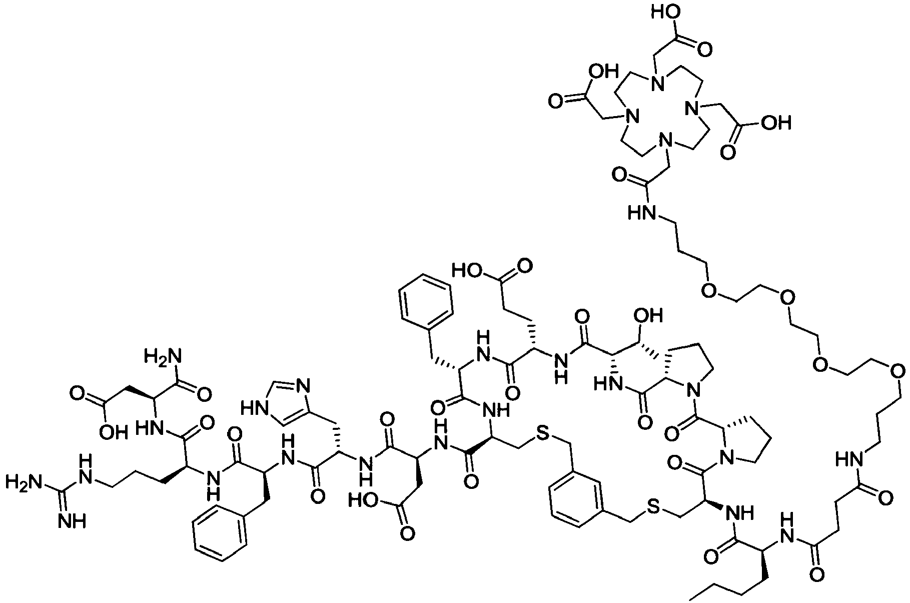
compound DOTA-Ttds-Met-[Cys(3MeBn)-Pro-Pro-Thr-Glu-Phe-Cys]-Asp-His-Phe-Arg-Asp-NH2 (3BP-3169) of the following formula 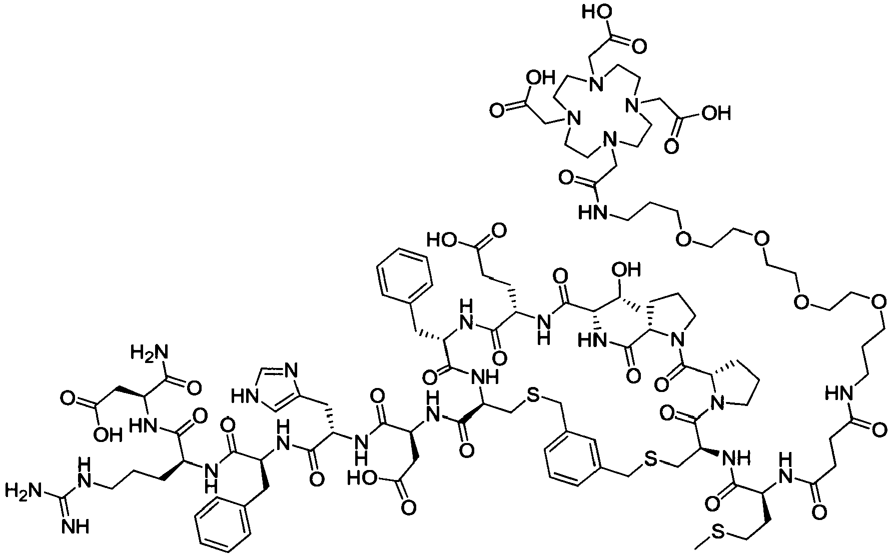
compound DOTA-Ttds-Leu-[Cys(3MeBn)-Pro-Pro-Thr-Glu-Phe-Cys]-Asp-His-Phe-Arg-Asp-NH2 (3BP-3172) of the following formula 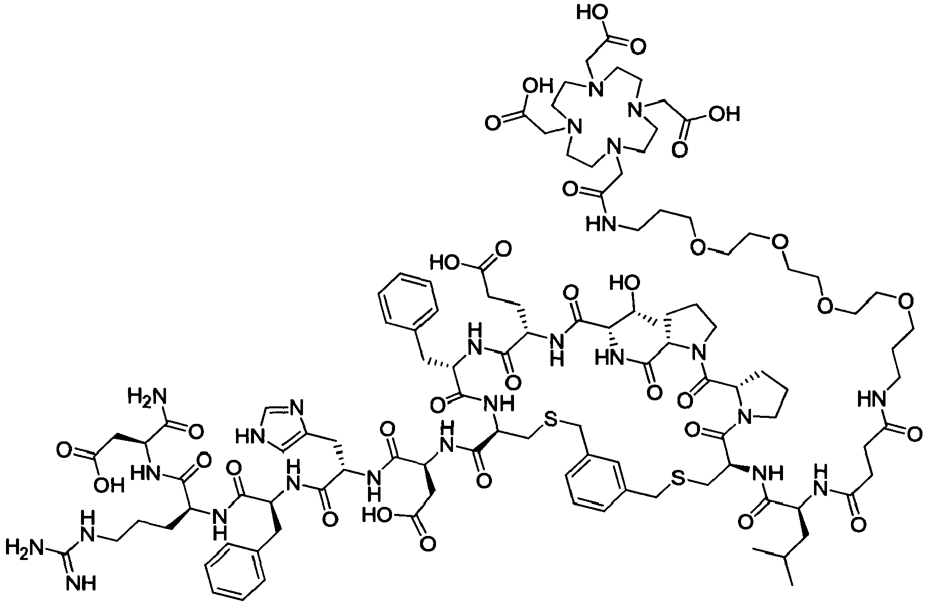
compound Ac-Met-[cys(3MeBn)-Pro-Pro-Thr-Glu-Phe-Cys]-Asp-His-Phe-Arg-Asp-NH2 (3BP-3175) of the following formula 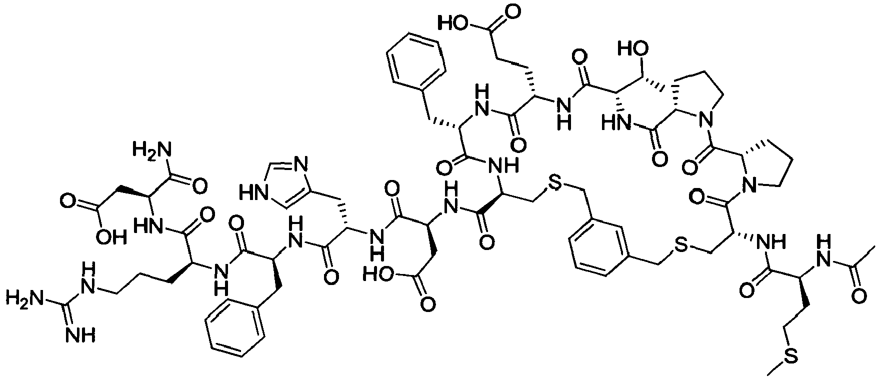
compound Ac-met-[Cys(3MeBn)-Pro-Pro-Thr-Glu-Phe-Cys]-Asp-His-Phe-Arg-Asp-NH2 (3BP-3187) of the following formula 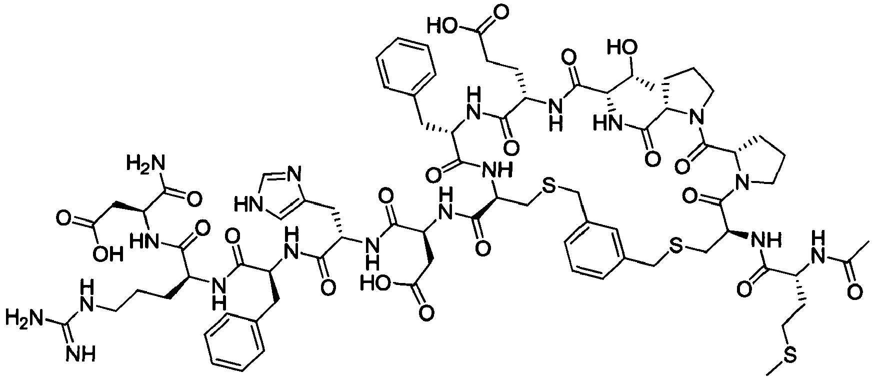
compound Ac-Met-[Cys(3MeBn)-Pro-Pro-Thr-Glu-Phe-Cys]-Asp-His-Nmf-Arg-Asp-NH2 (3BP-3188) of the following formula 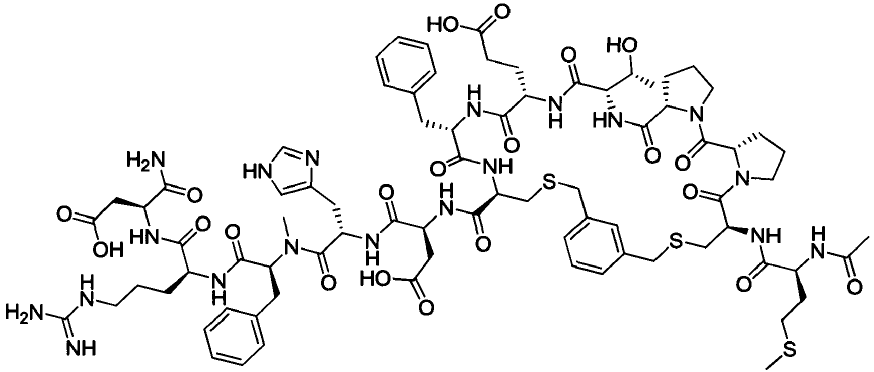
compound Ac-Met-[Cys(3MeBn)-Pro-Pro-Thr-Glu-Phe-Cys]-Asp-His-Tic-Arg-Asp-NH2 (3BP-3189) of the following formula 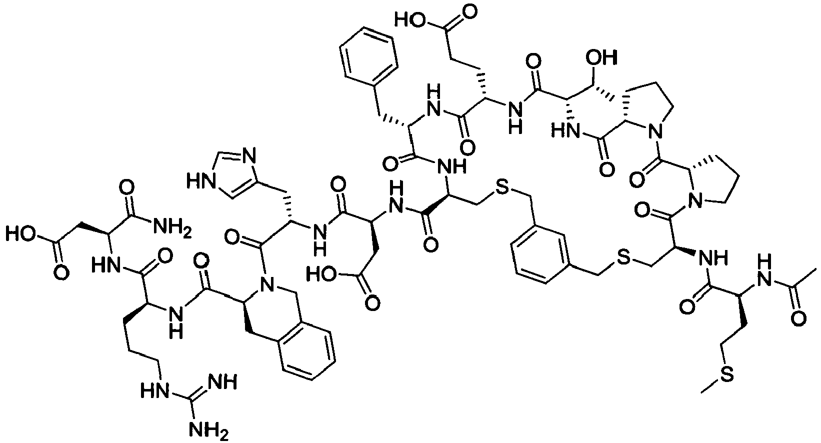
compound Ac-Met-[Cys(3MeBn)-Pro-Pro-Thr-Glu-Phe-Cys]-Asp-His-Aic-Arg-Asp-NH2 (3BP-3190) of the following formula 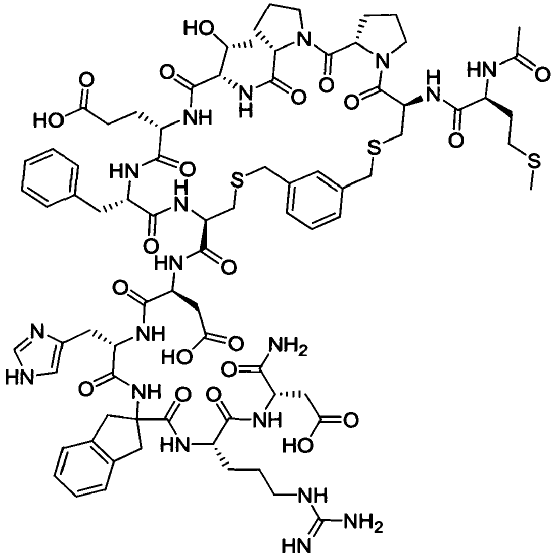
compound Ac-Met-[Cys(3MeBn)-Pro-Pro-Thr-Glu-Phe-Cys]-Asp-His-Ppa-Arg-Asp-NH2 (3BP-3191) of the following formula 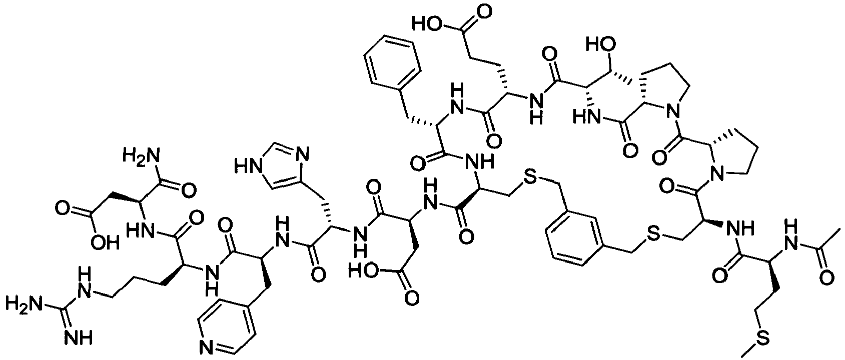
compound Ac-Met-[Cys(3MeBn)-Pro-Pro-Thr-Glu-Phe-Cys]-Asp-His-Mpa-Arg-Asp-NH2 (3BP-3192) of the following formula 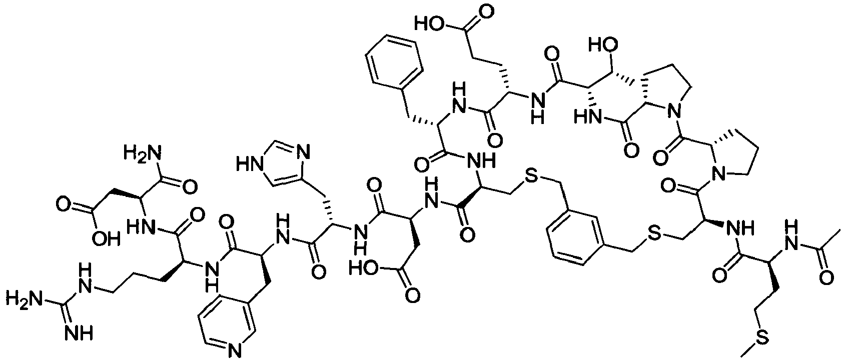
compound Ac-Met-[Cys(3MeBn)-Pro-Pro-Thr-Glu-Thi-Cys]-Asp-His-Phe-Arg-Asp-NH2 (3BP-3193) of the following formula 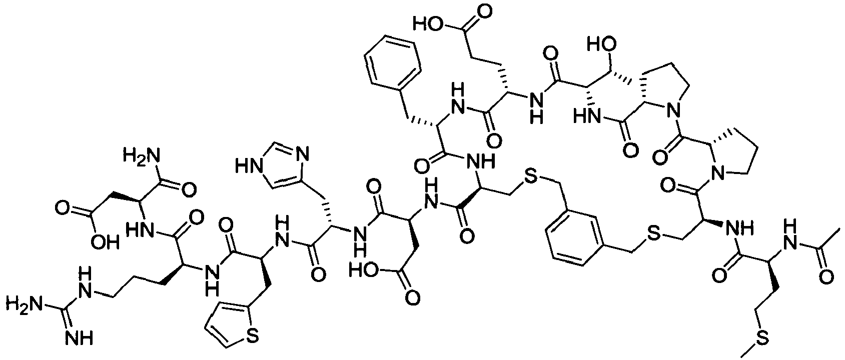
compound Ac-Met-[Cys(3MeBn)-Pro-Pro-Thr-Glu-Phe-Cys]-Asp-Ala-Phe-Arg-Asp-NH2 (3BP-3195) of the following formula 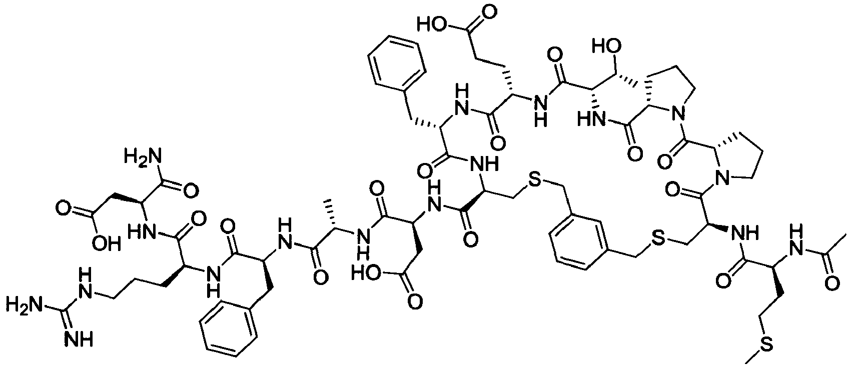
compound Ac-Met-[Cys(3MeBn)-Pro-Pro-Thr-Glu-Phe-Cys]-Asp-His-Ala-Arg-Asp-NH2 (3BP-3196) of the following formula 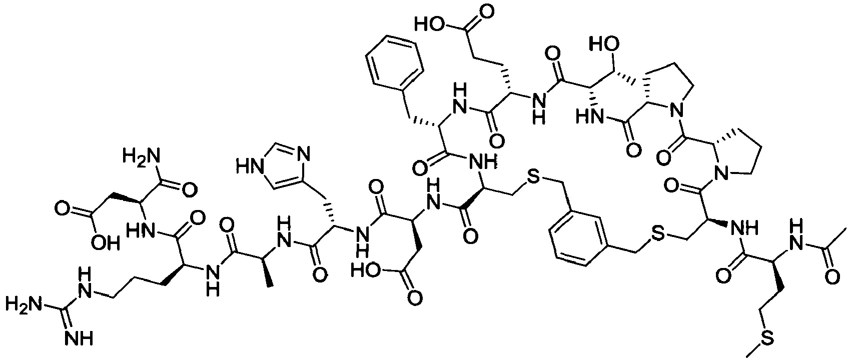
compound Ac-Met-[Cys(3MeBn)-Pro-Pro-Thr-Glu-Phe-Cys]-Asp-His-Phe-Arg-Ala-NH2 (3BP-3198) of the following formula 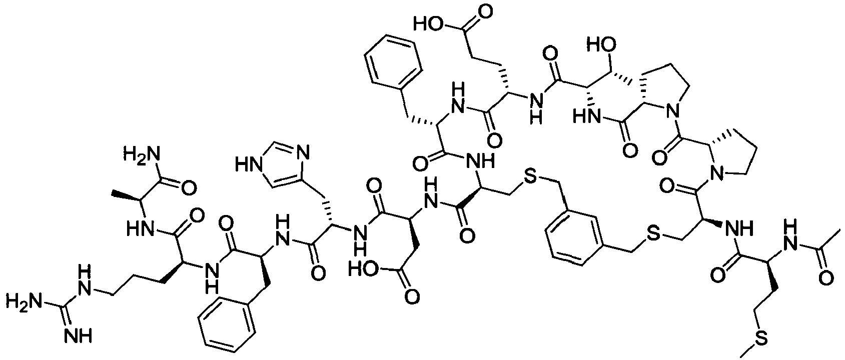
compound Ac-Met-[Cys(3MeBn)-Pro-Pro-Thr-Glu-Phe-Cys]-Asp-His-Phe-Arg-NH2 (3BP-3200) of the following formula 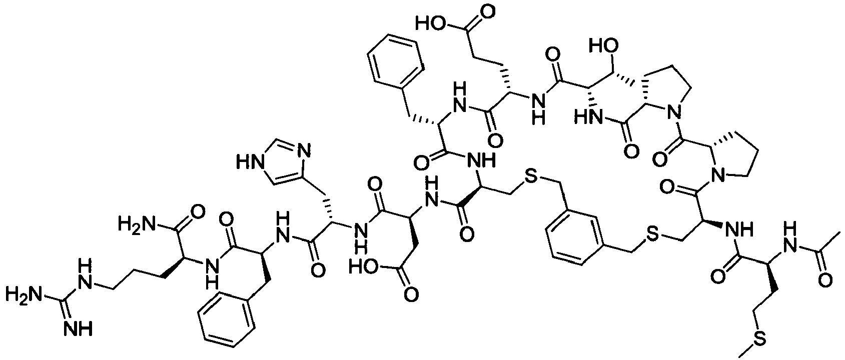
compound Ac-[Cys(3MeBn)-Pro-Pro-Thr-Glu-Phe-Cys]-Asp-His-Phe-Arg-Asp-NH2 (3BP-3202) of the following formula 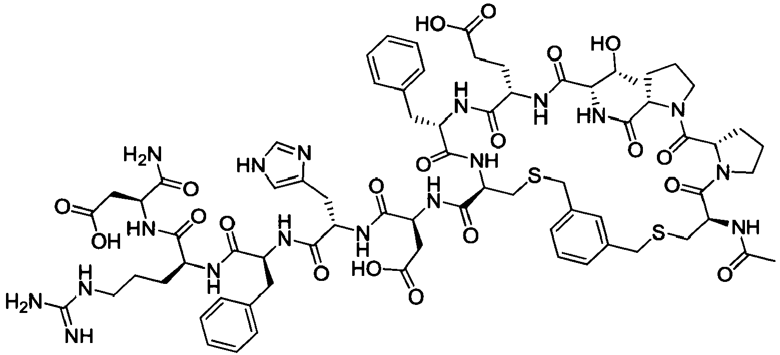
compound Ac-Met-[Cys(3MeBn)-Pro-Pro-Thr-Glu-Phe-Cys]-Asp-His-Amf-Arg-Asp-NH2 (3BP-3203) of the following formula 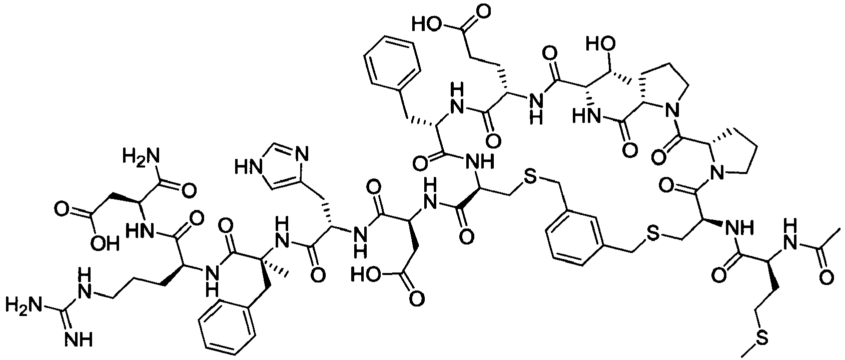
compound Ac-Met-[Cys(3MeBn)-Pro-Pro-Thr-Glu-Phe-Cys]-Asp-his-Phe-Arg-Asp-NH2 (3BP-3210) of the following formula 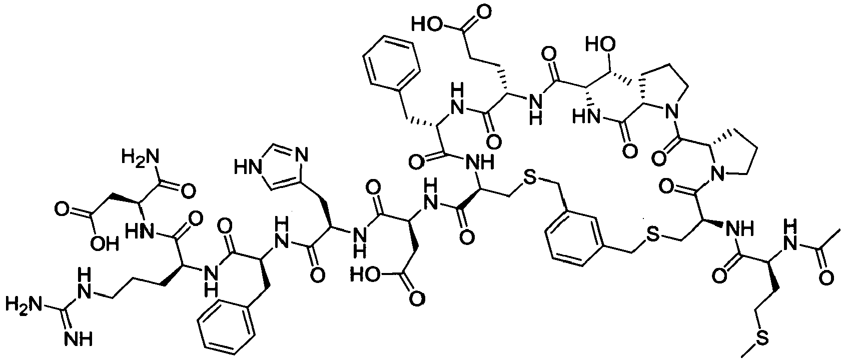
compound Ac-Met-[Cys(3MeBn)-Pro-Pro-Thr-Glu-Phe-Cys]-Asp-His-phe-Arg-Asp-NH2 (3BP-3211) of the following formula 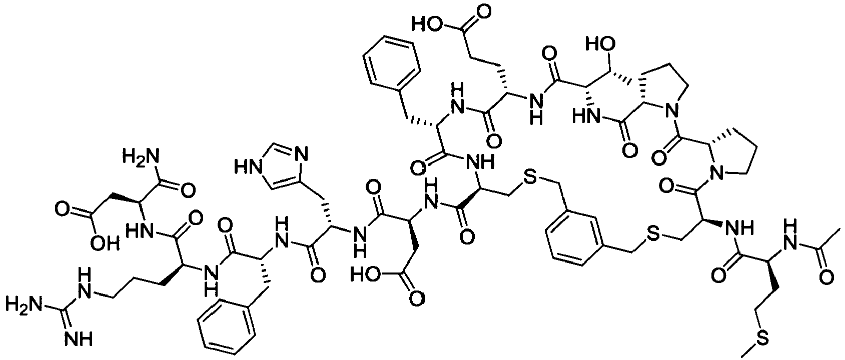
compound Ac-Met-[Cys(3MeBn)-Pro-Pro-Thr-Glu-Phe-Cys]-Asp-His-Phe-arg-Asp-NH2 (3BP-3212) of the following formula 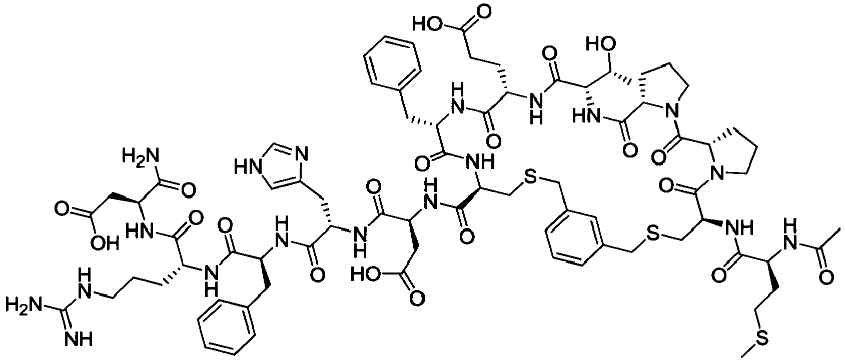
compound Ac-Met-[Cys(3MeBn)-Pro-Pro-Thr-Glu-Phe-Cys]-Asp-His-Phe-Arg-asp-NH2 (3BP-3213) of the following formula 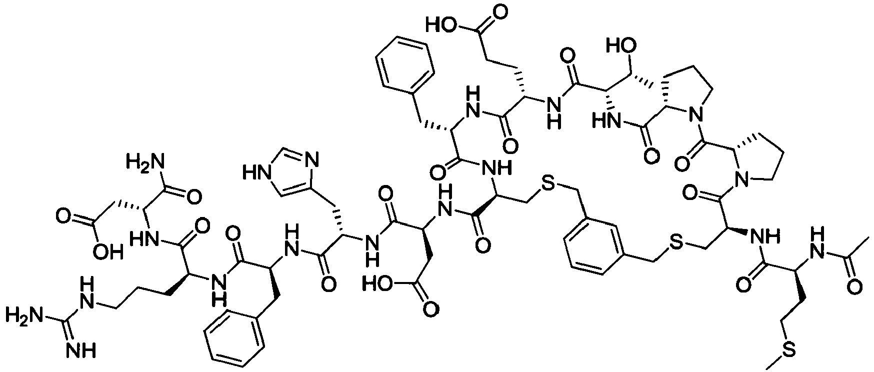
compound Ac-Met-[Cys(3MeBn)-Gly-Pro-Thr-Glu-Phe-Cys]-Asp-His-Phe-Arg-Asp-NH2 (3BP-3214) of the following formula 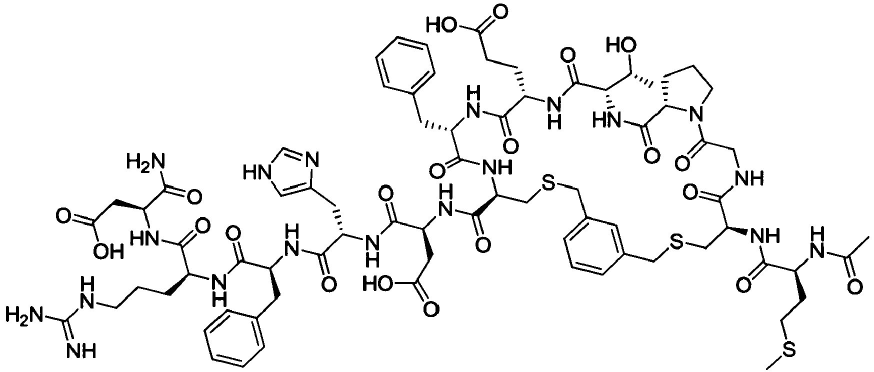
compound Hex-[Cys(3MeBn)-Pro-Pro-Thr-Glu-Phe-Cys]-Asp-His-Nmf-Arg-Ttds-Lys(DOTA)-NH2 (3BP-3275) of the following formula 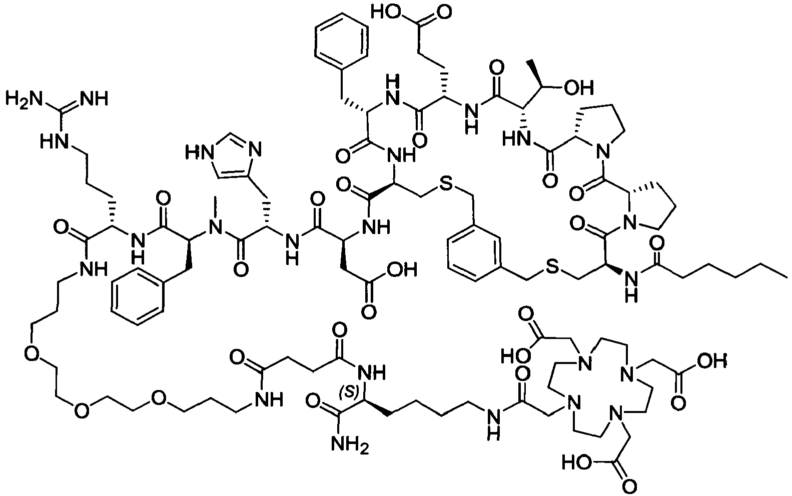
compound Hex-[Cys(3MeBn)-Pro-Pro-Thr-Glu-Phe-Cys]-Asp-His-phe-Arg-Ttds-Lys(DOTA)-NH2 (3BP-3276) of the following formula 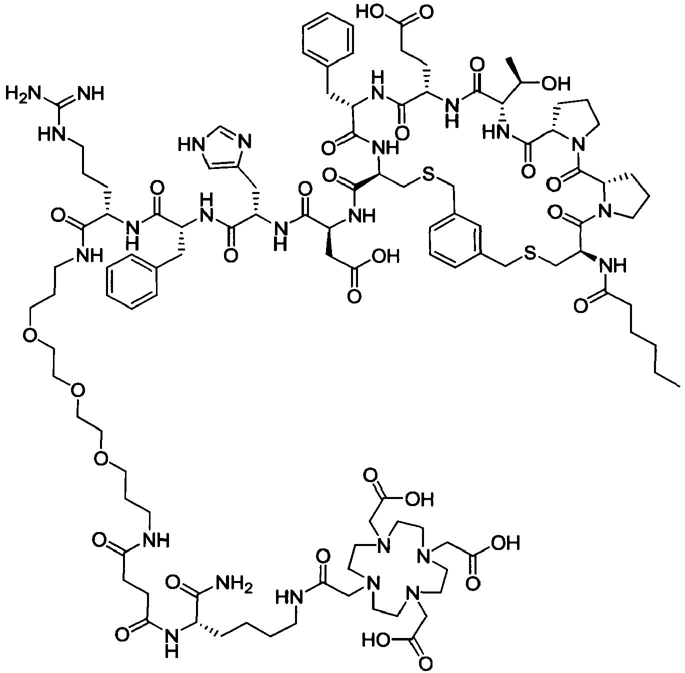
compound Hex-[Cys(3MeBn)-Pro-Pro-Thr-Glu-Phe-Cys]-Asp-His-Ppa-arg-Ttds-Lys(DOTA)-NH2 (3BP-3277) of the following formula 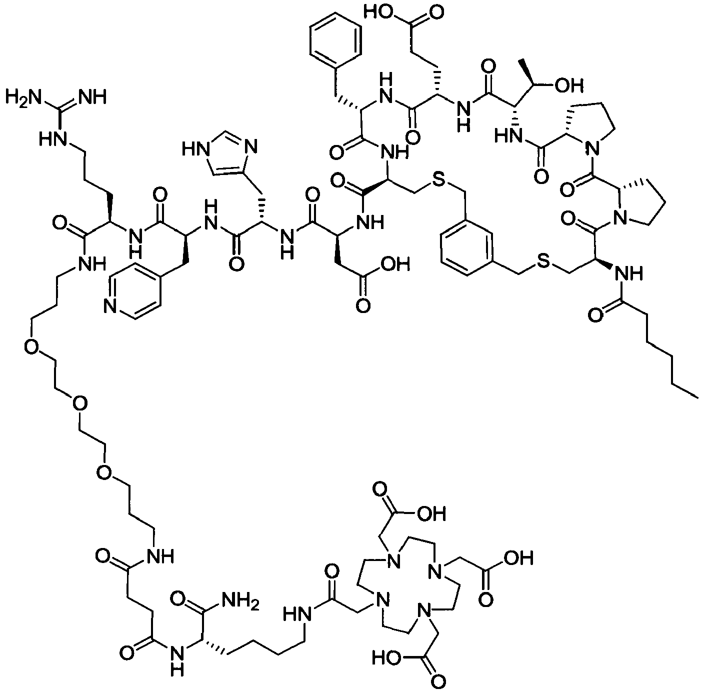
compound Hex-[Cys(3MeBn)-Pro-Pro-Thr-Glu-Phe-Cys]-Asp-NH2 (3BP-3288) of the following formula 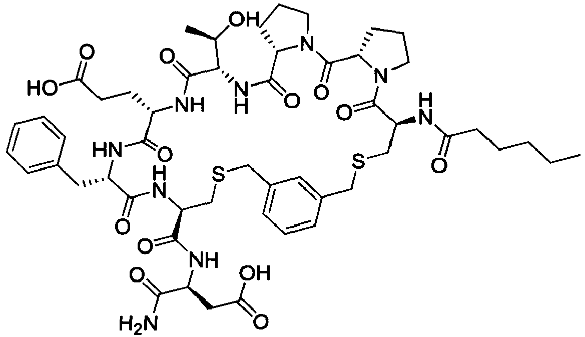
compound Hex-[Cys(3MeBn)-Pro-Pro-Thr-Glu-Phe-Cys]-Asp-Arg-NH2 (3BP-3299) of the following formula 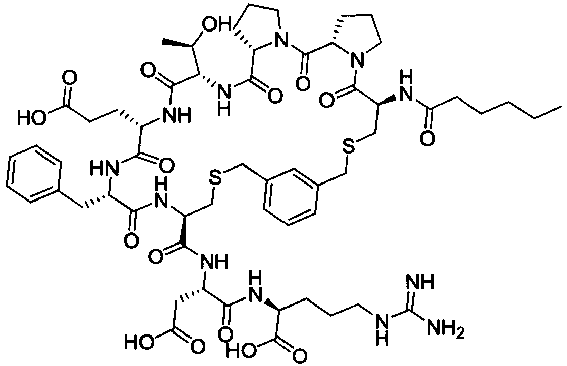
compound Hex-[Cys(3MeBn)-Pro-Pro-Thr-Glu-Phe-Cys]-Asp-Gab-Arg-NH2 (3BP-3300) of the following formula 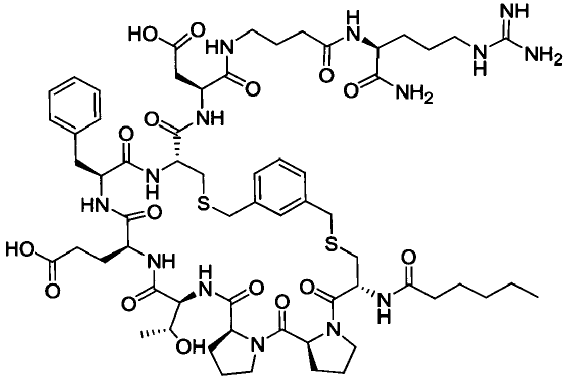
compound Hex-[Cys(3MeBn)-Pro-Pro-Thr-Glu-Phe-Cys]-Asp-Pamb-Arg-NH2 (3BP-3301) of the following formula 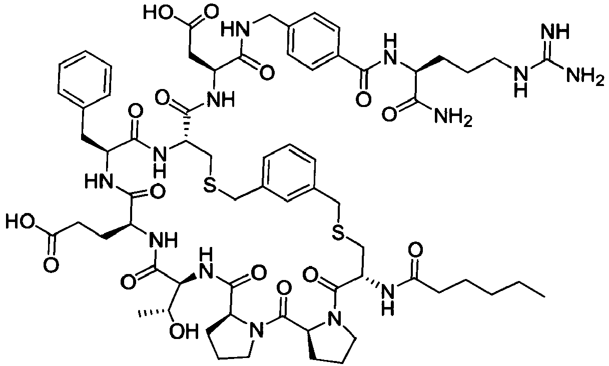
compound Hex-[Cys(3MeBn)-Pro-Pro-Thr-Glu-Phe-Cys]-Asp-Cmp-Arg-NH2 (3BP-3302) of the following formula 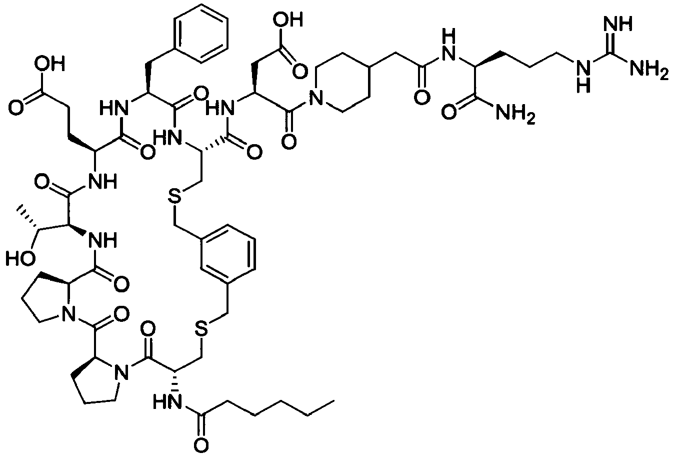
compound Hex-[Cys(3MeBn)-Pro-Pro-Thr-Glu-Phe-Cys]-Pamb-Arg-NH2 (3BP-3303) of the following formula 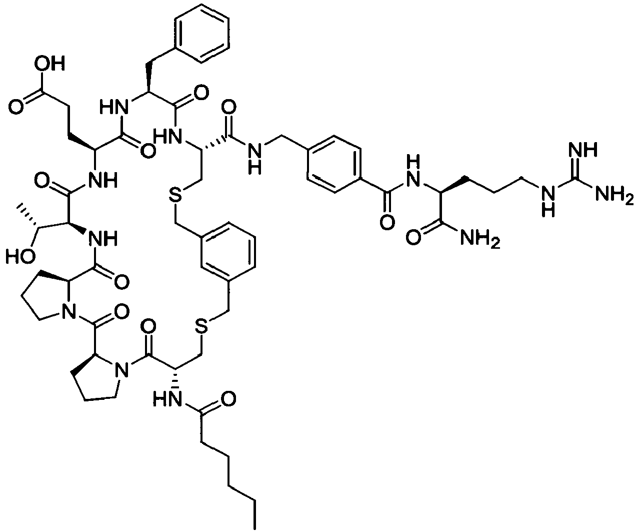
compound DOTA-Ttds-Nle-[Cys(3MeBn)-Pro-Pro-Thr-Glu-Phe-Cys]-NH2 (3BP-3319) of the following formula 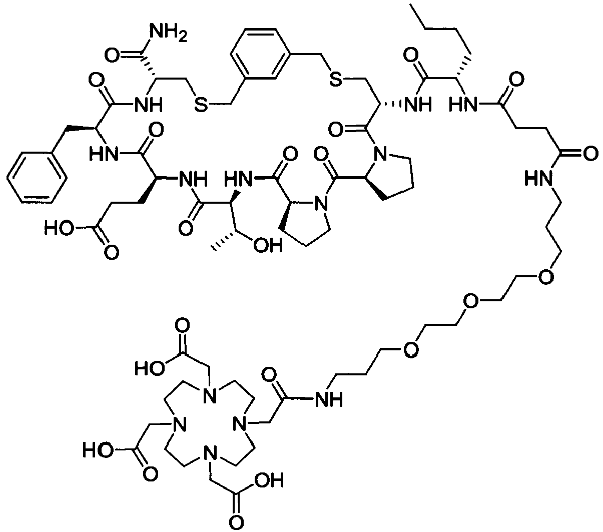
compound DOTA-Ttds-Nle-[Cys(3MeBn)-Pro-Pro-Thr-Glu-Phe-Cys]-Asp-NH2 (3BP-3320) of the following formula 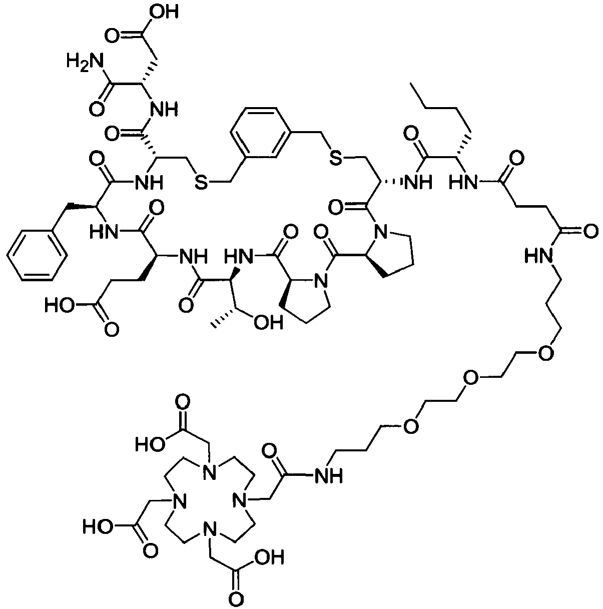
compound DOTA-Ttds-Nle-[Cys(3MeBn)-Pro-Pro-Thr-Glu-Phe-Cys]-Asp-Pamb-Arg-NH2 (3BP-3321) of the following formula 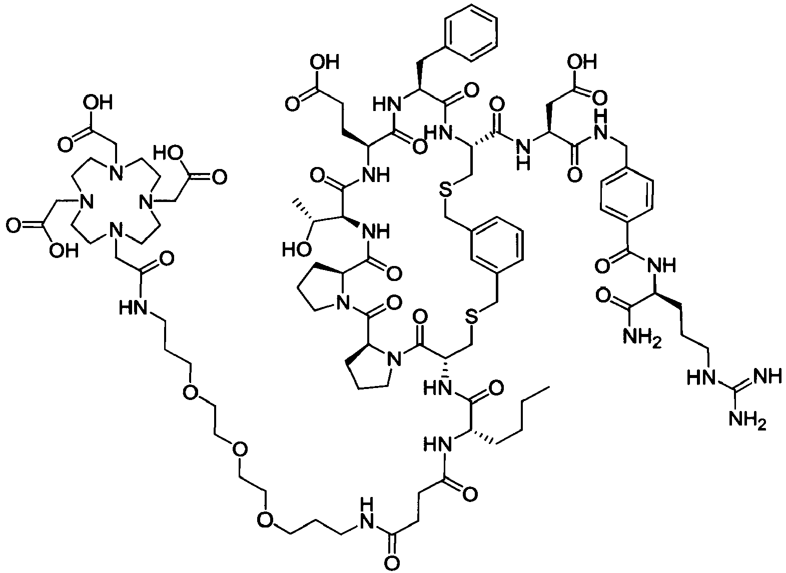
compound Hex-[Cys(3MeBn)-Pro-Pro-Thr-Glu-Phe-Cys]-Asp-Mamb-Arg-NH2 (3BP-3324) of the following formula 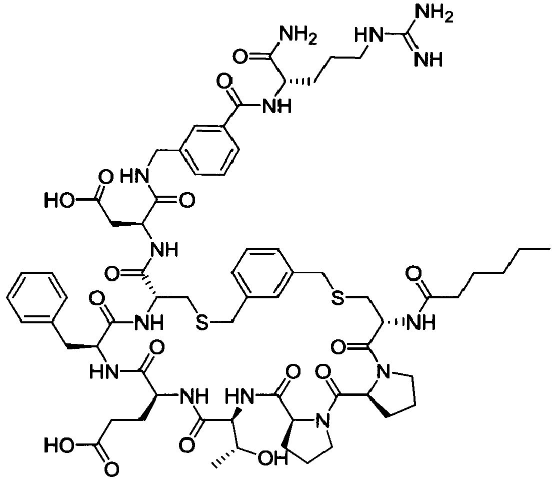
compound Hex-[Cys(3MeBn)-Pro-Pro-Thr-Gln-Phe-Cys]-NH2 (3BP-3349) of the following formula 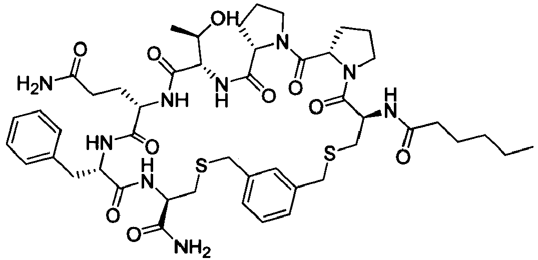
compound Hex-[Cys(3MeBn)-Pro-Pro-Thr-Glu-Phe-Cys]-Bal-OH (3BP-3371) of the following formula 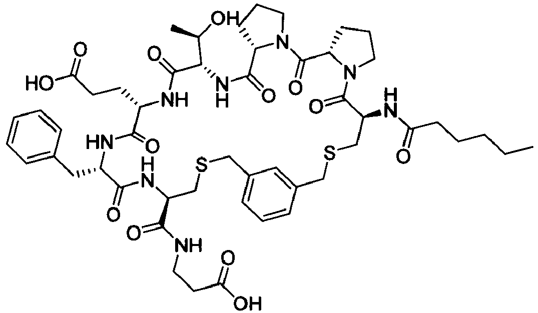
compound Hex-[Cys(3MeBn)-Pro-Pro-Thr-Gln-Phe-Cys]-Asp-Ttds-Lys(DOTA)-NH2 (3BP-3395) of the following formula 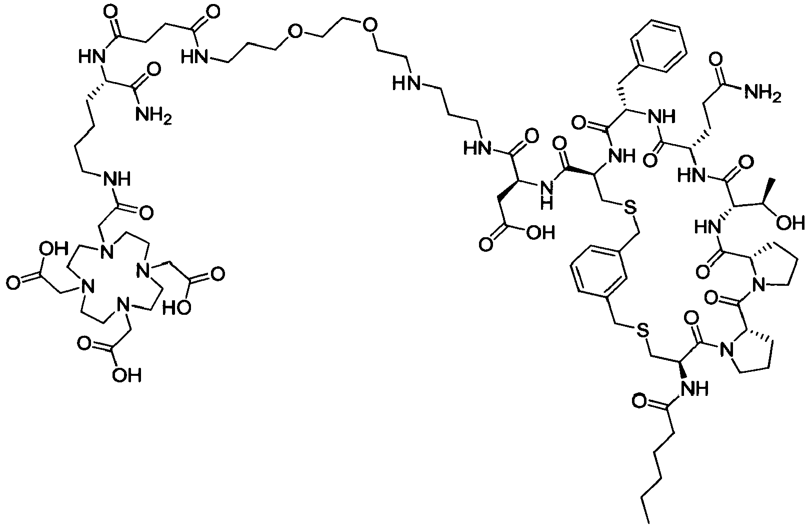
compound Hex-[Cys(3MeBn)-Pro-Pro-Thr-Glu-Phe-Cys]-Asp-Ttds-Lys(DOTA)-NH2 (3BP-3396) of the following formula 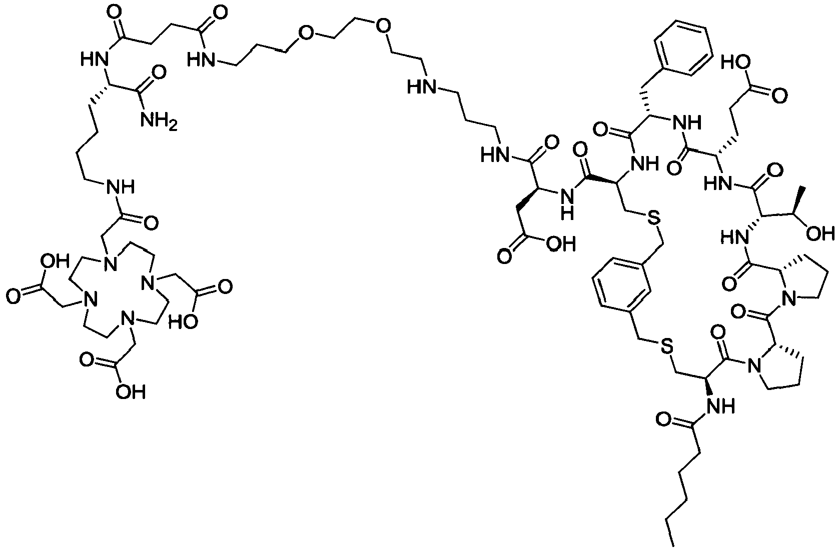
compound Hex-[Cys(3MeBn)-Pro-Pro-Thr-Gln-Phe-Cys]-Bhk(DOTA)-OH (3BP-3397) of the following formula 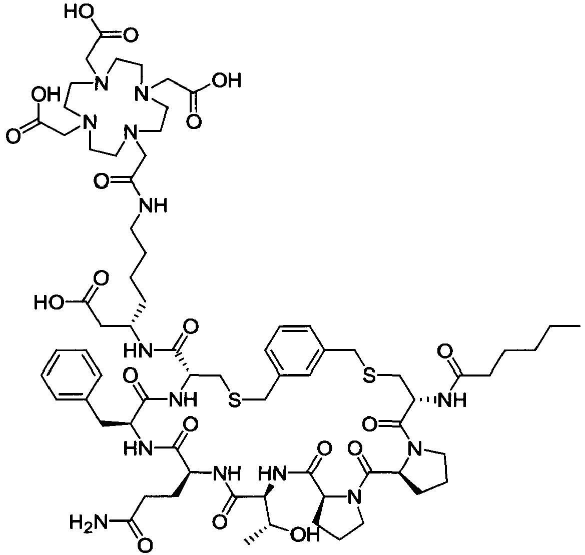
compound DOTA-Ttds-Nle-[Cys(3MeBn)-Pro-Pro-Thr-Gln-Phe-Cys]-Bal-OH (3BP-3398) of the following formula 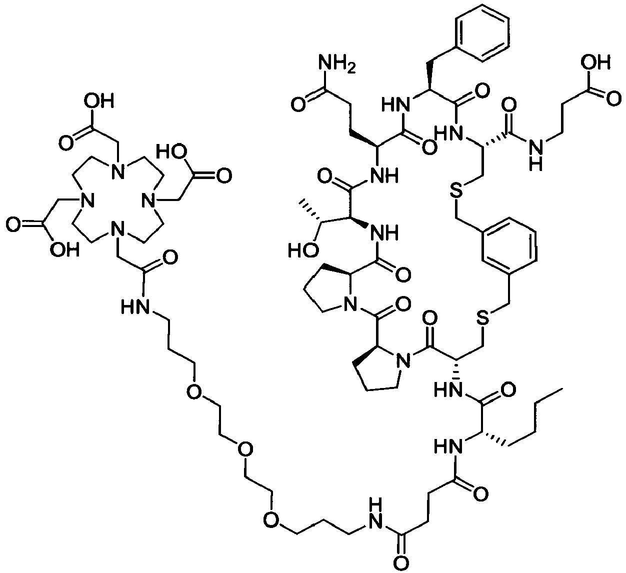
compound DOTA-Ttds-Nle-[Cys(3MeBn)-Pro-Pro-Thr-Gln-Phe-Cys]-Asp-NH2 (3BP-3401) of the following formula 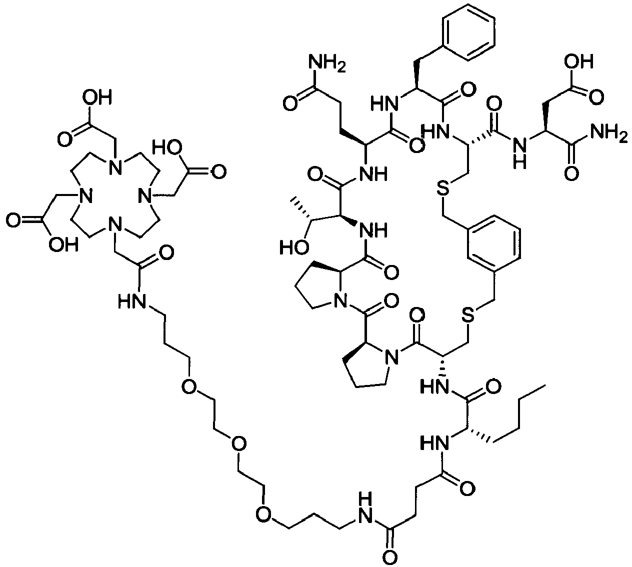
compound Hex-[Cys(3MeBn)-Pro-Pro-Thr-Gln-Phe-Cys]-Asp-Ape(DOTA) (3BP-3403) of the following formula 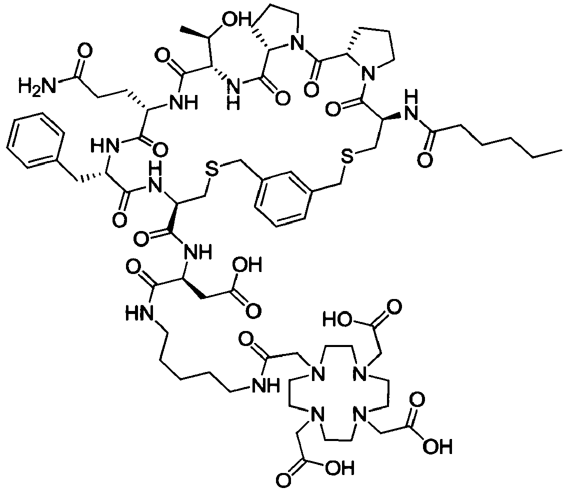
compound Hex-[Cys(3MeBn)-Pro-Pro-Thr-Gln-Phe-Cys]-Asp-Ttds-Ape(DOTA) (3BP-3404) of the following formula 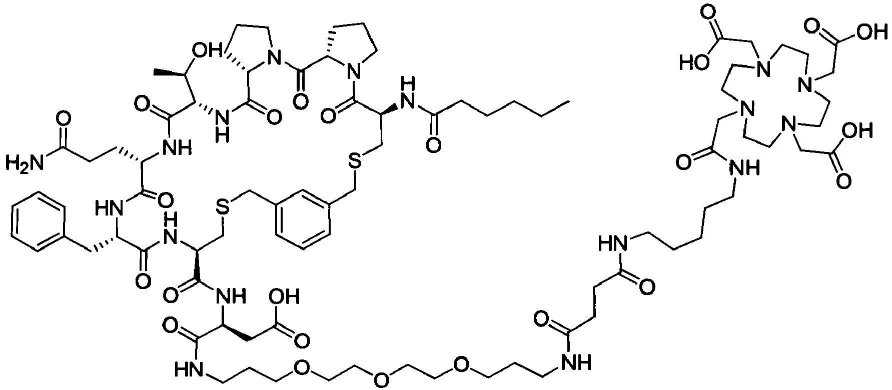
compound Hex-[Cys(tMeBn(DOTA-PP))-Pro-Pro-Thr-Gln-Phe-Cys]-Asp-NH2 (3BP-3407) of the following formula 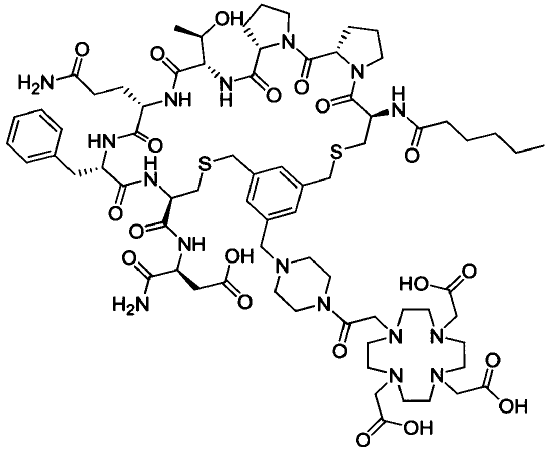
compound Hex-[Cys(3MeBn)-Pro-Pro-Thr-Gln-Otf-Cys]-NH2 (3BP-3409) of the following formula 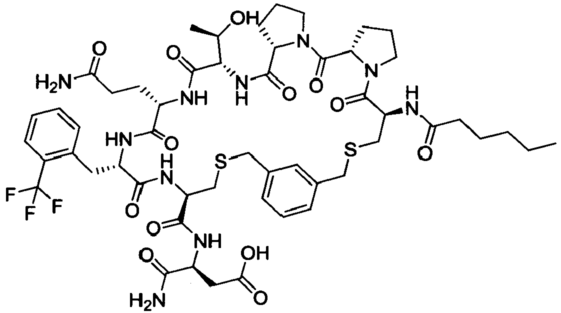
compound PentylNH-urea-[Cys(3MeBn)-Pro-Pro-Thr-Gln-Phe-Cys]-Asp-NH2 (3BP-3425) of the following formula 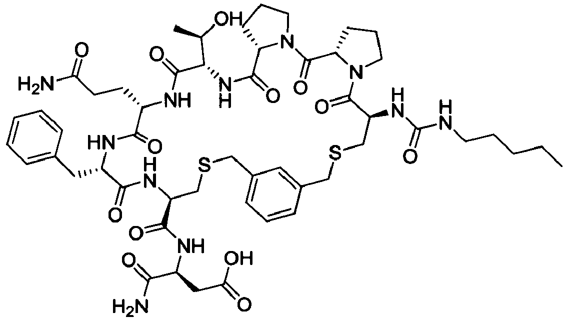
compound Hex-[Cys(tMeBn(DOTA-AET))-Pro-Pro-Thr-Gln-Phe-Cys]-Asp-NH2 (3BP-3426) of the following formula 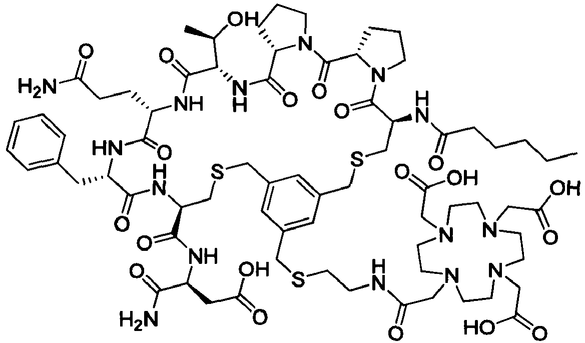
compound Hex-[Cys(3MeBn)-Pro-Pro-Thr-Gln-Phe-Cys]-Asp-NH2 (3BP-3476) of the following formula 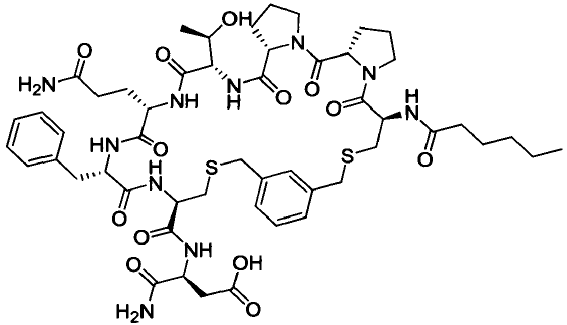
compound Hex-[Cys(3MeBn)-Pro-Pro-Thr-Gln-Phe-Cys]-Bhk(DOTA-Ttds)-OH (3BP-3489) of the following formula 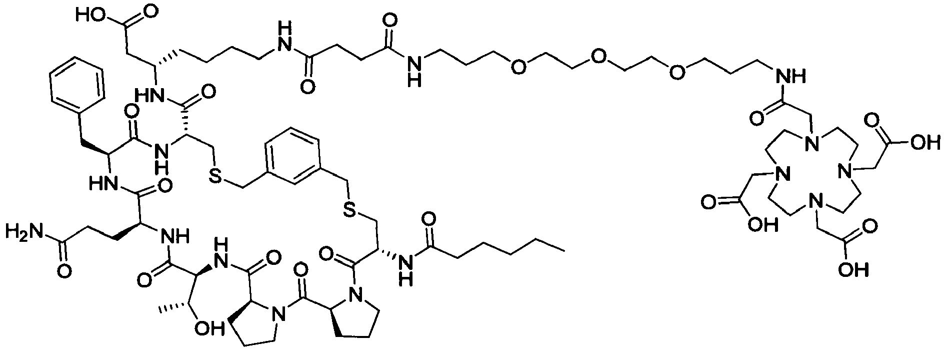
compound Pentyl-SO2-[Cys(3MeBn)-Pro-Pro-Thr-Gln-Phe-Cys]-Asp-NH2 (3BP-3514) of the following formula 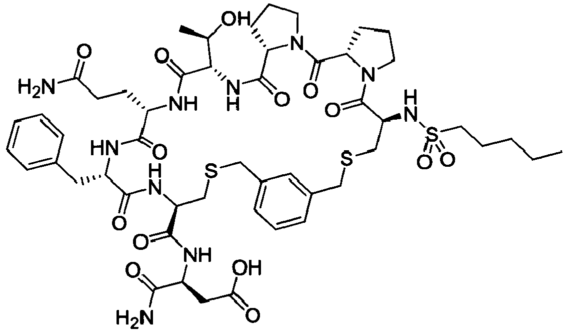
compound Hex-[Cys(2Lut)-Pro-Pro-Thr-Gln-Phe-Cys]-Asp-NH2 (3BP-3518) of the following formula 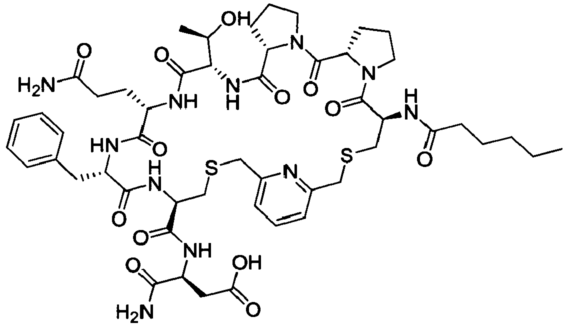
compound Hex-[Cys(3Lut)-Pro-Pro-Thr-Gln-Phe-Cys]-Asp-NH2 (3BP-3519) of the following formula 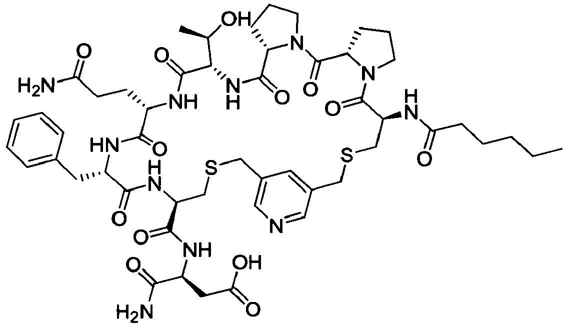
compound Hex-[Cys(tMeBn(DOTA-AET))-Pro-Pro-Thr-Gln-Phe-Cys]-OH (3BP-3554) of the following formula 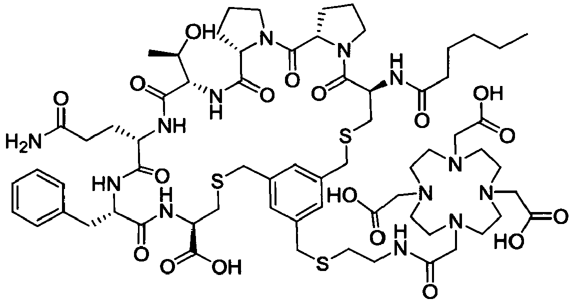
compound Hex-[Cys(tMeBn(DOTA-PP))-Pro-Pro-Thr-Gln-Phe-Cys]-OH (3BP-3555) of the following formula 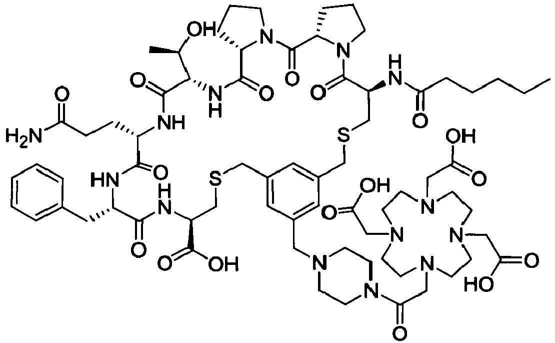
compound Hex-[Cys(tMeBn(InDOTA-PP))-Pro-Pro-Thr-Gln-Phe-Cys]-Asp-NH2 (3BP-3590) of the following formula 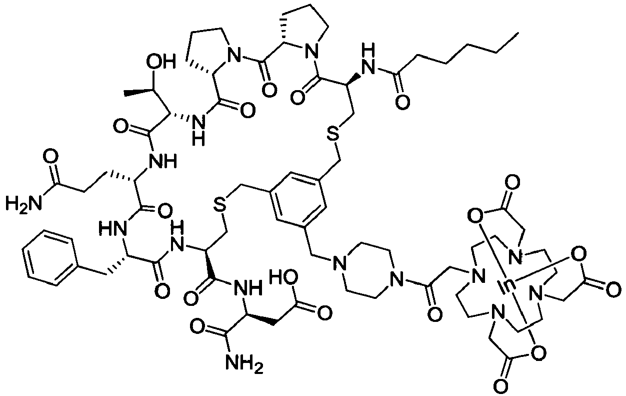
compound Hex-[Cys(tMeBn(LuDOTA-PP))-Pro-Pro-Thr-Gln-Phe-Cys]-Asp-NH2 (3BP-3591) of the following formula 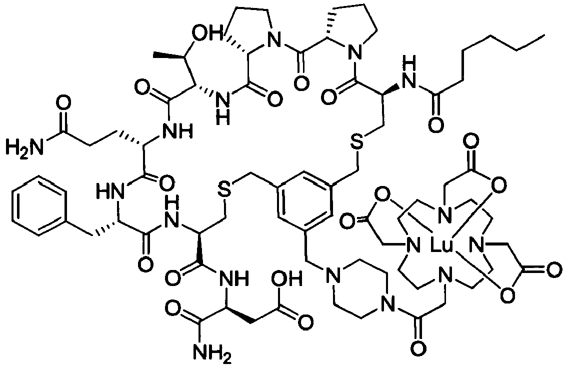
compound Hex-[Cys(tMeBn(GaDOTA-PP))-Pro-Pro-Thr-Gln-Phe-Cys]-Asp-NH2 (3BP-3592) of the following formula 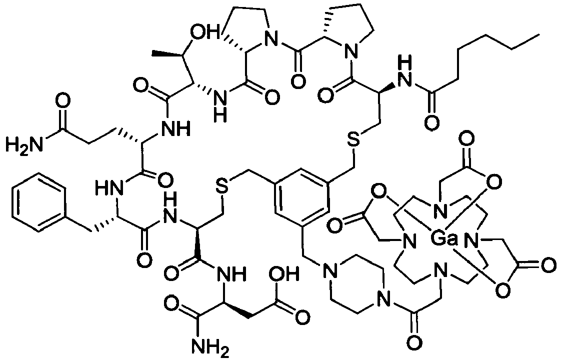
compound Hex-[Cys(tMeBn(InDOTA-AET))-Pro-Pro-Thr-Gln-Phe-Cys]-OH (3BP-3623) of the following formula 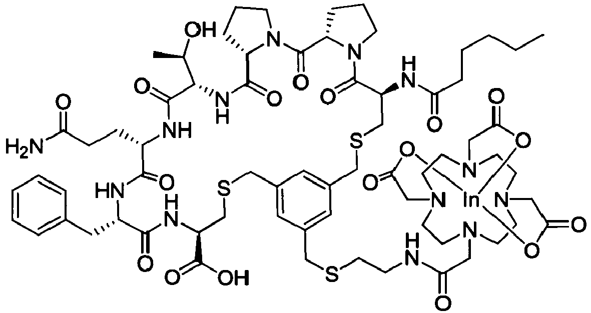
compound Hex-[Cys(tMeBn(LuDOTA-AET))-Pro-Pro-Thr-Gln-Phe-Cys]-OH (3BP-3624) of the following formula 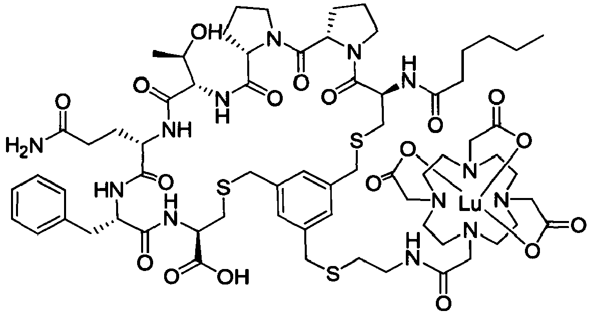
compound Hex-[Cys(tMeBn(DOTA-AET))-Pro-Pro-Thr-Gln-lNi-Cys]-OH (3BP-3650) of the following formula 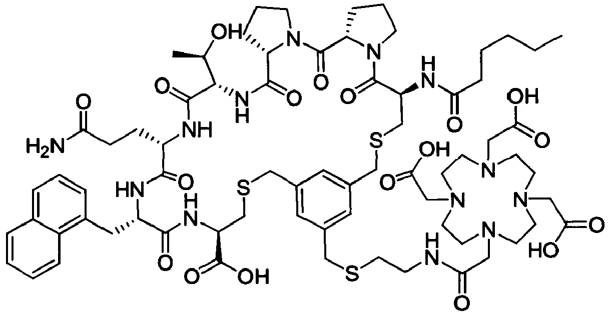
compound Hex-[Cys(tMeBn(DOTA-AET))-Pro-Pro-Thr-Gln-Phe-Cys]-Bal-OH (3BP-3651) of the following formula 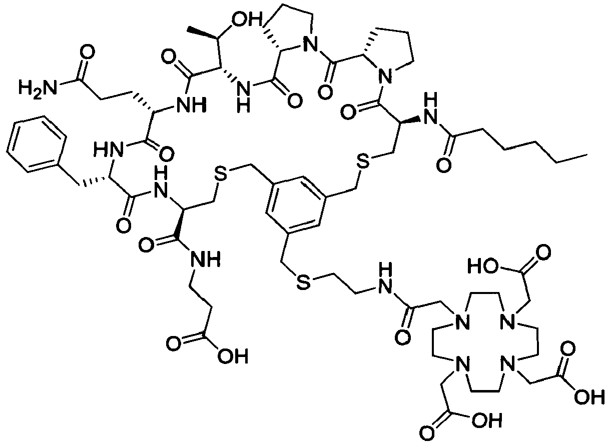
compound Hex-[Cys(tMeBn(DOTA-AET))-Pro-Pro-Thr-Gln-Phe-Cys]-NH2 (3BP-3652) of the following formula 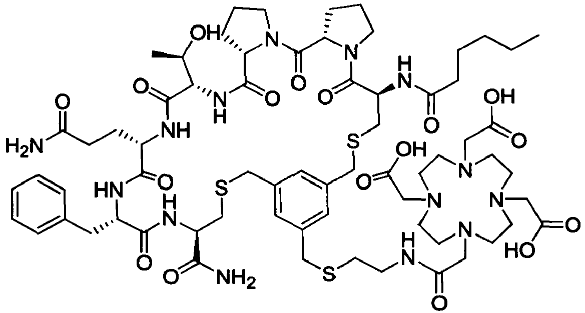
compound Hex-[Cys(tMeBn(DOTA-AET))-Pro-Pro-Thr-Glu-Phe-Cys]-NH2 (3BP-3653) of the following formula 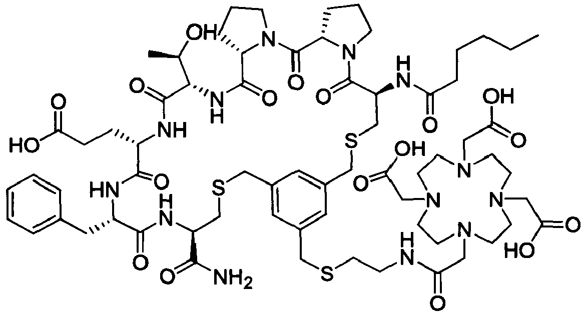
compound Hex-[Cys(tMeBn(DOTA-AET))-Pro-Pro-Thr-Gln-Phe-AET] (3BP-3654) of the following formula 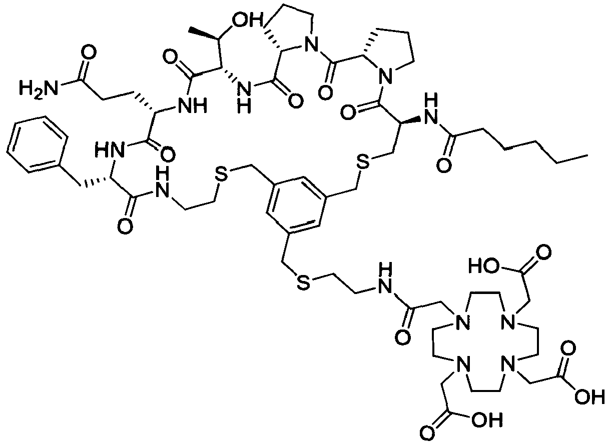
compound Hex-[Cys(tMeBn(DOTA-AET))-Pro-Pro-Thr-Gln-Phe-Cys]-Gly-OH (3BP-3656) of the following formula 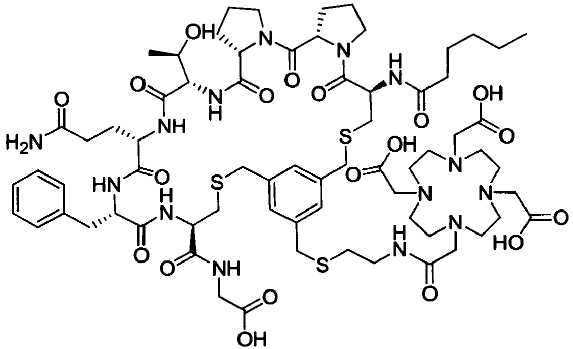
compound Hex-[Cys(tMeBn(DOTA-AET))-Pro-Pro-Thr-Gln-Phe-Cys]-Gab-OH (3BP-3657) of the following formula 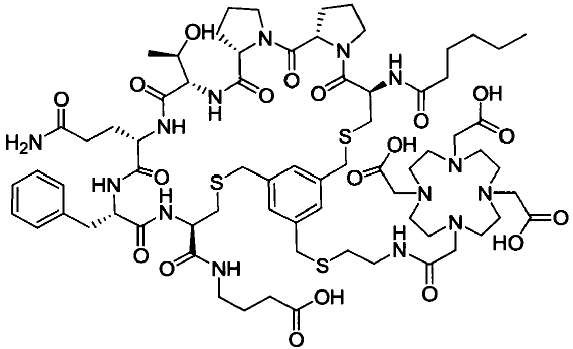
compound Hex-[Cys(tMeBn(DOTA-AET))-Pro-Pro-Thr-Gln-Phe-Cys]-Ser-OH (3BP-3658) of the following formula 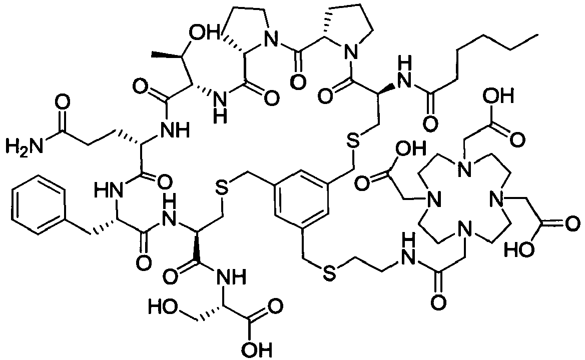
compound Hex-[Cys(tMeBn(DOTA-AET))-Pro-Pro-Thr-Gln-Phe-Cys]-Nmg-OH (3BP-3659) of the following formula 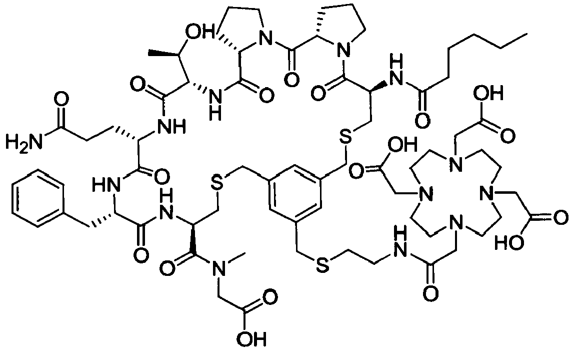
compound Hex-[Cys(tMeBn(DOTA-AET))-Pro-Pro-Thr-Gln-Phe-Cys]-Bhf-OH (3BP-3660) of the following formula 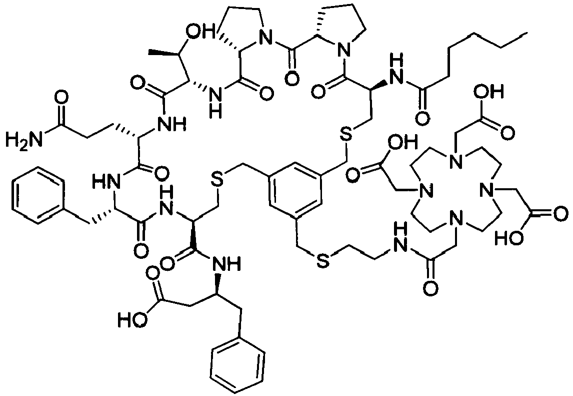
compound Hex-[Cys(tMeBn(EuDOTA-PP))-Pro-Pro-Thr-Gln-Phe-Cys]-Asp-NH2 (3BP-3661) of the following formula 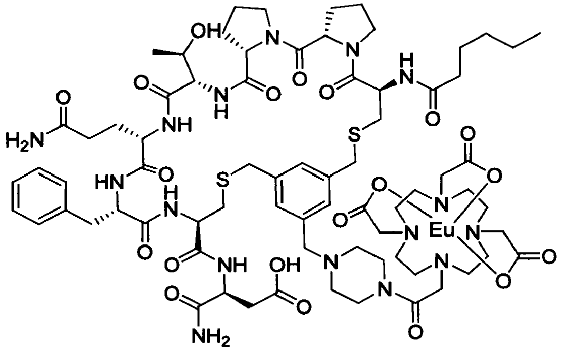
compound Hex-[Cys(tMeBn(EuDOTA-AET))-Pro-Pro-Thr-Gln-Phe-Cys]-OH (3BP-3662) of the following formula 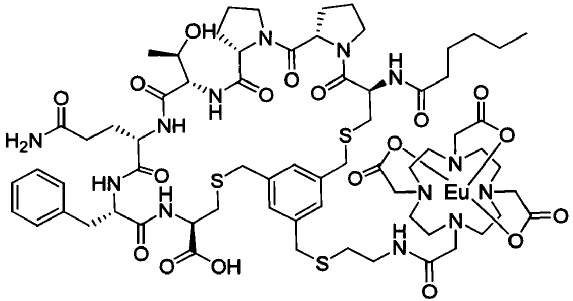
compound Hex-[Cys(tMeBn(DOTA-AET))-Pro-Pro-Thr-Gln-Mpa-Cys]-OH (3BP-3664) of the following formula 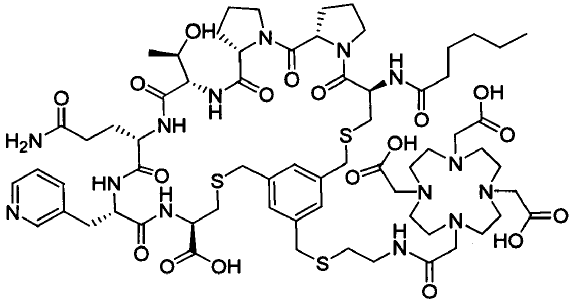
compound Hex-[Cys(tMeBn(DOTA-AET))-Pro-Pro-Thr-Gln-Phe-Cys]-Asp-OH (3BP-3665) of the following formula 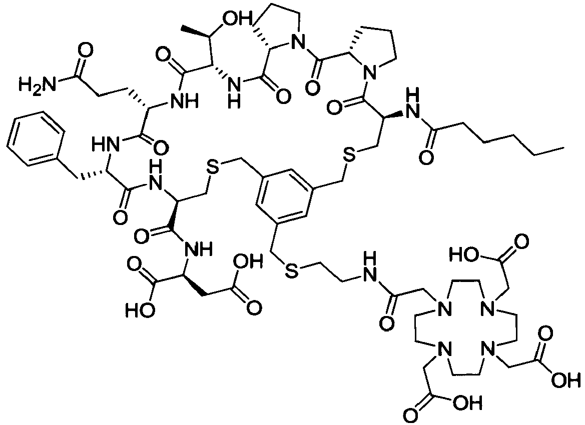
compound Hex-[Cys(tMeBn(DOTA-AET))-Nmg-Pro-Thr-Gln-Phe-Cys]-OH (3BP-3678) of the following formula 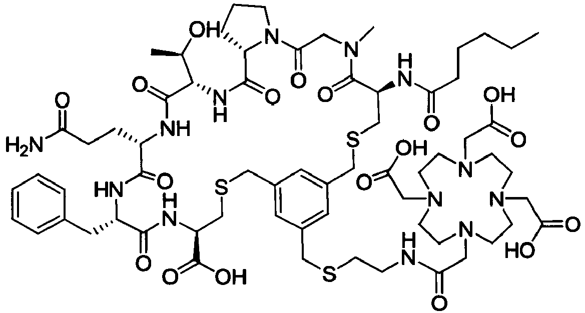
compound Hex-[Cys(tMeBn(DOTA-AET))-Pro-Hyp-Thr-Gln-Phe-Cys]-OH (3BP-3679) of the following formula 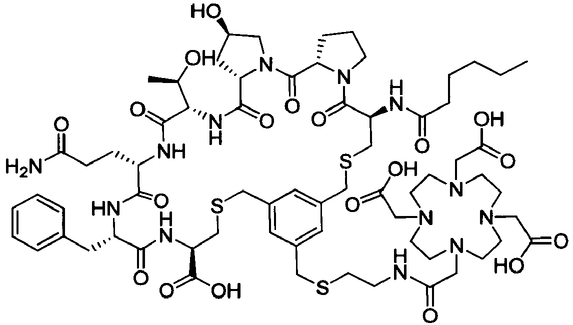
compound Hex-[Cys(tMeBn(DOTA-AET))-Pro-Pro-Thr-Gln-Otf-Cys]-OH (3BP-3680) of the following formula 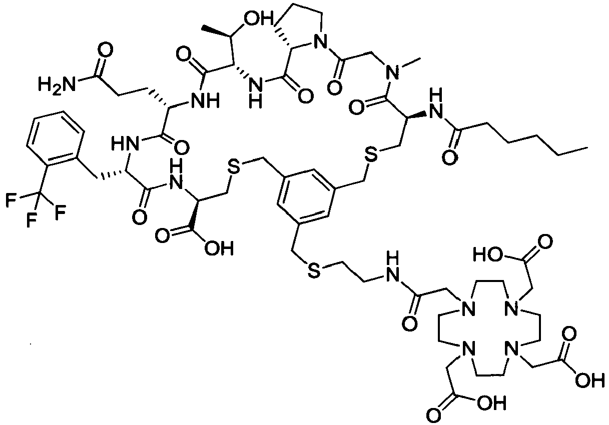
compound Hex-[Cys(tMeBn(DOTA-AET))-Pro-Pro-Thr-Gln-Phe-Cys]-asp-NH2 (3BP-3681) of the following formula 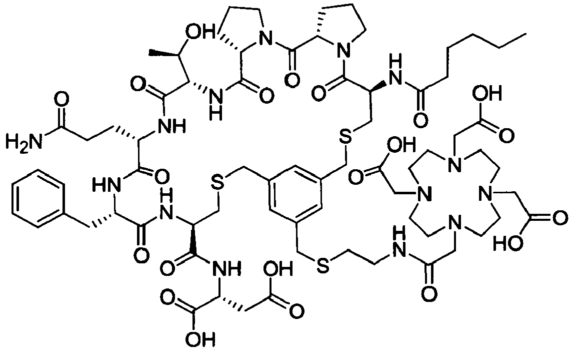
compound Pentyl-S02-[Cys(tMeBn(DOTA-AET))-Pro-Pro-Thr-Gln-Phe-Cys]-OH (3BP-3690) of the following formula 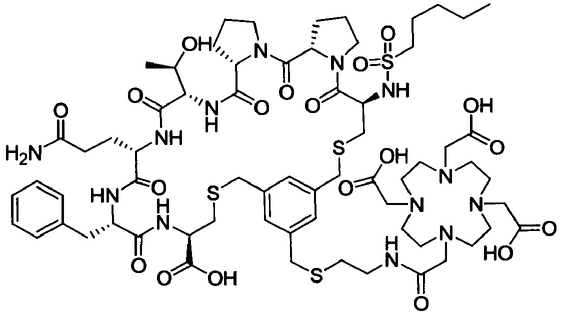
compound Pentyl-SO2-[Cys(tMeBn(DOTA-AET))-Pro-Pro-Thr-Glu-Phe-Cys]-OH (3BP-3691) of the following formula 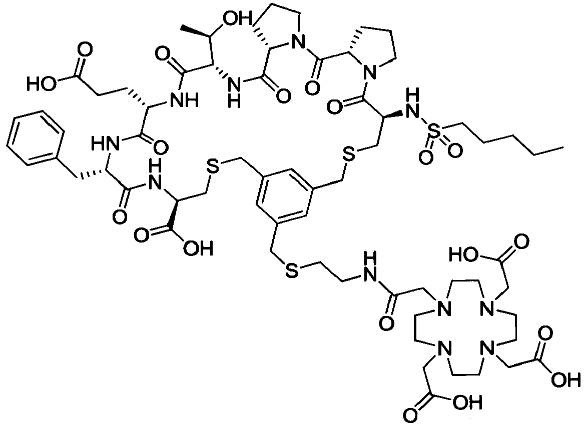
compound Pentyl-SO2-[Cys(tMeBn(DOTA-PP))-Pro-Pro-Thr-Gln-Phe-Cys]-Asp-NH2 (3BP-3692) of the following formula 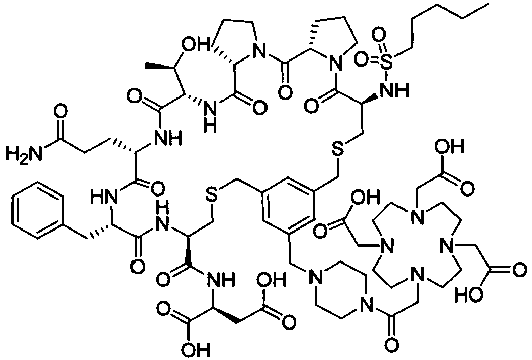
compound Hex-[Cys(tMeBn(InDOTA-AET))-Pro-Pro-Thr-Gln-Phe-Cys]-NH2 (3BP-3712) of the following formula 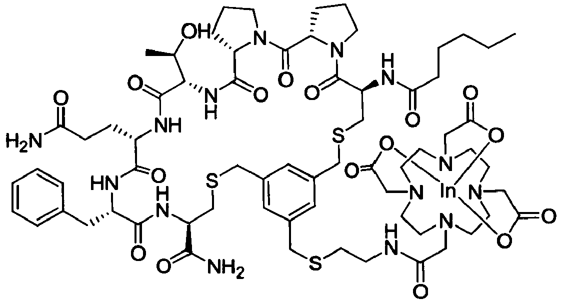
compound Hex-[Cys(tMeBn(InDOTA-AET))-Pro-Pro-Thr-Gln-Phe-AET] (3BP-3713) of the following formula 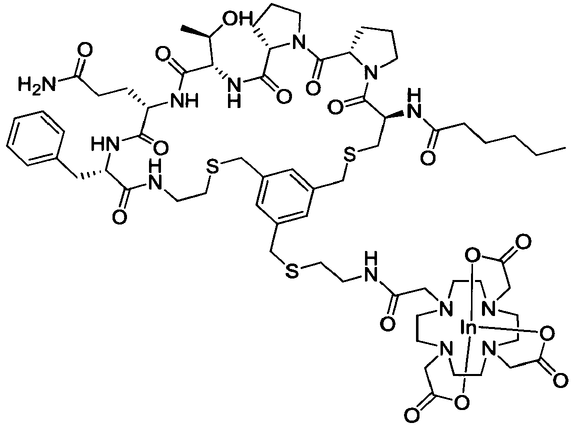
compound Hex-[Cys(tMeBn(InDOTA-AET))-Pro-Pro-Thr-Gln-Phe-Cys]-Gly-OH (3BP-3714) of the following formula 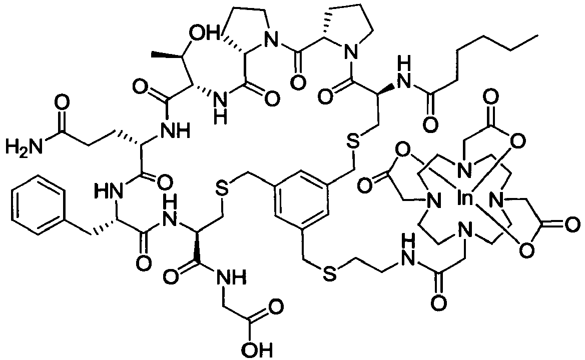
compound Hex-[Cys(tMeBn(InDOTA-AET))-Pro-Pro-Thr-Gln-Phe-Cys]-Nmg-OH (3BP-3715) of the following formula 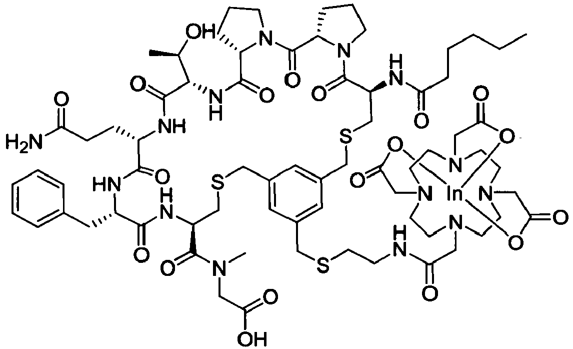
compound Hex-[Cys(tMeBn(InDOTA-AET))-Nmg-Pro-Thr-Gln-Phe-Cys]-OH (3BP-3716) of the following formula 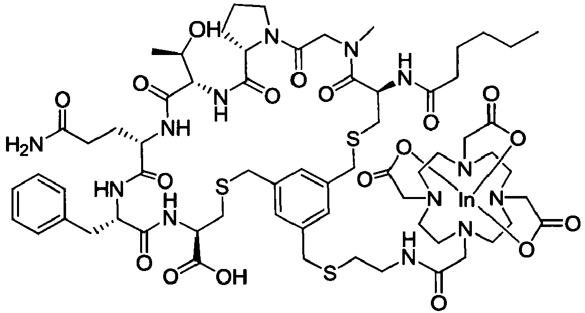
compound Pentyl-S02-[Cys(tMeBn(InDOTA-PP))-Pro-Pro-Thr-Gln-Phe-Cys]-Asp-NH2 (3BP-3717) of the following formula 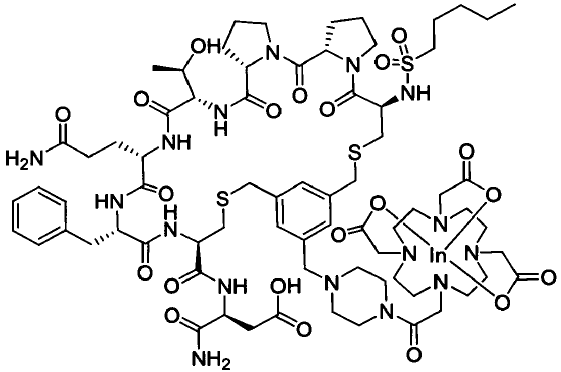
compound Hex-[Cys(tMeBn(DOTA-AET))-Pro-Pro-Thr-Gln-Phe-Cys]-Bal-NH2 (3BP-3736) of the following formula 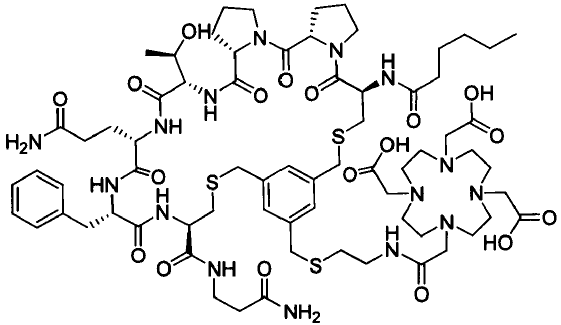
compound Hex-[Cys(tMeBn(DOTA-AET))-Pro-Pro-Thr-Gln-Phe-Cys]-Nmg-NH2 (3BP-3737) of the following formula 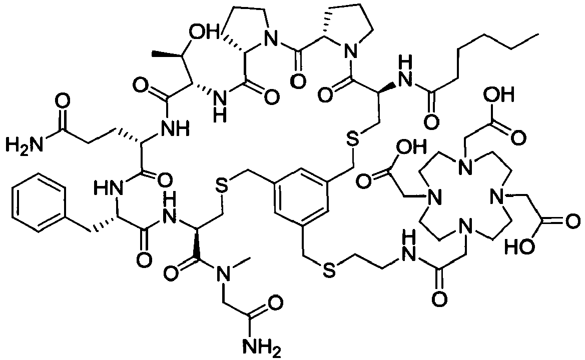
compound Hex-[Cys(tMeBn(DOTA-AET))-Nmg-Pro-Thr-Gln-Phe-Cys]-NH2 (3BP-3744) of the following formula 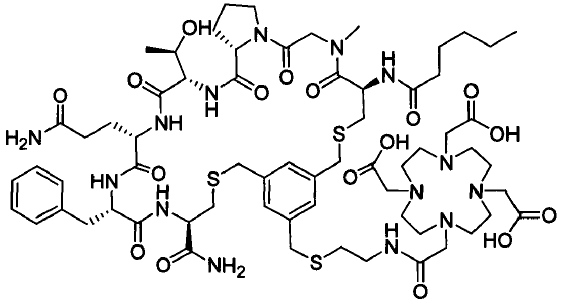
compound Hex-[Cys(tMeBn(DOTA-AET))-Pro-Pro-Thr-Gln-Phe-Cysol] (3BP-3767) of the following formula 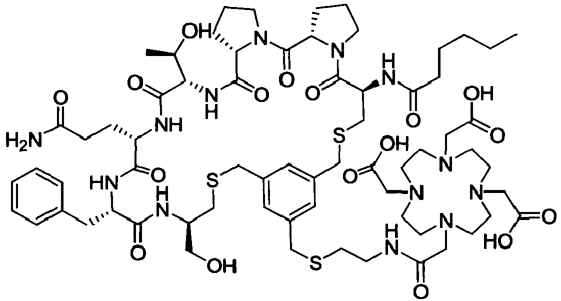
compound Hex-[Cys(tMeBn(InDOTA-PP))-Pro-Pro-Thr-Gln-Phe-Cys]-OH (3BP-3770) of the following formula 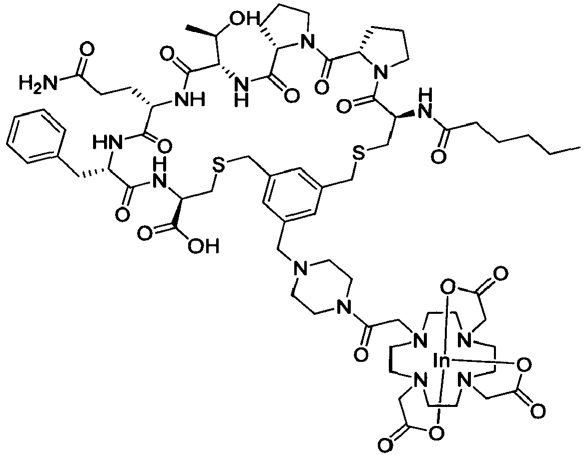
and compound Hex-[Cys(tMeBn(DOTA-PP))-Nmg-Pro-Thr-Gln-Phe-Cys]-OH (3BP-3771) of the following formula

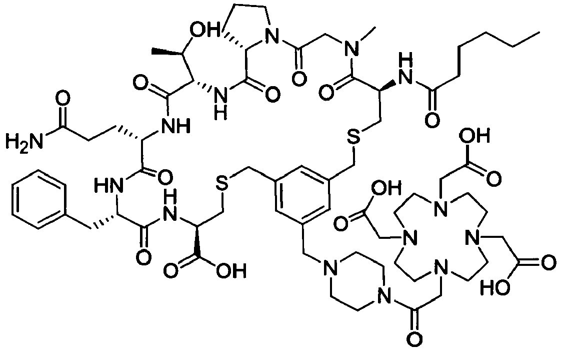

18. The compound of any one of claims 1 to 17, wherein the compound comprises a diagnostically active nuclide or a therapeutically active nuclide, wherein, preferably, the diagnostically active nuclide is a diagnostically active radionuclide, more preferably selected from the group consisting of ⁴³Sc, ⁴⁴Sc , ⁵¹Mn , ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵5Tb, ²⁰¹Tl, ²⁰³Pb, ¹⁸F, ⁷⁶Br, ⁷⁷Br, ¹²³I, ¹²⁴I, ¹²⁵I, preferably ⁴¹Sc, ⁴⁴Sc, ⁶⁴Cu, ⁶⁷Ga, ⁶⁸Ga, ⁸⁶Y, ⁸⁹Zr, ^{99m}Tc, ¹¹¹In, ¹⁵²Tb, ¹⁵5Tb, ²⁰³Pb, ¹⁸F, ⁷⁶Br, ⁷⁷Br, ¹²³I, ¹²⁴I, ¹²⁵I and most preferably ⁶⁴Cu, ⁶⁸Ga, ⁸⁹Zr, ^{99m}Tc, ¹¹¹In, ¹⁸F, ¹²³I, and ¹²⁴I and wherein the therapeutically active nuclide is a therapeutically active radionuclide, more preferably selected from the group consisting of ⁴⁷Sc , ⁶⁷Cu, ⁸⁹Sr, ⁹⁰Y, ¹⁵³Sm, ¹⁴⁹Tb, ¹⁶¹Tb, ¹⁷⁷Lu, ¹⁸⁶Re, ¹⁸⁸Re, ²¹²Pb , ²¹³Bi, ²²³Ra, ²²⁵Ac, ²²⁶Th, ²²⁷Th, ¹³¹I, ²¹¹At, preferably ⁴⁷Sc, ⁶⁷Cu, ⁹⁰Y, ¹⁷⁷Lu, ¹⁸⁸Re, ²¹²Pb, ²¹³Bi, ²²⁵Ac, ²²⁷Th, ¹³¹I, ²¹¹At and most preferably ⁹⁰Y, ¹⁷⁷Lu, ²²⁵Ac , ²²⁷Th, ¹³¹I and ²¹¹At.

19. The compound of any one of claims 1 to 18, for use in a method for the diagnosis of a disease, for use in a method for the treatment of a disease, for use in a method for the identification of a subject, wherein the subject is likely to respond or likely not to respond to a treatment of a disease, wherein the method for the identification of a subject comprises carrying out a method of diagnosis using the compound of any one of claims 1 to 18, preferably a method for the diagnosis of a disease as described in any one of the preceding claims, or for use in a method for the selection of a subject from a group of subjects, wherein the subject is likely to respond or likely not to respond to a treatment of a disease, wherein the method for the selection of a subject from a group of subjects comprises carrying out a method of diagnosis using the compound of any one of claims 1 to 18, preferably a method for the diagnosis of a disease as described in any one of the preceding claims or for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosis using the compound of any one of claims 1 to 18, preferably a method for the diagnosis of a disease as described in any one of the preceding claims.

20. A composition, preferably a pharmaceutical composition, wherein the composition comprises a compound according to any one of claims 1 to 18 and a pharmaceutically acceptable excipient.

21. A kit comprising a compound according to any one of claims 1 to 18, one or more optional excipient(s) and optionally one or more device(s), whereby the device(s) is/are selected from the group comprising a labeling device, a purification device, a handling device, a radioprotection device, an analytical device or an administration device.
